# EUROPEAN PATENT SPECIFICATION

(11) **EP 3 320 091 B1**
(45) Date of publication and mention of the grant of the patent: **11.11.2020**
(21) Application number: 16738385.0
(22) Date of filing: 06.07.2016
(51) Int. Cl.: C12N 15/10, C12N 15/64, C12N 5/10, C12N 15/63, C12N 15/81

(54) **GUIDE RNA ASSEMBLY VECTOR**
GUIDE-RNS-ASSEMBLIERUNGSVEKTOR
VECTEUR D'ASSEMBLAGE D'ARN GUIDE

(30) Priority: 06.07.2015 EP 15175444
(43) Date of publication of application: 16.05.2018
(73) Proprietor: DSM IP Assets B.V., 6411 TE Heerlen (NL)
(72) Inventor: DE WAAL, Paulus Petrus, 6100 AA Echt (NL); ROUBOS, Johannes Andries, 6100 AA Echt (NL); KLAASSEN, Paul, 6100 AA Echt (NL); VERWAAL, René, 6100 AA Echt (NL)
(74) Representative: DSM Intellectual Property
(86) International application number: PCT/EP2016/066016
(87) International publication number: WO 2017/005807

(56) References cited:
- WO-A1-2015/095804
- ANDREW A. HORWITZ ET AL: "Efficient Multiplexed Integration of Synergistic Alleles and Metabolic Pathways in Yeasts via CRISPR-Cas", CELL SYSTEMS, vol. 1, no. 1, 12 March 2015 (2015-03-12), pages 88-96, XP055230552, ISSN: 2405-4712, DOI: 10.1016/j.cels.2015.02.001 cited in the application
- OWEN W RYAN ET AL: "Selection of chromosomal DNA libraries using a multiplex CRISPR system", ELIFE, vol. 3, 19 August 2014 (2014-08-19), XP055175718, DOI: 10.7554/eLife.03703
- TADAS JAKOCIUNAS ET AL: "Multiplex metabolic pathway engineering using CRISPR/Cas9 in Saccharomyces cerevisiae", METABOLIC ENGINEERING, vol. 28, 28 January 2015 (2015-01-28), pages 213-222, XP055175723, ISSN: 1096-7176, DOI: 10.1016/j.ymben.2015.01.008
- ZEHUA BAO ET AL: "Homology-Integrated CRISPR-Cas (HI-CRISPR) System for One-Step Multigene Disruption in Saccharomyces cerevisiae", ACS SYNTHETIC BIOLOGY, 10 September 2014 (2014-09-10), XP055175736, ISSN: 2161-5063, DOI: 10.1021/sb500255k
- A. M. KABADI ET AL: "Multiplex CRISPR/Cas9-based genome engineering from a single lentiviral vector", NUCLEIC ACIDS RESEARCH, vol. 42, no. 19, 13 August 2014 (2014-08-13), pages e147-e147, XP055177310, ISSN: 0305-1048, DOI: 10.1093/nar/gku749
- TETSUSHI SAKUMA ET AL: "Multiplex genome engineering in human cells using all-in-one CRISPR/Cas9 vector system", SCIENTIFIC REPORTS, vol. 4, 23 June 2014 (2014-06-23), XP055196391, DOI: 10.1038/srep05400
- MANS ROBERT ET AL: "CRISPR/Cas9: a molecular Swiss army knife for simultaneous introduction of multiple genetic modifications in Saccharomyces cerevisiae", FEMS YEAST RESEARCH, vol. 15, no. 2, March 2015 (2015-03), XP002762726,
- NADINE ECKERT-BOULET ET AL: "Optimization of ordered plasmid assembly by gap repair in Saccharomyces cerevisiae", YEAST, vol. 29, no. 8, 17 July 2012 (2012-07-17), pages 323-334, XP055235000, GB ISSN: 0749-503X, DOI: 10.1002/yea.2912
- XIQUAN LIANG ET AL: "Rapid and highly efficient mammalian cell engineering via Cas9 protein transfection", JOURNAL OF BIOTECHNOLOGY, vol. 208, 21 May 2015 (2015-05-21), pages 44-53, XP055196365, ISSN: 0168-1656, DOI: 10.1016/j.jbiotec.2015.04.024

### SEQUENCE LISTING

<110> DSM IP Assets B.V.
<120> GUIDE RNA ASSEMBLY VECTOR
<130> 31203-WO-PCT
<160> 119
<170> PatentIn version 3.5
<210> 1
   <211> 20
   <212> DNA
   <213> artificial sequence
<220>
   <223> Preferred termination sequence in yeast
<400> 1
   tttttttgtt ttttatgtct 20
<210> 2
   <211> 7240
   <212> DNA
   <213> artificial sequence
<220>
   <223> polynucleotide sequence to be integrated in the integration locus on chromosome 14 of S. cerevisiae, comprising the Cas9 expression cassette and the natMX marker and including the 5'- and 3'-flanking regions
<400> 2
   ttgagcggac gccgaaacgt tgtttttttt ttgctttagt ccagataata accttttata       60
   attttctttt tagggaggaa gaccggtcta agctcttaga ggttctcgca tacccaagta    120
   aaagctaaga ccgaagcaaa cacgcaggat aattttccgg tttactctca ctgcagcttt    180
   agtatagatg cagatctggt attgagagag gggggccgga acgaaaagat tcaggggaga    240
   gaaaggcccg ggcgtcgggg aacaaaacca cttttgcaca ccttaatttt ttagctactc    300
   tgtttctccg ggcttgtatg gcacataaat aaaattctgc tactttccat tatctggtca    360
   tcacttaccg atgtggagag ccttgccctt gaaaaataag ataagacctc cgattagggt    420
   atttgaaatt aaccgctgta gaatgaaata aggacgcttg tttgacattt tttagttgct    480
   ttctctatct accaccattt gcatagcttc aaaatgtttc tactcctttt ttactcttcc    540
   agattttctc ggactccgcg catcgccgta ccacttcaaa acacccaagc acagcatact    600
   aaatttcccc tctttcttcc tctagggtgt cgttaattac ccgtactaaa ggtttggaaa    660
   agaaaaaaga gaccgcctcg tttctttttc ttcgtcgaaa aaggcaataa aaatttttat     720
   cacgtttctt tttcttgaaa attttttttt tgattttttt ctctttcgat gacctcccat    780
   tgatatttaa gttaataaac ggtcttcaat ttctcaagtt tcagtttcat ttttcttgtt    840
   ctattacaac tttttttact tcttgctcat tagaaagaaa gcatagcaat ctaatctaag    900
   ttttctagaa ctagtggatc ccccgggaaa aatggacaag aagtactcca ttgggctcga    960
   tatcggcaca aacagcgtcg gttgggccgt cattacggac gagtacaagg tgccgagcaa   1020
   aaaattcaaa gttctgggca ataccgatcg ccacagcata aagaagaacc tcattggcgc   1080
   cctcctgttc gactccgggg agacggccga agccacgcgg ctcaaaagaa cagcacggcg   1140
   cagatatacc cgcagaaaga atcggatctg ctacctgcag gagatcttta gtaatgagat   1200
   ggctaaggtg gatgactctt tcttccatag gctggaggag tcctttttgg tggaggagga   1260
   taaaaagcac gagcgccacc caatctttgg caatatcgtg gacgaggtgg cgtaccatga   1320
   aaagtaccca accatatatc atctgaggaa gaagcttgta gacagtactg ataaggctga   1380
   cttgcggttg atctatctcg cgctggcgca tatgatcaaa tttcggggac acttcctcat   1440
   cgagggggac ctgaacccag acaacagcga tgtcgacaaa ctctttatcc aactggttca   1500
   gacttacaat cagcttttcg aagagaaccc gatcaacgca tccggagttg acgccaaagc   1560
   aatcctgagc gctaggctgt ccaaatcccg gcggctcgaa aacctcatcg cacagctccc   1620
   tggggagaag aagaacggcc tgtttggtaa tcttatcgcc ctgtcactcg ggctgacccc   1680
   caactttaaa tctaacttcg acctggccga agatgccaag cttcaactga gcaaagacac   1740
   ctacgatgat gatctcgaca atctgctggc ccagatcggc gaccagtacg cagacctttt   1800
   tttggcggca aagaacctgt cagacgccat tctgctgagt gatattctgc gagtgaacac   1860
   ggagatcacc aaagctccgc tgagcgctag tatgatcaag cgctatgatg agcaccacca   1920
   agacttgact ttgctgaagg cccttgtcag acagcaactg cctgagaagt acaaggaaat   1980
   tttcttcgat cagtctaaaa atggctacgc cggatacatt gacggcggag caagccagga   2040
   ggaattttac aaatttatta agcccatctt ggaaaaaatg gacggcaccg aggagctgct   2100
   ggtaaagctt aacagagaag atctgttgcg caaacagcgc actttcgaca atggaagcat   2160
   cccccaccag attcacctgg gcgaactgca cgctatcctc aggcggcaag aggatttcta   2220
   cccctttttg aaagataaca gggaaaagat tgagaaaatc ctcacatttc ggatacccta  2280
   ctatgtaggc cccctcgccc ggggaaattc cagattcgcg tggatgactc gcaaatcaga   2340
   agagaccatc actccctgga acttcgagga agtcgtggat aagggggcct ctgcccagtc   2400
   cttcatcgaa aggatgacta actttgataa aaatctgcct aacgaaaagg tgcttcctaa   2460
   acactctctg ctgtacgagt acttcacagt ttataacgag ctcaccaagg tcaaatacgt   2520
   cacagaaggg atgagaaagc cagcattcct gtctggagag cagaagaaag ctatcgtgga   2580
   cctcctcttc aagacgaacc ggaaagttac cgtgaaacag ctcaaagaag actatttcaa   2640
   aaagattgaa tgtttcgact ctgttgaaat cagcggagtg gaggatcgct tcaacgcatc   2700
   cctgggaacg tatcacgatc tcctgaaaat cattaaagac aaggacttcc tggacaatga 2760
   ggagaacgag gacattcttg aggacattgt cctcaccctt acgttgtttg aagataggga 2820
   gatgattgaa gaacgcttga aaacttacgc tcatctcttc gacgacaaag tcatgaaaca 2880
   gctcaagagg cgccgatata caggatgggg gcggctgtca agaaaactga tcaatgggat 2940
   ccgagacaag cagagtggaa agacaatcct ggattttctt aagtccgatg gatttgccaa 3000
   ccggaacttc atgcagttga tccatgatga ctctctcacc tttaaggagg acatccagaa 3060
   agcacaagtt tctggccagg gggacagtct tcacgagcac atcgctaatc ttgcaggtag 3120
   cccagctatc aaaaagggaa tactgcagac cgttaaggtc gtggatgaac tcgtcaaagt 3180
   aatgggaagg cataagcccg agaatatcgt tatcgagatg gcccgagaga accaaactac 3240
   ccagaaggga cagaagaaca gtagggaaag gatgaagagg attgaagagg gtataaaaga 3300
   actggggtcc caaatcctta aggaacaccc agttgaaaac acccagcttc agaatgagaa 3360
   gctctacctg tactacctgc agaacggcag ggacatgtac gtggatcagg aactggacat 3420
   caatcggctc tccgactacg acgtggatca tatcgtgccc cagtcttttc tcaaagatga 3480
   ttctattgat aataaagtgt tgacaagatc cgataaaaat agagggaaga gtgataacgt 3540
   cccctcagaa gaagttgtca agaaaatgaa aaattattgg cggcagctgc tgaacgccaa 3600
   actgatcaca caacggaagt tcgataatct gactaaggct gaacgaggtg gcctgtctga 3660
   gttggataaa gccggcttca tcaaaaggca gcttgttgag acacgccaga tcaccaagca 3720
   cgtggcccaa attctcgatt cacgcatgaa caccaagtac gatgaaaatg acaaactgat 3780
   tcgagaggtg aaagttatta ctctgaagtc taagctggtc tcagatttca gaaaggactt 3840
   tcagttttat aaggtgagag agatcaacaa ttaccaccat gcgcatgatg cctacctgaa 3900
   tgcagtggta ggcactgcac ttatcaaaaa atatcccaag cttgaatctg aatttgttta 3960
   cggagactat aaagtgtacg atgttaggaa aatgatcgca aagtctgagc aggaaatagg 4020
   caaggccacc gctaagtact tcttttacag caatattatg aattttttca agaccgagat 4080
   tacactggcc aatggagaga ttcggaagcg accacttatc gaaacaaacg gagaaacagg 4140
   agaaatcgtg tgggacaagg gtagggattt cgcgacagtc cggaaggtcc tgtccatgcc 4200
   gcaggtgaac atcgttaaaa agaccgaagt acagaccgga ggcttctcca aggaaagtat 4260
   cctcccgaaa aggaacagcg acaagctgat cgcacgcaaa aaagattggg accccaagaa 4320
   atacggcgga ttcgattctc ctacagtcgc ttacagtgta ctggttgtgg ccaaagtgga 4380
   gaaagggaag tctaaaaaac tcaaaagcgt caaggaactg ctgggcatca caatcatgga 4440
   gcgatcaagc ttcgaaaaaa accccatcga ctttctcgag gcgaaaggat ataaagaggt 4500
   caaaaaagac ctcatcatta agcttcccaa gtactctctc tttgagcttg aaaacggccg 4560
   gaaacgaatg ctcgctagtg cgggcgagct gcagaaaggt aacgagctgg cactgccctc 4620
   taaatacgtt aatttcttgt atctggccag ccactatgaa aagctcaaag ggtctcccga 4680
   agataatgag cagaagcagc tgttcgtgga acaacacaaa cactaccttg atgagatcat 4740
   cgagcaaata agcgaattct ccaaaagagt gatcctcgcc gacgctaacc tcgataaggt 4800
   gctttctgct tacaataagc acagggataa gcccatcagg gagcaggcag aaaacattat 4860
   ccacttgttt actctgacca acttgggcgc gcctgcagcc ttcaagtact tcgacaccac 4920
   catagacaga aagcggtaca cctctacaaa ggaggtcctg gacgccacac tgattcatca 4980
   gtcaattacg gggctctatg aaacaagaat cgacctctct cagctcggtg gagacagcag 5040
   ggctgacccc aagaagaaga ggaaggtgtg atctcttctc gagtcatgta attagttatg 5100
   tcacgcttac attcacgccc tccccccaca tccgctctaa ccgaaaagga aggagttaga 5160
   caacctgaag tctaggtccc tatttatttt tttatagtta tgttagtatt aagaacgtta 5220
   tttatatttc aaatttttct tttttttctg tacagacgcg tgtacgcatg taacattata 5280
   ctgaaaacct tgcttgagaa ggttttggga cgctcgaagg ctttaatttg cgggcgtacg 5340
   ctgcaggtcg acgaattcta ccgttcgtat aatgtatgct atacgaagtt atagatctgt 5400
   ttagcttgcc ttgtccccgc cgggtcaccc ggccagcgac atggaggccc agaataccct 5460
   ccttgacagt cttgacgtgc gcagctcagg ggcatgatgt gactgtcgcc cgtacattta 5520
   gcccatacat ccccatgtat aatcatttgc atccatacat tttgatggcc gcacggcgcg 5580
   aagcaaaaat tacggctcct cgctgcagac ctgcgagcag ggaaacgctc ccctcacaga 5640
   cgcgttgaat tgtccccacg ccgcgcccct gtagagaaat ataaaaggtt aggatttgcc 5700
   actgaggttc ttctttcata tacttccttt taaaatcttg ctaggataca gttctcacat 5760
   cacatccgaa cataaacaac catgggtacc actcttgacg acacggctta ccggtaccgc 5820
   accagtgtcc cgggggacgc cgaggccatc gaggcactgg atgggtcctt caccaccgac 5880
   accgtcttcc gcgtcaccgc caccggggac ggcttcaccc tgcgggaggt gccggtggac 5940
   ccgcccctga ccaaggtgtt ccccgacgac gaatcggacg acgaatcgga cgacggggag 6000
   gacggcgacc cggactcccg gacgttcgtc gcgtacgggg acgacggcga cctggcgggc 6060
   ttcgtggtcg tctcgtactc cggctggaac cgccggctga ccgtcgagga catcgaggtc 6120
   gccccggagc accgggggca cggggtcggg cgcgcgttga tggggctcgc gacggagttc 6180
   gcccgcgagc ggggcgccgg gcacctctgg ctggaggtca ccaacgtcaa cgcaccggcg 6240
   atccacgcgt accggcggat ggggttcacc ctctgcggcc tggacaccgc cctgtacgac 6300
   ggcaccgcct cggacggcga gcaggcgctc tacatgagca tgccctgccc ctaatcagta 6360
   ctgacaataa aaagattctt gttttcaaga acttgtcatt tgtatagttt ttttatattg 6420
   tagttgttct attttaatca aatgttagcg tgatttatat tttttttcgc ctcgacatca 6480
   tctgcccaga tgcgaagtta agtgcgcaga aagtaatatc atgcgtcaat cgtatgtgaa     6540
   tgctggtcgc tatactgctg tcgattcgat actaacgccg ccatccagtg tcgaaaacga     6600
   gctcataact tcgtataatg tatgctatac gaacggtaga attcgatatc agatccacta     6660
   gtggcctcat cttgatttat gtcttgtttg aggagatttc ggataaatat tcttcgaggg     6720
   gaaaatgtcg taaaaataaa aacaataaag aaacaaaacg attaaaagaa aaactcaacc     6780
   atcctcaaaa gtcctcctct tttttttcac gtgctgcgct gatgtaagca gcaggacatg      6840
   gcaggaagaa aatcggagaa ttgtggccgc gacacctgac aaacaatcct caggcaaatg     6900
   taaaattgta catgaaaggc tgcgtagtca atgtcttaga aggccggccc gcatgatcca     6960
   gaagcagacc ataaaaaaaa taaaaattcg cggtctggca gcaggcataa gatgcatagc     7020
   gttatcctaa atgacatcac gatgataaat cctccgccgc atgatgcttt tgatttgcct     7080
   aagggcctgc catcgtgctg gctcaaacta ttgagggtca acataccttg aaaatccaag     7140
   taaaaggatg gatatcgtta tactaaaagc aacacagaaa aggtccacgt cagttccaca     7200
   caataacatt tacgtagtgt tcacgcgaag cagttacatc                          7240
<210> 3
   <211> 1875
   <212> DNA
   <213> artificial sequence
<220>
   <223> polynucleotide sequence of Brick 1 comprising the 2µ plasmid
<400> 3
   gttttagagc tagaaatagc aagttaaaat aaggctagtc cgttatcaac ttgaaaaagt 60
   ggcaccgagt cggtggtgct ttttttgttt tttatgtctt cgagtcatgt aattagttat 120
   gtcgtggcat gctagctccg gattatcgat gataagctgt caaacatgag aattaattcc 180
   acggactata gactatacct agtatactcc gtctactgta cgatacactt ccgctcaggt 240
   ccttgtcctt taacgaggcc ttaccactct tttgttactc tattgatcca gctcagcaaa 300
   ggcagtgtga tctaagattc tatcttcgcg atgtagtaaa actagctaga ccgagaaaga 360
   gactagaaat gcaaaaggca cttctacaat ggctgccatc attattatcc gatgtgacgc 420
   tgcagcttct caatgatatt cgaatacgct ttgaggagat acagcctaat atccgacaaa 480
   ctgttttaca gatttacgat cgtacttgtt acccatcatt gaattttgaa catccgaacc 540
   tgggagtttt ccctgaaaca gatagtatat ttgaacctgt ataataatat atagtctagc 600
   gctttacgga agacaatgta tgtatttcgg ttcctggaga aactattgca tctattgcat 660
   aggtaatctt gcacgtcgca tccccggttc attttctgcg tttccatctt gcacttcaat 720
   agcatatctt tgttaacgaa gcatctgtgc ttcattttgt agaacaaaaa tgcaacgcga 780
   gagcgctaat tttcaaacaa aagaatctga gctgcatttt tacagaacag aaatgcaacg 840
   cgaaagcgct attttaccaa cgaagaatct gtgcttcatt tttgtaaaac aaaaatgcaa      900
   cgcgagagcg ctaatttttc aaacaaagaa tctgagctgc atttttacag aacagaaatg      960
   caacgcgaga gcgctatttt accaacaaag aatctatact tcttttttgt tctacaaaaa     1020
   tgcatcccga gagcgctatt tttctaacaa agcatcttag attacttttt ttctcctttg     1080
   tgcgctctat aatgcagtct cttgataact ttttgcactg taggtccgtt aaggttagaa     1140
   gaaggctact ttggtgtcta ttttctcttc cataaaaaaa gcctgactcc acttcccgcg     1200
   tttactgatt actagcgaag ctgcgggtgc attttttcaa gataaaggca tccccgatta     1260
   tattctatac cgatgtggat tgcgcatact ttgtgaacag aaagtgatag cgttgatgat     1320
   tcttcattgg tcagaaaatt atgaacggtt tcttctattt tgtctctata tactacgtat     1380
   aggaaatgtt tacattttcg tattgttttc gattcactct atgaatagtt cttactacaa     1440
   tttttttgtc taaagagtaa tactagagat aaacataaaa aatgtagagg tcgagtttag     1500
   atgcaagttc aaggagcgaa aggtggatgg gtaggttata tagggatata gcacagagat     1560
   atatagcaaa gagatacttt tgagcaatgt ttgtggaaag cggctgttct ttgaaaagat     1620
   aatgtatgat tatgctttca ctcatattta tacagaaact tgatgttttc tttcgagtat     1680
   atacaaggtg attacatgta cgtttgaagt acaactctag attttgtagt gccctcttgg     1740
   gctagcggta aaggtgcgca ttttttcaca ccctacaatg ttctgttcaa aagattttgg     1800
   tcaaacgctg tagaagtgaa agttggtgcg catgtttcgg cgttcgaaac ttctccgcag     1860
   tgaaagataa atgat                                                      1875
<210> 4
   <211> 2125
   <212> DNA
   <213> artificial sequence
<220>
   <223> polynucleotide sequence of Brick 2 comprising the expression cassette of Dasher GFP, flanked upstream by the S. cerevisae TDH3 promoter, and flanked downstream by the S. cerevisiae ADH1 terminator sequence
<400> 4
   gttttagagc tagaaatagc aagttaaaat aaggctagtc cgttatcaac ttgaaaaagt       60
   ggcaccgagt cggtggtgct ttttttgttt tttatgtctt cgagtcatgt aattagttat      120
   gtctggccgg atcgatgtac acaaccgact gcacccaaac gaacacaaat cttagcagtg      180
   cttagtcaaa aaattagcct tttaattctg ctgtaacccg tacatgccca aaataggggg      240
   cgggttacac agaatatata acatcgtagg tgtctgggtg aacagtttat tcctggcatc      300
   cactaaatat aatggagccc gctttttaag ctggcatcca gaaaaaaaaa gaatcccagc      360
   accaaaatat tgttttcttc accaaccatc agttcatagg tccattctct tagcgcaact      420
   acagagaaca ggggcacaaa caggcaaaaa acgggcacaa cctcaatgga gtgatgcaac 480
   ctgcctggag taaatgatga cacaaggcaa ttgacccacg catgtatcta tctcattttc 540
   ttacaccttc tattaccttc tgctctctct gatttggaaa aagctgaaaa aaaaggttga 600
   aaccagttcc ctgaaattat tcccctactt gactaataag tatataaaga cggtaggtat 660
   tgattgtaat tctgtaaatc tatttcttaa acttcttaaa ttctactttt atagttagtc 720
   ttttttttag ttttaaaaca ccaagaactt agtttcgaat aaacacacat aaacaaacaa 780
   aatgaccgca ctaacagaag gagctaaact attcgaaaag gagattcctt acattacaga 840
   attagagggt gatgtcgaag gaatgaaatt cattatcaag ggcgagggta ctggtgacgc 900
   tactaccggt acgattaaag caaagtacat ctgtacaaca ggtgaccttc ctgttccgtg 960
   ggctactctg gtgagcactt tgtcttatgg agttcaatgt tttgctaaat acccttcgca 1020
   cattaaagac tttttcaaaa gtgcaatgcc tgagggctat actcaggaga gaacaatatc 1080
   tttcgaagga gatggtgtgt ataagactag ggctatggtc acgtatgaaa gaggatccat 1140
   ctacaataga gtaactttaa ctggtgaaaa cttcaaaaag gacggtcaca tccttagaaa 1200
   gaatgttgcc tttcaatgcc caccatccat cttgtacatt ttgccagaca cagttaacaa 1260
   tggtatcaga gttgagttta accaagctta tgacatagag ggtgtcaccg aaaagttggt 1320
   tacaaaatgt tcacagatga atcgtcccct ggcaggatca gctgccgtcc atatcccacg 1380
   ttaccatcat atcacttatc ataccaagct gtccaaagat cgtgatgaga gaagggatca 1440
   catgtgtttg gttgaagtgg taaaggccgt ggatttggat acttaccaag gttgataaag 1500
   cgaatttctt atgatttatg atttttatta ttaaataagt tataaaaaaa ataagtgtat 1560
   acaaatttta aagtgactct taggttttaa aacgaaaatt cttattcttg agtaactctt 1620
   tcctgtaggt caggttgctt tctcaggtat agcatgaggt cgctcttatt gaccacacct 1680
   ctaccggcat gccgagcaaa tgcctgcaaa tcgctcccca tttcacccaa ttgtagatat 1740
   gctaactcca gcaatgagtt gatgaatctc ggtgtgtatt ttatgtcctc agaggacaac 1800
   ctcagaaagc ctgtatgcga agccacaatc ctttccaaca gaccatacta agtgaattct 1860
   ttgaaaagat aatgtatgat tatgctttca ctcatattta tacagaaact tgatgttttc 1920
   tttcgagtat atacaaggtg attacatgta cgtttgaagt acaactctag attttgtagt 1980
   gccctcttgg gctagcggta aaggtgcgca ttttttcaca ccctacaatg ttctgttcaa 2040
   aagattttgg tcaaacgctg tagaagtgaa agttggtgcg catgtttcgg cgttcgaaac 2100
   ttctccgcag tgaaagataa atgat 2125
<210> 5
   <211> 1846
   <212> DNA
   <213> artificial sequence
<220>
   <223> polynucleotide sequence of Brick 3 comprising the dominant resistance marker KanMX
<400> 5
   gttttagagc tagaaatagc aagttaaaat aaggctagtc cgttatcaac ttgaaaaagt 60
   ggcaccgagt cggtggtgct ttttttgttt tttatgtctt cgagtcatgt aattagttat 120
   gtctgagatc tgtttagctt gcctcgtccc cgccgggtca cccggccagc gacatggagg 180
   cccagaatac cctccttgac agtcttgacg tgcgcagctc aggggcatga tgtgactgtc 240
   gcccgtacat ttagcccata catccccatg tataatcatt tgcatccata cattttgatg 300
   gccgcacggc gcgaagcaaa aattacggct cctcgctgca gacctgcgag cagggaaacg 360
   ctcccctcac agacgcgttg aattgtcccc acgccgcgcc cctgtagaga aatataaaag 420
   gttaggattt gccactgagg ttcttctttc atatacttcc ttttaaaatc ttgctaggat 480
   acagttctca catcacatcc gaacataaac aaccatgggt aaggaaaaga ctcacgtttc 540
   gaggccgcga ttaaattcca acatggatgc tgatttatat gggtataaat gggctcgcga 600
   taatgtcggg caatcaggtg cgacaatcta tcgattgtat gggaagcccg atgcgccaga 660
   gttgtttctg aaacatggca aaggtagcgt tgccaatgat gttacagatg agatggtcag 720
   actaaactgg ctgacggaat ttatgcctct tccgaccatc aagcatttta tccgtactcc 780
   tgatgatgca tggttactca ccactgcgat ccccggcaaa acagcattcc aggtattaga 840
   agaatatcct gattcaggtg aaaatattgt tgatgcgctg gcagtgttcc tgcgccggtt 900
   gcattcgatt cctgtttgta attgtccttt taacagcgat cgcgtatttc gtctcgctca 960
   ggcgcaatca cgaatgaata acggtttggt tgatgcgagt gattttgatg acgagcgtaa 1020
   tggctggcct gttgaacaag tctggaaaga aatgcataag cttttgccat tctcaccgga 1080
   ttcagtcgtc actcatggtg atttctcact tgataacctt atttttgacg aggggaaatt 1140
   aataggttgt attgatgttg gacgagtcgg aatcgcagac cgataccagg atcttgccat 1200
   cctatggaac tgcctcggtg agttttctcc ttcattacag aaacggcttt ttcaaaaata 1260
   tggtattgat aatcctgata tgaataaatt gcagtttcat ttgatgctcg atgagttttt 1320
   ctaatcagta ctgacaataa aaagattctt gttttcaaga acttgtcatt tgtatagttt 1380
   ttttatattg tagttgttct attttaatca aatgttagcg tgatttatat tttttttcgc 1440
   ctcgacatca tctgcccaga tgcgaagtta agtgcgcaga aagtaatatc atgcgtcaat 1500
   cgtatgtgaa tgctggtcgc tatactgctg tcgattcgat actaacgccg ccatccagtg 1560
   tcgaaaacga gctcgaattc tttgaaaaga taatgtatga ttatgctttc actcatattt 1620
   atacagaaac ttgatgtttt ctttcgagta tatacaaggt gattacatgt acgtttgaag 1680
   tacaactcta gattttgtag tgccctcttg ggctagcggt aaaggtgcgc attttttcac 1740
   accctacaat gttctgttca aaagattttg gtcaaacgct gtagaagtga aagttggtgc 1800
   gcatgtttcg gcgttcgaaa cttctccgca gtgaaagata aatgat 1846
<210> 6
   <211> 2047
   <212> DNA
   <213> artificial sequence
<220>
   <223> polynucleotide sequence of Brick 4 comprising the kanR gene, a bacterial selection marker, and pUC ORI
<400> 6
   gttttagagc tagaaatagc aagttaaaat aaggctagtc cgttatcaac ttgaaaaagt      60
   ggcaccgagt cggtggtgct ttttttgttt tttatgtctt cgagtcatgt aattagttat     120
   gtctgcgagc ggtatcagct cactcaaagg cggtaatacg gttatccaca gaatcagggg     180
   ataacgcagg aaagaacatg tgagcaaaag gccagcaaaa ggccaggaac cgtaaaaagg     240
   ccgcgttgct ggcgtttttc cataggctcc gcccccctga cgagcatcac aaaaatcgac     300
   gctcaagtca gaggtggcga aacccgacag gactataaag ataccaggcg tttccccctg     360
   gaagctccct cgtgcgctct cctgttccga ccctgccgct taccggatac ctgtccgcct     420
   ttctcccttc gggaagcgtg gcgctttctc atagctcacg ctgtaggtat ctcagttcgg     480
   tgtaggtcgt tcgctccaag ctgggctgtg tgcacgaacc ccccgttcag cccgaccgct     540
   gcgccttatc cggtaactat cgtcttgagt ccaacccggt aagacacgac ttatcgccac     600
   tggcagcagc cactggtaac aggattagca gagcgaggta tgtaggcggt gctacagagt     660
   tcttgaagtg gtggcctaac tacggctaca ctagaagaac agtatttggt atctgcgctc     720
   tgctgaagcc agttaccttc ggaaaaagag ttggtagctc ttgatccggc aaacaaacca     780
   ccgctggtag cggtggtttt tttgtttgca agcagcagat tacgcgcaga aaaaaaggat     840
   ctcaagaaga tcctttgatc ttttctacgg ggtctgacgc tcagtggaac gacgcgcgcg     900
   taactcacgt taagggattt tggtcatgag cttgcgccgt cccgtcaagt cagcgtaatg     960
   ctctgctttt agaaaaactc atcgagcatc aaatgaaact gcaatttatt catatcagga    1020
   ttatcaatac catatttttg aaaaagccgt ttctgtaatg aaggagaaaa ctcaccgagg    1080
   cagttccata ggatggcaag atcctggtat cggtctgcga ttccgactcg tccaacatca    1140
   atacaaccta ttaatttccc ctcgtcaaaa ataaggttat caagtgagaa atcaccatga    1200
   gtgacgactg aatccggtga gaatggcaaa agtttatgca tttctttcca gacttgttca    1260
   acaggccagc cattacgctc gtcatcaaaa tcactcgcat caaccaaacc gttattcatt    1320
   cgtgattgcg cctgagcgag gcgaaatacg cgatcgctgt taaaaggaca attacaaaca    1380
   ggaatcgagt gcaaccggcg caggaacact gccagcgcat caacaatatt ttcacctgaa 1440
   tcaggatatt cttctaatac ctggaacgct gtttttccgg ggatcgcagt ggtgagtaac 1500
   catgcatcat caggagtacg gataaaatgc ttgatggtcg gaagtggcat aaattccgtc 1560
   agccagttta gtctgaccat ctcatctgta acatcattgg caacgctacc tttgccatgt 1620
   ttcagaaaca actctggcgc atcgggcttc ccatacaagc gatagattgt cgcacctgat 1680
   tgcccgacat tatcgcgagc ccatttatac ccatataaat cagcatccat gttggaattt 1740
   aatcgcggcc tcgacgtttc ccgttgaata tggctgaatt ctttgaaaag ataatgtatg 1800
   attatgcttt cactcatatt tatacagaaa cttgatgttt tctttcgagt atatacaagg 1860
   tgattacatg tacgtttgaa gtacaactct agattttgta gtgccctctt gggctagcgg 1920
   taaaggtgcg cattttttca caccctacaa tgttctgttc aaaagatttt ggtcaaacgc 1980
   tgtagaagtg aaagttggtg cgcatgtttc ggcgttcgaa acttctccgc agtgaaagat 2040
   aaatgat 2047
<210> 7
   <211> 60
   <212> DNA
   <213> artificial sequence
<220>
   <223> forward primer gR1-HXT1-f
<400> 7
   tgaaagataa atgatgttgt agtcagcgcc tctctgtttt agagctagaa atagcaagtt 60
<210> 8
   <211> 60
   <212> DNA
   <213> artificial sequence
<220>
   <223> forward primer gR2-HXT2-f
<400> 8
   tgaaagataa atgattgggt taaccttagg acgtcgtttt agagctagaa atagcaagtt 60
<210> 9
   <211> 60
   <212> DNA
   <213> artificial sequence
<220>
   <223> forward primer gR3-HXT3-f
<400> 9
   tgaaagataa atgatggtgg ctagtatttg tttcggtttt agagctagaa atagcaagtt 60
<210> 10
   <211> 60
   <212> DNA
   <213> artificial sequence
<220>
   <223> forward primer gR4-GAL2-f
<400> 10
   tgaaagataa atgattctaa ctctgcctgg acggcgtttt agagctagaa atagcaagtt 60
<210> 11
   <211> 60
   <212> DNA
   <213> artificial sequence
<220>
   <223> reverse primer gR1-HXT1-r
<400> 11
   ttctagctct aaaacagaga ggcgctgact acaacatcat ttatctttca ctgcggagaa 60
<210> 12
   <211> 60
   <212> DNA
   <213> artificial sequence
<220>
   <223> reverse primer gR2-HXT2-r
<400> 12
   ttctagctct aaaacgacgt cctaaggtta acccaatcat ttatctttca ctgcggagaa 60
<210> 13
   <211> 60
   <212> DNA
   <213> artificial sequence
<220>
   <223> reverse primer gR3-HXT3-r
<400> 13
   ttctagctct aaaaccgaaa caaatactag ccaccatcat ttatctttca ctgcggagaa 60
<210> 14
   <211> 60
   <212> DNA
   <213> artificial sequence
<220>
   <223> reverse primer gR4-GAL2-r
<400> 14
   ttctagctct aaaacgccgt ccaggcagag ttagaatcat ttatctttca ctgcggagaa 60
<210> 15
   <211> 1945
   <212> DNA
   <213> artificial sequence
<220>
   <223> extended Brick 1
<400> 15
   tgaaagataa atgatgttgt agtcagcgcc tctctgtttt agagctagaa atagcaagtt 60
   aaaataaggc tagtccgtta tcaacttgaa aaagtggcac cgagtcggtg gtgctttttt 120
   tgttttttat gtcttcgagt catgtaatta gttatgtcgt ggcatgctag ctccggatta 180
   tcgatgataa gctgtcaaac atgagaatta attccacgga ctatagacta tacctagtat 240
   actccgtcta ctgtacgata cacttccgct caggtccttg tcctttaacg aggccttacc 300
   actcttttgt tactctattg atccagctca gcaaaggcag tgtgatctaa gattctatct 360
   tcgcgatgta gtaaaactag ctagaccgag aaagagacta gaaatgcaaa aggcacttct 420
   acaatggctg ccatcattat tatccgatgt gacgctgcag cttctcaatg atattcgaat 480
   acgctttgag gagatacagc ctaatatccg acaaactgtt ttacagattt acgatcgtac 540
   ttgttaccca tcattgaatt ttgaacatcc gaacctggga gttttccctg aaacagatag 600
   tatatttgaa cctgtataat aatatatagt ctagcgcttt acggaagaca atgtatgtat 660
   ttcggttcct ggagaaacta ttgcatctat tgcataggta atcttgcacg tcgcatcccc 720
   ggttcatttt ctgcgtttcc atcttgcact tcaatagcat atctttgtta acgaagcatc 780
   tgtgcttcat tttgtagaac aaaaatgcaa cgcgagagcg ctaatttttc aaacaaagaa 840
   tctgagctgc atttttacag aacagaaatg caacgcgaaa gcgctatttt accaacgaag 900
   aatctgtgct tcatttttgt aaaacaaaaa tgcaacgcga gagcgctaat ttttcaaaca 960
   aagaatctga gctgcatttt tacagaacag aaatgcaacg cgagagcgct attttaccaa 1020
   caaagaatct atacttcttt tttgttctac aaaaatgcat cccgagagcg ctatttttct 1080
   aacaaagcat cttagattac tttttttctc ctttgtgcgc tctataatgc agtctcttga 1140
   taactttttg cactgtaggt ccgttaaggt tagaagaagg ctactttggt gtctattttc 1200
   tcttccataa aaaaagcctg actccacttc ccgcgtttac tgattactag cgaagctgcg 1260
   ggtgcatttt ttcaagataa aggcatcccc gattatattc tataccgatg tggattgcgc 1320
   atactttgtg aacagaaagt gatagcgttg atgattcttc attggtcaga aaattatgaa 1380
   cggtttcttc tattttgtct ctatatacta cgtataggaa atgtttacat tttcgtattg 1440
   ttttcgattc actctatgaa tagttcttac tacaattttt ttgtctaaag agtaatacta 1500
   gagataaaca taaaaaatgt agaggtcgag tttagatgca agttcaagga gcgaaaggtg 1560
   gatgggtagg ttatataggg atatagcaca gagatatata gcaaagagat acttttgagc 1620
   aatgtttgtg gaaagcggct gttctttgaa aagataatgt atgattatgc tttcactcat 1680
   atttatacag aaacttgatg ttttctttcg agtatataca aggtgattac atgtacgttt 1740
   gaagtacaac tctagatttt gtagtgccct cttgggctag cggtaaaggt gcgcattttt 1800
   tcacacccta caatgttctg ttcaaaagat tttggtcaaa cgctgtagaa gtgaaagttg 1860
   gtgcgcatgt ttcggcgttc gaaacttctc cgcagtgaaa gataaatgat tgggttaacc 1920
   ttaggacgtc gttttagagc tagaa 1945
<210> 16
   <211> 2195
   <212> DNA
   <213> artificial sequence
<220>
   <223> extended Brick 2
<400> 16
   tgaaagataa atgattgggt taaccttagg acgtcgtttt agagctagaa atagcaagtt 60
   aaaataaggc tagtccgtta tcaacttgaa aaagtggcac cgagtcggtg gtgctttttt 120
   tgttttttat gtcttcgagt catgtaatta gttatgtctg gccggatcga tgtacacaac 180
   cgactgcacc caaacgaaca caaatcttag cagtgcttag tcaaaaaatt agccttttaa 240
   ttctgctgta acccgtacat gcccaaaata gggggcgggt tacacagaat atataacatc 300
   gtaggtgtct gggtgaacag tttattcctg gcatccacta aatataatgg agcccgcttt 360
   ttaagctggc atccagaaaa aaaaagaatc ccagcaccaa aatattgttt tcttcaccaa 420
   ccatcagttc ataggtccat tctcttagcg caactacaga gaacaggggc acaaacaggc 480
   aaaaaacggg cacaacctca atggagtgat gcaacctgcc tggagtaaat gatgacacaa 540
   ggcaattgac ccacgcatgt atctatctca ttttcttaca ccttctatta ccttctgctc 600
   tctctgattt ggaaaaagct gaaaaaaaag gttgaaacca gttccctgaa attattcccc 660
   tacttgacta ataagtatat aaagacggta ggtattgatt gtaattctgt aaatctattt 720
   cttaaacttc ttaaattcta cttttatagt tagtcttttt tttagtttta aaacaccaag 780
   aacttagttt cgaataaaca cacataaaca aacaaaatga ccgcactaac agaaggagct 840
   aaactattcg aaaaggagat tccttacatt acagaattag agggtgatgt cgaaggaatg 900
   aaattcatta tcaagggcga gggtactggt gacgctacta ccggtacgat taaagcaaag 960
   tacatctgta caacaggtga ccttcctgtt ccgtgggcta ctctggtgag cactttgtct 1020
   tatggagttc aatgttttgc taaataccct tcgcacatta aagacttttt caaaagtgca 1080
   atgcctgagg gctatactca ggagagaaca atatctttcg aaggagatgg tgtgtataag 1140
   actagggcta tggtcacgta tgaaagagga tccatctaca atagagtaac tttaactggt 1200
   gaaaacttca aaaaggacgg tcacatcctt agaaagaatg ttgcctttca atgcccacca 1260
   tccatcttgt acattttgcc agacacagtt aacaatggta tcagagttga gtttaaccaa 1320
   gcttatgaca tagagggtgt caccgaaaag ttggttacaa aatgttcaca gatgaatcgt 1380
   cccctggcag gatcagctgc cgtccatatc ccacgttacc atcatatcac ttatcatacc 1440
   aagctgtcca aagatcgtga tgagagaagg gatcacatgt gtttggttga agtggtaaag 1500
   gccgtggatt tggatactta ccaaggttga taaagcgaat ttcttatgat ttatgatttt 1560
   tattattaaa taagttataa aaaaaataag tgtatacaaa ttttaaagtg actcttaggt 1620
   tttaaaacga aaattcttat tcttgagtaa ctctttcctg taggtcaggt tgctttctca 1680
   ggtatagcat gaggtcgctc ttattgacca cacctctacc ggcatgccga gcaaatgcct 1740
   gcaaatcgct ccccatttca cccaattgta gatatgctaa ctccagcaat gagttgatga 1800
   atctcggtgt gtattttatg tcctcagagg acaacctcag aaagcctgta tgcgaagcca 1860
   caatcctttc caacagacca tactaagtga attctttgaa aagataatgt atgattatgc 1920
   tttcactcat atttatacag aaacttgatg ttttctttcg agtatataca aggtgattac 1980
   atgtacgttt gaagtacaac tctagatttt gtagtgccct cttgggctag cggtaaaggt 2040
   gcgcattttt tcacacccta caatgttctg ttcaaaagat tttggtcaaa cgctgtagaa 2100
   gtgaaagttg gtgcgcatgt ttcggcgttc gaaacttctc cgcagtgaaa gataaatgat 2160
   ggtggctagt atttgtttcg gttttagagc tagaa 2195
<210> 17
   <211> 1916
   <212> DNA
   <213> artificial sequence
<220>
   <223> extended Brick 3
<400> 17
   tgaaagataa atgatggtgg ctagtatttg tttcggtttt agagctagaa atagcaagtt        60
   aaaataaggc tagtccgtta tcaacttgaa aaagtggcac cgagtcggtg gtgctttttt       120
   tgttttttat gtcttcgagt catgtaatta gttatgtctg agatctgttt agcttgcctc       180
   gtccccgccg ggtcacccgg ccagcgacat ggaggcccag aataccctcc ttgacagtct       240
   tgacgtgcgc agctcagggg catgatgtga ctgtcgcccg tacatttagc ccatacatcc       300
   ccatgtataa tcatttgcat ccatacattt tgatggccgc acggcgcgaa gcaaaaatta       360
   cggctcctcg ctgcagacct gcgagcaggg aaacgctccc ctcacagacg cgttgaattg       420
   tccccacgcc gcgcccctgt agagaaatat aaaaggttag gatttgccac tgaggttctt       480
   ctttcatata cttcctttta aaatcttgct aggatacagt tctcacatca catccgaaca       540
   taaacaacca tgggtaagga aaagactcac gtttcgaggc cgcgattaaa ttccaacatg       600
   gatgctgatt tatatgggta taaatgggct cgcgataatg tcgggcaatc aggtgcgaca       660
   atctatcgat tgtatgggaa gcccgatgcg ccagagttgt ttctgaaaca tggcaaaggt       720
   agcgttgcca atgatgttac agatgagatg gtcagactaa actggctgac ggaatttatg       780
   cctcttccga ccatcaagca ttttatccgt actcctgatg atgcatggtt actcaccact 840
   gcgatccccg gcaaaacagc attccaggta ttagaagaat atcctgattc aggtgaaaat 900
   attgttgatg cgctggcagt gttcctgcgc cggttgcatt cgattcctgt ttgtaattgt 960
   ccttttaaca gcgatcgcgt atttcgtctc gctcaggcgc aatcacgaat gaataacggt 1020
   ttggttgatg cgagtgattt tgatgacgag cgtaatggct ggcctgttga acaagtctgg 1080
   aaagaaatgc ataagctttt gccattctca ccggattcag tcgtcactca tggtgatttc 1140
   tcacttgata accttatttt tgacgagggg aaattaatag gttgtattga tgttggacga 1200
   gtcggaatcg cagaccgata ccaggatctt gccatcctat ggaactgcct cggtgagttt 1260
   tctccttcat tacagaaacg gctttttcaa aaatatggta ttgataatcc tgatatgaat 1320
   aaattgcagt ttcatttgat gctcgatgag tttttctaat cagtactgac aataaaaaga 1380
   ttcttgtttt caagaacttg tcatttgtat agttttttta tattgtagtt gttctatttt 1440
   aatcaaatgt tagcgtgatt tatatttttt ttcgcctcga catcatctgc ccagatgcga 1500
   agttaagtgc gcagaaagta atatcatgcg tcaatcgtat gtgaatgctg gtcgctatac 1560
   tgctgtcgat tcgatactaa cgccgccatc cagtgtcgaa aacgagctcg aattctttga 1620
   aaagataatg tatgattatg ctttcactca tatttataca gaaacttgat gttttctttc 1680
   gagtatatac aaggtgatta catgtacgtt tgaagtacaa ctctagattt tgtagtgccc 1740
   tcttgggcta gcggtaaagg tgcgcatttt ttcacaccct acaatgttct gttcaaaaga 1800
   ttttggtcaa acgctgtaga agtgaaagtt ggtgcgcatg tttcggcgtt cgaaacttct 1860
   ccgcagtgaa agataaatga ttctaactct gcctggacgg cgttttagag ctagaa 1916
<210> 18
   <211> 2127
   <212> DNA
   <213> artificial sequence
<220>
   <223> extended Brick 4
<400> 18
   cgcagtgaaa gataaatgat tctaactctg cctggacggc gttttagagc tagaaatagc        60
   aagttaaaat aaggctagtc cgttatcaac ttgaaaaagt ggcaccgagt cggtggtgct       120
   tttttgtttt tttatgtctt cgagtcatgt aattagttat gtctgcgagc ggtatcagct       180
   cactcaaagg cggtaatacg gttatccaca gaatcagggg ataacgcagg aaagaacatg       240
   tgagcaaaag gccagcaaaa ggccaggaac cgtaaaaagg ccgcgttgct ggcgtttttc       300
   cataggctcc gcccccctga cgagcatcac aaaaatcgac gctcaagtca gaggtggcga       360
   aacccgacag gactataaag ataccaggcg tttccccctg gaagctccct cgtgcgctct       420
   cctgttccga ccctgccgct taccggatac ctgtccgcct ttctcccttc gggaagcgtg 480
   gcgctttctc atagctcacg ctgtaggtat ctcagttcgg tgtaggtcgt tcgctccaag 540
   ctgggctgtg tgcacgaacc ccccgttcag cccgaccgct gcgccttatc cggtaactat 600
   cgtcttgagt ccaacccggt aagacacgac ttatcgccac tggcagcagc cactggtaac 660
   aggattagca gagcgaggta tgtaggcggt gctacagagt tcttgaagtg gtggcctaac 720
   tacggctaca ctagaagaac agtatttggt atctgcgctc tgctgaagcc agttaccttc 780
   ggaaaaagag ttggtagctc ttgatccggc aaacaaacca ccgctggtag cggtggtttt 840
   tttgtttgca agcagcagat tacgcgcaga aaaaaaggat ctcaagaaga tcctttgatc 900
   ttttctacgg ggtctgacgc tcagtggaac gacgcgcgcg taactcacgt taagggattt 960
   tggtcatgag cttgcgccgt cccgtcaagt cagcgtaatg ctctgctttt agaaaaactc 1020
   atcgagcatc aaatgaaact gcaatttatt catatcagga ttatcaatac catatttttg 1080
   aaaaagccgt ttctgtaatg aaggagaaaa ctcaccgagg cagttccata ggatggcaag 1140
   atcctggtat cggtctgcga ttccgactcg tccaacatca atacaaccta ttaatttccc 1200
   ctcgtcaaaa ataaggttat caagtgagaa atcaccatga gtgacgactg aatccggtga 1260
   gaatggcaaa agtttatgca tttctttcca gacttgttca acaggccagc cattacgctc 1320
   gtcatcaaaa tcactcgcat caaccaaacc gttattcatt cgtgattgcg cctgagcgag 1380
   gcgaaatacg cgatcgctgt taaaaggaca attacaaaca ggaatcgagt gcaaccggcg 1440
   caggaacact gccagcgcat caacaatatt ttcacctgaa tcaggatatt cttctaatac 1500
   ctggaacgct gtttttccgg ggatcgcagt ggtgagtaac catgcatcat caggagtacg 1560
   gataaaatgc ttgatggtcg gaagtggcat aaattccgtc agccagttta gtctgaccat 1620
   ctcatctgta acatcattgg caacgctacc tttgccatgt ttcagaaaca actctggcgc 1680
   atcgggcttc ccatacaagc gatagattgt cgcacctgat tgcccgacat tatcgcgagc 1740
   ccatttatac ccatataaat cagcatccat gttggaattt aatcgcggcc tcgacgtttc 1800
   ccgttgaata tggctgaatt ctttgaaaag ataatgtatg attatgcttt cactcatatt 1860
   tatacagaaa cttgatgttt tctttcgagt atatacaagg tgattacatg tacgtttgaa 1920
   gtacaactct agattttgta gtgccctctt gggctagcgg taaaggtgcg cattttttca 1980
   caccctacaa tgttctgttc aaaagatttt ggtcaaacgc tgtagaagtg aaagttggtg 2040
   cgcatgtttc ggcgttcgaa acttctccgc agtgaaagat aaatgatgtt gtagtcagcg 2100
   cctctctgtt ttagagctag aaatagc 2127
<210> 19
   <211> 382
   <212> DNA
   <213> artificial sequence
<220>
   <223> donor DNA used in the CRISPR-CAS9-mediated deletion of genes HXT5-HXT1-HXT4 in S. cerevisiae strain BIE272
<400> 19
   tagtcgaacg gttctccctc taagaataag aaactatcaa aagaaagaga aaagtcgatt      60
   gaataatttt tctatatata atatacgcaa acaagattcg ctttcacttt gcaattttac     120
   ttcatagctt tgttaaaacc agcaaaaaat attatttttc tagaaaaaag aatatattag     180
   aggtaaagaa agatcagtta attaaataat atagacttcc ttcaaggaat aaaaataata     240
   tttgatcttc aatttttaac gctttatgaa atatatattt atgatttaat agtttataga     300
   taatacttta tcaaactaca attttgaatt atgttttttg tttcatagtt ctggaagagg      360
   ttaggaatag gccgaattct cc                                              382
<210> 20
   <211> 468
   <212> DNA
   <213> artificial sequence
<220>
   <223> donor DNA used in the CRISPR-CAS9-mediated deletion of gene HXT2 in S. cerevisiae strain BIE272
<400> 20
   cggtacgccg cctaacgata gtaataggcc acgcaactgg cgtggacgac aacaataagt      60
   cgcccatttt ttatgttttc aaaacctagc aacccccacc aaacttgtca tcgttcccgg     120
   attcacaaat gatataaaaa gcgattacaa ttctacattc taaccagatt tgagatttcc     180
   tctttctcaa ttcctcttat attagattat aagaacaaca aattaaatta caaaaagact     240
   tataaagcaa catagagatt atacttaaac tagcactgat ttttttaagg ctaatggcta     300
   ctaatacttt aatagatgat cttcatactt ttttatttaa cgatttttaa tgatgttttt     360
   atttgtacca ctcatttatc tagatttttt taatactgat caaatcttac ggactcgacg     420
   ttaaaaagtt cctacatacg tctggtactt gaaacgctgc ttcgaggt                  468
<210> 21
   <211> 422
   <212> DNA
   <213> artificial sequence
<220>
   <223> donor DNA used in the CRISPR-CAS9-mediated deletion of genes HXT3-HXT6-HXT7 in S. cerevisiae strain BIE272
<400> 21
   gactatatcc ccagagaatt tctccagaag agaagaaaag tcaaagtttt ttttcgcttg       60
   ggggttgcat ataaatacag gcgctgtttt atcttcagca tgaatattcc ataattttac      120
   ttaatagctt ttcataaata atagaatcac aaacaaaatt tacatctgag ttaaacaatc      180
   tttgcgaaca cttttattaa ttcatgatca cgctctaatt tgtgcatttg aaatgtactc      240
   taattctaat tttatatttt taatgatatc ttgaaaagta aatacgtttt taatatatac      300
   aaaataatac agtttaattt tcaagttttt gatcatttgt tctcagaaag ttgagtggga      360
   cggagacaaa gaaactttaa agagaaatgc aaagtgggaa gaagtcagtt gtttaccgac      420
   cg                                                                     422
<210> 22
   <211> 414
   <212> DNA
   <213> artificial sequence
<220>
   <223> donor DNA used in the CRISPR-CAS9-mediated deletion of gene GAL2 in S. cerevisiae strain BIE272
<400> 22
   tggagatagg attagttttg tagacatata taaacaatca gtaattggat tgaaaatttg       60
   gtgttgtgaa ttgctcttca ttatgcacct tattcaatta tcatcaagaa tagcaatagt      120
   taagtaaaca caagattaac ataataaaaa aaataattct ttcataatgg cagttgagga      180
   gaacaatatg cctgttgttt cacagcaacc ccaagctggt gaagacgtga cctatcgtta      240
   gaagaaattc aagaattatg ggaagaaggt gttttacctt ggaaatctga aggctggatt      300
   ccttcatcca gaagaggtaa taattacgat ttagaggatt tacaacatga cgacaaaccg      360
   tggtacaagg ccatgctaga ataatgcgtt tgaagtgaga cgctccatca tctc            414
<210> 23
   <211> 11742
   <212> DNA
   <213> artificial sequence
<220>
   <223> CAS9 expression vector pCSN061
<400> 23
   tcgcgcgttt cggtgatgac ggtgaaaacc tctgacacat gcagctcccg gagacggtca      60
   cagcttgtct gtaagcggat gccgggagca gacaagcccg tcagggcgcg tcagcgggtg     120
   ttggcgggtg tcggggctgg cttaactatg cggcatcaga gcagattgta ctgagagtgc     180
   accataaacg acattactat atatataata taggaagcat ttaatagaca gcatcgtaat     240
   atatgtgtac tttgcagtta tgacgccaga tggcagtagt ggaagatatt ctttattgaa     300
   aaatagcttg tcaccttacg tacaatcttg atccggagct tttctttttt tgccgattaa     360
   gaattaattc ggtcgaaaaa agaaaaggag agggccaaga gggagggcat tggtgactat     420
   tgagcacgtg agtatacgtg attaagcaca caaaggcagc ttggagtatg tctgttatta     480
   atttcacagg tagttctggt ccattggtga aagtttgcgg cttgcagagc acagaggccg     540
   cagaatgtgc tctagattcc gatgctgact tgctgggtat tatatgtgtg cccaatagaa 600
   agagaacaat tgacccggtt attgcaagga aaatttcaag tcttgtaaaa gcatataaaa 660
   atagttcagg cactccgaaa tacttggttg gcgtgtttcg taatcaacct aaggaggatg 720
   ttttggctct ggtcaatgat tacggcattg atatcgtcca actgcatgga gatgagtcgt 780
   ggcaagaata ccaagagttc ctcggtttgc cagttattaa aagactcgta tttccaaaag 840
   actgcaacat actactcagt gcagcttcac agaaacctca ttcgtttatt cccttgtttg 900
   attcagaagc aggtgggaca ggtgaacttt tggattggaa ctcgatttct gactgggttg 960
   gaaggcaaga gagccccgaa agcttacatt ttatgttagc tggtggactg acgccagaaa 1020
   atgttggtga tgcgcttaga ttaaatggcg ttattggtgt tgatgtaagc ggaggtgtgg 1080
   agacaaatgg tgtaaaagac tctaacaaaa tagcaaattt cgtcaaaaat gctaagaaat 1140
   aggttattac tgagtagtat ttatttaagt attgtttgtg cacttgccta tgcggtgtga 1200
   aataccgcac agatgcgtaa ggagaaaata ccgcatcagg aaattgtaaa cgttaatatt 1260
   ttgttaaaat tcgcgttaaa tttttgttaa atcagctcat tttttaacca ataggccgaa 1320
   atcggcaaaa tcccttataa atcaaaagaa tagaccgaga tagggttgag tgttgttcca 1380
   gtttggaaca agagtccact attaaagaac gtggactcca acgtcaaagg gcgaaaaacc 1440
   gtctatcagg gcgatggccc actacgtgaa ccatcaccct aatcaagttt tttggggtcg 1500
   aggtgccgta aagcactaaa tcggaaccct aaagggagcc cccgatttag agcttgacgg 1560
   ggaaagccgg cgaacgtggc gagaaaggaa gggaagaaag cgaaaggagc gggcgctagg 1620
   gcgctggcaa gtgtagcggt cacgctgcgc gtaaccacca cacccgccgc gcttaatgcg 1680
   ccgctacagg gcgcgtcgcg ccattcgcca ttcaggctgc gcaactgttg ggaagggcga 1740
   tcggtgcggg cctcttcgct attacgccag ctggcgaaag ggggatgtgc tgcaaggcga 1800
   ttaagttggg taacgccagg gttttcccag tcacgacgtt gtaaaacgac ggccagtgag 1860
   cgcgcgtaat acgactcact atagggcgaa ttgggtacct ttcttttttt tgcggtcacc 1920
   cccatgtggc ggggaggcag aggagtaggt agagcaacga atcctactat ttatccaaat 1980
   tagtctagga actctttttc tagatttttt agatttgagg gcaagcgctg ttaacgactc 2040
   agaaatgtaa gcactacgga gtagaacgag aaatccgcca taggtggaaa tcctagcaaa 2100
   atcttgctta ccctagctag cctcaggtaa gctagcctta gcctgtcaaa tttttttcaa 2160
   aatttggtaa gtttctacta gcaaagcaaa cacggttcaa caaaccgaaa actccactca 2220
   ttatacgtgg aaaccgaaac aaaaaaacaa aaaccaaaat actcgccaat gagaaagttg 2280
   ctgcgtttct actttcgagg aagaggaact gagaggattg actacgaaag gggcaaaaac 2340
   gagtcgtatt ctcccattat tgtctgctac cacgcggtct agtagaataa gcaaccagtc 2400
   aacgctaaga caggtaatca aaataccagt ctgctggcta cgggctagtt tttacctctt 2460
   ttagaaccca ctgtaaaagt ccgttgtaaa gcccgttctc actgttggcg tttttttttt 2520
   tttggtttag tttcttattt ttcatttttt tctttcatga ccaaaaacaa acaaatctcg 2580
   cgatttgtac tgcggccact ggggcgtggc caaaaaaatg acaaatttag aaaccttagt 2640
   ttctgatttt tcctgttatg aggagatatg ataaaaaata ttactgcttt attgtttttt 2700
   ttttatctac tgaaatagag aaacttaccc aaggaggagg caaaaaaaag agtatatata 2760
   cagcagctac cattcagatt ttaatatatt cttttctctt cttctacact attattataa 2820
   taattttact atattcattt ttagcttaaa acctcataga atattattct tcagtcactc 2880
   gcttaaatac ttatcaaaaa tggacaagaa atactctatt ggtttggata tcgggaccaa 2940
   ctccgtcggt tgggctgtca tcaccgacga atacaaggtt ccatccaaga aattcaaggt 3000
   cttgggtaac actgacagac actctatcaa gaagaatttg atcggtgctt tgttgttcga 3060
   ctccggtgaa accgctgaag ctaccagatt gaagcgtacc gctcgtcgta gatacactag 3120
   acgtaaaaac cgtatttgtt acttgcaaga aatcttttct aacgaaatgg ccaaggttga 3180
   cgactctttc ttccacagat tggaagaatc tttcttggtt gaagaagaca agaagcacga 3240
   aagacatcca atcttcggta acatcgttga cgaagttgct taccacgaaa aataccctac 3300
   catctaccat ttgagaaaga agttggtcga ttccaccgac aaggctgatt tgagattgat 3360
   ctatttggcc ttggctcaca tgatcaagtt cagaggtcac ttcttgattg aaggtgactt 3420
   gaacccagac aactctgacg tcgacaaatt gttcatccaa ttggtccaaa cctacaacca 3480
   attattcgag gaaaacccaa ttaacgcttc tggtgttgat gctaaggcca tcttatctgc 3540
   ccgtttgtcc aagtctagac gtttggaaaa cttgattgct caattgcctg gtgaaaagaa 3600
   aaacggtttg ttcggtaact tgatcgcttt gtccttgggt ttgaccccaa acttcaagtc 3660
   caacttcgac ttggctgaag atgccaagtt gcaattgtcc aaggacacct acgacgacga 3720
   cttagacaac ttgttggctc aaatcggtga ccaatacgcc gacttgttct tggctgccaa 3780
   aaacttatct gacgctatct tgttgtctga catcttgaga gttaacactg aaattaccaa 3840
   ggctccattg tctgcttcta tgatcaaaag atacgacgaa caccaccaag atctgacttt 3900
   gttgaaggct ttggttagac aacaattgcc agaaaagtac aaggaaatct tcttcgacca 3960
   atccaaaaat ggttacgccg gttacattga cggtggtgct tctcaggaag aattctacaa 4020
   gttcatcaag ccaattttgg aaaagatgga tggtactgaa gaattattgg ttaagttgaa 4080
   cagagaagac ttattgagaa agcaacgtac cttcgataac ggttctatcc cacaccaaat 4140
   ccacttgggt gaattgcacg ccattttgag aagacaggaa gatttctatc cattcctaaa 4200
   ggacaacaga gaaaagatcg aaaagatctt aactttcaga atcccatact acgtcggtcc 4260
   attggccaga ggtaattcta gattcgcttg gatgaccaga aagtctgaag aaaccatcac 4320
   cccatggaac ttcgaagaag tcgtcgacaa gggtgcttct gcccaatctt tcatcgaaag 4380
   aatgaccaac tttgataaga acttgccaaa cgagaaggtc ttgccaaagc actctttgtt 4440
   gtacgaatac ttcaccgtct acaacgaatt aaccaaggtt aaatacgtta ctgaaggtat 4500
   gagaaagcca gctttcctat ccggtgaaca aaagaaggct attgttgact tgttgtttaa 4560
   gaccaacaga aaggtcactg ttaagcaatt gaaggaagac tacttcaaga agattgaatg 4620
   tttcgattcc gtcgaaatct ccggtgttga agaccgtttc aatgcttctt tgggcaccta 4680
   ccacgatttg ttaaagatca tcaaggacaa ggacttttta gataacgaag aaaacgaaga 4740
   catcttggaa gatatcgttt tgaccttgac tcttttcgag gacagagaaa tgattgaaga 4800
   gagattgaag acctacgctc acttgttcga cgataaagtt atgaagcaac taaagagaag 4860
   aagatacact ggttggggta gattgtccag aaagttgatt aacggtatca gagacaagca 4920
   atccggtaag actattttag actttttgaa atccgatggt ttcgctaaca gaaactttat 4980
   gcaattgatt cacgacgatt ctttgacttt caaggaagac attcaaaaag cccaagtctc 5040
   tggtcaaggt gattctttgc acgaacacat cgctaacttg gctggttctc cagctattaa 5100
   gaagggtatc ttacaaaccg tcaaggtcgt tgatgaattg gtcaaagtca tgggtagaca 5160
   caagccagaa aatattgtca tcgaaatggc tagagaaaac caaactactc aaaagggtca 5220
   aaagaactct agagaacgta tgaagagaat tgaagaaggt atcaaggagt tgggttctca 5280
   aattttgaaa gaacacccag tcgaaaacac tcaattacaa aacgaaaagc tatacttgta 5340
   ctacttgcaa aacggtcgtg acatgtacgt cgaccaagaa ttggatatca acagattgtc 5400
   tgactacgat gtcgatcata tcgtcccaca atcgttcttg aaggacgatt ccattgacaa 5460
   caaagttttg actagatctg acaagaacag aggtaagtct gataacgttc catctgaaga 5520
   agttgttaag aagatgaaga actactggag acaattgttg aatgctaagt tgatcactca 5580
   aagaaagttc gacaacttga ccaaggctga aagaggtggt ttgtccgaat tggacaaagc 5640
   cggtttcatc aagagacaat tagtcgaaac tagacaaatc accaagcatg ttgctcaaat 5700
   cttggattcc agaatgaaca ctaagtacga tgaaaacgac aaactaatta gagaagttaa 5760
   ggtcatcact ttgaagtcta agttggtttc tgacttcaga aaggacttcc aattttacaa 5820
   ggtcagagaa atcaacaact accatcacgc tcacgatgcc tacttgaacg ctgttgtcgg 5880
   tactgcctta atcaaaaagt acccaaagtt ggaatctgaa ttcgtttacg gtgactacaa 5940
   ggtttacgat gttagaaaga tgatcgccaa gtctgaacaa gaaattggta aggccactgc 6000
   taagtacttc ttctactcta acatcatgaa ctttttcaag actgaaatca ctttagctaa 6060
   cggtgaaatt agaaagcgtc cattgattga aaccaatggt gaaactggtg aaattgtctg 6120
   ggacaagggt agagatttcg ctaccgtcag aaaggttttg tctatgccac aagttaacat 6180
   cgtcaagaag actgaagttc aaactggtgg tttctctaag gaatccattt tgccaaagag 6240
   aaactctgac aagttgattg ctagaaagaa ggactgggat cctaagaagt acggtggttt 6300
   cgactctcca actgttgctt actccgtttt ggtcgttgct aaggttgaaa agggtaagtc 6360
   taagaagttg aagtctgtta aggaattgtt gggtatcacc atcatggaaa gatcctcctt 6420
   cgaaaagaac ccaatcgact ttttggaagc taagggttac aaggaagtca agaaggattt 6480
   gatcattaag ttaccaaaat actccttgtt cgaattggaa aacggtagaa agagaatgtt 6540
   ggcctccgct ggtgaactac aaaaaggtaa cgaattggct ttaccatcta agtacgttaa 6600
   cttcttgtac ttggcttccc actacgaaaa gttgaaaggt tccccagaag acaacgaaca 6660
   aaagcaattg tttgttgaac aacacaagca ctacttggat gaaattattg aacaaatctc 6720
   cgaattctcc aagagagtca ttttggctga tgctaactta gataaggttt tatccgctta 6780
   caacaagcac agagacaaac caatcagaga acaagctgaa aacatcattc atttgttcac 6840
   tttaaccaac ttgggtgctc cagctgcttt caaatacttc gacactacca ttgacagaaa 6900
   gagatacact tccaccaaag aagttttaga tgctactttg attcaccaat ctattaccgg 6960
   tttgtacgaa accagaattg acttgtctca attgggtggt gattccagag ctgatccaaa 7020
   gaagaagaga aaggtgtaaa ggagttaaag gcaaagtttt cttttctaga gccgttccca 7080
   caaataatta tacgtatatg cttcttttcg tttactatat atctatattt acaagccttt 7140
   attcactgat gcaatttgtt tccaaatact tttttggaga tctcataact agatatcatg 7200
   atggcgcaac ttggcgctat cttaattact ctggctgcca ggcccgtgta gagggccgca 7260
   agaccttctg tacgccatat agtctctaag aacttgaaca agtttctaga cctattgccg 7320
   cctttcggat cgctattgtt gcggccgcca gctgaagctt cgtacgctgc aggtcgacga 7380
   attctaccgt tcgtataatg tatgctatac gaagttatag atctgtttag cttgcctcgt 7440
   ccccgccggg tcacccggcc agcgacatgg aggcccagaa taccctcctt gacagtcttg 7500
   acgtgcgcag ctcaggggca tgatgtgact gtcgcccgta catttagccc atacatcccc 7560
   atgtataatc atttgcatcc atacattttg atggccgcac ggcgcgaagc aaaaattacg 7620
   gctcctcgct gcagacctgc gagcagggaa acgctcccct cacagacgcg ttgaattgtc 7680
   cccacgccgc gcccctgtag agaaatataa aaggttagga tttgccactg aggttcttct 7740
   ttcatatact tccttttaaa atcttgctag gatacagttc tcacatcaca tccgaacata 7800
   aacaaccatg ggtaaggaaa agactcacgt ttcgaggccg cgattaaatt ccaacatgga 7860
   tgctgattta tatgggtata aatgggctcg cgataatgtc gggcaatcag gtgcgacaat 7920
   ctatcgattg tatgggaagc ccgatgcgcc agagttgttt ctgaaacatg gcaaaggtag 7980
   cgttgccaat gatgttacag atgagatggt cagactaaac tggctgacgg aatttatgcc 8040
   tcttccgacc atcaagcatt ttatccgtac tcctgatgat gcatggttac tcaccactgc 8100
   gatccccggc aaaacagcat tccaggtatt agaagaatat cctgattcag gtgaaaatat 8160
   tgttgatgcg ctggcagtgt tcctgcgccg gttgcattcg attcctgttt gtaattgtcc 8220
   ttttaacagc gatcgcgtat ttcgtctcgc tcaggcgcaa tcacgaatga ataacggttt 8280
   ggttgatgcg agtgattttg atgacgagcg taatggctgg cctgttgaac aagtctggaa 8340
   agaaatgcat aagcttttgc cattctcacc ggattcagtc gtcactcatg gtgatttctc 8400
   acttgataac cttatttttg acgaggggaa attaataggt tgtattgatg ttggacgagt 8460
   cggaatcgca gaccgatacc aggatcttgc catcctatgg aactgcctcg gtgagttttc 8520
   tccttcatta cagaaacggc tttttcaaaa atatggtatt gataatcctg atatgaataa 8580
   attgcagttt catttgatgc tcgatgagtt tttctaatca gtactgacaa taaaaagatt 8640
   cttgttttca agaacttgtc atttgtatag tttttttata ttgtagttgt tctattttaa 8700
   tcaaatgtta gcgtgattta tatttttttt cgcctcgaca tcatctgccc agatgcgaag 8760
   ttaagtgcgc agaaagtaat atcatgcgtc aatcgtatgt gaatgctggt cgctatactg 8820
   ctgtcgattc gatactaacg ccgccatcca gtgtcgaaaa cgagctcata acttcgtata 8880
   atgtatgcta tacgaacggt agaattcgaa tcagatccac tagtggccta tgcggccgcc 8940
   accgcggtgg agctccagct tttgttccct ttagtgaggg ttaattgcgc gcttggcgta 9000
   atcatggtca tagctgtttc ctgtgtgaaa ttgttatccg ctcacaattc cacacaacat 9060
   aggagccgga agcataaagt gtaaagcctg gggtgcctaa tgagtgaggt aactcacatt 9120
   aattgcgttg cgctcactgc ccgctttcca gtcgggaaac ctgtcgtgcc agctgcatta 9180
   atgaatcggc caacgcgcgg ggagaggcgg tttgcgtatt gggcgctctt ccgcttcctc 9240
   gctcactgac tcgctgcgct cggtcgttcg gctgcggcga gcggtatcag ctcactcaaa 9300
   ggcggtaata cggttatcca cagaatcagg ggataacgca ggaaagaaca tgtgagcaaa 9360
   aggccagcaa aaggccagga accgtaaaaa ggccgcgttg ctggcgtttt tccataggct 9420
   ccgcccccct gacgagcatc acaaaaatcg acgctcaagt cagaggtggc gaaacccgac 9480
   aggactataa agataccagg cgtttccccc tggaagctcc ctcgtgcgct ctcctgttcc 9540
   gaccctgccg cttaccggat acctgtccgc ctttctccct tcgggaagcg tggcgctttc 9600
   tcatagctca cgctgtaggt atctcagttc ggtgtaggtc gttcgctcca agctgggctg 9660
   tgtgcacgaa ccccccgttc agcccgaccg ctgcgcctta tccggtaact atcgtcttga 9720
   gtccaacccg gtaagacacg acttatcgcc actggcagca gccactggta acaggattag 9780
   cagagcgagg tatgtaggcg gtgctacaga gttcttgaag tggtggccta actacggcta 9840
   cactagaagg acagtatttg gtatctgcgc tctgctgaag ccagttacct tcggaaaaag 9900
   agttggtagc tcttgatccg gcaaacaaac caccgctggt agcggtggtt tttttgtttg 9960
   caagcagcag attacgcgca gaaaaaaagg atctcaagaa gatcctttga tcttttctac 10020
   ggggtctgac gctcagtgga acgaaaactc acgttaaggg attttggtca tgagattatc 10080
   aaaaaggatc ttcacctaga tccttttaaa ttaaaaatga agttttaaat caatctaaag 10140
   tatatatgag taaacttggt ctgacagtta ccaatgctta atcagtgagg cacctatctc 10200
   agcgatctgt ctatttcgtt catccatagt tgcctgactc cccgtcgtgt agataactac 10260
   gatacgggag ggcttaccat ctggccccag tgctgcaatg ataccgcgag acccacgctc 10320
   accggctcca gatttatcag caataaacca gccagccgga agggccgagc gcagaagtgg 10380
   tcctgcaact ttatccgcct ccatccagtc tattaattgt tgccgggaag ctagagtaag 10440
   tagttcgcca gttaatagtt tgcgcaacgt tgttgccatt gctacaggca tcgtggtgtc 10500
   acgctcgtcg tttggtatgg cttcattcag ctccggttcc caacgatcaa ggcgagttac 10560
   atgatccccc atgttgtgca aaaaagcggt tagctccttc ggtcctccga tcgttgtcag 10620
   aagtaagttg gccgcagtgt tatcactcat ggttatggca gcactgcata attctcttac 10680
   tgtcatgcca tccgtaagat gcttttctgt gactggtgag tactcaacca agtcattctg 10740
   agaatagtgt atgcggcgac cgagttgctc ttgcccggcg tcaatacggg ataataccgc 10800
   gccacatagc agaactttaa aagtgctcat cattggaaaa cgttcttcgg ggcgaaaact 10860
   ctcaaggatc ttaccgctgt tgagatccag ttcgatgtaa cccactcgtg cacccaactg 10920
   atcttcagca tcttttactt tcaccagcgt ttctgggtga gcaaaaacag gaaggcaaaa 10980
   tgccgcaaaa aagggaataa gggcgacacg gaaatgttga atactcatac tcttcctttt 11040
   tcaatattat tgaagcattt atcagggtta ttgtctcatg agcggataca tatttgaatg 11100
   tatttagaaa aataaacaaa taggggttcc gcgcacattt ccccgaaaag tgccacctgg 11160
   gtccttttca tcacgtgcta taaaaataat tataatttaa attttttaat ataaatatat 11220
   aaattaaaaa tagaaagtaa aaaaagaaat taaagaaaaa atagtttttg ttttccgaag 11280
   atgtaaaaga ctctaggggg atcgccaaca aatactacct tttatcttgc tcttcctgct 11340
   ctcaggtatt aatgccgaat tgtttcatct tgtctgtgta gaagaccaca cacgaaaatc 11400
   ctgtgatttt acattttact tatcgttaat cgaatgtata tctatttaat ctgcttttct 11460
   tgtctaataa atatatatgt aaagtacgct ttttgttgaa attttttaaa cctttgttta 11520
   tttttttttc ttcattccgt aactcttcta ccttctttat ttactttcta aaatccaaat 11580
   acaaaacata aaaataaata aacacagagt aaattcccaa attattccat cattaaaaga 11640
   tacgaggcgc gtgtaagtta caggcaagcg atccgtccta agaaaccatt attatcatga 11700
   cattaaccta taaaaatagg cgtatcacga ggccctttcg tc 11742
<210> 24
   <211> 1609
   <212> DNA
   <213> artificial sequence
<220>
   <223> Brick 3b (natMX-fragment)
<400> 24
   gttttagagc tagaaatagc aagttaaaat aaggctagtc cgttatcaac ttgaaaaagt 60
   ggcaccgagt cggtggtgct ttttttgttt tttatgtctt cgagtcatgt aattagttat 120
   gtctgagatc tgtttagctt gcctcgtccc cgccgggtca cccggccagc gacatggagg 180
   cccagaatac cctccttgac agtcttgacg tgcgcagctc aggggcatga tgtgactgtc 240
   gcccgtacat ttagcccata catccccatg tataatcatt tgcatccata cattttgatg 300
   gccgcacggc gcgaagcaaa aattacggct cctcgctgca gacctgcgag cagggaaacg 360
   ctcccctcac agacgcgttg aattgtcccc acgccgcgcc cctgtagaga aatataaaag 420
   gttaggattt gccactgagg ttcttctttc atatacttcc ttttaaaatc ttgctaggat 480
   acagttctca catcacatcc gaacataaac aaccatgggt accactcttg acgacacggc 540
   ttaccggtac cgcaccagtg tcccggggga cgccgaggcc atcgaggcac tggatgggtc 600
   cttcaccacc gacaccgtct tccgcgtcac cgccaccggg gacggcttca ccctgcggga 660
   ggtgccggtg gacccgcccc tgaccaaggt gttccccgac gacgaatcgg acgacgaatc 720
   ggacgacggg gaggacggcg acccggactc ccggacgttc gtcgcgtacg gggacgacgg 780
   cgacctggcg ggcttcgtgg tcgtctcgta ctccggctgg aaccgccggc tgaccgtcga 840
   ggacatcgag gtcgccccgg agcaccgggg gcacggggtc gggcgcgcgt tgatggggct 900
   cgcgacggag ttcgcccgcg agcggggcgc cgggcacctc tggctggagg tcaccaacgt 960
   caacgcaccg gcgatccacg cgtaccggcg gatggggttc accctctgcg gcctggacac 1020
   cgccctgtac gacggcaccg cctcggacgg cgagcaggcg ctctacatga gcatgccctg 1080
   cccctaatca gtactgacaa taaaaagatt cttgttttca agaacttgtc atttgtatag 1140
   tttttttata ttgtagttgt tctattttaa tcaaatgtta gcgtgattta tatttttttt 1200
   cgcctcgaca tcatctgccc agatgcgaag ttaagtgcgc agaaagtaat atcatgcgtc 1260
   aatcgtatgt gaatgctggt cgctatactg ctgtcgattc gatactaacg ccgccatcca 1320
   gtgtcgaaaa cgagctcgaa ttctttgaaa agataatgta tgattatgct ttcactcata 1380
   tttatacaga aacttgatgt tttctttcga gtatatacaa ggtgattaca tgtacgtttg 1440
   aagtacaact ctagattttg tagtgccctc ttgggctagc ggtaaaggtg cgcatttttt 1500
   cacaccctac aatgttctgt tcaaaagatt ttggtcaaac gctgtagaag tgaaagttgg 1560
   tgcgcatgtt tcggcgttcg aaacttctcc gcagtgaaag ataaatgat 1609
<210> 25
   <211> 61
   <212> DNA
   <213> artificial sequence
<220>
   <223> forward primer g1-INT59-f
<400> 25
   tgaaagataa atgatcagaa aactcttagc ttttccgttt tagagctaga aatagcaagt 60
   t 61
<210> 26
   <211> 61
   <212> DNA
   <213> artificial sequence
<220>
   <223> forward primer g2-YPRc-f
<400> 26
   tgaaagataa atgatccaat atggtatgcc gagtctgttt tagagctaga aatagcaagt 60
   t 61
<210> 27
   <211> 61
   <212> DNA
   <213> artificial sequence
<220>
   <223> forward primer g3-INT1-f
<400> 27
   tgaaagataa atgatctatt agaaccaggg aggtccgttt tagagctaga aatagcaagt 60
   t 61
<210> 28
   <211> 50
   <212> DNA
   <213> artificial sequence
<220>
   <223> forward primer Brick4_to3-f
<400> 28
   gccatccagt gtcgaaaacg agctcgcgag cggtatcagc tcactcaaag 50
<210> 29
   <211> 61
   <212> DNA
   <213> artificial sequence
<220>
   <223> reverse primer g1-INT59-r
<400> 29
   ttctagctct aaaacggaaa agctaagagt tttctgatca tttatctttc actgcggaga 60
   a 61
<210> 30
   <211> 61
   <212> DNA
   <213> artificial sequence
<220>
   <223> reverse primer g2-YPRc-r
<400> 30
   ttctagctct aaaacagact cggcatacca tattggatca tttatctttc actgcggaga 60
   a 61
<210> 31
   <211> 61
   <212> DNA
   <213> artificial sequence
<220>
   <223> reverse primer g3-INT1-r
<400> 31
   ttctagctct aaaacggacc tccctggttc taatagatca tttatctttc actgcggaga 60
   a 61
<210> 32
   <211> 50
   <212> DNA
   <213> artificial sequence
<220>
   <223> reverse primer Brick 3_to4-r
<400> 32
   ctttgagtga gctgataccg ctcgcgagct cgttttcgac actggatggc 50
<210> 33
   <211> 1945
   <212> DNA
   <213> artificial sequence
<220>
   <223> extended Brick 1 (Example 4)
<400> 33
   tgaaagataa atgatagaaa actcttagct tttccgtttt agagctagaa atagcaagtt      60
   aaaataaggc tagtccgtta tcaacttgaa aaagtggcac cgagtcggtg gtgctttttt     120
   tgttttttat gtcttcgagt catgtaatta gttatgtcgt ggcatgctag ctccggatta     180
   tcgatgataa gctgtcaaac atgagaatta attccacgga ctatagacta tacctagtat     240
   actccgtcta ctgtacgata cacttccgct caggtccttg tcctttaacg aggccttacc     300
   actcttttgt tactctattg atccagctca gcaaaggcag tgtgatctaa gattctatct 360
   tcgcgatgta gtaaaactag ctagaccgag aaagagacta gaaatgcaaa aggcacttct 420
   acaatggctg ccatcattat tatccgatgt gacgctgcag cttctcaatg atattcgaat 480
   acgctttgag gagatacagc ctaatatccg acaaactgtt ttacagattt acgatcgtac 540
   ttgttaccca tcattgaatt ttgaacatcc gaacctggga gttttccctg aaacagatag 600
   tatatttgaa cctgtataat aatatatagt ctagcgcttt acggaagaca atgtatgtat 660
   ttcggttcct ggagaaacta ttgcatctat tgcataggta atcttgcacg tcgcatcccc 720
   ggttcatttt ctgcgtttcc atcttgcact tcaatagcat atctttgtta acgaagcatc 780
   tgtgcttcat tttgtagaac aaaaatgcaa cgcgagagcg ctaatttttc aaacaaagaa 840
   tctgagctgc atttttacag aacagaaatg caacgcgaaa gcgctatttt accaacgaag 900
   aatctgtgct tcatttttgt aaaacaaaaa tgcaacgcga gagcgctaat ttttcaaaca 960
   aagaatctga gctgcatttt tacagaacag aaatgcaacg cgagagcgct attttaccaa 1020
   caaagaatct atacttcttt tttgttctac aaaaatgcat cccgagagcg ctatttttct 1080
   aacaaagcat cttagattac tttttttctc ctttgtgcgc tctataatgc agtctcttga 1140
   taactttttg cactgtaggt ccgttaaggt tagaagaagg ctactttggt gtctattttc 1200
   tcttccataa aaaaagcctg actccacttc ccgcgtttac tgattactag cgaagctgcg 1260
   ggtgcatttt ttcaagataa aggcatcccc gattatattc tataccgatg tggattgcgc 1320
   atactttgtg aacagaaagt gatagcgttg atgattcttc attggtcaga aaattatgaa 1380
   cggtttcttc tattttgtct ctatatacta cgtataggaa atgtttacat tttcgtattg 1440
   ttttcgattc actctatgaa tagttcttac tacaattttt ttgtctaaag agtaatacta 1500
   gagataaaca taaaaaatgt agaggtcgag tttagatgca agttcaagga gcgaaaggtg 1560
   gatgggtagg ttatataggg atatagcaca gagatatata gcaaagagat acttttgagc 1620
   aatgtttgtg gaaagcggct gttctttgaa aagataatgt atgattatgc tttcactcat 1680
   atttatacag aaacttgatg ttttctttcg agtatataca aggtgattac atgtacgttt 1740
   gaagtacaac tctagatttt gtagtgccct cttgggctag cggtaaaggt gcgcattttt 1800
   tcacacccta caatgttctg ttcaaaagat tttggtcaaa cgctgtagaa gtgaaagttg 1860
   gtgcgcatgt ttcggcgttc gaaacttctc cgcagtgaaa gataaatgat caatatggta 1920
   tgccgagtct gttttagagc tagaa 1945
<210> 34
   <211> 2195
   <212> DNA
   <213> artificial sequence
<220>
   <223> extended Brick 2 (Example 4)
<400> 34
   tgaaagataa atgatcaata tggtatgccg agtctgtttt agagctagaa atagcaagtt 60
   aaaataaggc tagtccgtta tcaacttgaa aaagtggcac cgagtcggtg gtgctttttt 120
   tgttttttat gtcttcgagt catgtaatta gttatgtctg gccggatcga tgtacacaac 180
   cgactgcacc caaacgaaca caaatcttag cagtgcttag tcaaaaaatt agccttttaa 240
   ttctgctgta acccgtacat gcccaaaata gggggcgggt tacacagaat atataacatc 300
   gtaggtgtct gggtgaacag tttattcctg gcatccacta aatataatgg agcccgcttt 360
   ttaagctggc atccagaaaa aaaaagaatc ccagcaccaa aatattgttt tcttcaccaa 420
   ccatcagttc ataggtccat tctcttagcg caactacaga gaacaggggc acaaacaggc 480
   aaaaaacggg cacaacctca atggagtgat gcaacctgcc tggagtaaat gatgacacaa 540
   ggcaattgac ccacgcatgt atctatctca ttttcttaca ccttctatta ccttctgctc 600
   tctctgattt ggaaaaagct gaaaaaaaag gttgaaacca gttccctgaa attattcccc 660
   tacttgacta ataagtatat aaagacggta ggtattgatt gtaattctgt aaatctattt 720
   cttaaacttc ttaaattcta cttttatagt tagtcttttt tttagtttta aaacaccaag 780
   aacttagttt cgaataaaca cacataaaca aacaaaatga ccgcactaac agaaggagct 840
   aaactattcg aaaaggagat tccttacatt acagaattag agggtgatgt cgaaggaatg 900
   aaattcatta tcaagggcga gggtactggt gacgctacta ccggtacgat taaagcaaag 960
   tacatctgta caacaggtga ccttcctgtt ccgtgggcta ctctggtgag cactttgtct 1020
   tatggagttc aatgttttgc taaataccct tcgcacatta aagacttttt caaaagtgca 1080
   atgcctgagg gctatactca ggagagaaca atatctttcg aaggagatgg tgtgtataag 1140
   actagggcta tggtcacgta tgaaagagga tccatctaca atagagtaac tttaactggt 1200
   gaaaacttca aaaaggacgg tcacatcctt agaaagaatg ttgcctttca atgcccacca 1260
   tccatcttgt acattttgcc agacacagtt aacaatggta tcagagttga gtttaaccaa 1320
   gcttatgaca tagagggtgt caccgaaaag ttggttacaa aatgttcaca gatgaatcgt 1380
   cccctggcag gatcagctgc cgtccatatc ccacgttacc atcatatcac ttatcatacc 1440
   aagctgtcca aagatcgtga tgagagaagg gatcacatgt gtttggttga agtggtaaag 1500
   gccgtggatt tggatactta ccaaggttga taaagcgaat ttcttatgat ttatgatttt 1560
   tattattaaa taagttataa aaaaaataag tgtatacaaa ttttaaagtg actcttaggt 1620
   tttaaaacga aaattcttat tcttgagtaa ctctttcctg taggtcaggt tgctttctca 1680
   ggtatagcat gaggtcgctc ttattgacca cacctctacc ggcatgccga gcaaatgcct 1740
   gcaaatcgct ccccatttca cccaattgta gatatgctaa ctccagcaat gagttgatga 1800
   atctcggtgt gtattttatg tcctcagagg acaacctcag aaagcctgta tgcgaagcca      1860
   caatcctttc caacagacca tactaagtga attctttgaa aagataatgt atgattatgc      1920
   tttcactcat atttatacag aaacttgatg ttttctttcg agtatataca aggtgattac      1980
   atgtacgttt gaagtacaac tctagatttt gtagtgccct cttgggctag cggtaaaggt      2040
   gcgcattttt tcacacccta caatgttctg ttcaaaagat tttggtcaaa cgctgtagaa      2100
   gtgaaagttg gtgcgcatgt ttcggcgttc gaaacttctc cgcagtgaaa gataaatgat      2160
   tattagaacc agggaggtcc gttttagagc tagaa                                  2195
<210> 35
   <211> 1397
   <212> DNA
   <213> artificial sequence
<220>
   <223> extended Brick 3b (Example 4)
<400> 35
   tgaaagataa atgattatta gaaccaggga ggtccgtttt agagctagaa atagcaagtt      60
   aaaataaggc tagtccgtta tcaacttgaa aaagtggcac cgagtcggtg gtgctttttt     120
   tgttttttat gtcttcgagt catgtaatta gttatgtctg agatctgttt agcttgcctc    180
   gtccccgccg ggtcacccgg ccagcgacat ggaggcccag aataccctcc ttgacagtct     240
   tgacgtgcgc agctcagggg catgatgtga ctgtcgcccg tacatttagc ccatacatcc     300
   ccatgtataa tcatttgcat ccatacattt tgatggccgc acggcgcgaa gcaaaaatta     360
   cggctcctcg ctgcagacct gcgagcaggg aaacgctccc ctcacagacg cgttgaattg     420
   tccccacgcc gcgcccctgt agagaaatat aaaaggttag gatttgccac tgaggttctt     480
   ctttcatata cttcctttta aaatcttgct aggatacagt tctcacatca catccgaaca     540
   taaacaacca tgggtaccac tcttgacgac acggcttacc ggtaccgcac cagtgtcccg     600
   ggggacgccg aggccatcga ggcactggat gggtccttca ccaccgacac cgtcttccgc     660
   gtcaccgcca ccggggacgg cttcaccctg cgggaggtgc cggtggaccc gcccctgacc     720
   aaggtgttcc ccgacgacga atcggacgac gaatcggacg acggggagga cggcgacccg     780
   gactcccgga cgttcgtcgc gtacggggac gacggcgacc tggcgggctt cgtggtcgtc     840
   tcgtactccg gctggaaccg ccggctgacc gtcgaggaca tcgaggtcgc cccggagcac     900
   cgggggcacg gggtcgggcg cgcgttgatg gggctcgcga cggagttcgc ccgcgagcgg     960
   ggcgccgggc acctctggct ggaggtcacc aacgtcaacg caccggcgat ccacgcgtac    1020
   cggcggatgg ggttcaccct ctgcggcctg gacaccgccc tgtacgacgg caccgcctcg    1080
   gacggcgagc aggcgctcta catgagcatg ccctgcccct aatcagtact gacaataaaa    1140
   agattcttgt tttcaagaac ttgtcatttg tatagttttt ttatattgta gttgttctat    1200
   tttaatcaaa tgttagcgtg atttatattt tttttcgcct cgacatcatc tgcccagatg    1260
   cgaagttaag tgcgcagaaa gtaatatcat gcgtcaatcg tatgtgaatg ctggtcgcta    1320
   tactgctgtc gattcgatac taacgccgcc atccagtgtc gaaaacgagc tcgcgagcgg    1380
   tatcagctca ctcaaag                                                   1397
<210> 36
   <211> 1983
   <212> DNA
   <213> artificial sequence
<220>
   <223> extended Brick 4 (Example 4)
<400> 36
   gccatccagt gtcgaaaacg agctcgcgag cggtatcagc tcactcaaag gcggtaatac 60
   ggttatccac agaatcaggg gataacgcag gaaagaacat gtgagcaaaa ggccagcaaa 120
   aggccaggaa ccgtaaaaag gccgcgttgc tggcgttttt ccataggctc cgcccccctg 180
   acgagcatca caaaaatcga cgctcaagtc agaggtggcg aaacccgaca ggactataaa 240
   gataccaggc gtttccccct ggaagctccc tcgtgcgctc tcctgttccg accctgccgc 300
   ttaccggata cctgtccgcc tttctccctt cgggaagcgt ggcgctttct catagctcac 360
   gctgtaggta tctcagttcg gtgtaggtcg ttcgctccaa gctgggctgt gtgcacgaac 420
   cccccgttca gcccgaccgc tgcgccttat ccggtaacta tcgtcttgag tccaacccgg 480
   taagacacga cttatcgcca ctggcagcag ccactggtaa caggattagc agagcgaggt 540
   atgtaggcgg tgctacagag ttcttgaagt ggtgggctaa ctacggctac actagaagaa 600
   cagtatttgg tatctgcgct ctgctgaagc cagttacctt cggaaaaaga gttggtagct 660
   cttgatccgg caaacaaacc accgctggta gcggtggttt ttttgtttgc aagcagcaga 720
   ttacgcgcag aaaaaaagga tctcaagaag atcctttgat cttttctacg gggtctgacg 780
   ctcagtggaa cgacgcgcgc gtaactcacg ttaagggatt ttggtcatga gcttgcgccg 840
   tcccgtcaag tcagcgtaat gctctgcttt tagaaaaact catcgagcat caaatgaaac 900
   tgcaatttat tcatatcagg attatcaata ccatattttt gaaaaagccg tttctgtaat 960
   gaaggagaaa actcaccgag gcagttccat aggatggcaa gatcctggta tcggtctgcg 1020
   attccgactc gtccaacatc aatacaacct attaatttcc cctcgtcaaa aataaggtta 1080
   tcaagtgaga aatcaccatg agtgacgact gaatccggtg agaatggcaa aagtttatgc 1140
   atttctttcc agacttgttc aacaggccag ccattacgct cgtcatcaaa atcactcgca 1200
   tcaaccaaac cgttattcat tcgtgattgc gcctgagcga ggcgaaatac gcgatcgctg 1260
   ttaaaaggac aattacaaac aggaatcgag tgcaaccggc gcaggaacac tgccagcgca 1320
   tcaacaatat tttcacctga atcaggatat tcttctaata cctggaacgc tgtttttccg 1380
   gggatcgcag tggtgagtaa ccatgcatca tcaggagtac ggataaaatg cttgatggtc 1440
   ggaagtggca taaattccgt cagccagttt agtctgacca tctcatctgt aacatcattg 1500
   gcaacgctac ctttgccatg tttcagaaac aactctggcg catcgggctt cccatacaag 1560
   cgatagattg tcgcacctga ttgcccgaca ttatcgcgag cccatttata cccatataaa 1620
   tcagcatcca tgttggaatt taatcgcggc ctcgacgttt cccgttgaat atggctgaat 1680
   tctttgaaaa gataatgtat gattatgctt tcactcatat ttatacagaa acttgatgtt 1740
   ttctttcgag tatatacaag gtgattacat gtacgtttga agtacaactc tagattttgt 1800
   agtgccctct tgggctagcg gtaaaggtgc gcattttttc acaccctaca atgttctgtt 1860
   caaaagattt tggtcaaacg ctgtagaagt gaaagttggt gcgcatgttt cggcgttcga 1920
   aacttctccg cagtgaaaga taaatgatag aaaactctta gcttttccgt tttagagcta 1980
   gaa 1983
<210> 37
<400> 37
   000
<210> 38
   <211> 2540
   <212> DNA
   <213> artificial sequence
<220>
   <223> con5 - Low p (KlTDH2p) - crtE - ScTDH3t - conA expression cassette
<400> 38
   aagcgacttc caatcgcttt gcatatccag taccacaccc acaggcgttt gtgccgtaaa      60
   aactaaaacg agcccccacc aaagaacaaa aaagaaggtg ctgggccccc actttcttcc     120
   cttgcacgtg ataggaagat ggctacagaa acaagaagat ggaaatcgaa ggaaagaggg     180
   agactggaag ctgtaaaaac tgaaatgaaa aaaaaaaaaa aaaaaaaaaa caagaagctg     240
   aaaatggaag actgaaattt gaaaaatggt aaaaaaaaaa aagaaacacg aagctaaaaa     300
   cctggattcc attttgagaa gaagcaagaa aggtaagtat ggtaacgacc gtacaggcaa     360
   gcgcgaaggc aaatggaaaa gctggagtcc ggaagataat catttcatct tcttttgtta     420
   gaacagaaca gtggatgtcc ctcatctcgg taacgtattg tccatgccct agaactctct     480
   gtccctaaaa agaggacaaa aacccaatgg tttccccagc ttccagtgga gccaccgatc     540
   ccactggaaa ccactggaca ggaagagaaa atcacggact tcctctattg aaggataatt     600
   caacactttc accagatccc aaatgtcccg cccctattcc cgtgttccat cacgtaccat     660
   aacttaccat ttcatcacgt tctctatggc acactggtac tgcttcgact gctttgcttc     720
   atcttctcta tgggccaatg agctaatgag cacaatgtgc tgcgaaataa agggatatct 780
   aatttatatt attacattat aatatgtact agtgtggtta ttggtaattg tacttaattt 840
   tgatatataa agggtggatc tttttcattt tgaatcagaa ttggaattgc aacttgtctc 900
   ttgtcactat tacttaatag taattatatt tcttattaac ctttttttta agtcaaaaca 960
   ccaaggacaa gaactactct tcaaaggtat ttcaagttat catacgtgtc acacacgctt 1020
   cacagtttca agtaaaaaaa aagaatatta cacaatggac tacgctaaca tcttgactgc 1080
   cattcctttg gaattcaccc cacaagatga cattgtcttg ttggaaccat accactactt 1140
   aggtaagaac ccaggtaagg aaatcagatc tcaattgatt gaagctttca actactggtt 1200
   agatgtcaag aaggaagact tggaagttat ccaaaatgtt gttggtatgt tgcacaccgc 1260
   ttctttgttg atggatgatg ttgaagattc ttccgtcttg agaagaggtt ctccagttgc 1320
   tcatttgatc tacggtattc cacaaaccat caacactgct aactacgttt acttcttggc 1380
   ttaccaagaa atcttcaaat tgcgtccaac tccaattcca atgccagtta tcccaccatc 1440
   ttctgcttct ttgcaatctt ctgtctcctc cgcctcctct tcctcttctg cctcctctga 1500
   aaacggtggt acctccactc caaactccca aatcccattc tccaaggaca cctacttgga 1560
   caaggttatc actgacgaaa tgttgtcttt gcaccgtggt caaggtttgg aattattctg 1620
   gagagactct ttgacctgtc catctgaaga agaatacgtc aagatggtct tgggtaagac 1680
   cggtggtttg ttcagaattg ctgtcagatt gatgatggcc aagtctgaat gtgacattga 1740
   ctttgttcaa ttggttaact tgatttccat ctacttccaa atcagagatg actacatgaa 1800
   cttgcaatcc tctgaatacg ctcacaacaa gaacttcgct gaagacttga ctgaaggtaa 1860
   gttctccttc ccaaccattc actccattca cgctaaccca tcttccagat tggttatcaa 1920
   cactttacaa aagaagtcca cttctccaga aatcttacat cactgtgtca actacatgag 1980
   aactgaaacc cactctttcg aatacactca agaagtcttg aacactttat ctggtgcttt 2040
   ggaaagagaa ttgggtagat tacaaggtga atttgctgaa gctaactcca agatcgattt 2100
   gggtgacgtt gaatctgaag gtagaaccgg taagaacgtc aaattggaag ccatcttgaa 2160
   gaaattggct gatatccctc tataaagtga atttacttta aatcttgcat ttaaataaat 2220
   tttcttttta tagctttatg acttagtttc aatttatata ctattttaat gacattttcg 2280
   attcattgat tgaaagcttt gtgttttttc ttgatgcgct attgcattgt tcttgtcttt 2340
   ttcgccacat gtaatatctg tagtagatac ctgatacatt gtggatgctg agtgaaattt 2400
   tagttaataa tggaggcgct cttaataatt ttggggatat tggctttttt ttttaaagtt 2460
   tacaaatgaa ttttttccgc caggatcctc ttgcccatcg aacgtacaag tactcctctg 2520
   ttctctcctt cctttgcttt 2540
<210> 39
   <211> 2540
   <212> DNA
   <213> artificial sequence
<220>
   <223> con5 - Med p (KlPGK1p) - crtE - ScTDH3t - conA expression cassette
<400> 39
   aagcgacttc caatcgcttt gcatatccag taccacaccc acaggcgttt gtgcgttcct      60
   catcactaga agccgaactg ttgtcttcag tggggattgg ttcgacattt tgccaattgc     120
   tgtcgatgta ccctttcaaa gccatgtacc ttaaatcttc atccttggca agtagattca     180
   tcgggtgtgt ttgaagtaag aatatttgct tgtttttatg gtatcaaagg tatatgttgt     240
   agaagacaat ttccggtaat ccaattgtct gtctgctcag tttagcacat gtatagtacg     300
   ttgcacatag tctacaatat tcagcattca gcattcagta tacagcatat ggctaaatga     360
   tcacaaatgt gattgatgat ttgacacgac tagaaaagag aacgaaaaag ggaaattcca     420
   tgtcacgtgc gttggcacgt gacatggaat atcgaagaaa gaaaaaaaaa acgatctcgt     480
   cctagtggaa gcccagagtc tggtcccccc ggagtcttcc caaaacaaga agctgacaca     540
   tgttgacaca gaacacccca cagcaaatgc accacgctac gtagatcagg aagcttaact     600
   ctagcgacct gtcgctcgcc ccacagaacc tcacccgaga accacacatt acacgccgcc     660
   agctcccact atactcatct tgcttccctt aagcgttctc acgattcgtt cgctgccctt     720
   cttcaagagt cttctgattc taattctcat tcgaaatcct ctacagttaa tgaattgctt     780
   gacatgacat tcattgtctc atggttttgg ctttttggct tttgtctttt aaagctatat     840
   caactttaca tataaatata cgtcaaaagg ggattcatta attagaaaat tctctttttc     900
   aatagttgct attcattatc aatctattca actcaattgg ttattatttt catctttttg     960
   tcatcctaaa ccatcaacaa tatttaaata tatctgttgc tacattaaga gttacttcag    1020
   aaataacaaa aaaatcgatc aagaattaat aaaaatggac tacgctaaca tcttgactgc    1080
   cattcctttg gaattcaccc cacaagatga cattgtcttg ttggaaccat accactactt    1140
   aggtaagaac ccaggtaagg aaatcagatc tcaattgatt gaagctttca actactggtt    1200
   agatgtcaag aaggaagact tggaagttat ccaaaatgtt gttggtatgt tgcacaccgc    1260
   ttctttgttg atggatgatg ttgaagattc ttccgtcttg agaagaggtt ctccagttgc    1320
   tcatttgatc tacggtattc cacaaaccat caacactgct aactacgttt acttcttggc    1380
   ttaccaagaa atcttcaaat tgcgtccaac tccaattcca atgccagtta tcccaccatc    1440
   ttctgcttct ttgcaatctt ctgtctcctc cgcctcctct tcctcttctg cctcctctga    1500
   aaacggtggt acctccactc caaactccca aatcccattc tccaaggaca cctacttgga    1560
   caaggttatc actgacgaaa tgttgtcttt gcaccgtggt caaggtttgg aattattctg 1620
   gagagactct ttgacctgtc catctgaaga agaatacgtc aagatggtct tgggtaagac 1680
   cggtggtttg ttcagaattg ctgtcagatt gatgatggcc aagtctgaat gtgacattga 1740
   ctttgttcaa ttggttaact tgatttccat ctacttccaa atcagagatg actacatgaa 1800
   cttgcaatcc tctgaatacg ctcacaacaa gaacttcgct gaagacttga ctgaaggtaa 1860
   gttctccttc ccaaccattc actccattca cgctaaccca tcttccagat tggttatcaa 1920
   cactttacaa aagaagtcca cttctccaga aatcttacat cactgtgtca actacatgag 1980
   aactgaaacc cactctttcg aatacactca agaagtcttg aacactttat ctggtgcttt 2040
   ggaaagagaa ttgggtagat tacaaggtga atttgctgaa gctaactcca agatcgattt 2100
   gggtgacgtt gaatctgaag gtagaaccgg taagaacgtc aaattggaag ccatcttgaa 2160
   gaaattggct gatatccctc tataaagtga atttacttta aatcttgcat ttaaataaat 2220
   tttcttttta tagctttatg acttagtttc aatttatata ctattttaat gacattttcg     2280
   attcattgat tgaaagcttt gtgttttttc ttgatgcgct attgcattgt tcttgtcttt     2340
   ttcgccacat gtaatatctg tagtagatac ctgatacatt gtggatgctg agtgaaattt 2400
   tagttaataa tggaggcgct cttaataatt ttggggatat tggctttttt ttttaaagtt 2460
   tacaaatgaa ttttttccgc caggatcctc ttgcccatcg aacgtacaag tactcctctg 2520
   ttctctcctt cctttgcttt 2540
<210> 40
   <211> 2140
   <212> DNA
   <213> artificial sequence
<220>
   <223> con5 - Strong p (ScFBA1p) - crtE - ScTDH3t - conA expression cassette
<400> 40
   aagcgacttc caatcgcttt gcatatccag taccacaccc acaggcgttt gtgcctactt      60
   ggcttcacat acgttgcata cgtcgatata gataataatg ataatgacag caggattatc     120
   gtaatacgta atagttgaaa atctcaaaaa tgtgtgggtc attacgtaaa taatgatagg     180
   aatgggattc ttctattttt cctttttcca ttctagcagc cgtcgggaaa acgtggcatc     240
   ctctctttcg ggctcaattg gagtcacgct gccgtgagca tcctctcttt ccatatctaa     300
   caactgagca cgtaaccaat ggaaaagcat gagcttagcg ttgctccaaa aaagtattgg     360
   atggttaata ccatttgtct gttctcttct gactttgact cctcaaaaaa aaaaaatcta     420
   caatcaacag atcgcttcaa ttacgccctc acaaaaactt ttttccttct tcttcgccca     480
   cgttaaattt tatccctcat gttgtctaac ggatttctgc acttgattta ttataaaaag     540
   acaaagacat aatacttctc tatcaatttc agttattgtt cttccttgcg ttattcttct 600
   gttcttcttt ttcttttgtc atatataacc ataaccaagt aatacatatt caaaatggac 660
   tacgctaaca tcttgactgc cattcctttg gaattcaccc cacaagatga cattgtcttg 720
   ttggaaccat accactactt aggtaagaac ccaggtaagg aaatcagatc tcaattgatt 780
   gaagctttca actactggtt agatgtcaag aaggaagact tggaagttat ccaaaatgtt 840
   gttggtatgt tgcacaccgc ttctttgttg atggatgatg ttgaagattc ttccgtcttg 900
   agaagaggtt ctccagttgc tcatttgatc tacggtattc cacaaaccat caacactgct 960
   aactacgttt acttcttggc ttaccaagaa atcttcaaat tgcgtccaac tccaattcca 1020
   atgccagtta tcccaccatc ttctgcttct ttgcaatctt ctgtctcctc cgcctcctct 1080
   tcctcttctg cctcctctga aaacggtggt acctccactc caaactccca aatcccattc 1140
   tccaaggaca cctacttgga caaggttatc actgacgaaa tgttgtcttt gcaccgtggt 1200
   caaggtttgg aattattctg gagagactct ttgacctgtc catctgaaga agaatacgtc 1260
   aagatggtct tgggtaagac cggtggtttg ttcagaattg ctgtcagatt gatgatggcc 1320
   aagtctgaat gtgacattga ctttgttcaa ttggttaact tgatttccat ctacttccaa 1380
   atcagagatg actacatgaa cttgcaatcc tctgaatacg ctcacaacaa gaacttcgct 1440
   gaagacttga ctgaaggtaa gttctccttc ccaaccattc actccattca cgctaaccca 1500
   tcttccagat tggttatcaa cactttacaa aagaagtcca cttctccaga aatcttacat 1560
   cactgtgtca actacatgag aactgaaacc cactctttcg aatacactca agaagtcttg 1620
   aacactttat ctggtgcttt ggaaagagaa ttgggtagat tacaaggtga atttgctgaa 1680
   gctaactcca agatcgattt gggtgacgtt gaatctgaag gtagaaccgg taagaacgtc 1740
   aaattggaag ccatcttgaa gaaattggct gatatccctc tataaagtga atttacttta 1800
   aatcttgcat ttaaataaat tttcttttta tagctttatg acttagtttc aatttatata 1860
   ctattttaat gacattttcg attcattgat tgaaagcttt gtgttttttc ttgatgcgct 1920
   attgcattgt tcttgtcttt ttcgccacat gtaatatctg tagtagatac ctgatacatt 1980
   gtggatgctg agtgaaattt tagttaataa tggaggcgct cttaataatt ttggggatat 2040
   tggctttttt ttttaaagtt tacaaatgaa ttttttccgc caggatcctc ttgcccatcg 2100
   aacgtacaag tactcctctg ttctctcctt cctttgcttt 2140
<210> 41
   <211> 3431
   <212> DNA
   <213> artificial sequence
<220>
   <223> conA - Low p (KlYDRp) - crtYB - ScPDC1t - conB expression cassette
<400> 41
   ttgcccatcg aacgtacaag tactcctctg ttctctcctt cctttgcttt gtgcttttct 60
   ttttttgcgg tcacccccat gtggcgggga ggcagaggag taggtagagc aacgaatcct 120
   actatttatc caaattagtc taggaactct ttttctagat tttttagatt tgagggcaag 180
   cgctgttaac gactcagaaa tgtaagcact acggagtaga acgagaaatc cgccataggt 240
   ggaaatccta gcaaaatctt gcttacccta gctagcctca ggtaagctag ccttagcctg 300
   tcaaattttt ttcaaaattt ggtaagtttc tactagcaaa gcaaacacgg ttcaacaaac 360
   cgaaaactcc actcattata cgtggaaacc gaaacaaaaa aacaaaaacc aaaatactcg 420
   ccaatgagaa agttgctgcg tttctacttt cgaggaagag gaactgagag gattgactac 480
   gaaaggggca aaaacgagtc gtattctccc attattgtct gctaccacgc ggtctagtag 540
   aataagcaac cagtcaacgc taagacaggt aatcaaaata ccagtctgct ggctacgggc 600
   tagtttttac ctcttttaga acccactgta aaagtccgtt gtaaagcccg ttctcactgt 660
   tggcgttttt ttttttttgg tttagtttct tatttttcat ttttttcttt catgaccaaa 720
   aacaaacaaa tctcgcgatt tgtactgcgg ccactggggc gtggccaaaa aaatgacaaa 780
   tttagaaacc ttagtttctg atttttcctg ttatgaggag atatgataaa aaatattact 840
   gctttattgt ttttttttta tctactgaaa tagagaaact tacccaagga ggaggcaaaa 900
   aaaagagtat atatacagca ggtaccattc agattttaat atattctttt ctcttcttct 960
   acactattat tataataatt ttactatatt catttttagc ttaaaacctc atagaatatt 1020
   attcttcagt cactcgctta aatacttatc aaaaatgacc gctttggctt actaccaaat 1080
   ccacttgatc tacactttgc caatcttagg tttgctaggt ttgttgactt ctccaatttt 1140
   gaccaaattc gacatctaca agatttctat cttagtcttt attgctttct ctgctaccac 1200
   tccatgggac tcctggatca tcagaaacgg tgcctggacc tacccatctg ctgaatctgg 1260
   tcaaggtgtt ttcggtacct ttttggatgt cccatacgaa gaatacgcct tctttgttat 1320
   ccaaaccgtc atcaccggtt tggtttacgt tttggctacc agacatttgt tgccatcttt 1380
   ggctctacca aagacccgtt cttctgcctt gtctctagct ttgaaggctt taatcccatt 1440
   gccaatcatc tatttgttca ccgctcatcc atctccatcc ccagatcctt tggttactga 1500
   ccactacttc tacatgagag ctttgtcttt gttgatcacc ccaccaacca tgttgttggc 1560
   tgctttatct ggtgaatacg ctttcgactg gaaatctggt agagctaagt ccaccattgc 1620
   tgccatcatg atcccaactg tctacttgat ctgggttgac tacgttgccg ttggtcaaga 1680
   ctcctggtcc atcaacgatg aaaagattgt cggttggaga ttaggtggtg tcttgccaat 1740
   tgaagaagct atgttcttct tattgaccaa cttgatgatc gttttgggtt tgtctgcctg 1800
   tgaccacact caagccttgt acttgttgca cggtagaact atctacggta acaagaagat 1860
   gccatcttct ttcccattaa tcactccacc agttttgtcc ttgttcttct cctccagacc      1920
   atactcctcc caaccaaaga gagatttgga attggctgtc aagttgttgg aaaagaagtc      1980
   cagatctttc ttcgttgctt ctgccggttt cccatctgaa gtcagagaaa gattggttgg      2040
   tttgtacgct ttctgtcgtg tcaccgatga cttgattgac tctccagaag tttcctccaa      2100
   cccacacgct accattgaca tggtttccga tttcttgact ttattattcg gtcctccatt      2160
   gcacccatct caaccagaca agattttgtc ttctccatta ttaccacctt cccacccatc      2220
   cagaccaact ggtatgtacc cattaccacc acctccatct ttgtctccag ctgaattggt      2280
   ccaattcttg actgaacgtg tcccagttca ataccacttc gctttcagat tgttggccaa      2340
   attgcaaggt ttgattccaa gatacccatt ggatgaatta ttgagaggtt acaccactga      2400
   cttgatcttc ccattgtcca ctgaagccgt ccaagctaga aagaccccaa ttgaaactac      2460
   tgctgacttg ttggactacg gtttgtgtgt tgccggttct gttgctgaat tgttggtcta      2520
   cgtttcctgg gcttccgctc catcccaagt tccagctact attgaagaaa gagaagctgt      2580
   tttggtcgcc tctcgtgaaa tgggtaccgc tttgcaattg gtcaacattg ccagagatat      2640
   caagggtgac gctactgaag gtagattcta cttgccattg tctttctttg gtttgagaga      2700
   tgaatccaaa ttggccattc caactgactg gactgaacca agacctcaag atttcgacaa      2760
   attgttgtct ctatctccat cttccacttt accatcctct aacgcttctg aatccttcag      2820
   attcgaatgg aagacctact ctttgccatt ggttgcttac gctgaagatt tggctaagca      2880
   ctcttacaag ggtattgaca gattaccaac tgaagtccaa gctggtatga gagctgcttg      2940
   tgcttcttac ttgttgattg gtcgtgaaat caaggttgtc tggaagggtg atgtcggtga      3000
   aagaagaacc gttgctggtt ggagaagagt cagaaaggtt ttgtctgttg tcatgtccgg      3060
   ttgggaaggt caataaagcg atttaatctc taattattag ttaaagtttt ataagcattt      3120
   ttatgtaacg aaaaataaat tggttcatat tattactgca ctgtcactta ccatggaaag      3180
   accagacaag aagttgccga cagtctgttg aattggcctg gttaggctta agtctgggtc      3240
   cgcttcttta caaatttgga gaatttctct taaacgatat gtatattctt ttcgttggaa      3300
   aagatgtctt ccaaaaaaaa aaccgatgaa ttagtggaac caaggaaaaa aaaagaggta      3360
   tccttgatta aggaacacct ccggatcgat gtacacaacc gactgcaccc aaacgaacac      3420
   aaatcttagc a                                                           3431
<210> 42
   <211> 3431
   <212> DNA
   <213> artificial sequence
<220>
   <223> conA - Med p (KlTEF2p) - crtYB - ScPDC1t - conB expression cassette
<400> 42
   ttgcccatcg aacgtacaag tactcctctg ttctctcctt cctttgcttt gtgcgagcct      60
   gtccaagcaa atgccttctc ataaatggtg ccaaagaccc gcaagcccaa agcaattacc     120
   ccccaaaaag aaatgatata gtgcaagata cgtatatgac catgacttga ctaggtgaaa     180
   cagtgcagaa acagccgcac aaaagcagcc ctaaccctca gagtcgattt tactctttca     240
   ggtaataaag cctcgacatc aattttagac agaagccagg ctggcctcga gattatagcc     300
   ataggcaagc aagaggagag aaggggaggc cccccatggg gggcctcccc cccgctgtca     360
   aggtttggca gaacctagct tcattaggcc actagcccag cctaaaacgt caacgggcag     420
   gaggaacact cccacaagac ggcgtagtat tctcgattca taaccatttt ctcaatcgaa     480
   ttacacagaa cacaccgtac aaacctctct atcataacta cttaatagtc acacacgtac     540
   tcgtctaaat acacatcatc gtcctacaag ttcatcaaag tgttggacag acaactatac     600
   cagcatggat ctcttgtatc ggttcttttc tcccgctctc tcgcaataac aatgaacact     660
   gggtcaatca tagcctacac aggtgaacag agtagcgttt atacagggtt tatacggtga     720
   ttcctacggc aaaaattttt catttctaaa aaaaaaaaga aaaatttttc tttccaacgc     780
   tagaaggaaa agaaaaatct aattaaattg atttggtgat tttctgagag ttcccttttt     840
   catatatcga attttgaata taaaaggaga tcgaaaaaat ttttctattc aatctgtttt     900
   ctggttttat ttgatagttt ttttgtgtat tattattatg gattagtact ggtttatatg     960
   ggtttttctg tataacttct ttttatttta gtttgtttaa tcttattttg agttacatta    1020
   tagttcccta actgcaagag aagtaacatt aaaaatgacc gctttggctt actaccaaat    1080
   ccacttgatc tacactttgc caatcttagg tttgctaggt ttgttgactt ctccaatttt    1140
   gaccaaattc gacatctaca agatttctat cttagtcttt attgctttct ctgctaccac    1200
   tccatgggac tcctggatca tcagaaacgg tgcctggacc tacccatctg ctgaatctgg    1260
   tcaaggtgtt ttcggtacct ttttggatgt cccatacgaa gaatacgcct tctttgttat    1320
   ccaaaccgtc atcaccggtt tggtttacgt tttggctacc agacatttgt tgccatcttt    1380
   ggctctacca aagacccgtt cttctgcctt gtctctagct ttgaaggctt taatcccatt    1440
   gccaatcatc tatttgttca ccgctcatcc atctccatcc ccagatcctt tggttactga    1500
   ccactacttc tacatgagag ctttgtcttt gttgatcacc ccaccaacca tgttgttggc    1560
   tgctttatct ggtgaatacg ctttcgactg gaaatctggt agagctaagt ccaccattgc    1620
   tgccatcatg atcccaactg tctacttgat ctgggttgac tacgttgccg ttggtcaaga    1680
   ctcctggtcc atcaacgatg aaaagattgt cggttggaga ttaggtggtg tcttgccaat    1740
   tgaagaagct atgttcttct tattgaccaa cttgatgatc gttttgggtt tgtctgcctg    1800
   tgaccacact caagccttgt acttgttgca cggtagaact atctacggta acaagaagat 1860
   gccatcttct ttcccattaa tcactccacc agttttgtcc ttgttcttct cctccagacc 1920
   atactcctcc caaccaaaga gagatttgga attggctgtc aagttgttgg aaaagaagtc 1980
   cagatctttc ttcgttgctt ctgccggttt cccatctgaa gtcagagaaa gattggttgg 2040
   tttgtacgct ttctgtcgtg tcaccgatga cttgattgac tctccagaag tttcctccaa 2100
   cccacacgct accattgaca tggtttccga tttcttgact ttattattcg gtcctccatt 2160
   gcacccatct caaccagaca agattttgtc ttctccatta ttaccacctt cccacccatc 2220
   cagaccaact ggtatgtacc cattaccacc acctccatct ttgtctccag ctgaattggt 2280
   ccaattcttg actgaacgtg tcccagttca ataccacttc gctttcagat tgttggccaa 2340
   attgcaaggt ttgattccaa gatacccatt ggatgaatta ttgagaggtt acaccactga 2400
   cttgatcttc ccattgtcca ctgaagccgt ccaagctaga aagaccccaa ttgaaactac 2460
   tgctgacttg ttggactacg gtttgtgtgt tgccggttct gttgctgaat tgttggtcta 2520
   cgtttcctgg gcttccgctc catcccaagt tccagctact attgaagaaa gagaagctgt 2580
   tttggtcgcc tctcgtgaaa tgggtaccgc tttgcaattg gtcaacattg ccagagatat 2640
   caagggtgac gctactgaag gtagattcta cttgccattg tctttctttg gtttgagaga 2700
   tgaatccaaa ttggccattc caactgactg gactgaacca agacctcaag atttcgacaa 2760
   attgttgtct ctatctccat cttccacttt accatcctct aacgcttctg aatccttcag 2820
   attcgaatgg aagacctact ctttgccatt ggttgcttac gctgaagatt tggctaagca 2880
   ctcttacaag ggtattgaca gattaccaac tgaagtccaa gctggtatga gagctgcttg 2940
   tgcttcttac ttgttgattg gtcgtgaaat caaggttgtc tggaagggtg atgtcggtga 3000
   aagaagaacc gttgctggtt ggagaagagt cagaaaggtt ttgtctgttg tcatgtccgg 3060
   ttgggaaggt caataaagcg atttaatctc taattattag ttaaagtttt ataagcattt 3120
   ttatgtaacg aaaaataaat tggttcatat tattactgca ctgtcactta ccatggaaag 3180
   accagacaag aagttgccga cagtctgttg aattggcctg gttaggctta agtctgggtc 3240
   cgcttcttta caaatttgga gaatttctct taaacgatat gtatattctt ttcgttggaa 3300
   aagatgtctt ccaaaaaaaa aaccgatgaa ttagtggaac caaggaaaaa aaaagaggta 3360
   tccttgatta aggaacacct ccggatcgat gtacacaacc gactgcaccc aaacgaacac 3420
   aaatcttagc a 3431
<210> 43
   <211> 3031
   <212> DNA
   <213> artificial sequence
<220>
   <223> conA - Strong p (ScTEF1p) - crtYB - ScPDC1t - conB expression cassette
<400> 43
   ttgcccatcg aacgtacaag tactcctctg ttctctcctt cctttgcttt gtgcttggct 60
   gataatagcg tataaacaat gcatactttg tacgttcaaa atacaatgca gtagatatat 120
   ttatgcatat tacatataat acatatcaca taggaagcaa caggcgcgtt ggacttttaa 180
   ttttcgagga ccgcgaatcc ttacatcaca cccaatcccc cacaagtgat cccccacaca 240
   ccatagcttc aaaatgtttc tactcctttt ttactcttcc agattttctc ggactccgcg 300
   catcgccgta ccacttcaaa acacccaagc acagcatact aaatttcccc tctttcttcc 360
   tctagggtgt cgttaattac ccgtactaaa ggtttggaaa agaaaaaaga caccgcctcg 420
   tttctttttc ttcgtcgaaa aaggcaataa aaatttttat cacgtttctt tttcttgaaa 480
   attttttttt ttgatttttt tctctttcga tgacctccca ttgatattta agttaataaa 540
   cggtcttcaa tttctcaagt ttcagtttca tttttcttgt tctattacaa ctttttttac 600
   ttcttgctca ttagaaagaa agcatagcaa tctaatctaa gttttaatta caaaatgacc 660
   gctttggctt actaccaaat ccacttgatc tacactttgc caatcttagg tttgctaggt 720
   ttgttgactt ctccaatttt gaccaaattc gacatctaca agatttctat cttagtcttt 780
   attgctttct ctgctaccac tccatgggac tcctggatca tcagaaacgg tgcctggacc 840
   tacccatctg ctgaatctgg tcaaggtgtt ttcggtacct ttttggatgt cccatacgaa 900
   gaatacgcct tctttgttat ccaaaccgtc atcaccggtt tggtttacgt tttggctacc 960
   agacatttgt tgccatcttt ggctctacca aagacccgtt cttctgcctt gtctctagct 1020
   ttgaaggctt taatcccatt gccaatcatc tatttgttca ccgctcatcc atctccatcc 1080
   ccagatcctt tggttactga ccactacttc tacatgagag ctttgtcttt gttgatcacc 1140
   ccaccaacca tgttgttggc tgctttatct ggtgaatacg ctttcgactg gaaatctggt 1200
   agagctaagt ccaccattgc tgccatcatg atcccaactg tctacttgat ctgggttgac 1260
   tacgttgccg ttggtcaaga ctcctggtcc atcaacgatg aaaagattgt cggttggaga 1320
   ttaggtggtg tcttgccaat tgaagaagct atgttcttct tattgaccaa cttgatgatc 1380
   gttttgggtt tgtctgcctg tgaccacact caagccttgt acttgttgca cggtagaact 1440
   atctacggta acaagaagat gccatcttct ttcccattaa tcactccacc agttttgtcc 1500
   ttgttcttct cctccagacc atactcctcc caaccaaaga gagatttgga attggctgtc 1560
   aagttgttgg aaaagaagtc cagatctttc ttcgttgctt ctgccggttt cccatctgaa 1620
   gtcagagaaa gattggttgg tttgtacgct ttctgtcgtg tcaccgatga cttgattgac 1680
   tctccagaag tttcctccaa cccacacgct accattgaca tggtttccga tttcttgact 1740
   ttattattcg gtcctccatt gcacccatct caaccagaca agattttgtc ttctccatta 1800
   ttaccacctt cccacccatc cagaccaact ggtatgtacc cattaccacc acctccatct    1860
   ttgtctccag ctgaattggt ccaattcttg actgaacgtg tcccagttca ataccacttc    1920
   gctttcagat tgttggccaa attgcaaggt ttgattccaa gatacccatt ggatgaatta    1980
   ttgagaggtt acaccactga cttgatcttc ccattgtcca ctgaagccgt ccaagctaga    2040
   aagaccccaa ttgaaactac tgctgacttg ttggactacg gtttgtgtgt tgccggttct    2100
   gttgctgaat tgttggtcta cgtttcctgg gcttccgctc catcccaagt tccagctact    2160
   attgaagaaa gagaagctgt tttggtcgcc tctcgtgaaa tgggtaccgc tttgcaattg    2220
   gtcaacattg ccagagatat caagggtgac gctactgaag gtagattcta cttgccattg    2280
   tctttctttg gtttgagaga tgaatccaaa ttggccattc caactgactg gactgaacca    2340
   agacctcaag atttcgacaa attgttgtct ctatctccat cttccacttt accatcctct    2400
   aacgcttctg aatccttcag attcgaatgg aagacctact ctttgccatt ggttgcttac    2460
   gctgaagatt tggctaagca ctcttacaag ggtattgaca gattaccaac tgaagtccaa    2520
   gctggtatga gagctgcttg tgcttcttac ttgttgattg gtcgtgaaat caaggttgtc    2580
   tggaagggtg atgtcggtga aagaagaacc gttgctggtt ggagaagagt cagaaaggtt    2640
   ttgtctgttg tcatgtccgg ttgggaaggt caataaagcg atttaatctc taattattag    2700
   ttaaagtttt ataagcattt ttatgtaacg aaaaataaat tggttcatat tattactgca    2760
   ctgtcactta ccatggaaag accagacaag aagttgccga cagtctgttg aattggcctg    2820
   gttaggctta agtctgggtc cgcttcttta caaatttgga gaatttctct taaacgatat    2880
   gtatattctt ttcgttggaa aagatgtctt ccaaaaaaaa aaccgatgaa ttagtggaac    2940
   caaggaaaaa aaaagaggta tccttgatta aggaacacct ccggatcgat gtacacaacc    3000
   gactgcaccc aaacgaacac aaatcttagc a                                   3031
<210> 44
   <211> 2758
   <212> DNA
   <213> artificial sequence
<220>
   <223> conB - Low p (ScPRE3p) - crtI - ScTAL1t - conC expression cassette
<400> 44
   cggatcgatg tacacaaccg actgcaccca aacgaacaca aatcttagca gtgccaaaca      60
   ttaatttgtt ctgcatactt tgaacctttc agaaaataaa aaacattacg cgcatactta     120
   ccctgctcgc gaagaagagt aacactaacg cattctatgg gcaattgaag acagtattca     180
   gtacaagaca tagtccgttt ccttgagtca attcctatag cattatgaac tagccgcctt     240
   taagagtgcc aagctgttca acaccgatca tttttgatga tttggcgttt ttgttatatt     300
   gatagatttc ttttgaattt tgtcattttc acttttccac tcgcaacgga atccggtggc 360
   aaaaaaggga aaagcattga aatgcaatct ttaacagtat tttaaacaag ttgcgacacg 420
   gtgtacaatt acgataagaa ttgctacttc aaagtacaca cagaaagtta acatgaatgg 480
   aattcaagtg gacatcaatc gtttgaaaaa gggcgaagtc agtttaggta cctcaatgta 540
   tgtatataag aatttttcct cccactttat tgtttctaaa agttcaatga agtaaagtct 600
   caattggcct tattactaac taataggtat cttataatca cctaataaaa tagaatgggt 660
   aaggaacaag accaagacaa gccaactgcc atcatcgttg gttgtggtat cggtggtatt 720
   gctaccgctg ccagattagc taaggaaggt ttccaagtta ccgtctttga aaagaacgac 780
   tactccggtg gtagatgttc tttgattgaa agagatggtt acagattcga ccaaggtcca 840
   tctttgttgc tattaccaga cttgttcaag caaaccttcg aagatttggg tgaaaagatg 900
   gaagactggg ttgatttgat caagtgtgaa ccaaactacg tttgtcactt ccatgatgaa 960
   gaaactttca ccttctccac tgacatggct ttattgaaga gagaagtcga aagatttgaa 1020
   ggtaaagatg gtttcgacag attcttgtct ttcatccaag aagctcacag acattacgaa 1080
   ttggctgttg tccacgtctt gcaaaagaac ttcccaggtt tcgctgcttt cttgagatta 1140
   caattcatcg gtcaaatctt agctttgcac ccatttgaat ccatctggac cagagtttgt 1200
   cgttacttca agactgacag attgagaaga gtcttctcct ttgccgttat gtacatgggt 1260
   caatctccat actctgctcc aggtacctac tccttgttgc aatacactga attgactgaa 1320
   ggtatctggt acccaagagg tggtttctgg caagttccaa acactttgtt gcaaatcgtc 1380
   aagagaaaca acccatctgc taagttcaac ttcaacgctc cagtttctca agttttgttg 1440
   tctccagcta aggacagagc taccggtgtc agattagaat ctggtgaaga acaccacgct 1500
   gatgttgtca ttgtcaatgc tgacttggtc tacgcttctg aacatttgat tccagatgat 1560
   gctagaaaca agatcggtca attaggtgaa gttaagcgtt cctggtgggc tgatttggtt 1620
   ggtggtaaga agttgaaggg ttcttgttct tctttgtctt tctactggtc tatggacaga 1680
   atcgttgacg gtttgggtgg tcacaacatc ttcttggctg aagacttcaa gggttccttc 1740
   gacaccattt tcgaagaatt gggtttgcca gctgacccat ctttctatgt taacgttcca 1800
   tccagaattg acccttctgc tgctccagaa ggtaaggatg ccattgtcat cttagtccca 1860
   tgtggtcaca tcgatgcttc caaccctcaa gactacaaca aattggttgc cagagccaga 1920
   aagttcgtca tccaaacctt gtctgccaag ttgggtctac cagatttcga aaagatgatt 1980
   gttgctgaaa aggttcacga tgctccatcc tgggaaaagg aattcaactt gaaggacggt 2040
   tccattttgg gtttggctca caacttcatg caagtcttgg gtttcagacc atccaccaga 2100
   cacccaaagt acgacaaatt gttctttgtc ggtgcttcta cccacccagg tactggtgtt 2160
   ccaattgtct tggctggtgc caaattgact gctaaccaag ttttggaatc cttcgatcgt     2220
   tctccagctc cagatcctaa catgtctttg tctgttccat acggtaagcc attgaaatcc     2280
   aacggtactg gtattgactc tcaagtccaa ttgaaattca tggacttgga acgttgggtt     2340
   tacctattag tcttgttgat tggtgctgtt atcgccagat ccgtcggtgt cttggccttt     2400
   taaaggaagt atctcggaaa tattaattta ggccatgtcc ttatgcacgt ttcttttgat     2460
   acttacgggt acatgtacac aagtatatct atatatataa attaatgaaa atcccctatt     2520
   tatatatatg actttaacga gacagaacag ttttttattt tttatcctat ttgatgaatg     2580
   atacagtttc ttattcacgt gttataccca caccaaatcc aatagcaata ccggccatca     2640
   caatcactgt ttcggcagcc cctaagatca gacaaaacat ccggaaccac cttaaatcaa     2700
   cgtccctcac gctttccggc atcttccaga ccacagtata tccatccgcc tcctgttg       2758
<210> 45
   <211> 2758
   <212> DNA
   <213> artificial sequence
<220>
   <223> conB - Med p (ScACT1p) - crtI - ScTAL1t - conC expression cassette
<400> 45
   cggatcgatg tacacaaccg actgcaccca aacgaacaca aatcttagca gtgcaacata 60
   tatacacaat tacagtaaca ataacaagag gacagatact accaaaatgt gtggggaagc 120
   gggtaagctg ccacagcaat taatgcacaa catttaacct acattcttcc ttatcggatc 180
   ctcaaaaccc ttaaaaacat atgcctcacc ctaacatatt ttccaattaa ccctcaatat 240
   ttctctgtca cccggcctct attttccatt ttcttcttta cccgccacgc gtttttttct 300
   ttcaaatttt tttcttcttt cttctttttc ttccacgtcc tcttgcataa ataaataaac 360
   cgttttgaaa ccaaactcgc ctctctctct cctttttgaa atatttttgg gtttgtttga 420
   tcctttcctt cccaatctct cttgtttaat atatattcat ttatatcacg ctctcttttt 480
   atcttccttt ttttcctctc tcttgtattc ttccttcccc tttctactca aaccaagaag 540
   aaaaagaaaa ggtcaatctt tgttaaagaa taggatcttc tactacatca gcttttagat 600
   ttttcacgct tactgctttt ttcttcccaa gatcgaaaat ttactgaatt aacaatgggt 660
   aaggaacaag accaagacaa gccaactgcc atcatcgttg gttgtggtat cggtggtatt 720
   gctaccgctg ccagattagc taaggaaggt ttccaagtta ccgtctttga aaagaacgac 780
   tactccggtg gtagatgttc tttgattgaa agagatggtt acagattcga ccaaggtcca 840
   tctttgttgc tattaccaga cttgttcaag caaaccttcg aagatttggg tgaaaagatg 900
   gaagactggg ttgatttgat caagtgtgaa ccaaactacg tttgtcactt ccatgatgaa 960
   gaaactttca ccttctccac tgacatggct ttattgaaga gagaagtcga aagatttgaa 1020
   ggtaaagatg gtttcgacag attcttgtct ttcatccaag aagctcacag acattacgaa 1080
   ttggctgttg tccacgtctt gcaaaagaac ttcccaggtt tcgctgcttt cttgagatta 1140
   caattcatcg gtcaaatctt agctttgcac ccatttgaat ccatctggac cagagtttgt 1200
   cgttacttca agactgacag attgagaaga gtcttctcct ttgccgttat gtacatgggt 1260
   caatctccat actctgctcc aggtacctac tccttgttgc aatacactga attgactgaa 1320
   ggtatctggt acccaagagg tggtttctgg caagttccaa acactttgtt gcaaatcgtc 1380
   aagagaaaca acccatctgc taagttcaac ttcaacgctc cagtttctca agttttgttg 1440
   tctccagcta aggacagagc taccggtgtc agattagaat ctggtgaaga acaccacgct 1500
   gatgttgtca ttgtcaatgc tgacttggtc tacgcttctg aacatttgat tccagatgat 1560
   gctagaaaca agatcggtca attaggtgaa gttaagcgtt cctggtgggc tgatttggtt 1620
   ggtggtaaga agttgaaggg ttcttgttct tctttgtctt tctactggtc tatggacaga 1680
   atcgttgacg gtttgggtgg tcacaacatc ttcttggctg aagacttcaa gggttccttc 1740
   gacaccattt tcgaagaatt gggtttgcca gctgacccat ctttctatgt taacgttcca 1800
   tccagaattg acccttctgc tgctccagaa ggtaaggatg ccattgtcat cttagtccca 1860
   tgtggtcaca tcgatgcttc caaccctcaa gactacaaca aattggttgc cagagccaga 1920
   aagttcgtca tccaaacctt gtctgccaag ttgggtctac cagatttcga aaagatgatt 1980
   gttgctgaaa aggttcacga tgctccatcc tgggaaaagg aattcaactt gaaggacggt 2040
   tccattttgg gtttggctca caacttcatg caagtcttgg gtttcagacc atccaccaga 2100
   cacccaaagt acgacaaatt gttctttgtc ggtgcttcta cccacccagg tactggtgtt 2160
   ccaattgtct tggctggtgc caaattgact gctaaccaag ttttggaatc cttcgatcgt 2220
   tctccagctc cagatcctaa catgtctttg tctgttccat acggtaagcc attgaaatcc 2280
   aacggtactg gtattgactc tcaagtccaa ttgaaattca tggacttgga acgttgggtt 2340
   tacctattag tcttgttgat tggtgctgtt atcgccagat ccgtcggtgt cttggccttt 2400
   taaaggaagt atctcggaaa tattaattta ggccatgtcc ttatgcacgt ttcttttgat 2460
   acttacgggt acatgtacac aagtatatct atatatataa attaatgaaa atcccctatt 2520
   tatatatatg actttaacga gacagaacag ttttttattt tttatcctat ttgatgaatg 2580
   atacagtttc ttattcacgt gttataccca caccaaatcc aatagcaata ccggccatca 2640
   caatcactgt ttcggcagcc cctaagatca gacaaaacat ccggaaccac cttaaatcaa 2700
   cgtccctcac gctttccggc atcttccaga ccacagtata tccatccgcc tcctgttg 2758
<210> 46
   <211> 3158
   <212> DNA
   <213> artificial sequence
<220>
   <223> conB - Strong p (KlENO1p) - crtI - ScTAL1t - conC expression cassette
<400> 46
   cggatcgatg tacacaaccg actgcaccca aacgaacaca aatcttagca gtgccgtatc 60
   cctatctgga ttaacatcac tgccacagat cgaattgcaa gaagccacac ttcacgtgat 120
   ccactcgttc atcaggtttg tagcttcatg gcgcaggact tctgatgatg aactatctgg 180
   ctcatccgga tggatcacaa ggatggatac cctcagacag tacgtttccg tatggagcga 240
   tcttacaaga accaacagtt gatcctatta cttttttttt attttttgtc cctccgggat 300
   ggcaagaggg acaaagaaga atcttcgttc ttctttcttg ttctcaactt cccagcttcc 360
   gtgtgattac cctccgggac aacagaaaaa ctggcattcg gtatcccggg aatctgctga 420
   gaaggaaaga aaacgaaaaa aaaattgtac atttgtgtca cattatgaat tacaggaagt 480
   cagaaaacag gcagcacatg tctcgcacat gcatgtccat cagacgagac attatgagac 540
   atgcacgcgt gtgagagaca tagcaaaagt ctctccagta cacacagaaa gacacgttca 600
   caatccaggc accccacaga gaaaaaaaaa agaagaagcc cggaagctgg cacgccatca 660
   tcaaccaccg ctcggtttac acgcatccca actgtctttt ttttctggaa tcctataata 720
   actggcatct ggaaatcacg ttgtatgttg caccatagtg actggctgtc tgactagcaa 780
   acattgattc cctgattccc atttggctca attttgatga gaaacagttg attgattctt 840
   gtcaattttt ttttctttgg accaccacca accaattgac attgaagtac tttcccatga 900
   tttgaggtta tataaaagga cgttcaaatc actttcaagg ttaattcagt tttgtcaatt 960
   gatttaagtt caattgttaa caaatttaat ttaattcgaa acaaaccaaa ccaattcatt 1020
   tgaattaaca aaccaaccca caaaacaaaa aaaaatgggt aaggaacaag accaagacaa 1080
   gccaactgcc atcatcgttg gttgtggtat cggtggtatt gctaccgctg ccagattagc 1140
   taaggaaggt ttccaagtta ccgtctttga aaagaacgac tactccggtg gtagatgttc 1200
   tttgattgaa agagatggtt acagattcga ccaaggtcca tctttgttgc tattaccaga 1260
   cttgttcaag caaaccttcg aagatttggg tgaaaagatg gaagactggg ttgatttgat 1320
   caagtgtgaa ccaaactacg tttgtcactt ccatgatgaa gaaactttca ccttctccac 1380
   tgacatggct ttattgaaga gagaagtcga aagatttgaa ggtaaagatg gtttcgacag 1440
   attcttgtct ttcatccaag aagctcacag acattacgaa ttggctgttg tccacgtctt 1500
   gcaaaagaac ttcccaggtt tcgctgcttt cttgagatta caattcatcg gtcaaatctt 1560
   agctttgcac ccatttgaat ccatctggac cagagtttgt cgttacttca agactgacag 1620
   attgagaaga gtcttctcct ttgccgttat gtacatgggt caatctccat actctgctcc 1680
   aggtacctac tccttgttgc aatacactga attgactgaa ggtatctggt acccaagagg     1740
   tggtttctgg caagttccaa acactttgtt gcaaatcgtc aagagaaaca acccatctgc     1800
   taagttcaac ttcaacgctc cagtttctca agttttgttg tctccagcta aggacagagc     1860
   taccggtgtc agattagaat ctggtgaaga acaccacgct gatgttgtca ttgtcaatgc     1920
   tgacttggtc tacgcttctg aacatttgat tccagatgat gctagaaaca agatcggtca     1980
   attaggtgaa gttaagcgtt cctggtgggc tgatttggtt ggtggtaaga agttgaaggg     2040
   ttcttgttct tctttgtctt tctactggtc tatggacaga atcgttgacg gtttgggtgg     2100
   tcacaacatc ttcttggctg aagacttcaa gggttccttc gacaccattt tcgaagaatt     2160
   gggtttgcca gctgacccat ctttctatgt taacgttcca tccagaattg acccttctgc     2220
   tgctccagaa ggtaaggatg ccattgtcat cttagtccca tgtggtcaca tcgatgcttc     2280
   caaccctcaa gactacaaca aattggttgc cagagccaga aagttcgtca tccaaacctt     2340
   gtctgccaag ttgggtctac cagatttcga aaagatgatt gttgctgaaa aggttcacga     2400
   tgctccatcc tgggaaaagg aattcaactt gaaggacggt tccattttgg gtttggctca     2460
   caacttcatg caagtcttgg gtttcagacc atccaccaga cacccaaagt acgacaaatt     2520
   gttctttgtc ggtgcttcta cccacccagg tactggtgtt ccaattgtct tggctggtgc     2580
   caaattgact gctaaccaag ttttggaatc cttcgatcgt tctccagctc cagatcctaa     2640
   catgtctttg tctgttccat acggtaagcc attgaaatcc aacggtactg gtattgactc     2700
   tcaagtccaa ttgaaattca tggacttgga acgttgggtt tacctattag tcttgttgat     2760
   tggtgctgtt atcgccagat ccgtcggtgt cttggccttt taaaggaagt atctcggaaa     2820
   tattaattta ggccatgtcc ttatgcacgt ttcttttgat acttacgggt acatgtacac     2880
   aagtatatct atatatataa attaatgaaa atcccctatt tatatatatg actttaacga     2940
   gacagaacag tttttttatt tttatcctat ttgatgaatg atacagtttc ttattcacgt     3000
   gttataccca caccaaatcc aatagcaata ccggccatca caatcactgt ttcggcagcc     3060
   cctaagatca gacaaaacat ccggaaccac cttaaatcaa cgtccctcac gctttccggc     3120
   atcttccaga ccacagtata tccatccgcc tcctgttg                             3158
<210> 47
   <211> 2534
   <212> DNA
   <213> artificial sequence
<220>
   <223> donor expression cassette sequence with 50 bp LF and RF flanks (PCR fragment). Homology to INT1 - Low p (KlTDH2p) - crtE - ScTDH3t - Homology to INT1
<400> 47
   catgcctatc cggcggcaac attattactc tggtatacgg gcgtaactcc acgtaaaaac      60
   taaaacgagc ccccaccaaa gaacaaaaaa gaaggtgctg ggcccccact ttcttccctt    120
   gcacgtgata ggaagatggc tacagaaaca agaagatgga aatcgaagga aagagggaga     180
   ctggaagctg taaaaactga aatgaaaaaa aaaaaaaaaa aaaaaaaaca agaagctgaa     240
   aatggaagac tgaaatttga aaaatggtaa aaaaaaaaaa gaaacacgaa gctaaaaacc     300
   tggattccat tttgagaaga agcaagaaag gtaagtatgg taacgaccgt acaggcaagc     360
   gcgaaggcaa atggaaaagc tggagtccgg aagataatca tttcatcttc ttttgttaga     420
   acagaacagt ggatgtccct catctcggta acgtattgtc catgccctag aactctctgt     480
   ccctaaaaag aggacaaaaa cccaatggtt tccccagctt ccagtggagc caccgatccc     540
   actggaaacc actggacagg aagagaaaat cacggacttc ctctattgaa ggataattca     600
   acactttcac cagatcccaa atgtcccgcc cctattcccg tgttccatca cgtaccataa     660
   cttaccattt catcacgttc tctatggcac actggtactg cttcgactgc tttgcttcat     720
   cttctctatg ggccaatgag ctaatgagca caatgtgctg cgaaataaag ggatatctaa     780
   tttatattat tacattataa tatgtactag tgtggttatt ggtaattgta cttaattttg     840
   atatataaag ggtggatctt tttcattttg aatcagaatt ggaattgcaa cttgtctctt     900
   gtcactatta cttaatagta attatatttc ttattaacct tttttttaag tcaaaacacc     960
   aaggacaaga actactcttc aaaggtattt caagttatca tacgtgtcac acacgcttca    1020
   cagtttcaag taaaaaaaaa gaatattaca caatggacta cgctaacatc ttgactgcca    1080
   ttcctttgga attcacccca caagatgaca ttgtcttgtt ggaaccatac cactacttag    1140
   gtaagaaccc aggtaaggaa atcagatctc aattgattga agctttcaac tactggttag    1200
   atgtcaagaa ggaagacttg gaagttatcc aaaatgttgt tggtatgttg cacaccgctt    1260
   ctttgttgat ggatgatgtt gaagattctt ccgtcttgag aagaggttct ccagttgctc    1320
   atttgatcta cggtattcca caaaccatca acactgctaa ctacgtttac ttcttggctt    1380
   accaagaaat cttcaaattg cgtccaactc caattccaat gccagttatc ccaccatctt    1440
   ctgcttcttt gcaatcttct gtctcctccg cctcctcttc ctcttctgcc tcctctgaaa    1500
   acggtggtac ctccactcca aactcccaaa tcccattctc caaggacacc tacttggaca    1560
   aggttatcac tgacgaaatg ttgtctttgc accgtggtca aggtttggaa ttattctgga    1620
   gagactcttt gacctgtcca tctgaagaag aatacgtcaa gatggtcttg ggtaagaccg    1680
   gtggtttgtt cagaattgct gtcagattga tgatggccaa gtctgaatgt gacattgact    1740
   ttgttcaatt ggttaacttg atttccatct acttccaaat cagagatgac tacatgaact    1800
   tgcaatcctc tgaatacgct cacaacaaga acttcgctga agacttgact gaaggtaagt    1860
   tctccttccc aaccattcac tccattcacg ctaacccatc ttccagattg gttatcaaca    1920
   ctttacaaaa gaagtccact tctccagaaa tcttacatca ctgtgtcaac tacatgagaa 1980
   ctgaaaccca ctctttcgaa tacactcaag aagtcttgaa cactttatct ggtgctttgg 2040
   aaagagaatt gggtagatta caaggtgaat ttgctgaagc taactccaag atcgatttgg 2100
   gtgacgttga atctgaaggt agaaccggta agaacgtcaa attggaagcc atcttgaaga 2160
   aattggctga tatccctcta taaagtgaat ttactttaaa tcttgcattt aaataaattt 2220
   tctttttata gctttatgac ttagtttcaa tttatatact attttaatga cattttcgat 2280
   tcattgattg aaagctttgt gttttttctt gatgcgctat tgcattgttc ttgtcttttt 2340
   cgccacatgt aatatctgta gtagatacct gatacattgt ggatgctgag tgaaatttta 2400
   gttaataatg gaggcgctct taataatttt ggggatattg gctttttttt ttaaagttta 2460
   caaatgaatt ttttccgcca ggatgaagta tggatctttg aaaccctttt attttatttt 2520
   acttttttta gaat 2534
<210> 48
   <211> 2530
   <212> DNA
   <213> artificial sequence
<220>
   <223> donor expression cassette sequence with 50 bp LF and RF flanks (PCR fragment). Homology to INT1 - Med p (KlPGK1p) - crtE - ScTDH3t - Homology to INT1
<400> 48
   catgcctatc cggcggcaac attattactc tggtatacgg gcgtaactcc acctcatcac 60
   tagaagccga actgttgtct tcagtgggga ttggttcgac attttgccaa ttgctgtcga 120
   tgtacccttt caaagccatg taccttaaat cttcatcctt ggcaagtaga ttcatcgggt 180
   gtgtttgaag taagaatatt tgcttgtttt tatggtatca aaggtatatg ttgtagaaga 240
   caatttccgg taatccaatt gtctgtctgc tcagtttagc acatgtatag tacgttgcac 300
   atagtctaca atattcagca ttcagcattc agtatacagc atatggctaa atgatcacaa 360
   atgtgattga tgatttgaca cgactagaaa agagaacgaa aaagggaaat tccatgtcac 420
   gtgcgttggc acgtgacatg gaatatcgaa gaaagaaaaa aaaaacgatc tcgtcctagt 480
   ggaagcccag agtctggtcc ccccggagtc ttcccaaaac aagaagctga cacatgttga 540
   cacagaacac cccacagcaa atgcaccacg ctacgtagat caggaagctt aactctagcg 600
   acctgtcgct cgccccacag aacctcaccc gagaaccaca cattacacgc cgccagctcc 660
   cactatactc atcttgcttc ccttaagcgt tctcacgatt cgttcgctgc ccttcttcaa 720
   gagtcttctg attctaattc tcattcgaaa tcctctacag ttaatgaatt gcttgacatg 780
   acattcattg tctcatggtt ttggcttttt ggcttttgtc ttttaaagct atatcaactt 840
   tacatataaa tatacgtcaa aaggggattc attaattaga aaattctctt tttcaatagt 900
   tgctattcat tatcaatcta ttcaactcaa ttggttatta ttttcatctt tttgtcatcc 960
   taaaccatca acaatattta aatatatctg ttgctacatt aagagttact tcagaaataa 1020
   caaaaaaatc gatcaagaat taataaaaat ggactacgct aacatcttga ctgccattcc 1080
   tttggaattc accccacaag atgacattgt cttgttggaa ccataccact acttaggtaa 1140
   gaacccaggt aaggaaatca gatctcaatt gattgaagct ttcaactact ggttagatgt 1200
   caagaaggaa gacttggaag ttatccaaaa tgttgttggt atgttgcaca ccgcttcttt 1260
   gttgatggat gatgttgaag attcttccgt cttgagaaga ggttctccag ttgctcattt 1320
   gatctacggt attccacaaa ccatcaacac tgctaactac gtttacttct tggcttacca 1380
   agaaatcttc aaattgcgtc caactccaat tccaatgcca gttatcccac catcttctgc 1440
   ttctttgcaa tcttctgtct cctccgcctc ctcttcctct tctgcctcct ctgaaaacgg 1500
   tggtacctcc actccaaact cccaaatccc attctccaag gacacctact tggacaaggt 1560
   tatcactgac gaaatgttgt ctttgcaccg tggtcaaggt ttggaattat tctggagaga 1620
   ctctttgacc tgtccatctg aagaagaata cgtcaagatg gtcttgggta agaccggtgg 1680
   tttgttcaga attgctgtca gattgatgat ggccaagtct gaatgtgaca ttgactttgt 1740
   tcaattggtt aacttgattt ccatctactt ccaaatcaga gatgactaca tgaacttgca 1800
   atcctctgaa tacgctcaca acaagaactt cgctgaagac ttgactgaag gtaagttctc 1860
   cttcccaacc attcactcca ttcacgctaa cccatcttcc agattggtta tcaacacttt 1920
   acaaaagaag tccacttctc cagaaatctt acatcactgt gtcaactaca tgagaactga 1980
   aacccactct ttcgaataca ctcaagaagt cttgaacact ttatctggtg ctttggaaag 2040
   agaattgggt agattacaag gtgaatttgc tgaagctaac tccaagatcg atttgggtga 2100
   cgttgaatct gaaggtagaa ccggtaagaa cgtcaaattg gaagccatct tgaagaaatt 2160
   ggctgatatc cctctataaa gtgaatttac tttaaatctt gcatttaaat aaattttctt 2220
   tttatagctt tatgacttag tttcaattta tatactattt taatgacatt ttcgattcat 2280
   tgattgaaag ctttgtgttt tttcttgatg cgctattgca ttgttcttgt ctttttcgcc 2340
   acatgtaata tctgtagtag atacctgata cattgtggat gctgagtgaa attttagtta 2400
   ataatggagg cgctcttaat aattttgggg atattggctt ttttttttaa agtttacaaa 2460
   tgaatttttt ccgccaggat gaagtatgga tctttgaaac ccttttattt tattttactt 2520
   tttttagaat 2530
<210> 49
   <211> 2130
   <212> DNA
   <213> artificial sequence
<220>
   <223> donor expression cassette sequence with 50 bp LF and RF flanks (PCR fragment). Homology to INT1 - Strong p (ScFBA1p) - crtE - ScTDH3t -Homology to INT1
<400> 49
   catgcctatc cggcggcaac attattactc tggtatacgg gcgtaactcc acttggcttc 60
   acatacgttg catacgtcga tatagataat aatgataatg acagcaggat tatcgtaata 120
   cgtaatagtt gaaaatctca aaaatgtgtg ggtcattacg taaataatga taggaatggg 180
   attcttctat ttttcctttt tccattctag cagccgtcgg gaaaacgtgg catcctctct 240
   ttcgggctca attggagtca cgctgccgtg agcatcctct ctttccatat ctaacaactg 300
   agcacgtaac caatggaaaa gcatgagctt agcgttgctc caaaaaagta ttggatggtt 360
   aataccattt gtctgttctc ttctgacttt gactcctcaa aaaaaaaaaa tctacaatca 420
   acagatcgct tcaattacgc cctcacaaaa acttttttcc ttcttcttcg cccacgttaa 480
   attttatccc tcatgttgtc taacggattt ctgcacttga tttattataa aaagacaaag 540
   acataatact tctctatcaa tttcagttat tgttcttcct tgcgttattc ttctgttctt 600
   ctttttcttt tgtcatatat aaccataacc aagtaataca tattcaaaat ggactacgct 660
   aacatcttga ctgccattcc tttggaattc accccacaag atgacattgt cttgttggaa 720
   ccataccact acttaggtaa gaacccaggt aaggaaatca gatctcaatt gattgaagct 780
   ttcaactact ggttagatgt caagaaggaa gacttggaag ttatccaaaa tgttgttggt 840
   atgttgcaca ccgcttcttt gttgatggat gatgttgaag attcttccgt cttgagaaga 900
   ggttctccag ttgctcattt gatctacggt attccacaaa ccatcaacac tgctaactac 960
   gtttacttct tggcttacca agaaatcttc aaattgcgtc caactccaat tccaatgcca 1020
   gttatcccac catcttctgc ttctttgcaa tcttctgtct cctccgcctc ctcttcctct 1080
   tctgcctcct ctgaaaacgg tggtacctcc actccaaact cccaaatccc attctccaag 1140
   gacacctact tggacaaggt tatcactgac gaaatgttgt ctttgcaccg tggtcaaggt 1200
   ttggaattat tctggagaga ctctttgacc tgtccatctg aagaagaata cgtcaagatg 1260
   gtcttgggta agaccggtgg tttgttcaga attgctgtca gattgatgat ggccaagtct 1320
   gaatgtgaca ttgactttgt tcaattggtt aacttgattt ccatctactt ccaaatcaga 1380
   gatgactaca tgaacttgca atcctctgaa tacgctcaca acaagaactt cgctgaagac 1440
   ttgactgaag gtaagttctc cttcccaacc attcactcca ttcacgctaa cccatcttcc 1500
   agattggtta tcaacacttt acaaaagaag tccacttctc cagaaatctt acatcactgt 1560
   gtcaactaca tgagaactga aacccactct ttcgaataca ctcaagaagt cttgaacact 1620
   ttatctggtg ctttggaaag agaattgggt agattacaag gtgaatttgc tgaagctaac 1680
   tccaagatcg atttgggtga cgttgaatct gaaggtagaa ccggtaagaa cgtcaaattg 1740
   gaagccatct tgaagaaatt ggctgatatc cctctataaa gtgaatttac tttaaatctt 1800
   gcatttaaat aaattttctt tttatagctt tatgacttag tttcaattta tatactattt 1860
   taatgacatt ttcgattcat tgattgaaag ctttgtgttt tttcttgatg cgctattgca 1920
   ttgttcttgt ctttttcgcc acatgtaata tctgtagtag atacctgata cattgtggat 1980
   gctgagtgaa attttagtta ataatggagg cgctcttaat aattttgggg atattggctt 2040
   ttttttttaa agtttacaaa tgaatttttt ccgccaggat gaagtatgga tctttgaaac 2100
   ccttttattt tattttactt tttttagaat 2130
<210> 50
   <211> 3428
   <212> DNA
   <213> artificial sequence
<220>
   <223> donor expression cassette sequence with 50 bp LF and RF flanks (PCR fragment). Homology to INT2 - Low p (KlYDR1p) - crtYB - ScPDC1t - Homology to INT2
<400> 50
   caaaccgcag attgcgtcac ggttctgcgg acgccgttag cccattggtg tcaagttttc 60
   tttttttgcg gtcaccccca tgtggcgggg aggcagagga gtaggtagag caacgaatcc 120
   tactatttat ccaaattagt ctaggaactc tttttctaga ttttttagat ttgagggcaa 180
   gcgctgttaa cgactcagaa atgtaagcac tacggagtag aacgagaaat ccgccatagg 240
   tggaaatcct agcaaaatct tgcttaccct agctagcctc aggtaagcta gccttagcct 300
   gtcaaatttt tttcaaaatt tggtaagttt ctactagcaa agcaaacacg gttcaacaaa 360
   ccgaaaactc cactcattat acgtggaaac cgaaacaaaa aaacaaaaac caaaatactc 420
   gccaatgaga aagttgctgc gtttctactt tcgaggaaga ggaactgaga ggattgacta 480
   cgaaaggggc aaaaacgagt cgtattctcc cattattgtc tgctaccacg cggtctagta 540
   gaataagcaa ccagtcaacg ctaagacagg taatcaaaat accagtctgc tggctacggg 600
   ctagttttta cctcttttag aacccactgt aaaagtccgt tgtaaagccc gttctcactg 660
   ttggcgtttt tttttttttg gtttagtttc ttatttttca tttttttctt tcatgaccaa 720
   aaacaaacaa atctcgcgat ttgtactgcg gccactgggg cgtggccaaa aaaatgacaa 780
   atttagaaac cttagtttct gatttttcct gttatgagga gatatgataa aaaatattac 840
   tgctttattg tttttttttt atctactgaa atagagaaac ttacccaagg aggaggcaaa 900
   aaaaagagta tatatacagc aggtaccatt cagattttaa tatattcttt tctcttcttc 960
   tacactatta ttataataat tttactatat tcatttttag cttaaaacct catagaatat 1020
   tattcttcag tcactcgctt aaatacttat caaaaatgac cgctttggct tactaccaaa 1080
   tccacttgat ctacactttg ccaatcttag gtttgctagg tttgttgact tctccaattt 1140
   tgaccaaatt cgacatctac aagatttcta tcttagtctt tattgctttc tctgctacca 1200
   ctccatggga ctcctggatc atcagaaacg gtgcctggac ctacccatct gctgaatctg 1260
   gtcaaggtgt tttcggtacc tttttggatg tcccatacga agaatacgcc ttctttgtta 1320
   tccaaaccgt catcaccggt ttggtttacg ttttggctac cagacatttg ttgccatctt 1380
   tggctctacc aaagacccgt tcttctgcct tgtctctagc tttgaaggct ttaatcccat 1440
   tgccaatcat ctatttgttc accgctcatc catctccatc cccagatcct ttggttactg 1500
   accactactt ctacatgaga gctttgtctt tgttgatcac cccaccaacc atgttgttgg 1560
   ctgctttatc tggtgaatac gctttcgact ggaaatctgg tagagctaag tccaccattg 1620
   ctgccatcat gatcccaact gtctacttga tctgggttga ctacgttgcc gttggtcaag 1680
   actcctggtc catcaacgat gaaaagattg tcggttggag attaggtggt gtcttgccaa 1740
   ttgaagaagc tatgttcttc ttattgacca acttgatgat cgttttgggt ttgtctgcct 1800
   gtgaccacac tcaagccttg tacttgttgc acggtagaac tatctacggt aacaagaaga 1860
   tgccatcttc tttcccatta atcactccac cagttttgtc cttgttcttc tcctccagac 1920
   catactcctc ccaaccaaag agagatttgg aattggctgt caagttgttg gaaaagaagt 1980
   ccagatcttt cttcgttgct tctgccggtt tcccatctga agtcagagaa agattggttg 2040
   gtttgtacgc tttctgtcgt gtcaccgatg acttgattga ctctccagaa gtttcctcca 2100
   acccacacgc taccattgac atggtttccg atttcttgac tttattattc ggtcctccat 2160
   tgcacccatc tcaaccagac aagattttgt cttctccatt attaccacct tcccacccat 2220
   ccagaccaac tggtatgtac ccattaccac cacctccatc tttgtctcca gctgaattgg 2280
   tccaattctt gactgaacgt gtcccagttc aataccactt cgctttcaga ttgttggcca 2340
   aattgcaagg tttgattcca agatacccat tggatgaatt attgagaggt tacaccactg 2400
   acttgatctt cccattgtcc actgaagccg tccaagctag aaagacccca attgaaacta 2460
   ctgctgactt gttggactac ggtttgtgtg ttgccggttc tgttgctgaa ttgttggtct 2520
   acgtttcctg ggcttccgct ccatcccaag ttccagctac tattgaagaa agagaagctg 2580
   ttttggtcgc ctctcgtgaa atgggtaccg ctttgcaatt ggtcaacatt gccagagata 2640
   tcaagggtga cgctactgaa ggtagattct acttgccatt gtctttcttt ggtttgagag 2700
   atgaatccaa attggccatt ccaactgact ggactgaacc aagacctcaa gatttcgaca 2760
   aattgttgtc tctatctcca tcttccactt taccatcctc taacgcttct gaatccttca 2820
   gattcgaatg gaagacctac tctttgccat tggttgctta cgctgaagat ttggctaagc 2880
   actcttacaa gggtattgac agattaccaa ctgaagtcca agctggtatg agagctgctt 2940
   gtgcttctta cttgttgatt ggtcgtgaaa tcaaggttgt ctggaagggt gatgtcggtg 3000
   aaagaagaac cgttgctggt tggagaagag tcagaaaggt tttgtctgtt gtcatgtccg 3060
   gttgggaagg tcaataaagc gatttaatct ctaattatta gttaaagttt tataagcatt 3120
   tttatgtaac gaaaaataaa ttggttcata ttattactgc actgtcactt accatggaaa 3180
   gaccagacaa gaagttgccg acagtctgtt gaattggcct ggttaggctt aagtctgggt 3240
   ccgcttcttt acaaatttgg agaatttctc ttaaacgata tgtatattct tttcgttgga 3300
   aaagatgtct tccaaaaaaa aaaccgatga attagtggaa ccaaggaaaa aaaaagaggt 3360
   atccttgatt aaggaacaga acaatactga ttgcgttacc ccaagcttat cccacgtagt 3420
   tttgtgtc 3428
<210> 51
   <211> 3420
   <212> DNA
   <213> artificial sequence
<220>
   <223> donor expression cassette sequence with 50 bp LF and RF flanks (PCR fragment). Homology to INT2 - Med p (KlTEF2p) - crtYB - ScPDC1t - Homology to INT2
<400> 51
   cgcagattgc gtcacggttc tgcggacgcc gttagcccat tggtgtcaag cctgtccaag      60
   caaatgcctt ctcataaatg gtgccaaaga cccgcaagcc caaagcaatt accccccaaa     120
   aagaaatgat atagtgcaag atacgtatat gaccatgact tgactaggtg aaacagtgca     180
   gaaacagccg cacaaaagca gccctaaccc tcagagtcga ttttactctt tcaggtaata     240
   aagcctcgac atcaatttta gacagaagcc aggctggcct cgagattata gccataggca     300
   agcaagagga gagaagggga ggccccccat ggggggcctc ccccccgctg tcaaggtttg     360
   gcagaaccta gcttcattag gccactagcc cagcctaaaa cgtcaacggg caggaggaac     420
   actcccacaa gacggcgtag tattctcgat tcataaccat tttctcaatc gaattacaca     480
   gaacacaccg tacaaacctc tctatcataa ctacttaata gtcacacacg tactcgtcta     540
   aatacacatc atcgtcctac aagttcatca aagtgttgga cagacaacta taccagcatg     600
   gatctcttgt atcggttctt ttctcccgct ctctcgcaat aacaatgaac actgggtcaa     660
   tcatagccta cacaggtgaa cagagtagcg tttatacagg gtttatacgg tgattcctac     720
   ggcaaaaatt tttcatttct aaaaaaaaaa agaaaaattt ttctttccaa cgctagaagg     780
   aaaagaaaaa tctaattaaa ttgatttggt gattttctga gagttccctt tttcatatat     840
   cgaattttga atataaaagg agatcgaaaa aatttttcta ttcaatctgt tttctggttt     900
   tatttgatag tttttttgtg tattattatt atggattagt actggtttat atgggttttt     960
   ctgtataact tctttttatt ttagtttgtt taatcttatt ttgagttaca ttatagttcc    1020
   ctaactgcaa gagaagtaac attaaaaatg accgctttgg cttactacca aatccacttg 1080
   atctacactt tgccaatctt aggtttgcta ggtttgttga cttctccaat tttgaccaaa 1140
   ttcgacatct acaagatttc tatcttagtc tttattgctt tctctgctac cactccatgg 1200
   gactcctgga tcatcagaaa cggtgcctgg acctacccat ctgctgaatc tggtcaaggt 1260
   gttttcggta cctttttgga tgtcccatac gaagaatacg ccttctttgt tatccaaacc 1320
   gtcatcaccg gtttggttta cgttttggct accagacatt tgttgccatc tttggctcta 1380
   ccaaagaccc gttcttctgc cttgtctcta gctttgaagg ctttaatccc attgccaatc 1440
   atctatttgt tcaccgctca tccatctcca tccccagatc ctttggttac tgaccactac 1500
   ttctacatga gagctttgtc tttgttgatc accccaccaa ccatgttgtt ggctgcttta 1560
   tctggtgaat acgctttcga ctggaaatct ggtagagcta agtccaccat tgctgccatc 1620
   atgatcccaa ctgtctactt gatctgggtt gactacgttg ccgttggtca agactcctgg 1680
   tccatcaacg atgaaaagat tgtcggttgg agattaggtg gtgtcttgcc aattgaagaa 1740
   gctatgttct tcttattgac caacttgatg atcgttttgg gtttgtctgc ctgtgaccac 1800
   actcaagcct tgtacttgtt gcacggtaga actatctacg gtaacaagaa gatgccatct 1860
   tctttcccat taatcactcc accagttttg tccttgttct tctcctccag accatactcc 1920
   tcccaaccaa agagagattt ggaattggct gtcaagttgt tggaaaagaa gtccagatct 1980
   ttcttcgttg cttctgccgg tttcccatct gaagtcagag aaagattggt tggtttgtac 2040
   gctttctgtc gtgtcaccga tgacttgatt gactctccag aagtttcctc caacccacac 2100
   gctaccattg acatggtttc cgatttcttg actttattat tcggtcctcc attgcaccca 2160
   tctcaaccag acaagatttt gtcttctcca ttattaccac cttcccaccc atccagacca 2220
   actggtatgt acccattacc accacctcca tctttgtctc cagctgaatt ggtccaattc 2280
   ttgactgaac gtgtcccagt tcaataccac ttcgctttca gattgttggc caaattgcaa 2340
   ggtttgattc caagataccc attggatgaa ttattgagag gttacaccac tgacttgatc 2400
   ttcccattgt ccactgaagc cgtccaagct agaaagaccc caattgaaac tactgctgac 2460
   ttgttggact acggtttgtg tgttgccggt tctgttgctg aattgttggt ctacgtttcc 2520
   tgggcttccg ctccatccca agttccagct actattgaag aaagagaagc tgttttggtc 2580
   gcctctcgtg aaatgggtac cgctttgcaa ttggtcaaca ttgccagaga tatcaagggt 2640
   gacgctactg aaggtagatt ctacttgcca ttgtctttct ttggtttgag agatgaatcc 2700
   aaattggcca ttccaactga ctggactgaa ccaagacctc aagatttcga caaattgttg 2760
   tctctatctc catcttccac tttaccatcc tctaacgctt ctgaatcctt cagattcgaa 2820
   tggaagacct actctttgcc attggttgct tacgctgaag atttggctaa gcactcttac 2880
   aagggtattg acagattacc aactgaagtc caagctggta tgagagctgc ttgtgcttct 2940
   tacttgttga ttggtcgtga aatcaaggtt gtctggaagg gtgatgtcgg tgaaagaaga 3000
   accgttgctg gttggagaag agtcagaaag gttttgtctg ttgtcatgtc cggttgggaa 3060
   ggtcaataaa gcgatttaat ctctaattat tagttaaagt tttataagca tttttatgta 3120
   acgaaaaata aattggttca tattattact gcactgtcac ttaccatgga aagaccagac 3180
   aagaagttgc cgacagtctg ttgaattggc ctggttaggc ttaagtctgg gtccgcttct 3240
   ttacaaattt ggagaatttc tcttaaacga tatgtatatt cttttcgttg gaaaagatgt 3300
   cttccaaaaa aaaaaccgat gaattagtgg aaccaaggaa aaaaaaagag gtatccttga 3360
   ttaaggaaca gaacaatact gattgcgtta ccccaagctt atcccacgta gttttgtgtc 3420
<210> 52
   <211> 3025
   <212> DNA
   <213> artificial sequence
<220>
   <223> donor expression cassette sequence with 50 bp LF and RF flanks (PCR fragment). Homology to INT2 - Strong p (ScTEF1p) - crtYB - ScPDC1t - Homology to INT2
<400> 52
   caaaccgcag attgcgtcac ggttctgcgg acgccgttag cccattggtg tcaaggctga 60
   taatagcgta taaacaatgc atactttgta cgttcaaaat acaatgcagt agatatattt 120
   atgcatatta catataatac atatcacata ggaagcaaca ggcgcgttgg acttttaatt 180
   ttcgaggacc gcgaatcctt acatcacacc caatccccca caagtgatcc cccacacacc 240
   atagcttcaa aatgtttcta ctcctttttt actcttccag attttctcgg actccgcgca 300
   tcgccgtacc acttcaaaac acccaagcac agcatactaa atttcccctc tttcttcctc 360
   tagggtgtcg ttaattaccc gtactaaagg tttggaaaag aaaaaagaca ccgcctcgtt 420
   tctttttctt cgtcgaaaaa ggcaataaaa atttttatca cgtttctttt tcttgaaaat 480
   tttttttttt gatttttttc tctttcgatg acctcccatt gatatttaag ttaataaacg 540
   gtcttcaatt tctcaagttt cagtttcatt tttcttgttc tattacaact ttttttactt 600
   cttgctcatt agaaagaaag catagcaatc taatctaagt tttaattaca aaatgaccgc 660
   tttggcttac taccaaatcc acttgatcta cactttgcca atcttaggtt tgctaggttt 720
   gttgacttct ccaattttga ccaaattcga catctacaag atttctatct tagtctttat 780
   tgctttctct gctaccactc catgggactc ctggatcatc agaaacggtg cctggaccta 840
   cccatctgct gaatctggtc aaggtgtttt cggtaccttt ttggatgtcc catacgaaga 900
   atacgccttc tttgttatcc aaaccgtcat caccggtttg gtttacgttt tggctaccag 960
   acatttgttg ccatctttgg ctctaccaaa gacccgttct tctgccttgt ctctagcttt 1020
   gaaggcttta atcccattgc caatcatcta tttgttcacc gctcatccat ctccatcccc    1080
   agatcctttg gttactgacc actacttcta catgagagct ttgtctttgt tgatcacccc    1140
   accaaccatg ttgttggctg ctttatctgg tgaatacgct ttcgactgga aatctggtag    1200
   agctaagtcc accattgctg ccatcatgat cccaactgtc tacttgatct gggttgacta    1260
   cgttgccgtt ggtcaagact cctggtccat caacgatgaa aagattgtcg gttggagatt    1320
   aggtggtgtc ttgccaattg aagaagctat gttcttctta ttgaccaact tgatgatcgt    1380
   tttgggtttg tctgcctgtg accacactca agccttgtac ttgttgcacg gtagaactat    1440
   ctacggtaac aagaagatgc catcttcttt cccattaatc actccaccag ttttgtcctt    1500
   gttcttctcc tccagaccat actcctccca accaaagaga gatttggaat tggctgtcaa    1560
   gttgttggaa aagaagtcca gatctttctt cgttgcttct gccggtttcc catctgaagt    1620
   cagagaaaga ttggttggtt tgtacgcttt ctgtcgtgtc accgatgact tgattgactc    1680
   tccagaagtt tcctccaacc cacacgctac cattgacatg gtttccgatt tcttgacttt    1740
   attattcggt cctccattgc acccatctca accagacaag attttgtctt ctccattatt    1800
   accaccttcc cacccatcca gaccaactgg tatgtaccca ttaccaccac ctccatcttt    1860
   gtctccagct gaattggtcc aattcttgac tgaacgtgtc ccagttcaat accacttcgc    1920
   tttcagattg ttggccaaat tgcaaggttt gattccaaga tacccattgg atgaattatt    1980
   gagaggttac accactgact tgatcttccc attgtccact gaagccgtcc aagctagaaa    2040
   gaccccaatt gaaactactg ctgacttgtt ggactacggt ttgtgtgttg ccggttctgt    2100
   tgctgaattg ttggtctacg tttcctgggc ttccgctcca tcccaagttc cagctactat    2160
   tgaagaaaga gaagctgttt tggtcgcctc tcgtgaaatg ggtaccgctt tgcaattggt    2220
   caacattgcc agagatatca agggtgacgc tactgaaggt agattctact tgccattgtc    2280
   tttctttggt ttgagagatg aatccaaatt ggccattcca actgactgga ctgaaccaag    2340
   acctcaagat ttcgacaaat tgttgtctct atctccatct tccactttac catcctctaa    2400
   cgcttctgaa tccttcagat tcgaatggaa gacctactct ttgccattgg ttgcttacgc    2460
   tgaagatttg gctaagcact cttacaaggg tattgacaga ttaccaactg aagtccaagc    2520
   tggtatgaga gctgcttgtg cttcttactt gttgattggt cgtgaaatca aggttgtctg    2580
   gaagggtgat gtcggtgaaa gaagaaccgt tgctggttgg agaagagtca gaaaggtttt    2640
   gtctgttgtc atgtccggtt gggaaggtca ataaagcgat ttaatctcta attattagtt    2700
   aaagttttat aagcattttt atgtaacgaa aaataaattg gttcatatta ttactgcact    2760
   gtcacttacc atggaaagac cagacaagaa gttgccgaca gtctgttgaa ttggcctggt    2820
   taggcttaag tctgggtccg cttctttaca aatttggaga atttctctta aacgatatgt    2880
   atattctttt cgttggaaaa gatgtcttcc aaaaaaaaaa ccgatgaatt agtggaacca     2940
   aggaaaaaaa aagaggtatc cttgattaag gaacagaaca atactgattg cgttacccca     3000
   agcttatccc acgtagtttt gtgtc                                           3025
<210> 53
   <211> 2748
   <212> DNA
   <213> artificial sequence
<220>
   <223> donor expression cassette sequence with 50 bp LF and RF flanks (PCR fragment). Homology to INT3 - Low p (ScPRE3p) - crtI - ScTAL1t - Homology to INT3
<400> 53
   gtccttgtat ggaagtatca aaggggacgt tcttcacctc cttggaacaa acattaattt      60
   gttctgcata ctttgaacct ttcagaaaat aaaaaacatt acgcgcatac ttaccctgct     120
   cgcgaagaag agtaacacta acgcattcta tgggcaattg aagacagtat tcagtacaag     180
   acatagtccg tttccttgag tcaattccta tagcattatg aactagccgc ctttaagagt     240
   gccaagctgt tcaacaccga tcatttttga tgatttggcg tttttgttat attgatagat     300
   ttcttttgaa ttttgtcatt ttcacttttc cactcgcaac ggaatccggt ggcaaaaaag     360
   ggaaaagcat tgaaatgcaa tctttaacag tattttaaac aagttgcgac acggtgtaca     420
   attacgataa gaattgctac ttcaaagtac acacagaaag ttaacatgaa tggaattcaa     480
   gtggacatca atcgtttgaa aaagggcgaa gtcagtttag gtacctcaat gtatgtatat     540
   aagaattttt cctcccactt tattgtttct aaaagttcaa tgaagtaaag tctcaattgg     600
   ccttattact aactaatagg tatcttataa tcacctaata aaatagaatg ggtaaggaac     660
   aagaccaaga caagccaact gccatcatcg ttggttgtgg tatcggtggt attgctaccg     720
   ctgccagatt agctaaggaa ggtttccaag ttaccgtctt tgaaaagaac gactactccg     780
   gtggtagatg ttctttgatt gaaagagatg gttacagatt cgaccaaggt ccatctttgt     840
   tgctattacc agacttgttc aagcaaacct tcgaagattt gggtgaaaag atggaagact     900
   gggttgattt gatcaagtgt gaaccaaact acgtttgtca cttccatgat gaagaaactt     960
   tcaccttctc cactgacatg gctttattga agagagaagt cgaaagattt gaaggtaaag    1020
   atggtttcga cagattcttg tctttcatcc aagaagctca cagacattac gaattggctg    1080
   ttgtccacgt cttgcaaaag aacttcccag gtttcgctgc tttcttgaga ttacaattca    1140
   tcggtcaaat cttagctttg cacccatttg aatccatctg gaccagagtt tgtcgttact    1200
   tcaagactga cagattgaga agagtcttct cctttgccgt tatgtacatg ggtcaatctc    1260
   catactctgc tccaggtacc tactccttgt tgcaatacac tgaattgact gaaggtatct    1320
   ggtacccaag aggtggtttc tggcaagttc caaacacttt gttgcaaatc gtcaagagaa    1380
   acaacccatc tgctaagttc aacttcaacg ctccagtttc tcaagttttg ttgtctccag 1440
   ctaaggacag agctaccggt gtcagattag aatctggtga agaacaccac gctgatgttg 1500
   tcattgtcaa tgctgacttg gtctacgctt ctgaacattt gattccagat gatgctagaa 1560
   acaagatcgg tcaattaggt gaagttaagc gttcctggtg ggctgatttg gttggtggta 1620
   agaagttgaa gggttcttgt tcttctttgt ctttctactg gtctatggac agaatcgttg 1680
   acggtttggg tggtcacaac atcttcttgg ctgaagactt caagggttcc ttcgacacca 1740
   ttttcgaaga attgggtttg ccagctgacc catctttcta tgttaacgtt ccatccagaa 1800
   ttgacccttc tgctgctcca gaaggtaagg atgccattgt catcttagtc ccatgtggtc 1860
   acatcgatgc ttccaaccct caagactaca acaaattggt tgccagagcc agaaagttcg 1920
   tcatccaaac cttgtctgcc aagttgggtc taccagattt cgaaaagatg attgttgctg 1980
   aaaaggttca cgatgctcca tcctgggaaa aggaattcaa cttgaaggac ggttccattt 2040
   tgggtttggc tcacaacttc atgcaagtct tgggtttcag accatccacc agacacccaa 2100
   agtacgacaa attgttcttt gtcggtgctt ctacccaccc aggtactggt gttccaattg 2160
   tcttggctgg tgccaaattg actgctaacc aagttttgga atccttcgat cgttctccag 2220
   ctccagatcc taacatgtct ttgtctgttc catacggtaa gccattgaaa tccaacggta 2280
   ctggtattga ctctcaagtc caattgaaat tcatggactt ggaacgttgg gtttacctat 2340
   tagtcttgtt gattggtgct gttatcgcca gatccgtcgg tgtcttggcc ttttaaagga 2400
   agtatctcgg aaatattaat ttaggccatg tccttatgca cgtttctttt gatacttacg 2460
   ggtacatgta cacaagtata tctatatata taaattaatg aaaatcccct atttatatat 2520
   atgactttaa cgagacagaa cagtttttta ttttttatcc tatttgatga atgatacagt 2580
   ttcttattca cgtgttatac ccacaccaaa tccaatagca ataccggcca tcacaatcac 2640
   tgtttcggca gcccctaaga tcagacaaaa catccggaac caccttaaat caacgtcgat 2700
   gggacgtcag cactgtactt gtttttgcga ctagattgta aatcattc 2748
<210> 54
   <211> 2749
   <212> DNA
   <213> artificial sequence
<220>
   <223> donor expression cassette sequence with 50 bp LF and RF flanks (PCR fragment). Homology to INT3 - Med p (ScACT1p) - crtI - ScTAL1t - Homology to INT3
<400> 54
   cgtccttgta tggaagtatc aaaggggacg ttcttcacct ccttggaaaa catatataca 60
   caattacagt aacaataaca agaggacaga tactaccaaa atgtgtgggg aagcgggtaa 120
   gctgccacag caattaatgc acaacattta acctacattc ttccttatcg gatcctcaaa 180
   acccttaaaa acatatgcct caccctaaca tattttccaa ttaaccctca atatttctct 240
   gtcacccggc ctctattttc cattttcttc tttacccgcc acgcgttttt ttctttcaaa 300
   tttttttctt ctttcttctt tttcttccac gtcctcttgc ataaataaat aaaccgtttt 360
   gaaaccaaac tcgcctctct ctctcctttt tgaaatattt ttgggtttgt ttgatccttt 420
   ccttcccaat ctctcttgtt taatatatat tcatttatat cacgctctct ttttatcttc 480
   ctttttttcc tctctcttgt attcttcctt cccctttcta ctcaaaccaa gaagaaaaag 540
   aaaaggtcaa tctttgttaa agaataggat cttctactac atcagctttt agatttttca 600
   cgcttactgc ttttttcttc ccaagatcga aaatttactg aattaacaat gggtaaggaa 660
   caagaccaag acaagccaac tgccatcatc gttggttgtg gtatcggtgg tattgctacc 720
   gctgccagat tagctaagga aggtttccaa gttaccgtct ttgaaaagaa cgactactcc 780
   ggtggtagat gttctttgat tgaaagagat ggttacagat tcgaccaagg tccatctttg 840
   ttgctattac cagacttgtt caagcaaacc ttcgaagatt tgggtgaaaa gatggaagac 900
   tgggttgatt tgatcaagtg tgaaccaaac tacgtttgtc acttccatga tgaagaaact 960
   ttcaccttct ccactgacat ggctttattg aagagagaag tcgaaagatt tgaaggtaaa 1020
   gatggtttcg acagattctt gtctttcatc caagaagctc acagacatta cgaattggct 1080
   gttgtccacg tcttgcaaaa gaacttccca ggtttcgctg ctttcttgag attacaattc 1140
   atcggtcaaa tcttagcttt gcacccattt gaatccatct ggaccagagt ttgtcgttac 1200
   ttcaagactg acagattgag aagagtcttc tcctttgccg ttatgtacat gggtcaatct 1260
   ccatactctg ctccaggtac ctactccttg ttgcaataca ctgaattgac tgaaggtatc 1320
   tggtacccaa gaggtggttt ctggcaagtt ccaaacactt tgttgcaaat cgtcaagaga 1380
   aacaacccat ctgctaagtt caacttcaac gctccagttt ctcaagtttt gttgtctcca 1440
   gctaaggaca gagctaccgg tgtcagatta gaatctggtg aagaacacca cgctgatgtt 1500
   gtcattgtca atgctgactt ggtctacgct tctgaacatt tgattccaga tgatgctaga 1560
   aacaagatcg gtcaattagg tgaagttaag cgttcctggt gggctgattt ggttggtggt 1620
   aagaagttga agggttcttg ttcttctttg tctttctact ggtctatgga cagaatcgtt 1680
   gacggtttgg gtggtcacaa catcttcttg gctgaagact tcaagggttc cttcgacacc 1740
   attttcgaag aattgggttt gccagctgac ccatctttct atgttaacgt tccatccaga 1800
   attgaccctt ctgctgctcc agaaggtaag gatgccattg tcatcttagt cccatgtggt 1860
   cacatcgatg cttccaaccc tcaagactac aacaaattgg ttgccagagc cagaaagttc 1920
   gtcatccaaa ccttgtctgc caagttgggt ctaccagatt tcgaaaagat gattgttgct 1980
   gaaaaggttc acgatgctcc atcctgggaa aaggaattca acttgaagga cggttccatt 2040
   ttgggtttgg ctcacaactt catgcaagtc ttgggtttca gaccatccac cagacaccca 2100
   aagtacgaca aattgttctt tgtcggtgct tctacccacc caggtactgg tgttccaatt 2160
   gtcttggctg gtgccaaatt gactgctaac caagttttgg aatccttcga tcgttctcca 2220
   gctccagatc ctaacatgtc tttgtctgtt ccatacggta agccattgaa atccaacggt 2280
   actggtattg actctcaagt ccaattgaaa ttcatggact tggaacgttg ggtttaccta 2340
   ttagtcttgt tgattggtgc tgttatcgcc agatccgtcg gtgtcttggc cttttaaagg 2400
   aagtatctcg gaaatattaa tttaggccat gtccttatgc acgtttcttt tgatacttac 2460
   gggtacatgt acacaagtat atctatatat ataaattaat gaaaatcccc tatttatata 2520
   tatgacttta acgagacaga acagtttttt attttttatc ctatttgatg aatgatacag 2580
   tttcttattc acgtgttata cccacaccaa atccaatagc aataccggcc atcacaatca 2640
   ctgtttcggc agcccctaag atcagacaaa acatccggaa ccaccttaaa tcaacgtcga 2700
   tgggacgtca gcactgtact tgtttttgcg actagattgt aaatcattc 2749
<210> 55
   <211> 3149
   <212> DNA
   <213> artificial sequence
<220>
   <223> donor expression cassette sequence with 50 bp LF and RF flanks (PCR fragment). Homology to INT3 - Strong p (KlENO1p) - crtI - ScTAL1t - Homology to INT3
<400> 55
   cgtccttgta tggaagtatc aaaggggacg ttcttcacct ccttggaacg tatccctatc 60
   tggattaaca tcactgccac agatcgaatt gcaagaagcc acacttcacg tgatccactc 120
   gttcatcagg tttgtagctt catggcgcag gacttctgat gatgaactat ctggctcatc 180
   cggatggatc acaaggatgg ataccctcag acagtacgtt tccgtatgga gcgatcttac 240
   aagaaccaac agttgatcct attacttttt ttttattttt tgtccctccg ggatggcaag 300
   agggacaaag aagaatcttc gttcttcttt cttgttctca acttcccagc ttccgtgtga 360
   ttaccctccg ggacaacaga aaaactggca ttcggtatcc cgggaatctg ctgagaagga 420
   aagaaaacga aaaaaaaatt gtacatttgt gtcacattat gaattacagg aagtcagaaa 480
   acaggcagca catgtctcgc acatgcatgt ccatcagacg agacattatg agacatgcac 540
   gcgtgtgaga gacatagcaa aagtctctcc agtacacaca gaaagacacg ttcacaatcc 600
   aggcacccca cagagaaaaa aaaaagaaga agcccggaag ctggcacgcc atcatcaacc 660
   accgctcggt ttacacgcat cccaactgtc ttttttttct ggaatcctat aataactggc 720
   atctggaaat cacgttgtat gttgcaccat agtgactggc tgtctgacta gcaaacattg 780
   attccctgat tcccatttgg ctcaattttg atgagaaaca gttgattgat tcttgtcaat      840
   ttttttttct ttggaccacc accaaccaat tgacattgaa gtactttccc atgatttgag      900
   gttatataaa aggacgttca aatcactttc aaggttaatt cagttttgtc aattgattta      960
   agttcaattg ttaacaaatt taatttaatt cgaaacaaac caaaccaatt catttgaatt     1020
   aacaaaccaa cccacaaaac aaaaaaaaat gggtaaggaa caagaccaag acaagccaac     1080
   tgccatcatc gttggttgtg gtatcggtgg tattgctacc gctgccagat tagctaagga     1140
   aggtttccaa gttaccgtct ttgaaaagaa cgactactcc ggtggtagat gttctttgat     1200
   tgaaagagat ggttacagat tcgaccaagg tccatctttg ttgctattac cagacttgtt     1260
   caagcaaacc ttcgaagatt tgggtgaaaa gatggaagac tgggttgatt tgatcaagtg     1320
   tgaaccaaac tacgtttgtc acttccatga tgaagaaact ttcaccttct ccactgacat     1380
   ggctttattg aagagagaag tcgaaagatt tgaaggtaaa gatggtttcg acagattctt     1440
   gtctttcatc caagaagctc acagacatta cgaattggct gttgtccacg tcttgcaaaa     1500
   gaacttccca ggtttcgctg ctttcttgag attacaattc atcggtcaaa tcttagcttt     1560
   gcacccattt gaatccatct ggaccagagt ttgtcgttac ttcaagactg acagattgag     1620
   aagagtcttc tcctttgccg ttatgtacat gggtcaatct ccatactctg ctccaggtac     1680
   ctactccttg ttgcaataca ctgaattgac tgaaggtatc tggtacccaa gaggtggttt     1740
   ctggcaagtt ccaaacactt tgttgcaaat cgtcaagaga aacaacccat ctgctaagtt     1800
   caacttcaac gctccagttt ctcaagtttt gttgtctcca gctaaggaca gagctaccgg     1860
   tgtcagatta gaatctggtg aagaacacca cgctgatgtt gtcattgtca atgctgactt     1920
   ggtctacgct tctgaacatt tgattccaga tgatgctaga aacaagatcg gtcaattagg     1980
   tgaagttaag cgttcctggt gggctgattt ggttggtggt aagaagttga agggttcttg     2040
   ttcttctttg tctttctact ggtctatgga cagaatcgtt gacggtttgg gtggtcacaa     2100
   catcttcttg gctgaagact tcaagggttc cttcgacacc attttcgaag aattgggttt     2160
   gccagctgac ccatctttct atgttaacgt tccatccaga attgaccctt ctgctgctcc     2220
   agaaggtaag gatgccattg tcatcttagt cccatgtggt cacatcgatg cttccaaccc     2280
   tcaagactac aacaaattgg ttgccagagc cagaaagttc gtcatccaaa ccttgtctgc     2340
   caagttgggt ctaccagatt tcgaaaagat gattgttgct gaaaaggttc acgatgctcc     2400
   atcctgggaa aaggaattca acttgaagga cggttccatt ttgggtttgg ctcacaactt     2460
   catgcaagtc ttgggtttca gaccatccac cagacaccca aagtacgaca aattgttctt     2520
   tgtcggtgct tctacccacc caggtactgg tgttccaatt gtcttggctg gtgccaaatt     2580
   gactgctaac caagttttgg aatccttcga tcgttctcca gctccagatc ctaacatgtc     2640
   tttgtctgtt ccatacggta agccattgaa atccaacggt actggtattg actctcaagt     2700
   ccaattgaaa ttcatggact tggaacgttg ggtttaccta ttagtcttgt tgattggtgc     2760
   tgttatcgcc agatccgtcg gtgtcttggc cttttaaagg aagtatctcg gaaatattaa     2820
   tttaggccat gtccttatgc acgtttcttt tgatacttac gggtacatgt acacaagtat     2880
   atctatatat ataaattaat gaaaatcccc tatttatata tatgacttta acgagacaga     2940
   acagtttttt attttttatc ctatttgatg aatgatacag tttcttattc acgtgttata     3000
   cccacaccaa atccaatagc aataccggcc atcacaatca ctgtttcggc agcccctaag     3060
   atcagacaaa acatccggaa ccaccttaaa tcaacgtcga tgggacgtca gcactgtact     3120
   tgtttttgcg actagattgt aaatcattc                                       3149
<210> 56
   <211> 71
   <212> DNA
   <213> artificial sequence
<220>
   <223> forward primer to obtain SEQ ID NO: 47
<400> 56
   catgcctatc cggcggcaac attattactc tggtatacgg gcgtaactcc acgtaaaaac       60
   taaaacgagc c                                                            71
<210> 57
   <211> 74
   <212> DNA
   <213> artificial sequence
<220>
   <223> reverse primer to obtain SEQ ID NO: 47, 48, 49
<400> 57
   attctaaaaa aagtaaaata aaataaaagg gtttcaaaga tccatacttc atcctggcgg       60
   aaaaaattca tttg                                                         74
<210> 58
   <211> 72
   <212> DNA
   <213> artificial sequence
<220>
   <223> forward primer to obtain SEQ ID NO: 48
<400> 58
   catgcctatc cggcggcaac attattactc tggtatacgg gcgtaactcc acctcatcac 60
   tagaagccga ac 72
<210> 59
   <211> 71
   <212> DNA
   <213> artificial sequence
<220>
   <223> forward primer to obtain SEQ ID NO: 49
<400> 59
   catgcctatc cggcggcaac attattactc tggtatacgg gcgtaactcc acttggcttc 60
   acatacgttg c 71
<210> 60
   <211> 84
   <212> DNA
   <213> artificial sequence
<220>
   <223> forward primer to obtain SEQ ID NO: 50
<400> 60
   caaaccgcag attgcgtcac ggttctgcgg acgccgttag cccattggtg tcaagttttc 60
   tttttttgcg gtcaccccca tgtg 84
<210> 61
   <211> 70
   <212> DNA
   <213> artificial sequence
<220>
   <223> reverse primer to obtain SEQ ID NO: 50, 51, 52
<400> 61
   gacacaaaac tacgtgggat aagcttgggg taacgcaatc agtattgttc tgttccttaa 60
   tcaaggatac 70
<210> 62
   <211> 73
   <212> DNA
   <213> artificial sequence
<220>
   <223> forward primer to obtain SEQ ID NO: 51
<400> 62
   cgcagattgc gtcacggttc tgcggacgcc gttagcccat tggtgtcaag cctgtccaag        60
   caaatgcctt ctc                                                           73
<210> 63
   <211> 80
   <212> DNA
   <213> artificial sequence
<220>
   <223> forward primer to obtain SEQ ID NO: 52
<400> 63
   caaaccgcag attgcgtcac ggttctgcgg acgccgttag cccattggtg tcaaggctga        60
   taatagcgta taaacaatgc                                                    80
<210> 64
   <211> 71
   <212> DNA
   <213> artificial sequence
<220>
   <223> forward primer to obtain SEQ ID NO: 53
<400> 64
   gtccttgtat ggaagtatca aaggggacgt tcttcacctc cttggaacaa acattaattt        60
   gttctgcata c                                                             71
<210> 65
   <211> 72
   <212> DNA
   <213> artificial sequence
<220>
   <223> reverse primer to obtain SEQ ID NO: 53, 54, 55
<400> 65
   gaatgattta caatctagtc gcaaaaacaa gtacagtgct gacgtcccat cgacgttgat        60
   ttaaggtggt tc                                                            72
<210> 66
   <211> 73
   <212> DNA
   <213> artificial sequence
<220>
   <223> forward primer to obtain SEQ ID NO: 54
<400> 66
   cgtccttgta tggaagtatc aaaggggacg ttcttcacct ccttggaaaa catatataca      60
   caattacagt aac                                                         73
<210> 67
   <211> 74
   <212> DNA
   <213> artificial sequence
<220>
   <223> forward primer to obtain SEQ ID NO: 55
<400> 67
   cgtccttgta tggaagtatc aaaggggacg ttcttcacct ccttggaacg tatccctatc      60
   tggattaaca tcac                                                        74
<210> 68
   <211> 584
   <212> DNA
   <213> artificial sequence
<220>
   <223> right flank sequence with overlap ScTDH3t - INT1 3' (part of donor) - INT1 3' genomic DNA. Homology with INT1 3' genomic DNA 524 bp
<400> 68
   aattttgggg atattggctt ttttttttaa agtttacaaa tgaatttttt ccgccaggat       60
   gaagtatgga tctttgaaac ccttttattt tattttactt tttttagaat gacctgttcc     120
   cgacactatg taagatctag cttttaacat attatggaaa cctgaaatgt aaaatctgaa     180
   tttttgtata tgtgtttata tttgggtagt tcttttgagg aaagcatgca tagacttgct     240
   gtacgaactt tatgtgactt gtagtgacgc tgtttcatga gactttagcc ctttgaacat     300
   attatcatat ctcagcttga aatactatag atttactttt gcagccattt cttggtgctc     360
   caaggttgtg cgtatctatt acttaatttc tgtccttgcc aagttttgca gcagggcggt     420
   cacaagactc ctctgccgtc attccttagt ccttcgggaa cacacttatt tatgtatttg     480
   tattctacaa ttctacggtg cacaagggtt gggcactgtt gagctcagca cgcaactatt     540
   gctggcatga agataagatt gatttttgga agaataagct tgtg                      584
<210> 69
   <211> 372
   <212> DNA
   <213> artificial sequence
<220>
   <223> left flank sequence with overlap KlYDR2p - INT2 5' (part of donor) - INT2 5' genomic DNA. Homology with INT2 5' genomic DNA 322 bp
<400> 69
   cattatatcg aggaaagccc tgcgaacgtg aagtgaaaag gacggttgtc ctttttcctg       60
   ttcttcgagg atcctgaaca ggatgtcagt agggcgggcc ttttcttttt attgtgcagg      120
   tacgcatgtt tctttgacac aaaatagtct ggattcgcca gccagtggca cttttgatgc      180
   catttccacc acagaaaacg tctgagtatt cagccatggg aggggagtgt tgaaagggga      240
   gaagcagccc acttctctgt cttgacacaa accgcagatt gcgtcacggt tctgcggacg      300
   ccgttagccc attggtgtca agttttcttt ttttgcggtc acccccatgt ggcggggagg      360
   cagaggagta gg                                                          372
<210> 70
   <211> 372
   <212> DNA
   <213> artificial sequence
<220>
   <223> left flank sequence with overlap KlTEF2p - INT2 5' (part of donor) - INT2 5' genomic DNA. Homology with INT2 5' genomic DNA 322 bp
<400> 70
   cattatatcg aggaaagccc tgcgaacgtg aagtgaaaag gacggttgtc ctttttcctg       60
   ttcttcgagg atcctgaaca ggatgtcagt agggcgggcc ttttcttttt attgtgcagg      120
   tacgcatgtt tctttgacac aaaatagtct ggattcgcca gccagtggca cttttgatgc      180
   catttccacc acagaaaacg tctgagtatt cagccatggg aggggagtgt tgaaagggga      240
   gaagcagccc acttctctgt cttgacacaa accgcagatt gcgtcacggt tctgcggacg      300
   ccgttagccc attggtgtca agcctgtcca agcaaatgcc ttctcataaa tggtgccaaa      360
   gacccgcaag cc                                                          372
<210> 71
   <211> 373
   <212> DNA
   <213> artificial sequence
<220>
   <223> left flank sequence with overlap ScTEFlp - INT2 5' (part of donor) - INT2 5' genomic DNA. Homology with INT2 5' genomic DNA 322 bp
<400> 71
   cattatatcg aggaaagccc tgcgaacgtg aagtgaaaag gacggttgtc ctttttcctg        60
   ttcttcgagg atcctgaaca ggatgtcagt agggcgggcc ttttcttttt attgtgcagg       120
   tacgcatgtt tctttgacac aaaatagtct ggattcgcca gccagtggca cttttgatgc       180
   catttccacc acagaaaacg tctgagtatt cagccatggg aggggagtgt tgaaagggga       240
   gaagcagccc acttctctgt cttgacacaa accgcagatt gcgtcacggt tctgcggacg       300
   ccgttagccc attggtgtca aggctgataa tagcgtataa acaatgcata ctttgtacgt       360
   tcaaaataca atg                                                          373
<210> 72
   <211> 608
   <212> DNA
   <213> artificial sequence
<220>
   <223> right flank sequence with overlap ScPDC1t - INT2 3' (part of donor) - INT2 3' genomic DNA. Homology with INT2 3' genomic DNA 524 bp
<400> 72
   gaattagtgg aaccaaggaa aaaaaaagag gtatccttga ttaaggaaca gaacaatact        60
   gattgcgtta ccccaagctt atcccacgta gttttgtgtc tctttcttgg gctcttggcg       120
   gccgtacgca tatagtatac taatgcggta acggctacct cctacgtctg tatcaaaaaa       180
   gttcgctctg gtcctatcaa tattttctcc cacacgagga ttctgtggtt ttgcagccac       240
   atctacagga tgttgacttt ttgtttgttt acgtagtttg tttcaacgct ttcgaagatg       300
   tggcgctggt gcaagtcatc taagttggtt actgataaca aattgtgaca aggatgtagt       360
   ggatgcgtag gagaccaaaa tgacatctga cacaaaaaaa agtagataac gaaaacagga       420
   caaaggtgtc attttgtttt gccaaggaga attataatct tagtgtgaag atactttcat       480
   tagacacata aatttacgtt cgacacactt ttttcttact cctcccctta tcaaggatac       540
   ctggttgatt ctacgtcgtc ctcttcactt tggttaattc acctttgccc ttcacctcgt       600
   ggtgtgcg                                                                608
<210> 73
   <211> 649
   <212> DNA
   <213> artificial sequence
<220>
   <223> left flank sequence with overlap ScPRE3p - INT3 5' (part of donor) - INT3 5' genomic DNA. Homology with INT3 5' genomic DNA 602 bp
<400> 73
   taaaggaggt gcacgcatta tggagaccac tacgatacga tagctgcgtt gttgttgaag        60
   gggtttctta aggttgtttt cgttgaaggt aaatattggt cgtttttgtg cagcatattg       120
   tcctctagat gcaaactctg caggtccatt tgcagtaaag tgagttgcct ctcgaagaat       180
   cattaatttc gtataaccgt cactattaaa gtcagaaaat aaattctgtc gtagacaatg       240
   ttaccataat gttcttgtcc attttgcata cactttaaat attcatttga tttctcaggg       300
   ttcatgatca taataaattg cgcattcgca aggcggtagt attataatgg ggtccatcat       360
   tctgtagcaa gaagttacag tacgctgttc aagcgttaaa caagataagt aatctcgaat       420
   gaaacattca tatttcgcat gagccaacat acagttgctg agtaatcttc attgcgctta       480
   tttatcggca ttgagattgt aaaggaagta aaacgcattt ttgcagatct gttctcttat       540
   gtatttttaa tcgtccttgt atggaagtat caaaggggac gttcttcacc tccttggaac       600
   aaacattaat ttgttctgca tactttgaac ctttcagaaa ataaaaaac                   649
<210> 74
   <211> 648
   <212> DNA
   <213> artificial sequence
<220>
   <223> left flank sequence with overlap ScACT1p - INT3 5' (part of donor) - INT3 5' genomic DNA. Homology with INT3 5' genomic DNA 602 bp
<400> 74
   taaaggaggt gcacgcatta tggagaccac tacgatacga tagctgcgtt gttgttgaag        60
   gggtttctta aggttgtttt cgttgaaggt aaatattggt cgtttttgtg cagcatattg       120
   tcctctagat gcaaactctg caggtccatt tgcagtaaag tgagttgcct ctcgaagaat       180
   cattaatttc gtataaccgt cactattaaa gtcagaaaat aaattctgtc gtagacaatg       240
   ttaccataat gttcttgtcc attttgcata cactttaaat attcatttga tttctcaggg       300
   ttcatgatca taataaattg cgcattcgca aggcggtagt attataatgg ggtccatcat       360
   tctgtagcaa gaagttacag tacgctgttc aagcgttaaa caagataagt aatctcgaat       420
   gaaacattca tatttcgcat gagccaacat acagttgctg agtaatcttc attgcgctta       480
   tttatcggca ttgagattgt aaaggaagta aaacgcattt ttgcagatct gttctcttat       540
   gtatttttaa tcgtccttgt atggaagtat caaaggggac gttcttcacc tccttggaaa       600
   acatatatac acaattacag taacaataac aagaggacag atactacc                    648
<210> 75
   <211> 649
   <212> DNA
   <213> artificial sequence
<220>
   <223> left flank sequence with overlap KlENOlp - INT3 5' (part of donor) - INT3 5' genomic DNA. Homology with INT3 5' genomic DNA 602 bp
<400> 75
   taaaggaggt gcacgcatta tggagaccac tacgatacga tagctgcgtt gttgttgaag      60
   gggtttctta aggttgtttt cgttgaaggt aaatattggt cgtttttgtg cagcatattg     120
   tcctctagat gcaaactctg caggtccatt tgcagtaaag tgagttgcct ctcgaagaat     180
   cattaatttc gtataaccgt cactattaaa gtcagaaaat aaattctgtc gtagacaatg     240
   ttaccataat gttcttgtcc attttgcata cactttaaat attcatttga tttctcaggg     300
   ttcatgatca taataaattg cgcattcgca aggcggtagt attataatgg ggtccatcat     360
   tctgtagcaa gaagttacag tacgctgttc aagcgttaaa caagataagt aatctcgaat     420
   gaaacattca tatttcgcat gagccaacat acagttgctg agtaatcttc attgcgctta     480
   tttatcggca ttgagattgt aaaggaagta aaacgcattt ttgcagatct gttctcttat     540
   gtatttttaa tcgtccttgt atggaagtat caaaggggac gttcttcacc tccttggaac     600
   gtatccctat ctggattaac atcactgcca cagatcgaat tgcaagaag                 649
<210> 76
   <211> 674
   <212> DNA
   <213> artificial sequence
<220>
   <223> right flank RF sequence with overlap ScTAL1t - INT3 3' (part of donor) - INT3 3' genomic DNA. Homology with INT3 3' genomic DNA 624 bp
<400> 76
   gcagccccta agatcagaca aaacatccgg aaccacctta aatcaacgtc gatgggacgt 60
   cagcactgta cttgtttttg cgactagatt gtaaatcatt ctttatttaa tctctttctt 120
   taactactgc ttaaagtata atttggtccg tagtttaata actatactaa gcgtaacaat 180
   gcatactgac attataagcc tgaacattac gagtttaagt tgtatgtagg cgttctgtaa 240
   gaggttactg cgtaaattat caacgaatgc attggtgtat ttgcgaaagc tacttctttt 300
   aacaagtatt tacataagaa taatggtgat ctgctcaact gatttggtga taactctaac 360
   ttttttagca acaatttaaa agataattcg aacatatata acagtaggaa gaatttgtgt 420
   acgtcaaatt aagataattt agcattacca aagttattaa cctaaacata aaatatatat 480
   gagacacatg tggaaatcgt atgaaacaac tgttatgaaa ctgacaagaa tgaatatata 540
   gagtaagctc cgcttgtaaa gaggaatcac ttaagtgtat aaatgtctcg acgattactt 600
   tagatccaag attgatgatt gatattactc tgtaatactt aagctctttt aatagctcac 660
   tgttgtatta cggg 674
<210> 77
   <211> 81
   <212> DNA
   <213> artificial sequence
<220>
   <223> forward primer primer to obtain SEQ ID NO: 68
<400> 77
   aattttgggg atattggctt ttttttttaa agtttacaaa tgaatttttt ccgccaggat 60
   gaagtatgga tctttgaaac c 81
<210> 78
   <211> 24
   <212> DNA
   <213> artificial sequence
<220>
   <223> reverse primer to obtain SEQ ID NO: 68
<400> 78
   cacaagctta ttcttccaaa aatc 24
<210> 79
   <211> 20
   <212> DNA
   <213> artificial sequence
<220>
   <223> forward primer to obtain SEQ ID NO: 69, 70 ,71
<400> 79
   cattatatcg aggaaagccc 20
<210> 80
   <211> 71
   <212> DNA
   <213> artificial sequence
<220>
   <223> reverse primer to obtain SEQ ID NO: 69
<400> 80
   cctactcctc tgcctccccg ccacatgggg gtgaccgcaa aaaaagaaaa cttgacacca 60
   atgggctaac g 71
<210> 81
   <211> 71
   <212> DNA
   <213> artificial sequence
<220>
   <223> reverse primer to obtain SEQ ID NO: 70
<400> 81
   ggcttgcggg tctttggcac catttatgag aaggcatttg cttggacagg cttgacacca 60
   atgggctaac g 71
<210> 82
   <211> 72
   <212> DNA
   <213> artificial sequence
<220>
   <223> reverse primer to obtain SEQ ID NO: 71
<400> 82
   cattgtattt tgaacgtaca aagtatgcat tgtttatacg ctattatcag ccttgacacc 60
   aatgggctaa cg 72
<210> 83
   <211> 72
   <212> DNA
   <213> artificial sequence
<220>
   <223> forward primer to obtain SEQ ID NO: 72
<400> 83
   gaattagtgg aaccaaggaa aaaaaaagag gtatccttga ttaaggaaca gaacaatact 60
   gattgcgtta cc 72
<210> 84
   <211> 20
   <212> DNA
   <213> artificial sequence
<220>
   <223> reverse primer to obtain SEQ ID NO: 72
<400> 84
   cgcacaccac gaggtgaagg 20
<210> 85
   <211> 23
   <212> DNA
   <213> artificial sequence
<220>
   <223> forward primer to obtain SEQ ID NO: 73, 74, 75
<400> 85
   taaaggaggt gcacgcatta tgg 23
<210> 86
   <211> 72
   <212> DNA
   <213> artificial sequence
<220>
   <223> reverse primer to obtain SEQ ID NO: 73
<400> 86
   gttttttatt ttctgaaagg ttcaaagtat gcagaacaaa ttaatgtttg ttccaaggag 60
   gtgaagaacg tc 72
<210> 87
   <211> 71
   <212> DNA
   <213> artificial sequence
<220>
   <223> reverse primer to obtain SEQ ID NO: 74
<400> 87
   ggtagtatct gtcctcttgt tattgttact gtaattgtgt atatatgttt tccaaggagg 60
   tgaagaacgt c 71
<210> 88
   <211> 72
   <212> DNA
   <213> artificial sequence
<220>
   <223> reverse primer to obtain SEQ ID NO: 75
<400> 88
   cttcttgcaa ttcgatctgt ggcagtgatg ttaatccaga tagggatacg ttccaaggag 60
   gtgaagaacg tc 72
<210> 89
   <211> 71
   <212> DNA
   <213> artificial sequence
<220>
   <223> forward primer to obtain SEQ ID NO: 76
<400> 89
   gcagccccta agatcagaca aaacatccgg aaccacctta aatcaacgtc gatgggacgt 60
   cagcactgta c 71
<210> 90
   <211> 20
   <212> DNA
   <213> artificial sequence
<220>
   <223> reverse primer to obtain SEQ ID NO: 76
<400> 90
   cccgtaatac aacagtgagc 20
<210> 91
   <211> 5712
   <212> DNA
   <213> artificial sequence
<220>
   <223> vector pRN1120
<400> 91
   tcgcgcgttt cggtgatgac ggtgaaaacc tctgacacat gcagctcccg gagacggtca      60
   cagcttgtct gtaagcggat gccgggagca gacaagcccg tcagggcgcg tcagcgggtg     120
   ttggcgggtg tcggggctgg cttaactatg cggcatcaga gcagattgta ctgagagtgc     180
   accatatcga ctacgtcgta aggccgtttc tgacagagta aaattcttga gggaactttc     240
   accattatgg gaaatggttc aagaaggtat tgacttaaac tccatcaaat ggtcaggtca     300
   ttgagtgttt tttatttgtt gtattttttt ttttttagag aaaatcctcc aatatcaaat     360
   taggaatcgt agtttcatga ttttctgtta cacctaactt tttgtgtggt gccctcctcc     420
   ttgtcaatat taatgttaaa gtgcaattct ttttccttat cacgttgagc cattagtatc     480
   aatttgctta cctgtattcc tttactatcc tcctttttct ccttcttgat aaatgtatgt     540
   agattgcgta tatagtttcg tctaccctat gaacatattc cattttgtaa tttcgtgtcg     600
   tttctattat gaatttcatt tataaagttt atgtacacct aggatccgtc gacactggat     660
   ggcggcgtta gtatcgaatc gacagcagta tagcgaccag cattcacata cgattgacgc     720
   atgatattac tttctgcgca cttaacttcg catctgggca gatgatgtcg aggcgaaaaa     780
   aaatataaat cacgctaaca tttgattaaa atagaacaac tacaatataa aaaaactata     840
   caaatgacaa gttcttgaaa acaagaatct ttttattgtc agtactaggg gcagggcatg     900
   ctcatgtaga gcgcctgctc gccgtccgag gcggtgccgt cgtacagggc ggtgtccagg 960
   ccgcagaggg tgaaccccat ccgccggtac gcgtggatcg ccggtgcgtt gacgttggtg 1020
   acctccagcc agaggtgccc ggcgccccgc tcgcgggcga actccgtcgc gagccccatc 1080
   aacgcgcgcc cgaccccgtg cccccggtgc tccggggcga cctcgatgtc ctcgacggtc 1140
   agccggcggt tccagccgga gtacgagacg accacgaagc ccgccaggtc gccgtcgtcc 1200
   ccgtacgcga cgaacgtccg ggagtccggg tcgccgtcct ccccggcgtc cgattcgtcg 1260
   tccgattcgt cgtcggggaa caccttggtc aggggcgggt ccaccggcac ctcccgcagg 1320
   gtgaagccgt ccccggtggc ggtgacgcgg aagacggtgt cggtggtgaa ggacccatcc 1380
   agtgcctcga tggcctcggc gtcccccggg acactggtgc ggtaccggta agccgtgtcg 1440
   tcaagagtgg tcattttaca tggttgttta tgttcggatg tgatgtgaga actgtatcct 1500
   agcaagattt taaaaggaag tatatgaaag aagaacctca gtggcaaatc ctaacctttt 1560
   atatttctct acaggggcgc ggcgtgggga caattcaacg cgtctgtgag gggagcgttt 1620
   ccctgctcgc aggtctgcag cgaggagccg taatttttgc ttcgcgccgt gcggccatca 1680
   aaatgtatgg atgcaaatga ttatacatgg ggatgtatgg gctaaatgta cgggcgacag 1740
   tcacatcatg cccctgagct gcgcacgtca agactgtcaa ggagggtatt ctgggcctcc 1800
   atgtcgctgg ccgggtgacc cggcggggac gaggccttaa gttcgaacgt acgagctccg 1860
   gcattgcgaa taccgctttc cacaaacatt gctcaaaagt atctctttgc tatatatctc 1920
   tgtgctatat ccctatataa cctacccatc cacctttcgc tccttgaact tgcatctaaa 1980
   ctcgacctct acatttttta tgtttatctc tagtattact ctttagacaa aaaaattgta 2040
   gtaagaacta ttcatagagt gaatcgaaaa caatacgaaa atgtaaacat ttcctatacg 2100
   tagtatatag agacaaaata gaagaaaccg ttcataattt tctgaccaat gaagaatcat 2160
   caacgctatc actttctgtt cacaaagtat gcgcaatcca catcggtata gaatataatc 2220
   ggggatgcct ttatcttgaa aaaatgcacc cgcagcttcg ctagtaatca gtaaacgcgg 2280
   gaagtggagt caggcttttt ttatggaaga gaaaatagac accaaagtag ccttcttcta 2340
   accttaacgg acctacagtg caaaaagtta tcaagagact gcattataga gcgcacaaag 2400
   gagaaaaaaa gtaatctaag atgctttgtt agaaaaatag cgctctcggg atgcattttt 2460
   gtagaacaaa aaagaagtat agattctttg ttggtaaaat agcgctctcg cgttgcattt 2520
   ctgttctgta aaaatgcagc tcagattctt tgtttgaaaa attagcgctc tcgcgttgca 2580
   tttttgtttt acaaaaatga agcacagatt cttcgttggt aaaatagcgc tttcgcgttg 2640
   catttctgtt ctgtaaaaat gcagctcaga ttctttgttt gaaaaattag cgctctcgcg 2700
   ttgcattttt gttctacaaa atgaagcaca gatgcttcgt taacaaagat atgctattga 2760
   agtgcaagat ggaaacgcag aaaatgaacc ggggatgcga cgtgcaagat tacctatgca 2820
   atagatgcaa tagtttctcc aggaaccgaa atacatacat tgtcttccgt aaagcgctag 2880
   actatatatt attatacagg ttcaaatata ctatctgttt cagggaaaac tcccaggttc 2940
   ggatgttcaa aattcaatga tgggtaacaa gtacgatcgt aaatctgtaa aacagtttgt 3000
   cggatattag gctgtatctc ctcaaagcgt attcgaatat cattgagaag ctgcagcgtc 3060
   acatcggata ataatgatgg cagccattgt agaagtgcct tttgcatttc tagtctcttt 3120
   ctcggtctag ctagttttac tacatcgcga agatagaatc ttagatcaca ctgcctttgc 3180
   tgagctggat caatagagta acaaaagagt ggtaaggcct cgttaaagga caaggacctg 3240
   agcggaagtg tatcgtacag tagacggagt atactaggta tagtctatag tccgtggaat 3300
   taattctcat gtttgacagc ttatcatcga taatccggag ctagcatgcg gccgctctag 3360
   aactagtgga tcccccgggc tgcaggaatt cgatatcaag cttatcgata ccgtcgacct 3420
   cgaggggggg cccggtaccc agcttttgtt ccctttagtg agggttaatt ccgagcttgg 3480
   cgtaatcatg gtcatagctg tttcctgtgt gaaattgtta tccgctcaca attccacaca 3540
   acataggagc cggaagcata aagtgtaaag cctggggtgc ctaatgagtg aggtaactca 3600
   cattaattgc gttgcgctca ctgcccgctt tccagtcggg aaacctgtcg tgccagctgc 3660
   attaatgaat cggccaacgc gcggggagag gcggtttgcg tattgggcgc tcttccgctt 3720
   cctcgctcac tgactcgctg cgctcggtcg ttcggctgcg gcgagcggta tcagctcact 3780
   caaaggcggt aatacggtta tccacagaat caggggataa cgcaggaaag aacatgtgag 3840
   caaaaggcca gcaaaaggcc aggaaccgta aaaaggccgc gttgctggcg tttttccata 3900
   ggctcggccc ccctgacgag catcacaaaa atcgacgctc aagtcagagg tggcgaaacc 3960
   cgacaggact ataaagatac caggcgttcc cccctggaag ctccctcgtg cgctctcctg 4020
   ttccgaccct gccgcttacc ggatacctgt ccgcctttct cccttcggga agcgtggcgc 4080
   tttctcaatg ctcacgctgt aggtatctca gttcggtgta ggtcgttcgc tccaagctgg 4140
   gctgtgtgca cgaacccccc gttcagcccg accgctgcgc cttatccggt aactatcgtc 4200
   ttgagtccaa cccggtaaga cacgacttat cgccactggc agcagccact ggtaacagga 4260
   ttagcagagc gaggtatgta ggcggtgcta cagagttctt gaagtggtgg cctaactacg 4320
   gctacactag aaggacagta tttggtatct gcgctctgct gaagccagtt accttcggaa 4380
   aaagagttgg tagctcttga tccggcaaac aaaccaccgc tggtagcggt ggtttttttg 4440
   tttgcaagca gcagattacg cgcagaaaaa aaggatctca agaagatcct ttgatctttt 4500
   ctacggggtc tgacgctcag tggaacgaaa actcacgtta agggattttg gtcatgagat 4560
   tatcaaaaag gatcttcacc tagatccttt taaattaaaa atgaagtttt aaatcaatct 4620
   aaagtatata tgagtaaact tggtctgaca gttaccaatg cttaatcagt gaggcaccta 4680
   tctcagcgat ctgtctattt cgttcatcca tagttgcctg actgcccgtc gtgtagataa 4740
   ctacgatacg ggagggctta ccatctggcc ccagtgctgc aatgataccg cgagacccac 4800
   gctcaccggc tccagattta tcagcaataa accagccagc cggaagggcc gagcgcagaa 4860
   gtggtcctgc aactttatcc gcctccatcc agtctattaa ttgttgccgg gaagctagag 4920
   taagtagttc gccagttaat agtttgcgca acgttgttgc cattgctaca ggcatcgtgg 4980
   tgtcacgctc gtcgtttggt atggcttcat tcagctccgg ttcccaacga tcaaggcgag 5040
   ttacatgatc ccccatgttg tgaaaaaaag cggttagctc cttcggtcct ccgatcgttg 5100
   tcagaagtaa gttggccgca gtgttatcac tcatggttat ggcagcactg cataattctc 5160
   ttactgtcat gccatccgta agatgctttt ctgtgactgg tgagtactca accaagtcat 5220
   tctgagaata gtgtatgcgg cgaccgagtt gctcttgccc ggcgtcaata cgggataata 5280
   ccgcgccaca tagcagaact ttaaaagtgc tcatcattgg aaaacgttct tcggggcgaa 5340
   aactctcaag gatcttaccg ctgttgagat ccagttcgat gtaacccact cgtgcaccca 5400
   actgatcttc agcatctttt actttcacca gcgtttctgg gtgagcaaaa acaggaaggc 5460
   aaaatgccgc aaaaaaggga ataagggcga cacggaaatg ttgaatactc atactcttcc 5520
   tttttcaata ttattgaagc atttatcagg gttattgtct catgagcgga tacatatttg 5580
   aatgtattta gaaaaataaa caaatagggg ttccgcgcac atttccccga aaagtgccac 5640
   ctgacgtcta agaaaccatt attatcatga cattaaccta taaaaatagg cgtatcacga 5700
   ggccctttcg tc 5712
<210> 92
   <211> 21
   <212> DNA
   <213> artificial sequence
<220>
   <223> forward primer to remove SapI restriction site in pRN1120
<400> 92
   tattgggccc tcttccgctt c 21
<210> 93
   <211> 18
   <212> DNA
   <213> artificial sequence
<220>
   <223> reverse primer to remove SapI restriction site in pRN1120
<400> 93
   cgcaaaccgc ctctcccc 18
<210> 94
   <211> 547
   <212> DNA
   <213> artificial sequence
<220>
   <223> gBlock allowing direct SapI cloning of the guide sequence, part of vector pGRN002
<400> 94
   ggagctagca tgcggccgct ctagaactag tggatccccc gggctgcagg aattctcttt        60
   gaaaagataa tgtatgatta tgctttcact catatttata cagaaacttg atgttttctt       120
   tcgagtatat acaaggtgat tacatgtacg tttgaagtac aactctagat tttgtagtgc       180
   cctcttgggc tagcggtaaa ggtgcgcatt ttttcacacc ctacaatgtt ctgttcaaaa       240
   gattttggtc aaacgctgta gaagtgaaag ttggtgcgca tgtttcggcg ttcgaaactt       300
   ctccgcagtg aaagataaat gatcagaaga gcctgaggtc gacggtatcg ataagcttga       360
   tatcaattcc ccgggggatc cactgctctt ctgttttaga gctagaaata gcaagttaaa       420
   ataaggctag tccgttatca acttgaaaaa gtggcaccga gtcggtggtg ctttttttgt      480
   tttttatgtc tctcgagggg gggcccggta cccagctttt gttcccttta gtgagggtta       540
   attccga                                                                547
<210> 95
   <211> 6121
   <212> DNA
   <213> artificial sequence
<220>
   <223> expression vector pGRN002
<400> 95
   tcgcgcgttt cggtgatgac ggtgaaaacc tctgacacat gcagctcccg gagacggtca        60
   cagcttgtct gtaagcggat gccgggagca gacaagcccg tcagggcgcg tcagcgggtg       120
   ttggcgggtg tcggggctgg cttaactatg cggcatcaga gcagattgta ctgagagtgc       180
   accatatcga ctacgtcgta aggccgtttc tgacagagta aaattcttga gggaactttc       240
   accattatgg gaaatggttc aagaaggtat tgacttaaac tccatcaaat ggtcaggtca       300
   ttgagtgttt tttatttgtt gtattttttt ttttttagag aaaatcctcc aatatcaaat        360
   taggaatcgt agtttcatga ttttctgtta cacctaactt tttgtgtggt gccctcctcc       420
   ttgtcaatat taatgttaaa gtgcaattct ttttccttat cacgttgagc cattagtatc       480
   aatttgctta cctgtattcc tttactatcc tcctttttct ccttcttgat aaatgtatgt       540
   agattgcgta tatagtttcg tctaccctat gaacatattc cattttgtaa tttcgtgtcg       600
   tttctattat gaatttcatt tataaagttt atgtacacct aggatccgtc gacactggat       660
   ggcggcgtta gtatcgaatc gacagcagta tagcgaccag cattcacata cgattgacgc       720
   atgatattac tttctgcgca cttaacttcg catctgggca gatgatgtcg aggcgaaaaa       780
   aaatataaat cacgctaaca tttgattaaa atagaacaac tacaatataa aaaaactata 840
   caaatgacaa gttcttgaaa acaagaatct ttttattgtc agtactaggg gcagggcatg 900
   ctcatgtaga gcgcctgctc gccgtccgag gcggtgccgt cgtacagggc ggtgtccagg 960
   ccgcagaggg tgaaccccat ccgccggtac gcgtggatcg ccggtgcgtt gacgttggtg 1020
   acctccagcc agaggtgccc ggcgccccgc tcgcgggcga actccgtcgc gagccccatc 1080
   aacgcgcgcc cgaccccgtg cccccggtgc tccggggcga cctcgatgtc ctcgacggtc 1140
   agccggcggt tccagccgga gtacgagacg accacgaagc ccgccaggtc gccgtcgtcc 1200
   ccgtacgcga cgaacgtccg ggagtccggg tcgccgtcct ccccggcgtc cgattcgtcg 1260
   tccgattcgt cgtcggggaa caccttggtc aggggcgggt ccaccggcac ctcccgcagg 1320
   gtgaagccgt ccccggtggc ggtgacgcgg aagacggtgt cggtggtgaa ggacccatcc 1380
   agtgcctcga tggcctcggc gtcccccggg acactggtgc ggtaccggta agccgtgtcg 1440
   tcaagagtgg tcattttaca tggttgttta tgttcggatg tgatgtgaga actgtatcct 1500
   agcaagattt taaaaggaag tatatgaaag aagaacctca gtggcaaatc ctaacctttt 1560
   atatttctct acaggggcgc ggcgtgggga caattcaacg cgtctgtgag gggagcgttt 1620
   ccctgctcgc aggtctgcag cgaggagccg taatttttgc ttcgcgccgt gcggccatca 1680
   aaatgtatgg atgcaaatga ttatacatgg ggatgtatgg gctaaatgta cgggcgacag 1740
   tcacatcatg cccctgagct gcgcacgtca agactgtcaa ggagggtatt ctgggcctcc 1800
   atgtcgctgg ccgggtgacc cggcggggac gaggccttaa gttcgaacgt acgagctccg 1860
   gcattgcgaa taccgctttc cacaaacatt gctcaaaagt atctctttgc tatatatctc 1920
   tgtgctatat ccctatataa cctacccatc cacctttcgc tccttgaact tgcatctaaa 1980
   ctcgacctct acatttttta tgtttatctc tagtattact ctttagacaa aaaaattgta 2040
   gtaagaacta ttcatagagt gaatcgaaaa caatacgaaa atgtaaacat ttcctatacg 2100
   tagtatatag agacaaaata gaagaaaccg ttcataattt tctgaccaat gaagaatcat 2160
   caacgctatc actttctgtt cacaaagtat gcgcaatcca catcggtata gaatataatc 2220
   ggggatgcct ttatcttgaa aaaatgcacc cgcagcttcg ctagtaatca gtaaacgcgg 2280
   gaagtggagt caggcttttt ttatggaaga gaaaatagac accaaagtag ccttcttcta 2340
   accttaacgg acctacagtg caaaaagtta tcaagagact gcattataga gcgcacaaag 2400
   gagaaaaaaa gtaatctaag atgctttgtt agaaaaatag cgctctcggg atgcattttt 2460
   gtagaacaaa aaagaagtat agattctttg ttggtaaaat agcgctctcg cgttgcattt 2520
   ctgttctgta aaaatgcagc tcagattctt tgtttgaaaa attagcgctc tcgcgttgca 2580
   tttttgtttt acaaaaatga agcacagatt cttcgttggt aaaatagcgc tttcgcgttg 2640
   catttctgtt ctgtaaaaat gcagctcaga ttctttgttt gaaaaattag cgctctcgcg 2700
   ttgcattttt gttctacaaa atgaagcaca gatgcttcgt taacaaagat atgctattga 2760
   agtgcaagat ggaaacgcag aaaatgaacc ggggatgcga cgtgcaagat tacctatgca 2820
   atagatgcaa tagtttctcc aggaaccgaa atacatacat tgtcttccgt aaagcgctag 2880
   actatatatt attatacagg ttcaaatata ctatctgttt cagggaaaac tcccaggttc 2940
   ggatgttcaa aattcaatga tgggtaacaa gtacgatcgt aaatctgtaa aacagtttgt 3000
   cggatattag gctgtatctc ctcaaagcgt attcgaatat cattgagaag ctgcagcgtc 3060
   acatcggata ataatgatgg cagccattgt agaagtgcct tttgcatttc tagtctcttt 3120
   ctcggtctag ctagttttac tacatcgcga agatagaatc ttagatcaca ctgcctttgc 3180
   tgagctggat caatagagta acaaaagagt ggtaaggcct cgttaaagga caaggacctg 3240
   agcggaagtg tatcgtacag tagacggagt atactaggta tagtctatag tccgtggaat 3300
   taattctcat gtttgacagc ttatcatcga taatccggag ctagcatgcg gccgctctag 3360
   aactagtgga tcccccgggc tgcaggaatt ctctttgaaa agataatgta tgattatgct 3420
   ttcactcata tttatacaga aacttgatgt tttctttcga gtatatacaa ggtgattaca 3480
   tgtacgtttg aagtacaact ctagattttg tagtgccctc ttgggctagc ggtaaaggtg 3540
   cgcatttttt cacaccctac aatgttctgt tcaaaagatt ttggtcaaac gctgtagaag 3600
   tgaaagttgg tgcgcatgtt tcggcgttcg aaacttctcc gcagtgaaag ataaatgatc 3660
   agaagagcct gaggtcgacg gtatcgataa gcttgatatc aattccccgg gggatccact 3720
   gctcttctgt tttagagcta gaaatagcaa gttaaaataa ggctagtccg ttatcaactt 3780
   gaaaaagtgg caccgagtcg gtggtgcttt ttttgttttt tatgtctctc gagggggggc 3840
   ccggtaccca gcttttgttc cctttagtga gggttaattc cgagcttggc gtaatcatgg 3900
   tcatagctgt ttcctgtgtg aaattgttat ccgctcacaa ttccacacaa cataggagcc 3960
   ggaagcataa agtgtaaagc ctggggtgcc taatgagtga ggtaactcac attaattgcg 4020
   ttgcgctcac tgcccgcttt ccagtcggga aacctgtcgt gccagctgca ttaatgaatc 4080
   ggccaacgcg cggggagagg cggtttgcgt attgggccct cttccgcttc ctcgctcact 4140
   gactcgctgc gctcggtcgt tcggctgcgg cgagcggtat cagctcactc aaaggcggta 4200
   atacggttat ccacagaatc aggggataac gcaggaaaga acatgtgagc aaaaggccag 4260
   caaaaggcca ggaaccgtaa aaaggccgcg ttgctggcgt ttttccatag gctcggcccc 4320
   cctgacgagc atcacaaaaa tcgacgctca agtcagaggt ggcgaaaccc gacaggacta 4380
   taaagatacc aggcgttccc ccctggaagc tccctcgtgc gctctcctgt tccgaccctg 4440
   ccgcttaccg gatacctgtc cgcctttctc ccttcgggaa gcgtggcgct ttctcaatgc 4500
   tcacgctgta ggtatctcag ttcggtgtag gtcgttcgct ccaagctggg ctgtgtgcac 4560
   gaaccccccg ttcagcccga ccgctgcgcc ttatccggta actatcgtct tgagtccaac 4620
   ccggtaagac acgacttatc gccactggca gcagccactg gtaacaggat tagcagagcg 4680
   aggtatgtag gcggtgctac agagttcttg aagtggtggc ctaactacgg ctacactaga 4740
   aggacagtat ttggtatctg cgctctgctg aagccagtta ccttcggaaa aagagttggt 4800
   agctcttgat ccggcaaaca aaccaccgct ggtagcggtg gtttttttgt ttgcaagcag 4860
   cagattacgc gcagaaaaaa aggatctcaa gaagatcctt tgatcttttc tacggggtct 4920
   gacgctcagt ggaacgaaaa ctcacgttaa gggattttgg tcatgagatt atcaaaaagg 4980
   atcttcacct agatcctttt aaattaaaaa tgaagtttta aatcaatcta aagtatatat 5040
   gagtaaactt ggtctgacag ttaccaatgc ttaatcagtg aggcacctat ctcagcgatc 5100
   tgtctatttc gttcatccat agttgcctga ctgcccgtcg tgtagataac tacgatacgg 5160
   gagggcttac catctggccc cagtgctgca atgataccgc gagacccacg ctcaccggct 5220
   ccagatttat cagcaataaa ccagccagcc ggaagggccg agcgcagaag tggtcctgca 5280
   actttatccg cctccatcca gtctattaat tgttgccggg aagctagagt aagtagttcg 5340
   ccagttaata gtttgcgcaa cgttgttgcc attgctacag gcatcgtggt gtcacgctcg 5400
   tcgtttggta tggcttcatt cagctccggt tcccaacgat caaggcgagt tacatgatcc 5460
   cccatgttgt gaaaaaaagc ggttagctcc ttcggtcctc cgatcgttgt cagaagtaag 5520
   ttggccgcag tgttatcact catggttatg gcagcactgc ataattctct tactgtcatg 5580
   ccatccgtaa gatgcttttc tgtgactggt gagtactcaa ccaagtcatt ctgagaatag 5640
   tgtatgcggc gaccgagttg ctcttgcccg gcgtcaatac gggataatac cgcgccacat 5700
   agcagaactt taaaagtgct catcattgga aaacgttctt cggggcgaaa actctcaagg 5760
   atcttaccgc tgttgagatc cagttcgatg taacccactc gtgcacccaa ctgatcttca 5820
   gcatctttta ctttcaccag cgtttctggg tgagcaaaaa caggaaggca aaatgccgca 5880
   aaaaagggaa taagggcgac acggaaatgt tgaatactca tactcttcct ttttcaatat 5940
   tattgaagca tttatcaggg ttattgtctc atgagcggat acatatttga atgtatttag 6000
   aaaaataaac aaataggggt tccgcgcaca tttccccgaa aagtgccacc tgacgtctaa 6060
   gaaaccatta ttatcatgac attaacctat aaaaataggc gtatcacgag gccctttcgt 6120
   c 6121
<210> 96
   <211> 53
   <212> DNA
   <213> artificial sequence
<220>
   <223> forward primer approach 1: 30 bp on either side
<400> 96
   ataaatgatc tattagaacc agggaggtcc gttttagagc tagaaatagc aag 53
<210> 97
   <211> 52
   <212> DNA
   <213> artificial sequence
<220>
   <223> reverse primer approach 1: 30 bp on either side
<400> 97
   gctctaaaac ggacctccct ggttctaata gatcatttat ctttcactgc gg 52
<210> 98
   <211> 6113
   <212> DNA
   <213> artificial sequence
<220>
   <223> PCR fragment approach 1: 30 bp on eiter side. The fragment was transformed to yeast allowing reconstitution of a circular vector by in vivo recombination
<400> 98
   ataaatgatc tattagaacc agggaggtcc gttttagagc tagaaatagc aagttaaaat      60
   aaggctagtc cgttatcaac ttgaaaaagt ggcaccgagt cggtggtgct ttttttgttt     120
   tttatgtctc tcgagggggg gcccggtacc cagcttttgt tccctttagt gagggttaat     180
   tccgagcttg gcgtaatcat ggtcatagct gtttcctgtg tgaaattgtt atccgctcac     240
   aattccacac aacataggag ccggaagcat aaagtgtaaa gcctggggtg cctaatgagt     300
   gaggtaactc acattaattg cgttgcgctc actgcccgct ttccagtcgg gaaacctgtc     360
   gtgccagctg cattaatgaa tcggccaacg cgcggggaga ggcggtttgc gtattgggcc     420
   ctcttccgct tcctcgctca ctgactcgct gcgctcggtc gttcggctgc ggcgagcggt     480
   atcagctcac tcaaaggcgg taatacggtt atccacagaa tcaggggata acgcaggaaa     540
   gaacatgtga gcaaaaggcc agcaaaaggc caggaaccgt aaaaaggccg cgttgctggc     600
   gtttttccat aggctcggcc cccctgacga gcatcacaaa aatcgacgct caagtcagag     660
   gtggcgaaac ccgacaggac tataaagata ccaggcgttc ccccctggaa gctccctcgt      720
   gcgctctcct gttccgaccc tgccgcttac cggatacctg tccgcctttc tcccttcggg     780
   aagcgtggcg ctttctcaat gctcacgctg taggtatctc agttcggtgt aggtcgttcg     840
   ctccaagctg ggctgtgtgc acgaaccccc cgttcagccc gaccgctgcg ccttatccgg     900
   taactatcgt cttgagtcca acccggtaag acacgactta tcgccactgg cagcagccac     960
   tggtaacagg attagcagag cgaggtatgt aggcggtgct acagagttct tgaagtggtg    1020
   gcctaactac ggctacacta gaaggacagt atttggtatc tgcgctctgc tgaagccagt    1080
   taccttcgga aaaagagttg gtagctcttg atccggcaaa caaaccaccg ctggtagcgg 1140
   tggttttttt gtttgcaagc agcagattac gcgcagaaaa aaaggatctc aagaagatcc 1200
   tttgatcttt tctacggggt ctgacgctca gtggaacgaa aactcacgtt aagggatttt 1260
   ggtcatgaga ttatcaaaaa ggatcttcac ctagatcctt ttaaattaaa aatgaagttt 1320
   taaatcaatc taaagtatat atgagtaaac ttggtctgac agttaccaat gcttaatcag 1380
   tgaggcacct atctcagcga tctgtctatt tcgttcatcc atagttgcct gactgcccgt 1440
   cgtgtagata actacgatac gggagggctt accatctggc cccagtgctg caatgatacc 1500
   gcgagaccca cgctcaccgg ctccagattt atcagcaata aaccagccag ccggaagggc 1560
   cgagcgcaga agtggtcctg caactttatc cgcctccatc cagtctatta attgttgccg 1620
   ggaagctaga gtaagtagtt cgccagttaa tagtttgcgc aacgttgttg ccattgctac 1680
   aggcatcgtg gtgtcacgct cgtcgtttgg tatggcttca ttcagctccg gttcccaacg 1740
   atcaaggcga gttacatgat cccccatgtt gtgaaaaaaa gcggttagct ccttcggtcc 1800
   tccgatcgtt gtcagaagta agttggccgc agtgttatca ctcatggtta tggcagcact 1860
   gcataattct cttactgtca tgccatccgt aagatgcttt tctgtgactg gtgagtactc 1920
   aaccaagtca ttctgagaat agtgtatgcg gcgaccgagt tgctcttgcc cggcgtcaat 1980
   acgggataat accgcgccac atagcagaac tttaaaagtg ctcatcattg gaaaacgttc 2040
   ttcggggcga aaactctcaa ggatcttacc gctgttgaga tccagttcga tgtaacccac 2100
   tcgtgcaccc aactgatctt cagcatcttt tactttcacc agcgtttctg ggtgagcaaa 2160
   aacaggaagg caaaatgccg caaaaaaggg aataagggcg acacggaaat gttgaatact 2220
   catactcttc ctttttcaat attattgaag catttatcag ggttattgtc tcatgagcgg 2280
   atacatattt gaatgtattt agaaaaataa acaaataggg gttccgcgca catttccccg 2340
   aaaagtgcca cctgacgtct aagaaaccat tattatcatg acattaacct ataaaaatag 2400
   gcgtatcacg aggccctttc gtctcgcgcg tttcggtgat gacggtgaaa acctctgaca 2460
   catgcagctc ccggagacgg tcacagcttg tctgtaagcg gatgccggga gcagacaagc 2520
   ccgtcagggc gcgtcagcgg gtgttggcgg gtgtcggggc tggcttaact atgcggcatc 2580
   agagcagatt gtactgagag tgcaccatat cgactacgtc gtaaggccgt ttctgacaga 2640
   gtaaaattct tgagggaact ttcaccatta tgggaaatgg ttcaagaagg tattgactta 2700
   aactccatca aatggtcagg tcattgagtg ttttttattt gttgtatttt ttttttttta 2760
   gagaaaatcc tccaatatca aattaggaat cgtagtttca tgattttctg ttacacctaa 2820
   ctttttgtgt ggtgccctcc tccttgtcaa tattaatgtt aaagtgcaat tctttttcct 2880
   tatcacgttg agccattagt atcaatttgc ttacctgtat tcctttacta tcctcctttt 2940
   tctccttctt gataaatgta tgtagattgc gtatatagtt tcgtctaccc tatgaacata     3000
   ttccattttg taatttcgtg tcgtttctat tatgaatttc atttataaag tttatgtaca     3060
   cctaggatcc gtcgacactg gatggcggcg ttagtatcga atcgacagca gtatagcgac     3120
   cagcattcac atacgattga cgcatgatat tactttctgc gcacttaact tcgcatctgg     3180
   gcagatgatg tcgaggcgaa aaaaaatata aatcacgcta acatttgatt aaaatagaac     3240
   aactacaata taaaaaaact atacaaatga caagttcttg aaaacaagaa tctttttatt     3300
   gtcagtacta ggggcagggc atgctcatgt agagcgcctg ctcgccgtcc gaggcggtgc     3360
   cgtcgtacag ggcggtgtcc aggccgcaga gggtgaaccc catccgccgg tacgcgtgga     3420
   tcgccggtgc gttgacgttg gtgacctcca gccagaggtg cccggcgccc cgctcgcggg     3480
   cgaactccgt cgcgagcccc atcaacgcgc gcccgacccc gtgcccccgg tgctccgggg     3540
   cgacctcgat gtcctcgacg gtcagccggc ggttccagcc ggagtacgag acgaccacga     3600
   agcccgccag gtcgccgtcg tccccgtacg cgacgaacgt ccgggagtcc gggtcgccgt     3660
   cctccccggc gtccgattcg tcgtccgatt cgtcgtcggg gaacaccttg gtcaggggcg     3720
   ggtccaccgg cacctcccgc agggtgaagc cgtccccggt ggcggtgacg cggaagacgg     3780
   tgtcggtggt gaaggaccca tccagtgcct cgatggcctc ggcgtccccc gggacactgg     3840
   tgcggtaccg gtaagccgtg tcgtcaagag tggtcatttt acatggttgt ttatgttcgg     3900
   atgtgatgtg agaactgtat cctagcaaga ttttaaaagg aagtatatga aagaagaacc     3960
   tcagtggcaa atcctaacct tttatatttc tctacagggg cgcggcgtgg ggacaattca     4020
   acgcgtctgt gaggggagcg tttccctgct cgcaggtctg cagcgaggag ccgtaatttt     4080
   tgcttcgcgc cgtgcggcca tcaaaatgta tggatgcaaa tgattataca tggggatgta     4140
   tgggctaaat gtacgggcga cagtcacatc atgcccctga gctgcgcacg tcaagactgt     4200
   caaggagggt attctgggcc tccatgtcgc tggccgggtg acccggcggg gacgaggcct     4260
   taagttcgaa cgtacgagct ccggcattgc gaataccgct ttccacaaac attgctcaaa     4320
   agtatctctt tgctatatat ctctgtgcta tatccctata taacctaccc atccaccttt     4380
   cgctccttga acttgcatct aaactcgacc tctacatttt ttatgtttat ctctagtatt     4440
   actctttaga caaaaaaatt gtagtaagaa ctattcatag agtgaatcga aaacaatacg     4500
   aaaatgtaaa catttcctat acgtagtata tagagacaaa atagaagaaa ccgttcataa     4560
   ttttctgacc aatgaagaat catcaacgct atcactttct gttcacaaag tatgcgcaat     4620
   ccacatcggt atagaatata atcggggatg cctttatctt gaaaaaatgc acccgcagct     4680
   tcgctagtaa tcagtaaacg cgggaagtgg agtcaggctt tttttatgga agagaaaata     4740
   gacaccaaag tagccttctt ctaaccttaa cggacctaca gtgcaaaaag ttatcaagag     4800
   actgcattat agagcgcaca aaggagaaaa aaagtaatct aagatgcttt gttagaaaaa     4860
   tagcgctctc gggatgcatt tttgtagaac aaaaaagaag tatagattct ttgttggtaa     4920
   aatagcgctc tcgcgttgca tttctgttct gtaaaaatgc agctcagatt ctttgtttga     4980
   aaaattagcg ctctcgcgtt gcatttttgt tttacaaaaa tgaagcacag attcttcgtt     5040
   ggtaaaatag cgctttcgcg ttgcatttct gttctgtaaa aatgcagctc agattctttg     5100
   tttgaaaaat tagcgctctc gcgttgcatt tttgttctac aaaatgaagc acagatgctt     5160
   cgttaacaaa gatatgctat tgaagtgcaa gatggaaacg cagaaaatga accggggatg     5220
   cgacgtgcaa gattacctat gcaatagatg caatagtttc tccaggaacc gaaatacata     5280
   cattgtcttc cgtaaagcgc tagactatat attattatac aggttcaaat atactatctg     5340
   tttcagggaa aactcccagg ttcggatgtt caaaattcaa tgatgggtaa caagtacgat     5400
   cgtaaatctg taaaacagtt tgtcggatat taggctgtat ctcctcaaag cgtattcgaa     5460
   tatcattgag aagctgcagc gtcacatcgg ataataatga tggcagccat tgtagaagtg     5520
   ccttttgcat ttctagtctc tttctcggtc tagctagttt tactacatcg cgaagataga     5580
   atcttagatc acactgcctt tgctgagctg gatcaataga gtaacaaaag agtggtaagg     5640
   cctcgttaaa ggacaaggac ctgagcggaa gtgtatcgta cagtagacgg agtatactag     5700
   gtatagtcta tagtccgtgg aattaattct catgtttgac agcttatcat cgataatccg     5760
   gagctagcat gcggccgctc tagaactagt ggatcccccg ggctgcagga attctctttg     5820
   aaaagataat gtatgattat gctttcactc atatttatac agaaacttga tgttttcttt     5880
   cgagtatata caaggtgatt acatgtacgt ttgaagtaca actctagatt ttgtagtgcc     5940
   ctcttgggct agcggtaaag gtgcgcattt tttcacaccc tacaatgttc tgttcaaaag     6000
   attttggtca aacgctgtag aagtgaaagt tggtgcgcat gtttcggcgt tcgaaacttc     6060
   tccgcagtga aagataaatg atctattaga accagggagg tccgttttag agc            6113
<210> 99
   <211> 73
   <212> DNA
   <213> artificial sequence
<220>
   <223> forward primer approach 1: 50 bp tail on one side
<400> 99
   aaacttctcc gcagtgaaag ataaatgatc tattagaacc agggaggtcc gttttagagc       60
   tagaaatagc aag                                                          73
<210> 100
   <211> 22
   <212> DNA
   <213> artificial sequence
<220>
   <223> reverse primer approach 1: 50 bp tail on one side
<400> 100
   gatcatttat ctttcactgc gg 22
<210> 101
   <211> 6103
   <212> DNA
   <213> artificial sequence
<220>
   <223> PCR fragment approach 2: 50 bp tail on one side. The fragment was transformed to yeast allowing reconstitution of a circular vector by in vivo recombination
<400> 101
   aaacttctcc gcagtgaaag ataaatgatc tattagaacc agggaggtcc gttttagagc      60
   tagaaatagc aagttaaaat aaggctagtc cgttatcaac ttgaaaaagt ggcaccgagt     120
   cggtggtgct ttttttgttt tttatgtctc tcgagggggg gcccggtacc cagcttttgt     180
   tccctttagt gagggttaat tccgagcttg gcgtaatcat ggtcatagct gtttcctgtg     240
   tgaaattgtt atccgctcac aattccacac aacataggag ccggaagcat aaagtgtaaa     300
   gcctggggtg cctaatgagt gaggtaactc acattaattg cgttgcgctc actgcccgct     360
   ttccagtcgg gaaacctgtc gtgccagctg cattaatgaa tcggccaacg cgcggggaga     420
   ggcggtttgc gtattgggcc ctcttccgct tcctcgctca ctgactcgct gcgctcggtc     480
   gttcggctgc ggcgagcggt atcagctcac tcaaaggcgg taatacggtt atccacagaa     540
   tcaggggata acgcaggaaa gaacatgtga gcaaaaggcc agcaaaaggc caggaaccgt     600
   aaaaaggccg cgttgctggc gtttttccat aggctcggcc cccctgacga gcatcacaaa     660
   aatcgacgct caagtcagag gtggcgaaac ccgacaggac tataaagata ccaggcgttc     720
   ccccctggaa gctccctcgt gcgctctcct gttccgaccc tgccgcttac cggatacctg     780
   tccgcctttc tcccttcggg aagcgtggcg ctttctcaat gctcacgctg taggtatctc     840
   agttcggtgt aggtcgttcg ctccaagctg ggctgtgtgc acgaaccccc cgttcagccc     900
   gaccgctgcg ccttatccgg taactatcgt cttgagtcca acccggtaag acacgactta     960
   tcgccactgg cagcagccac tggtaacagg attagcagag cgaggtatgt aggcggtgct    1020
   acagagttct tgaagtggtg gcctaactac ggctacacta gaaggacagt atttggtatc    1080
   tgcgctctgc tgaagccagt taccttcgga aaaagagttg gtagctcttg atccggcaaa    1140
   caaaccaccg ctggtagcgg tggttttttt gtttgcaagc agcagattac gcgcagaaaa    1200
   aaaggatctc aagaagatcc tttgatcttt tctacggggt ctgacgctca gtggaacgaa    1260
   aactcacgtt aagggatttt ggtcatgaga ttatcaaaaa ggatcttcac ctagatcctt    1320
   ttaaattaaa aatgaagttt taaatcaatc taaagtatat atgagtaaac ttggtctgac    1380
   agttaccaat gcttaatcag tgaggcacct atctcagcga tctgtctatt tcgttcatcc 1440
   atagttgcct gactgcccgt cgtgtagata actacgatac gggagggctt accatctggc 1500
   cccagtgctg caatgatacc gcgagaccca cgctcaccgg ctccagattt atcagcaata 1560
   aaccagccag ccggaagggc cgagcgcaga agtggtcctg caactttatc cgcctccatc 1620
   cagtctatta attgttgccg ggaagctaga gtaagtagtt cgccagttaa tagtttgcgc 1680
   aacgttgttg ccattgctac aggcatcgtg gtgtcacgct cgtcgtttgg tatggcttca 1740
   ttcagctccg gttcccaacg atcaaggcga gttacatgat cccccatgtt gtgaaaaaaa 1800
   gcggttagct ccttcggtcc tccgatcgtt gtcagaagta agttggccgc agtgttatca 1860
   ctcatggtta tggcagcact gcataattct cttactgtca tgccatccgt aagatgcttt 1920
   tctgtgactg gtgagtactc aaccaagtca ttctgagaat agtgtatgcg gcgaccgagt 1980
   tgctcttgcc cggcgtcaat acgggataat accgcgccac atagcagaac tttaaaagtg 2040
   ctcatcattg gaaaacgttc ttcggggcga aaactctcaa ggatcttacc gctgttgaga 2100
   tccagttcga tgtaacccac tcgtgcaccc aactgatctt cagcatcttt tactttcacc 2160
   agcgtttctg ggtgagcaaa aacaggaagg caaaatgccg caaaaaaggg aataagggcg 2220
   acacggaaat gttgaatact catactcttc ctttttcaat attattgaag catttatcag 2280
   ggttattgtc tcatgagcgg atacatattt gaatgtattt agaaaaataa acaaataggg 2340
   gttccgcgca catttccccg aaaagtgcca cctgacgtct aagaaaccat tattatcatg 2400
   acattaacct ataaaaatag gcgtatcacg aggccctttc gtctcgcgcg tttcggtgat 2460
   gacggtgaaa acctctgaca catgcagctc ccggagacgg tcacagcttg tctgtaagcg 2520
   gatgccggga gcagacaagc ccgtcagggc gcgtcagcgg gtgttggcgg gtgtcggggc 2580
   tggcttaact atgcggcatc agagcagatt gtactgagag tgcaccatat cgactacgtc 2640
   gtaaggccgt ttctgacaga gtaaaattct tgagggaact ttcaccatta tgggaaatgg 2700
   ttcaagaagg tattgactta aactccatca aatggtcagg tcattgagtg ttttttattt 2760
   gttgtatttt ttttttttta gagaaaatcc tccaatatca aattaggaat cgtagtttca 2820
   tgattttctg ttacacctaa ctttttgtgt ggtgccctcc tccttgtcaa tattaatgtt 2880
   aaagtgcaat tctttttcct tatcacgttg agccattagt atcaatttgc ttacctgtat 2940
   tcctttacta tcctcctttt tctccttctt gataaatgta tgtagattgc gtatatagtt 3000
   tcgtctaccc tatgaacata ttccattttg taatttcgtg tcgtttctat tatgaatttc 3060
   atttataaag tttatgtaca cctaggatcc gtcgacactg gatggcggcg ttagtatcga 3120
   atcgacagca gtatagcgac cagcattcac atacgattga cgcatgatat tactttctgc 3180
   gcacttaact tcgcatctgg gcagatgatg tcgaggcgaa aaaaaatata aatcacgcta 3240
   acatttgatt aaaatagaac aactacaata taaaaaaact atacaaatga caagttcttg 3300
   aaaacaagaa tctttttatt gtcagtacta ggggcagggc atgctcatgt agagcgcctg 3360
   ctcgccgtcc gaggcggtgc cgtcgtacag ggcggtgtcc aggccgcaga gggtgaaccc 3420
   catccgccgg tacgcgtgga tcgccggtgc gttgacgttg gtgacctcca gccagaggtg 3480
   cccggcgccc cgctcgcggg cgaactccgt cgcgagcccc atcaacgcgc gcccgacccc 3540
   gtgcccccgg tgctccgggg cgacctcgat gtcctcgacg gtcagccggc ggttccagcc 3600
   ggagtacgag acgaccacga agcccgccag gtcgccgtcg tccccgtacg cgacgaacgt 3660
   ccgggagtcc gggtcgccgt cctccccggc gtccgattcg tcgtccgatt cgtcgtcggg 3720
   gaacaccttg gtcaggggcg ggtccaccgg cacctcccgc agggtgaagc cgtccccggt 3780
   ggcggtgacg cggaagacgg tgtcggtggt gaaggaccca tccagtgcct cgatggcctc 3840
   ggcgtccccc gggacactgg tgcggtaccg gtaagccgtg tcgtcaagag tggtcatttt 3900
   acatggttgt ttatgttcgg atgtgatgtg agaactgtat cctagcaaga ttttaaaagg 3960
   aagtatatga aagaagaacc tcagtggcaa atcctaacct tttatatttc tctacagggg 4020
   cgcggcgtgg ggacaattca acgcgtctgt gaggggagcg tttccctgct cgcaggtctg 4080
   cagcgaggag ccgtaatttt tgcttcgcgc cgtgcggcca tcaaaatgta tggatgcaaa 4140
   tgattataca tggggatgta tgggctaaat gtacgggcga cagtcacatc atgcccctga 4200
   gctgcgcacg tcaagactgt caaggagggt attctgggcc tccatgtcgc tggccgggtg 4260
   acccggcggg gacgaggcct taagttcgaa cgtacgagct ccggcattgc gaataccgct 4320
   ttccacaaac attgctcaaa agtatctctt tgctatatat ctctgtgcta tatccctata 4380
   taacctaccc atccaccttt cgctccttga acttgcatct aaactcgacc tctacatttt 4440
   ttatgtttat ctctagtatt actctttaga caaaaaaatt gtagtaagaa ctattcatag 4500
   agtgaatcga aaacaatacg aaaatgtaaa catttcctat acgtagtata tagagacaaa 4560
   atagaagaaa ccgttcataa ttttctgacc aatgaagaat catcaacgct atcactttct 4620
   gttcacaaag tatgcgcaat ccacatcggt atagaatata atcggggatg cctttatctt 4680
   gaaaaaatgc acccgcagct tcgctagtaa tcagtaaacg cgggaagtgg agtcaggctt 4740
   tttttatgga agagaaaata gacaccaaag tagccttctt ctaaccttaa cggacctaca 4800
   gtgcaaaaag ttatcaagag actgcattat agagcgcaca aaggagaaaa aaagtaatct 4860
   aagatgcttt gttagaaaaa tagcgctctc gggatgcatt tttgtagaac aaaaaagaag 4920
   tatagattct ttgttggtaa aatagcgctc tcgcgttgca tttctgttct gtaaaaatgc 4980
   agctcagatt ctttgtttga aaaattagcg ctctcgcgtt gcatttttgt tttacaaaaa 5040
   tgaagcacag attcttcgtt ggtaaaatag cgctttcgcg ttgcatttct gttctgtaaa 5100
   aatgcagctc agattctttg tttgaaaaat tagcgctctc gcgttgcatt tttgttctac 5160
   aaaatgaagc acagatgctt cgttaacaaa gatatgctat tgaagtgcaa gatggaaacg     5220
   cagaaaatga accggggatg cgacgtgcaa gattacctat gcaatagatg caatagtttc     5280
   tccaggaacc gaaatacata cattgtcttc cgtaaagcgc tagactatat attattatac     5340
   aggttcaaat atactatctg tttcagggaa aactcccagg ttcggatgtt caaaattcaa     5400
   tgatgggtaa caagtacgat cgtaaatctg taaaacagtt tgtcggatat taggctgtat     5460
   ctcctcaaag cgtattcgaa tatcattgag aagctgcagc gtcacatcgg ataataatga     5520
   tggcagccat tgtagaagtg ccttttgcat ttctagtctc tttctcggtc tagctagttt     5580
   tactacatcg cgaagataga atcttagatc acactgcctt tgctgagctg gatcaataga     5640
   gtaacaaaag agtggtaagg cctcgttaaa ggacaaggac ctgagcggaa gtgtatcgta     5700
   cagtagacgg agtatactag gtatagtcta tagtccgtgg aattaattct catgtttgac     5760
   agcttatcat cgataatccg gagctagcat gcggccgctc tagaactagt ggatcccccg     5820
   ggctgcagga attctctttg aaaagataat gtatgattat gctttcactc atatttatac     5880
   agaaacttga tgttttcttt cgagtatata caaggtgatt acatgtacgt ttgaagtaca     5940
   actctagatt ttgtagtgcc ctcttgggct agcggtaaag gtgcgcattt tttcacaccc     6000
   tacaatgttc tgttcaaaag attttggtca aacgctgtag aagtgaaagt tggtgcgcat     6060
   gtttcggcgt tcgaaacttc tccgcagtga aagataaatg atc                       6103
<210> 102
   <211> 23
   <212> DNA
   <213> artificial sequence
<220>
   <223> forward primer to obtain the 5' flank A - connector 5 sequence to obtain 1 kB deletion of genomic DNA
<400> 102
   cactatagca atctggctat atg 23
<210> 103
   <211> 24
   <212> DNA
   <213> artificial sequence
<220>
   <223> reverse primer to obtain the 5' flank A and B - connector 5 sequence to obtain 1 kB deletion of genomic DNA
<400> 103
   aaacgcctgt gggtgtggta ctgg 24
<210> 104
   <211> 500
   <212> DNA
   <213> artificial sequence
<220>
   <223> fragment A (5' flank A - connector 5) to obtain 1 kB deletion of genomic DNA
<400> 104
   cactatagca atctggctat atgatatgca gaaaatagtg attactgtgc tctttgctgc      60
   atcatgcatc atatgtttgg cattctgtcc tgtcagatga atgggcgaaa caattcgggg     120
   agctttgttg cgacttggta cccggcaagc cgcgacctat attttgcatt aaacgagttc     180
   atcgtggatt tatcgccatg agccttagct tatcaagcct ctcacagaca attaagcaat     240
   gaaaaaggta taccatcggc gcagaatggt taactagtgg gttcatactg ctgtgttata     300
   gattgttacc taagtgatca ccaaaaaaaa gtgcaaaaag gaaaaaaaaa taagagacag     360
   gtaacttcca caagcttatt cttccaaaaa tcaatcttat cttcatgcca gcaatagttg     420
   cgtgctgagc tcaacagtgc ccaacccttg aagcgacttc caatcgcttt gcatatccag     480
   taccacaccc acaggcgttt                                                 500
<210> 105
   <211> 26
   <212> DNA
   <213> artificial sequence
<220>
   <223> forward primer to obtain the 5' flank B - connector 5 sequence to obtain 10 kB deletion of genomic DNA
<400> 105
   caatagtaat tttaaaaaca atgtgc 26
<210> 106
   <211> 500
   <212> DNA
   <213> artificial sequence
<220>
   <223> fragment B (5' flank B - connector 5) to obtain 10 kB deletion of genomic DNA
<400> 106
   caatagtaat tttaaaaaca atgtgcttat ccagaaattc cataaggagt agcgcttgga      60
   tccctcacct ttttcggtcg cggcagtctt caaaaccttt tttggctcgt catttttttt     120
   taactttgcc ttctttgtca cagccatatt ttgcctcgaa cgatcacctt tcatcgtagg     180
   ctatccaata gacgtcaaat gccttgctga ccactacaga actgtaatga acttcagcta     240
   tttttaacgt atgttattct ataattctat accactattg tcgttttgaa aaatgaaatt     300
   tcacttttcg gaagttgtcc gctaagaaca actaagtgat aacagtaaat tttaaaaatg     360
   ctttgaaaaa tgggttaagc aaaggttatt tactataact acgtagtgaa cgaaaccata     420
   tatctcttaa tttgcagcag ggattttata aagcgacttc caatcgcttt gcatatccag 480
   taccacaccc acaggcgttt 500
<210> 107
   <211> 22
   <212> DNA
   <213> artificial sequence
<220>
   <223> forward primer to obtain Connector 5 - TDH3p - GFP - ENOlt - Connector 3 PCR fragment
<400> 107
   aagcgacttc caatcgcttt gc 22
<210> 108
   <211> 24
   <212> DNA
   <213> artificial sequence
<220>
   <223> reverse primer to obtain Connector 5 - TDH3p - GFP - ENOlt - Connector 3 PCR fragment
<400> 108
   acttagtatg gtctgttgga aagg 24
<210> 109
   <211> 1726
   <212> DNA
   <213> artificial sequence
<220>
   <223> fragment C, Connector 5 - TDH3p - GFP - ENOlt - Connector 3 synthetic cassette
<400> 109
   aagcgacttc caatcgcttt gcatatccag taccacaccc acaggcgttt gtgcttagtc 60
   aaaaaattag ccttttaatt ctgctgtaac ccgtacatgc ccaaaatagg gggcgggtta 120
   cacagaatat ataacatcgt aggtgtctgg gtgaacagtt tattcctggc atccactaaa 180
   tataatggag cccgcttttt aagctggcat ccagaaaaaa aaagaatccc agcaccaaaa 240
   tattgttttc ttcaccaacc atcagttcat aggtccattc tcttagcgca actacagaga 300
   acaggggcac aaacaggcaa aaaacgggca caacctcaat ggagtgatgc aacctgcctg 360
   gagtaaatga tgacacaagg caattgaccc acgcatgtat ctatctcatt ttcttacacc 420
   ttctattacc ttctgctctc tctgatttgg aaaaagctga aaaaaaaggt tgaaaccagt 480
   tccctgaaat tattccccta cttgactaat aagtatataa agacggtagg tattgattgt 540
   aattctgtaa atctatttct taaacttctt aaattctact tttatagtta gtcttttttt 600
   tagttttaaa acaccaagaa cttagtttcg aataaacaca cataaacaaa caaaatgtct 660
   aaaggtgaag aattattcac tggtgttgtc ccaattttgg ttgaattaga tggtgatgtt 720
   aatggtcaca aattttctgt ctccggtgaa ggtgaaggtg atgctactta cggtaaattg 780
   accttaaaat tgatttgtac tactggtaaa ttgccagttc catggccaac cttagtcact 840
   actttaggtt atggtttgca atgttttgct agatacccag atcatatgaa acaacatgac 900
   tttttcaagt ctgccatgcc agaaggttat gttcaagaaa gaactatttt tttcaaagat 960
   gacggtaact acaagaccag agctgaagtc aagtttgaag gtgatacctt agttaataga 1020
   atcgaattaa aaggtattga ttttaaagaa gatggtaaca ttttaggtca caaattggaa 1080
   tacaactata actctcacaa tgtttacatc actgctgaca aacaaaagaa tggtatcaaa 1140
   gctaacttca aaattagaca caacattgaa gatggtggtg ttcaattagc tgaccattat 1200
   caacaaaata ctccaattgg tgatggtcca gtcttgttac cagacaacca ttacttatcc 1260
   tatcaatctg ccttatccaa agatccaaac gaaaagagag atcacatggt cttgttagaa 1320
   tttgttactg ctgctggtat tacccatggt atggatgaat tgtacaaata aaagcttttg 1380
   attaagcctt ctagtccaaa aaacacgttt ttttgtcatt tatttcattt tcttagaata 1440
   gtttagttta ttcattttat agtcacgaat gttttatgat tctatatagg gttgcaaaca 1500
   agcatttttc attttatgtt aaaacaattt caggtttacc ttttattctg cttgtggtga 1560
   cgcgtgtatc cgcccgctct tttggtcacc catgtattta attgcataaa taattcttaa 1620
   aagtggagct agtctatttc tatttacata cctctcattt ctcatttcct cccctcagaa 1680
   agcctgtatg cgaagccaca atcctttcca acagaccata ctaagt 1726
<210> 110
   <211> 72
   <212> DNA
   <213> artificial sequence
<220>
   <223> forward primer to obtain the connector 3 - 3' flank A sequence to obtain 1 kB deletion of genomic DNA
<400> 110
   agaaagcctg tatgcgaagc cacaatcctt tccaacagac catactaagt aagcgttgaa 60
   gtttcctctt tg 72
<210> 111
   <211> 20
   <212> DNA
   <213> artificial sequence
<220>
   <223> reverse primer to obtain the connector 3 - 3' flank A and B sequence to obtain 1 kB deletion of genomic DNA
<400> 111
   tgtcaactgg agagctatcg 20
<210> 112
   <211> 581
   <212> DNA
   <213> artificial sequence
<220>
   <223> fragment D (connector 3 - 3' flank A) to obtain 1 kB deletion of genomic DNA
<400> 112
   agaaagcctg tatgcgaagc cacaatcctt tccaacagac catactaagt aagcgttgaa       60
   gtttcctctt tgtatatttg agatcttcat tttatcggat tctttgtcat cagacaactt      120
   gttgagtggt actaaaggag tgcttttcat catccttttg gtgaacgatt tcaaatacgt      180
   tagtgttttc tgagctagtt ttgatcaatt caggtgattc gttatcagaa ctctcaggtt      240
   tgtattcgtg tccagttgtg tagcattcgc ctaacgtgta agcacggatt tcttcctcag      300
   aaatttcact gtatggaatc atgcccttct ttctcgcttc ttcgtcggta aatgcaccat      360
   agtaatcttt gtcatcatgt ctaacagtaa ttttgaatgg gaagaagaca catagccccc      420
   agtaaacgaa aaaagaaatc aaaaaggaga agaaagaatc accataaaag aatttaacaa      480
   tacctgagtc gtggaaatag ttattgttga cttcccaagc gataccaggt agaccgggag      540
   ccataccaca cacccaggca acgatagctc tccagttgac a                          581
<210> 113
   <211> 71
   <212> DNA
   <213> artificial sequence
<220>
   <223> forward primer to obtain the connector 3 - 3' flank B sequence to obtain 10 kB deletion of genomic DNA
<400> 113
   agaaagcctg tatgcgaagc cacaatcctt tccaacagac catactaagt ctgttgtgac       60
   agcatcttgt c                                                            71
<210> 114
   <211> 22
   <212> DNA
   <213> artificial sequence
<220>
   <223> reverse primer to obtain the connector 3 - 3' flank B sequence to obtain 10 kB deletion of genomic DNA
<400> 114
   cctagcagta gttgtaatgt gg 22
<210> 115
   <211> 607
   <212> DNA
   <213> artificial sequence
<220>
   <223> fragment E (connector 3 - 3' flank B) to obtain 10 kB deletion of genomic DNA
<400> 115
   agaaagcctg tatgcgaagc cacaatcctt tccaacagac catactaagt ctgttgtgac      60
   agcatcttgt ctattattta ttggatacgc aaaactcatt tttttaaaat tttttttata    120
   atttccttta aataaaaagg ttaaagctgg gaaaaatgca tctaaataat aaactaatga     180
   tttagtttcc ctcatttaaa atatctaaga aggacagaac tttgtttggg gtacttaccc     240
   ttgagtattt ttagcgatta ttcttggagt tctcagccag aagattaaaa gaatatatgt     300
   tactgaatgc agtgtgatac tcaagactaa gtagtcttaa caacaagtta acaagggcgt     360
   gtggtctagt ggtatgattc tcgctttggg cgacttcctg actaacagga agacaaagca     420
   tgcgagaggc cctgggttca attcccagct cgcccctttt atttttgatt atttatttcc     480
   tattggtcgg gtaacagatt ttttttattt ttttcaaggc ccttttgttt tgtaaacaaa     540
   gaaatttcac aaagtgagcg tctactaata ttgataatgc catttccaca ttacaactac     600
   tgctagg                                                               607
<210> 116
   <211> 15
   <212> DNA
   <213> artificial sequence
<220>
   <223> polynucleotide sequence of the first 15 polynucleotides (nucleotide 1-15) in the forward primers according to SEQ ID NO: 7 to 10
<400> 116
   tgaaagataa atgat 15
<210> 117
   <211> 25
   <212> DNA
   <213> artificial sequence
<220>
   <223> polynucleotide sequence of the last 25 polynucleotides (nucleotide 36-60) in the forward primers according to SEQ ID NO: 7 to 10
<400> 117
   gttttagagc tagaaatagc aagtt 25
<210> 118
   <211> 15
   <212> DNA
   <213> artificial sequence
<220>
   <223> polynucleotide sequence of the first 15 polynucleotides (nucleotide 1-15) in the reverse primers according to SEQ ID NO: 11 to 14
<400> 118
   ttctagctct aaaac 15
<210> 119
   <211> 25
   <212> DNA
   <213> artificial sequence
<220>
   <223> polynucleotide sequence of the last 25 polynucleotides (nucleotide 36-60) in the reverse primers according to SEQ ID NO: 11 to 14
<400> 119
   atcatttatc tttcactgcg gagaa 25

## Description

### Field of the invention

The present disclosure relates to a new method to assemble gRNA containing vectors to be used in CRISPR-CAS-mediated genomic transformations in a host cell.

### Background to the invention

Recent advances in genomics techniques and analysis methods have significantly accelerated the ability to e.g. catalog and map genetic factors associated with a diverse range of biological functions and diseases. Precise genome engineering technologies are needed to enable systematic reverse engineering of casual genetic variations by allowing selective perturbation of individual genetic elements, as well as to advance synthetic biology, biotechnological, and medical applications. Although genome-editing techniques such as designer zinc fingers, transcription activator-like effectors nucleases (TALENs), or homing meganucleases are available for producing targeted genome perturbations, there remains a need for new genome engineering technologies that are affordable, easy to set up, scalable, and amenable to targeting multiple positions within a genome. The engineering of meganucleases has been challenging for most academic researchers because the DNA recognition and cleavage functions of these enzymes are intertwined in a single domain. Robust construction of engineered zinc finger arrays has also proven to be difficult for many laboratories because of the need to account for context-dependent effects between individual finger domains in an array. There thus exists a pressing need for alternative and robust techniques for targeting of specific sequences within a host cell with a wide array of applications.
Very recently a new genome editing system, the CRISPR-Cas system, has been described (Sander *et al*., 2014; Hsu *et al.,* 2014; Doudna and Charpentier, 2104). The CRISPR-Cas system does not require the generation of customized proteins to target-specific sequences but rather a single Cas enzyme that can be programmed by a guide-polynucleotide to recognize a specific polynucleotide target; in other words, the Cas enzyme can be recruited to a specific polynucleotide target in a host cell genome using said guide-polynucleotide molecule.
To enable genome precision engineering in the yeast *Saccharomyces cerevisiae* using the RNA-guided CRISPR/CAS9 system, the essential components CAS9 protein and the crRNA-tracrRNA fusion transcript (referred to as guide-RNA or gRNA) are introduced in the yeast cell and act together. This can be efficiently achieved by expressing both the CAS9 protein and the gRNA from one or more deoxy ribo polynucleotides. It was shown that the CAS9 protein can be expressed from a single copy vector using a centromeric episomal vector (Sikorski and Hieter, 1989) together with a 2 µ vector (Christianson *et al.,* 1992) expressing the guide-RNA and introducing donor DNA in the transformation, resulting in cells with near 100% donor DNA recombination frequency as shown by the introduction of a desired mutation (DiCarlo *et al.,* 2013). This first study by DiCarlo and colleagues described the use of the CRISPR/CAS9 system to modify a single target in the genome within one yeast transformation round.

Expanding on the DiCarlo approach, others have described expression systems (e.g., yeast expression plasmids, use of *in vivo* recombination in yeast) to enable multiple modifications per transformation round, *i.e*., the expression of multiple gRNA sequences and donor DNA sequences (Ryan *et al.,* 2014; Bao *et al.,* 2015; Horwitz *et al.,* 2015; Jakočiu̅nas *et al.,* 2015; Mans *et al.,* 2015). The gRNA expression cassettes (under control of the *SNR52* promoter and *SUP4* terminator as described by DiCarlo *et al.,* 2013) are expressed from one or more high copy yeast expression vectors (*i*.*e*., bearing the sequence for the 2-µ selfish replication plasmid and a choice of dominant resistance or auxotrophic markers). For the construction of the (multi-)gRNA expression plasmids, so far two strategies are followed: either the plasmids are constructed before introduction to *S*. *cerevisiae,* for example by using an *in-vitro* DNA assembly method like Gibson cloning (Mans *et al.,* 2015), or USER cloning (Ryan *et al.,* 2014; Jakočiu̅nas *et al.,* 2015) and an intermediate cloning step in *E. coli.* In all these methods, which are rather laborious, full gRNA expression cassettes are used as building blocks in the cloning reaction. A second approach relies on the *in vivo* recombination capability of *S*. *cerevisiae* to assemble the fragments into a functional expression vector (Horwitz *et al.,* 2015). For example, a linearized backbone vector and multiple linear gRNA expression cassettes were co-transformed directly into *S*. *cerevisiae* to generate multiple functional expression vectors by gap repair. The latter method of direct transformation of PCR-amplified or synthetic DNA fragments and the subsequent *in vivo* recombination by *S*. *cerevisiae* has the advantage that no intermediate amplification step, for example via *E. coli* is needed, shortening time and circumventing possible recombination events in *E. coli.*

This gRNA transformation protocol and expression system resulted in either the triple deletion of three *S*. *cerevisiae* genes (*ADH5, HO, RHR2*) or the introduction of single nucleotide mutations in several *S*. *cerevisiae* genes leading to easily identifiable phenotypes (*e.g. SPT15* mutations and ethanol tolerance). A disadvantage of the latter method relies in the use of full expression cassettes for each guide RNA to be introduced which renders this method not very suitable for the use of standardized vector elements in the assembly. Furthermore the gap repair was dependent of 500 bp flanking sequences on each terminus of the gRNA expression cassettes. This method therefore requires the laborious construction of multiple gRNA expression cassettes comprising each approximately 1500 nucleotides. Another disadvantage relies in the efficiency of the CRISPR-Cas9 mediated genome editing which differed considerably between experiments (triple modification in one yeast transformation round leading to 64% with deletions and 9% with introduction of single nucleotide mutations). The latter might be due to the fact that not all gRNA's were equally expressed in the cell. An approach in which all gRNA are present in one single vector would be therefore preferred.

It is therefore desired to develop low-cost reliable methods for assembling of multiple gRNA -containing vectors *in vivo,* by using standard building blocks in the assembly.

### Summary of the invention

The present disclosure relates to the CRISPR-CAS system.

The present invention provides a method to produce a vector, preferably a circular vector comprising two or more guide-polynucleotide expression cassettes, wherein said two or more guide-polynucleotide expression cassette comprises a polynucleotide encoding a guide-polynucleotide operably linked to one or more control sequences which direct the expression of said guide-polynucleotide in a host cell, wherein said guide-polynucleotide comprises a guide-sequence that essentially is the reverse complement of a target-polynucleotide in a host cell and the guide-polynucleotide can direct binding of a Cas protein at a target-polynucleotide in a host cell to form a CRISPR-Cas complex, wherein the method comprises the following steps:
- providing two or more polynucleotides, wherein the two or more polynucleotides comprise a polynucleotide sequence to be extended, wherein the polynucleotide sequence to be extended comprises at the 5'-end and/or at the 3'-end to be extended a fragment of a guide-polynucleotide expression cassette, wherein said fragment of a guide-polynucleotide expression cassette at the 5'-end and/or at the 3'-end of the polynucleotide sequence to be extended, does not comprise a polynucleotide coding for a guide sequence;
- performing two or more overlap-extension PCR reactions by subjecting in each reaction one of said two or more polynucleotides and two suitable polynucleotide primers to yield one hybrid linear polynucleotide,
   wherein the two or more polynucleotide sequence to be extended and the suitable polynucleotide primers are selected so that each hybrid linear polynucleotide obtained in the two or more overlap-extension PCR reactions comprises at least a polynucleotide coding for a guide sequence and suitable 5'-termini and 3'-termini which allow assembly *in vivo* of the two or more hybrid linear polynucleotides and optionally of one or more additional linear polynucleotides with each other in a pre-defined order to yield a vector, preferably a circular vector comprising one or more functional guide-polynucleotide expression cassettes;
- subjecting the twoor more hybrid linear polynucleotides obtained in the overlap-extension PCR and optionally one or more additional linear polynucleotides to an assembly reaction yielding a vector, preferably a circular vector comprising one or more functional guide-polynucleotide expression cassettes, wherein the assembly reaction occurs *in vivo,* preferably wherein the guide-polynucleotide is a gRNA.

The present invention further provides a method of assembling a circular vector comprising two or more guide-polynucleotide expression cassettes *in vivo,* wherein said two or more guide-polynucleotide expression cassette comprises a polynucleotide encoding a guide-polynucleotide operably linked to one or more control sequences which direct the expression of said guide-polynucleotide in a host cell, wherein said guide-polynucleotide comprises a guide-sequence that essentially is the reverse complement of a target-polynucleotide in a host cell and the guide-polynucleotide can direct binding of a Cas protein at a target-polynucleotide in a host cell to form a CRISPR-Cas complex, wherein the method comprises the following steps:
providing two or more polynucleotides, wherein the two or more polynucleotides comprise a polynucleotide sequence to be extended, wherein the polynucleotide sequence to be extended comprises at the 5'-end and/or at the 3'-end to be extended a fragment of a guide polynucleotide expression cassette;
- performing two or more overlap-extension PCR reactions by subjecting in each reaction one of said one or more polynucleotides and two suitable polynucleotide primers wherein at least one suitable primer comprises a polynucleotide coding for a guide sequence to yield one hybrid linear polynucleotide,
- wherein the two or more polynucleotide sequences to be extended and the suitable polynucleotide primers are selected so that each hybrid linear polynucleotide obtained in the two or more overlap-extension PCR reactions comprises at least a polynucleotide coding for a guide sequence and suitable 5'-termini and 3'-termini which allow assembly *in vivo* of the two or more hybrid linear polynucleotides and optionally of one or more additional linear polynucleotides with each other in a pre-defined order to yield a circular vector comprising one or more functional guide-polynucleotide expression cassettes;-subjecting the two or more hybrid linear polynucleotides obtained in the overlap extension PCR and optionally one or more additional linear polynucleotides to an assembly reaction yielding the circular vector;
- providing one or more linear polynucleotides, wherein said one or more linear polynucleotides comprise at the 5'-terminus and/or at the 3'-terminus at least one guide-polynucleotide expression cassette or a fragment of at least one guide-polynucleotide expression cassette comprising at least the guide sequence;
- transforming said one or more linear polynucleotides, and optionally one or more additional linear polynucleotides into a host cell;
- allowing an homologous recombination reaction to take place between said one or more linear polynucleotides and optionally one or more additional linear polynucleotides, wherein said linear polynucleotides and additional linear polynucleotides have been selected to comprise suitable 5'-termini and 3'-termini which allow homologous recombination of said one or more linear polynucleotides and optionally of said one or more additional linear polynucleotides with each other in a pre-defined order to yield a circular vector comprising one or more functional guide-polynucleotide expression cassettes;
- selecting a host cell comprising a circular vector and wherein said vector comprises one or more functional guide-polynucleotide expression cassettes;
- recovering the circular vector.

The present invention also provides a method for producing a recombinant host cell comprising a circular vector, wherein said vector comprises two or more guide-polynucleotide expression cassettes, wherein said two or more guide-polynucleotide expression cassette comprises a polynucleotide encoding a guide-polynucleotide operably linked to one or more control sequences which direct the expression of said guide-polynucleotide in a host cell, wherein said guide-polynucleotide comprises a guide-sequence that essentially is the reverse complement of a target-polynucleotide in a host cell and the guide-polynucleotide can direct binding of a Cas protein at a target-polynucleotide in a host cell to form a CRISPR-Cas complex, wherein the method comprises the following steps:
- providing one or more linear polynucleotides, wherein said one or more linear polynucleotides comprise at the 5'-terminus and/or at the 3'-terminus at least one guide-polynucleotide expression cassette or a fragment thereof comprising at least the guide sequence;
- transforming said one or more linear polynucleotides, and optionally one or more additional linear polynucleotides into a host cell;
- allowing an homologous recombination reaction to take place between said one or more linear polynucleotides and optionally one or more additional linear polynucleotides, wherein said linear polynucleotides and additional linear polynucleotides have been selected to comprise suitable 5'-termini and 3'-termini which allow homologous recombination of said one or more linear polynucleotides and optionally of said one or more additional linear polynucleotides with each other in a pre-defined order to yield a vector, preferably a circular vector comprising one or more functional guide-polynucleotide expression cassettes;
- optionally selecting a host cell comprising a vector, preferably a circular vector and wherein said vector, preferably said circular vector comprises one or more functional guide-polynucleotide expression cassettes, wherein the one or more linear polynucleotides are hybrid linear polynucleotides obtained by
- providing two or more polynucleotides, wherein the two or more polynucleotides comprise a polynucleotide sequence to be extended, wherein the polynucleotide sequence to be extended comprises at the 5'-end and/or at the 3'-end to be extended a fragment of a guide-polynucleotide expression cassette, preferably wherein the fragment of a guide-polynucleotide expression cassette at the 5'-end and/or at the 3'-end of the polynucleotide sequence to be extended, does not comprise a polynucleotide coding for a guide sequence;
- performing two or more overlap-extension PCR reactions by subjecting in each reaction one of said two or more polynucleotides and two suitable polynucleotide primers to yield one hybrid linear polynucleotide,
wherein the two or more polynucleotide sequence to be extended and the suitable polynucleotide primers are selected so that each hybrid linear polynucleotide obtained in the two or more overlap-extension PCR reactions comprises at least a polynucleotide coding for a guide sequence and suitable 5'-termini and 3'-termini which allow assembly *in vivo* of the two or more hybrid linear polynucleotides and optionally of one or more additional linear polynucleotides with each other in a pre-defined order to yield a circular vector comprising one or more functional guide-polynucleotide expression cassettes.

The present invention provides as well a method for producing a recombinant host cell comprising a circular vectorwherein said vector comprises two or more guide-polynucleotide expression cassettes, wherein said two or more guide-polynucleotide expression cassette comprises a polynucleotide encoding a guide-polynucleotide operably linked to one or more control sequences which direct the expression of said guide-polynucleotide in a host cell, wherein said guide-polynucleotide comprises a guide-sequence that essentially is the reverse complement of a target-polynucleotide in a host cell and the guide-polynucleotide can direct binding of a Cas protein at a target-polynucleotide in a host cell to form a CRISPR-Cas complex, wherein the method comprises the following steps:
- performing a method of assembling a circular vector comprising two or more guide-polynucleotide expression cassettes *in vivo* according to the present invention in a first host cell, preferably a first host cell belonging to the species *S*. *cerevisiae,* wherein said first host cell comprising the circular vector, is selected and the circular vector is recovered, wherein said circular vector comprises two or more functional guide-polynucleotide expression cassettes,
- transforming the circular vector in a second host cell;
- optionally isolating the second host cell comprising the the circular vector.

### Brief description of the drawings

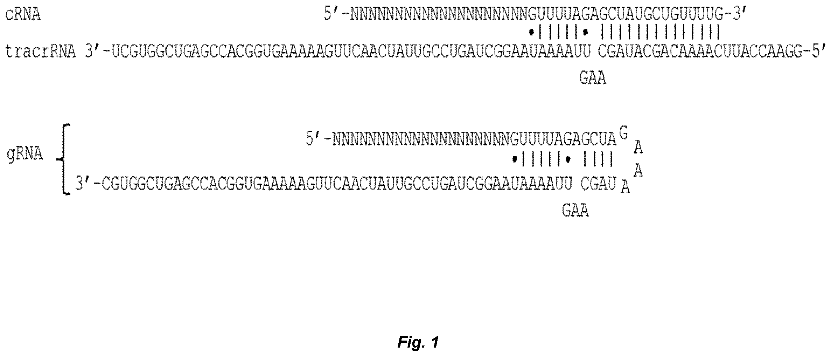

- Figure 1: depicts examples of typical guide-polynucleotides. Both guide-polynucleotides are guide-RNA's comprising a guide-sequence (crRNA) and a guide-polynucleotide structural component. In the upper figure, the guide-polynucleotide structural component is comprised of two separate molecules hybridized to each other; the individual molecules may be referred to as a tracr sequence and a tracr-mate sequence. In the lower figure, the guide-polynucleotide structural component is comprised of a single molecule with internal hybridization. This figure is adapted from Sander and Joung, 2014 and Mali et al., 2013.
- Figure 2: depicts the structure of bricks 1 to 4 and of the primers used in the OE-PCR reactions.
- Figure 3: depticts the structure of extended bricks 1 to 4 used in the assembly reaction *in vivo.*
- Figure 4: depticts how extended bricks 1 to 4 are assembled with each other in a pre-determined order in the assembly reaction *in vivo.*
- Figure 5: depicts a vector map of single copy (CEN/ARS) vector pCSN061 expressing CAS9 codon pair optimized for expression in S. cerevisiae. A KanMX marker is present on the vector.
- Figure 6: depicts the structure of bricks 1, 2, 3b, 4 and of the primers used in the OE-PCR reactions.
- Figure 7A: depicts the structure of extended bricks 1, 2, 3b and 4 used in the assembly reaction *in vivo.*
- Figure 7B: depticts how extended bricks 1 to 4 are assembled with each other in a pre-determined order in the assembly reaction *in vivo.*
- Figure 8: depicts a schematic representation of the donor DNA sequences used in the multiplex approach described in transformation Experiment 1. The donor DNA sequences (carotenoid gene expression cassettes) contain approximately 50 bp flanks sequences (INT 5' and INT 3'), that have homology with the intended integration sites (INT1, INT2 or INT3). Upon transformation, the donor DNA sequences integrate into genomic intended integration sites. Three different promoters were used for expression of crtE, crtYB and crtl, as represented by the white and two different grey colored rectangles.
- Figure: 9A depicts a schematic representation of the donor DNA, sequences used in the multiplex approach described in transformation Experiment 2. The donor DNA sequences (carotenoid gene expression cassettes) contain approximately 50 bp flanks sequences (INT 5' and INT 3'), that have homology with the intended integration sites (INT1, INT2 or INT3). The LF sequences contain overlap with integration sites in genomic DNA and the 50 bp flank sequences plus part of a specific promoter used for expression of crtE, crtYB and crtl (indicated by the small white and two different grey colored rectangles attached to the LF sequences). The RF sequences contain overlap with integration sites in genomic DNA and the 50 bp flank sequences plus part of a specific terminator used for expression of crtE, crtYB and crtl (indicated by the small white rectangle attached to the RF sequences).
- Figure: 9B depicts that upon transformation, the donor DNA sequences described in fig. 9A integrate into genomic intended integration sites. Three different promoters were used for expression of crtE, crtYB and crtl, as represented by the white and two different grey colored rectangles.
- Figure: 10 depicts a vector map of multi copy (2 micron) vector pGNR002, containing guide RNA expression cassette in which the guide sequence can be cloned/assembled by making use of the *Sap*I sites. A NatMX (nourseothricin) resistance marker is present on the vector.
- Figure 1: 11 depicts a graphical representation of the primers used in the OE-PCR reactions, in which vector pGRN002 was used as template, used for 1gRNA-vector assembly approach and 1 gRNA-vector assembly approach 2.

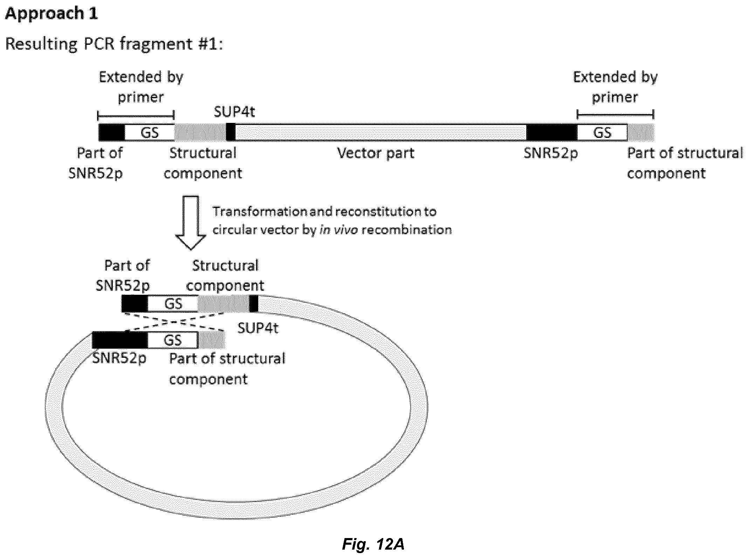

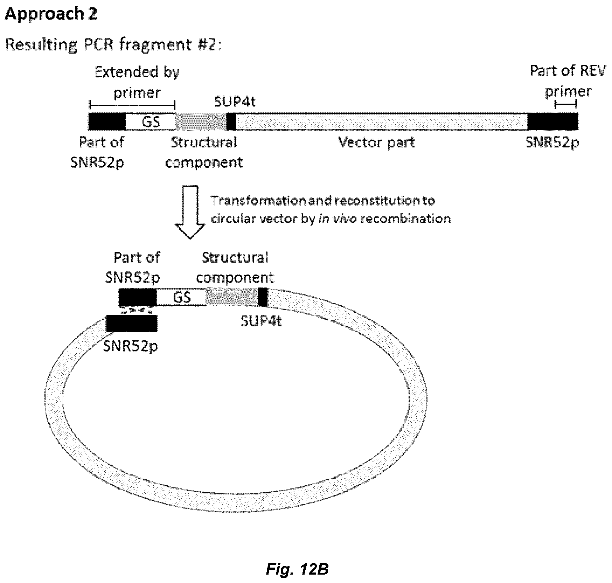

- Figure 12A and 12B: depict a graphical representation of PCR fragment #1 (one gRNA-vector approach 1) and PCR fragment #2 (one gRNA-vector approach 2), used in the *in vivo* assembly reaction in yeast. GS: 20 bp guide sequence.

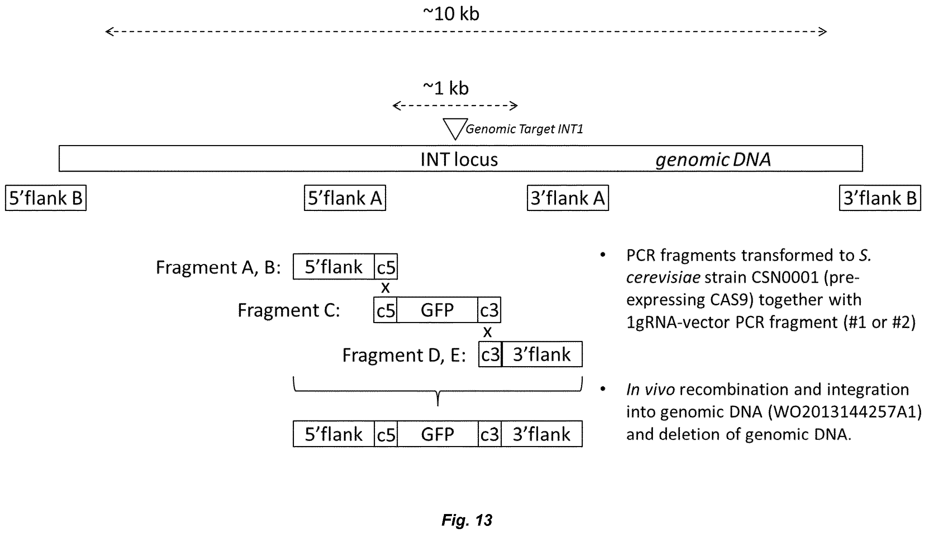

- Figure 13: depicts a graphical representation of the strategy for deletion of approximately 1 or approximately 10 kb of genomic DNA around the INT1 locus using 1 gRNA-vector assembly approach 1 or 1gRNA-vector assembly approach 2.

### Description of the sequence listing

SEQ ID NO: 1 sets out a preferred termination polynucleotide sequence in yeast.
SEQ ID NO: 2 sets out the polynucleotide sequence to be integrated in the integration locus on chromosome 14 of *S*. *cerevisiae,* comprising the Cas9 expression cassette and the *natMX* marker and including the 5'- and 3'- flanking regions to the integration locus.
SEQ ID NO: 3 sets out the polynucleotide sequence of Brick 1 comprising the 2µ plasmid.
SEQ ID NO: 4 sets out the polynucleotide sequence of Brick 2 comprising the expression cassette of Dasher GFP, flanked upstream by the *S*. *cerevisae TDH3* promoter, and flanked downstream by the *S*. *cerevisiae ADH1* terminator sequence.
SEQ ID NO: 5 sets out the polynucleotide sequence of Brick 3 comprising the dominant resistance marker *KanMX.*
SEQ ID NO: 6 sets out the polynucleotide sequence of Brick 4 comprising the *kanR* gene, a bacterial selection marker, and pUC ORI.
SEQ ID NO: 7 sets out the polynucleotide sequence of forward primer gR1-HXT1-f.
SEQ ID NO: 8 sets out the polynucleotide sequence of forward primer gR2-HXT2-f.
SEQ ID NO: 9 sets out the polynucleotide sequence of forward primer gR3-HXT3-f.
SEQ ID NO: 10 sets out the polynucleotide sequence of forward primer gR4-GAL2-f.
SEQ ID NO: 11 sets out the polynucleotide sequence of reverse primer gR1-HXT1-r.
SEQ ID NO: 12 sets out the polynucleotide sequence of reverse primer gR2-HXT2-r.
SEQ ID NO: 13 sets out the polynucleotide sequence of reverse primer gR3-HXT3-r.
SEQ ID NO: 14 sets out the polynucleotide sequence of reverse primer gR4-GAL2-r.
SEQ ID NO: 15 sets out the polynucleotide sequence of extended Brick 1.
SEQ ID NO: 16 sets out the polynucleotide sequence of extended Brick 2.
SEQ ID NO: 17 sets out the polynucleotide sequence of extended Brick 3.
SEQ ID NO: 18 sets out the polynucleotide sequence of extended Brick 4.
SEQ ID NO: 19 sets out the polynucleotide sequence of the donor DNA used in the CRISPR-CAS9-mediated deletion of genes *HXT5-HXT1-HXT4* in *S*. *cerevisiae* strain BIE272.
SEQ ID NO: 20 sets out the polynucleotide sequence of the donor DNA used in the CRISPR-CAS9-mediated deletion of gene *HXT2* in *S*. *cerevisiae* strain BIE272.
SEQ ID NO: 21 sets out the polynucleotide sequence of the donor DNA used in the CRISPR-CAS9-mediated deletion of genes *HXT3-HXT6-HXT7* in *S*. *cerevisiae* strain BIE272.
SEQ ID NO: 22 sets out the polynucleotide sequence of the donor DNA used in the CRISPR-CAS9-mediated deletion of gene *GAL2* in *S*. *cerevisiae* strain BIE272.
SEQ ID NO: 23 sets out the polynucleotide sequence of CAS9 expression vector pCSN061.
SEQ ID NO: 24 sets out the polynucleotide sequence of Brick 3b (natMX-fragment).
SEQ ID NO: 25 sets out the polynucleotide sequence of forward primer g1-INT59-f.
SEQ ID NO: 26 sets out the polynucleotide sequence of forward primer g2-YPRc-f.
SEQ ID NO: 27 sets out the polynucleotide sequence of forward primer g3-INT1-f.
SEQ ID NO: 28 sets out the polynucleotide sequence of forward primer Brick4_t03-f.
SEQ ID NO: 29 sets out the polynucleotide sequence of reverse primer g1-INT59-r.
SEQ ID NO: 30 sets out the polynucleotide sequence of reverse primer g2-YPRc-r.
SEQ ID NO: 31 sets out the polynucleotide sequence of reverse primer g3-INT1-r.
SEQ ID NO: 32 sets out the polynucleotide sequence of reverse primer Brick 3_to4-r.
SEQ ID NO: 33 sets out the polynucleotide sequence of extended Brick 1 (example 4).
SEQ ID NO: 34 sets out the polynucleotide sequence of extended Brick 2 (example 4).
SEQ ID NO: 35 sets out the polynucleotide sequence of extended Brick 3b (Example 4).
SEQ ID NO: 36 sets out the polynucleotide sequence of extended Brick 4 (Example 4).
SEQ ID NO: 37 EMPTY.
SEQ ID NO: 38 sets out the polynucleotide sequence of the synthetic con5 - Low p (KITDH2p) - crtE - ScTDH3t - conA expression cassette.
SEQ ID NO: 39 sets out the polynucleotide sequence of the synthetic con5 - Med p (KIPGK1p) - crtE - ScTDH3t - conA expression cassette.
SEQ ID NO: 40 sets out the polynucleotide sequence of the synthetic con5 - Strong p (ScFBA1p) - crtE - ScTDH3t - conA expression cassette.
SEQ ID NO: 41 sets out the polynucleotide sequence of the synthetic conA - Low p (KIYDRp) - crtYB - ScPDC1t - conB expression cassette.
SEQ ID NO: 42 sets out the polynucleotide sequence of the synthetic conA - Med p (KITEF2p) - crtYB - ScPDC1t - conB expression cassette.
SEQ ID NO: 43 sets out the polynucleotide sequence of the synthetic conA - Strong p (ScTEF1p) - crtYB - ScPDC1t - conB expression cassette.
SEQ ID NO: 44 sets out the polynucleotide sequence of the synthetic conB - Low p (ScPRE3p) - crtl - ScTAL1t - conC expression cassette.
SEQ ID NO: 45 sets out the polynucleotide sequence of the synthetic conB - Med p (ScACT1p) - crtl - ScTAL1t - conC expression cassette.
SEQ ID NO: 46 sets out the polynucleotide sequence of the synthetic conB - Strong p (KIENO1p) - crtl - ScTAL1t - conC expression cassette.
SEQ ID NO: 47 sets out the polynucleotide sequence of the donor expression cassette sequence with 50 bp LF and RF flanks (PCR fragment). Homology to INT1 - Low p (KITDH2p) - crtE - ScTDH3t - Homology to INT1.
SEQ ID NO: 48 sets out the polynucleotide sequence of the donor expression cassette sequence with 50 bp LF and RF flanks (PCR fragment). Homology to INT1 - Med p (KIPGK1p) - crtE - ScTDH3t - Homology to INT1.
SEQ ID NO: 49 sets out the polynucleotide sequence of the donor expression cassette sequence with 50 bp LF and RF flanks (PCR fragment). Homology to INT1 - Strong p (ScFBA1p) - crtE - ScTDH3t -Homology to INT1.
SEQ ID NO: 50 sets out the polynucleotide sequence of the donor expression cassette sequence with 50 bp LF and RF flanks (PCR fragment). Homology to INT2 - Low p (KIYDR1p) - crtYB - ScPDC1t - Homology to INT2.
SEQ ID NO: 51 sets out the polynucleotide sequence of the donor expression cassette sequence with 50 bp LF and RF flanks (PCR fragment). Homology to INT2 - Med p (KITEF2p) - crtYB - ScPDC1t - Homology to INT2.
SEQ ID NO: 52 sets out the polynucleotide sequence of the donor expression cassette sequence with 50 bp LF and RF flanks (PCR fragment). Homology to INT2 - Strong p (ScTEF1p) - crtYB - ScPDC1t - Homology to INT2.
SEQ ID NO: 53 sets out the polynucleotide sequence of the donor expression cassette sequence with 50 bp LF and RF flanks (PCR fragment). Homology to INT3 - Low p (ScPRE3p) - crtl - ScTAL1t - Homology to INT3.
SEQ ID NO: 54 sets out the polynucleotide sequence of the donor expression cassette sequence with 50 bp LF and RF flanks (PCR fragment). Homology to INT3 - Med p (ScACT1p) - crtl - ScTAL1t - Homology to INT3.
SEQ ID NO: 55 sets out the polynucleotide sequence of the donor expression cassette sequence with 50 bp LF and RF flanks (PCR fragment). Homology to INT3 - Strong p (KIENO1p) - crtl - ScTAL1t - Homology to INT3.
SEQ ID NO: 56 sets out the polynucleotide sequence of the forward (FW) primer to obtain SEQ ID NO: 47.
SEQ ID NO: 57 sets out the polynucleotide sequence of the reverse (REV) primer to obtain SEQ ID NO: 47, 48, 49.
SEQ ID NO: 58 sets out the polynucleotide sequence of the FW primer to obtain SEQ ID NO: 48.
SEQ ID NO: 59 sets out the polynucleotide sequence of the FW primer to obtain SEQ ID NO: 49.
SEQ ID NO: 60 sets out the polynucleotide sequence of the FW primer to obtain SEQ ID NO: 50.
SEQ ID NO: 61 sets out the polynucleotide sequence of the REV primer to obtain SEQ ID NO: 50, 51, 52.
SEQ ID NO: 62 sets out the polynucleotide sequence of the FW primer to obtain SEQ ID NO: 51.
SEQ ID NO: 63 sets out the polynucleotide sequence of the FW primer to obtain SEQ ID NO: 52.
SEQ ID NO: 64 sets out the polynucleotide sequence of the FW primer to obtain SEQ ID NO: 53.
SEQ ID NO: 65 sets out the polynucleotide sequence of the REV primer to obtain SEQ ID NO: 53, 54, 55.
SEQ ID NO: 66 sets out the polynucleotide sequence of the FW primer to obtain SEQ ID NO: 54.
SEQ ID NO: 67 sets out the polynucleotide sequence of the FW primer to obtain SEQ ID NO: 55.
SEQ ID NO: 68 sets out the polynucleotide sequence of the Right Flank (RF) sequence with overlap ScTDH3t - INT1 3' (part of donor) - INT1 3' genomic DNA. Homology with INT1 3' genomic DNA 524 bp.
SEQ ID NO: 69 sets out the polynucleotide sequence of the Left Flank (LF) sequence with overlap KIYDR2p - INT2 5' (part of donor) - INT2 5' genomic DNA. Homology with INT2 5' genomic DNA 322 bp.
SEQ ID NO: 70 sets out the polynucleotide sequence of the LF sequence with overlap KITEF2p - INT2 5' (part of donor) - INT2 5' genomic DNA. Homology with INT2 5' genomic DNA 322 bp.
SEQ ID NO: 71 sets out the polynucleotide sequence of the LF sequence with overlap ScTEF1p - INT2 5' (part of donor) - INT2 5' genomic DNA. Homology with INT2 5' genomic DNA 322 bp.
SEQ ID NO: 72 sets out the polynucleotide sequence of the RF sequence with overlap ScPDC1t - INT2 3' (part of donor) - INT2 3' genomic DNA. Homology with INT2 3' genomic DNA 524 bp.
SEQ ID NO: 73 sets out the polynucleotide sequence of the LF sequence with overlap ScPRE3p - INT3 5' (part of donor) - INT3 5' genomic DNA. Homology with INT3 5' genomic DNA 602 bp.
SEQ ID NO: 74 sets out the polynucleotide sequence of the LF sequence with overlap ScACT1p - INT3 5' (part of donor) - INT3 5' genomic DNA. Homology with INT3 5' genomic DNA 602 bp.
SEQ ID NO: 75 sets out the polynucleotide sequence of the LF sequence with overlap KIENO1p - INT3 5' (part of donor) - INT3 5' genomic DNA. Homology with INT3 5' genomic DNA 602 bp.
SEQ ID NO: 76 sets out the polynucleotide sequence of the RF sequence with overlap ScTAL - INT3 3' (part of donor) - INT3 3' genomic DNA. Homology with INT3 3' genomic DNA 624 bp.
SEQ ID NO: 77 sets out the polynucleotide sequence of the FW primer to obtain SEQ ID NO: 68.
SEQ ID NO: 78 sets out the polynucleotide sequence of the REV primer to obtain SEQ ID NO: 68.
SEQ ID NO: 79 sets out the polynucleotide sequence of the FW primer to obtain SEQ ID NO: 69, 70 ,71.
SEQ ID NO: 80 sets out the polynucleotide sequence of the REV primer to obtain SEQ ID NO: 69.
SEQ ID NO: 81 sets out the polynucleotide sequence of the REV primer to obtain SEQ ID NO: 70.
SEQ ID NO: 82 sets out the polynucleotide sequence of the REV primer to obtain SEQ ID NO: 71.
SEQ ID NO: 83 sets out the polynucleotide sequence of the FW primer to obtain SEQ ID NO: 72.
SEQ ID NO: 84 sets out the polynucleotide sequence of the REV primer to obtain SEQ ID NO: 72.
SEQ ID NO: 85 sets out the polynucleotide sequence of the FW primer to obtain SEQ ID NO: 73, 74, 75.
SEQ ID NO: 86 sets out the polynucleotide sequence of the REV primer to obtain SEQ ID NO: 73.
SEQ ID NO: 87 sets out the polynucleotide sequence of the REV primer to obtain SEQ ID NO: 74.
SEQ ID NO: 88 sets out the polynucleotide sequence of the REV primer to obtain SEQ ID NO: 75.
SEQ ID NO: 89 sets out the polynucleotide sequence of the FW primer to obtain SEQ ID NO: 76.
SEQ ID NO: 90 sets out the polynucleotide sequence of the REV primer to obtain SEQ ID NO: 76.
SEQ ID NO: 91 sets out the polynucleotide sequence of expression vector pRN1120.
SEQ ID NO: 92 sets out the polynucleotide sequence of FW primer to remove Sapl restriction site in pRN1120.
SEQ ID NO: 93 sets out the polynucleotide sequence of REV primer to remove Sapl restriction site in pRN1120.
SEQ ID NO: 94 sets out the polynucleotide sequence of gBlock allowing direct Sapl cloning of the guide sequence, part of vector pGRN002.
SEQ ID NO: 95 sets out the polynucleotide sequence of expression vector pGRN002.
SEQ ID NO: 96 sets out the polynucleotide sequence of FW primer approach 1: 30 bp on either side.
SEQ ID NO: 97 sets out the polynucleotide sequence of REV primer approach 1: 30 bp on either side.
SEQ ID NO: 98 sets out the polynucleotide sequence of PCR fragment approach 1: 30 bp on either side. The fragment was transformed to yeast allowing reconstitution of a circular vector by *in vivo* recombination.
SEQ ID NO: 99 sets out the polynucleotide sequence of FW primer approach 1: 50 bp tail on one side.
SEQ ID NO: 100 sets out the polynucleotide sequence of REV primer approach 1: 50 bp tail on one side.
SEQ ID NO: 101 sets out the polynucleotide sequence of PCR fragment approach 2: 50 bp tail on one side. The fragment was transformed to yeast allowing reconstitution of a circular vector by *in vivo* recombination.
SEQ ID NO: 102 sets out the polynucleotide sequence of FW primer to obtain the 5' flank A - connector 5 sequence to obtain approximately 1 kB deletion of genomic DNA.
SEQ ID NO: 103 sets out the polynucleotide sequence of REV primer to obtain the 5' flank A and B - connector 5 sequence to obtain approximately 1 kB deletion of genomic DNA.
SEQ ID NO: 104 sets out the polynucleotide sequence of fragment A (5' flank A - connector 5) to obtain approximately 1 kB deletion of genomic DNA.
SEQ ID NO: 105 sets out the polynucleotide sequence of FW primer to obtain the 5' flank B - connector 5 sequence to obtain approximately 10 kB deletion of genomic DNA.
SEQ ID NO: 106 sets out the polynucleotide sequence of fragment B (5' flank B - connector 5) to obtain approximately 10 kB deletion of genomic DNA.
SEQ ID NO: 107 sets out the polynucleotide sequence of FW primer to obtain Connector 5 - TDH3p - GFP - ENO1t - Connector 3 PCR fragment.
SEQ ID NO: 108 sets out the polynucleotide sequence of REV primer to obtain Connector 5 - TDH3p - GFP - ENO1t - Connector 3 PCR fragment.
SEQ ID NO: 109 sets out the polynucleotide sequence of fragment C, Connector 5 - TDH3p - GFP - ENO1t - Connector 3 synthetic cassette.
SEQ ID NO: 110 sets out the polynucleotide sequence of FW primer to obtain the connector 3 - 3' flank A sequence to obtain approximately 1 kB deletion of genomic DNA.
SEQ ID NO: 111 sets out the polynucleotide sequence of REV primer to obtain the connector 3 - 3' flank A and B sequence to obtain approximately 1 kB deletion of genomic DNA.
SEQ ID NO: 112 sets out the polynucleotide sequence of fragment D (connector 3 - 3' flank A) to obtain approximately 1 kB deletion of genomic DNA.
SEQ ID NO: 113 sets out the polynucleotide sequence of FW primer to obtain the connector 3 - 3' flank B sequence to obtain approximately 10 kB deletion of genomic DNA.
SEQ ID NO: 114 sets out the polynucleotide sequence of REV primer to obtain the connector 3 - 3' flank B sequence to obtain approximately 10 kB deletion of genomic DNA.
SEQ ID NO: 115 sets out the polynucleotide sequence of fragment E (connector 3 - 3' flank B) to obtain approximately 10 kB deletion of genomic DNA.
SEQ ID NO: 116 sets out the polynucleotide sequence of the first 15 polynucleotides (nucleotide 1-15) in the forward primers according to SEQ ID NO: 7 to 10.
SEQ ID NO: 117 sets out the polynucleotide sequence of the last 25 polynucleotides (nucleotide 36-60) in the forward primers according to SEQ ID NO: 7 to 10.
SEQ ID NO: 118 sets out the polynucleotide sequence of the first 15 polynucleotides (nucleotide 1-15) in the reverse primers according to SEQ ID NO: 11 to 14.
SEQ ID NO: 119 sets out the polynucleotide sequence of the last 25 polynucleotides (nucleotide 36-60) in the reverse primers according to SEQ ID NO: 11 to 14.

### Detailed description of the invention

We have surprisingly found that vectors comprising multiple gRNA-expression cassettes can be efficiently produced by applying re-usable, standardized vector elements together with overlap-extension-PCR (OE-PCR) to introduce the variable guide sequences. Due to the design of the multi-fragment assembly according to the invention, functional and multi-gRNA cassettes can be assembled straightforward at low cost. The method described herewith provides the flexibility to work with a set of standardized vector elements that can be re-used in a flexible way, e.g., a set of standardized marker cassettes, Origing of Replication cassettes, fluorescent/chromogenic cassettes, promoters, terminators etc.

In a first aspect the invention relates to a method to produce a circular vector comprising two or more guide-polynucleotide expression cassettes, wherein said two or more guide-polynucleotide expression cassettes comprise a polynucleotide encoding a guide-polynucleotide operably linked to one or more control sequences which direct the expression of said guide-polynucleotide in a host cell, wherein said guide-polynucleotide comprises a guide-sequence that essentially is the reverse complement of a target-polynucleotide in a host cell and the guide-polynucleotide can direct binding of a Cas protein at a target-polynucleotide in a host cell to form a CRISPR-Cas complex, wherein the method comprises the following steps:
- providing two or more polynucleotides, wherein the two or more polynucleotides comprise a polynucleotide sequence to be extended, wherein the polynucleotide sequence to be extended comprises at the 5'-end and/or at the 3'-end to be extended a fragment of a guide-polynucleotide expression cassette;
- performing two or more overlap-extension PCR reactions by subjecting in each reaction one of said one or more polynucleotides and two suitable polynucleotide primers to yield one hybrid linear polynucleotide,
   wherein two one or more polynucleotide sequence to be extended and the suitable polynucleotide primers are selected so that each hybrid linear polynucleotide obtained in the one or more overlap-extension PCR reactions comprises at least a polynucleotide coding for a guide sequence and suitable 5'-termini and 3'-termini which allow assembly *in vivo* of the two or more hybrid linear polynucleotides and optionally of one or more additional linear polynucleotides with each other in a pre-defined order to yield a vector, preferably a circular vector comprising one or more functional guide-polynucleotide expression cassettes;
- subjecting the two or more hybrid linear polynucleotides obtained in the overlap-extension PCR and optionally one or more additional linear polynucleotides to an assembly reaction yielding a vector, preferably a circular vector comprising one or more functional guide-polynucleotide expression cassettes,
   wherein the assembly reaction occurs *in vivo,* preferably wherein the guide-polynucleotide is a gRNA.

A " guide-polynucleotide expression cassette" is herewith defined as an expression cassette comprising a polynucleotide encoding a guide-polynucleotide operably linked to one or more control sequences which direct the expression of said guide-polynucleotide in a suitable host cell. In the context of the present invention a "guide-polynucleotide" comprises a guide-sequence that essentially is the reverse complement of a target-polynucleotide in a host cell and the guide-polynucleotide can direct binding of a Cas protein at a target-polynucleotide in a host cell to form a CRISPR-Cas complex.

The term "CRISPR-Cas complex" refers in the context of all embodiments of the present invention to a complex comprising a guide-polynucleotide hybridized to a target-polynucleotide and complexed with a Cas protein. In the most straightforward form, where a non-mutated Cas protein is used such as but not limited to the Cas9 protein of *Streptococcus pyogenes,* the formation of the CRISPR-Cas complex results in cleavage of one or both polynucleotide strands in or near (e.g. within 1, 2, 3, 4, 5, 6, 7, 8, 9, 10, 20, 50, or more base pairs from) the target-polynucleotide. Typically, a target-polynucleotide according to the present invention (defined below herein) is associated with a PAM sequence (defined below herein) and the PAM sequence is preferably immediately downstream (3') of the target-polynucleotide; the formation of the CRISPR-Cas complex typically results in cleavage of one or both polynucleotide strands 3 base pairs upstream (5') of the PAM sequence.

A "guide-polynucleotide" as intended herewith is a polynucleotide comprising at least a guide-sequence that is able to hybridize with the target-polynucleotide, e.g. a target polynucleotide in the genome of a host cell, and is able to direct sequence-specific binding of the CRISPR-Cas system to the target-polynucleotide to form a CRISPR-Cas complex. In one embodiment the guide-polynucleotide is a gRNA. In order to enable formation of an active CRISPR-Cas complex, the guide-polynucleotide preferably also comprises a sequence that has a specific secondary structure and allows binding of the Cas protein to the guide-polynucleotide. Such sequence is known in the art as tracrRNA, tracr sequence, tracr scaffold or guide-polynucleotide structural component, these terms are used interchangeably herein; wherein the tracr is the abbreviation for transactivating CRISPR; tracrRNA thus means transactivating CRISPR RNA. The tracrRNA in the original CRISPR-Cas system is the endogenous bacterial RNA that links the crRNA (guide-sequence) to the Cas nuclease, being able to bind any crRNA. A guide-polynucleotide structural component may be comprised of a single polynucleotide molecule or may be comprised of two or more molecules hybridized to each other; such hybridizing components of a guide-polynucleotide structural component may be referred to as a tracr sequence and a tracr-mate sequence. Accordingly, the guide-polynucleotide preferably also comprises a tracr sequence and/or a tracr-mate sequence. The guide-polynucleotide is a polynucleotide according to the general definition of a polynucleotide set out here below; a preferred guide-polynucleotide comprises ribonucleotides, a more preferred guide-polynucleotide is a RNA (guide-RNA or gRNA). Two examples of typical guide-polynucleotide structures are depicted in Figure 1.

In the context of the present invention, a "guide-sequence" is referred to as essentially the reverse complement of a target-sequence or of a target-polynucleotide, e.g. a target-sequence or target-polynucleotide present in the genome of a host cell, if the subject sequence is able to hybridize with the target-sequence or target-polynucleotide, preferably under physiological conditions as in a host cell. The degree of complementarity between a guide-sequence and its corresponding target-sequence, when optimally aligned using a suitable alignment algorithm, is preferably higher than 50%, 60%, 75%, 80%, 85%, 90%, 95%, 97.5%, 99% sequence identity. Optimal alignment may be determined using any suitable algorithm for aligning sequences, preferably an algorithm as defined herein under "Sequence identity". When the target-polynucleotide is a double stranded polynucleotide, the subject sequence, such as a guide-sequence, may be able to hybridize with either strand of the target-polynucleotide e.g. a coding strand or a non-coding strand. Preferably, a guide-sequence according to the present invention targets a target-sequence that is unique in the target. Preferably, a guide-sequence according to the present invention has 100% sequence identity with the 8, 9, 10, 11, 12, 13, 14, 15, 16, 17, 18, 19 or 20, more preferably 8, 9, 10, 11 or 12 nucleotides in the target-polynucleotide immediately adjacent to a PAM sequence. A guide-sequence according to the present invention preferably is 8-30, more preferably 10-30, more preferably 15-30, more preferably 17-27, more preferably 17-20, more preferably 17, 18, 19, 20, 21, 22, 23, 24, 25, 26, or 27 nucleotides in length. The ability of a guide-sequence to direct sequence-specific binding of a CRISPR-Cas system to a target-sequence to form a CRISPR-Cas complex may be assessed by any suitable assay. For example, the components of a CRISPR system sufficient to form a CRISPR-Cas complex, including the guide-sequence to be tested, may be provided to a host cell having the corresponding target-sequence, such, as by transfection with vectors encoding the components of the CRISPR-Cas system, followed by an assessment of preferential cleavage within the target-sequence, such as by the Surveyor assay (Surveyor® Mutation Detection Kits distributed by Integrated DNA Technologies, Leuven, Belgium) or another sequence analysis assay such as sequencing. Cleavage of a target-polynucleotide may be evaluated in a test tube by providing the target-polynucleotide, components of a CRISPR-Cas system, including the guide-sequence to be tested and a control guide-sequence different from the test guide-sequence, and comparing binding or rate of cleavage at the target-sequence between the test and control guide-sequence reactions. Other assays are possible, and are known to a person skilled in the art. A guide-polynucleotide structural component is believed to be necessary for formation of an active CRISPR-Cas complex. The guide-polynucleotide structural component is believed not necessarily to be operably linked to the guide-sequence; however, a guide-polynucleotide structural component may be operably linked to a guide-sequence within a guide-polynucleotide. A guide-polynucleotide structural component according to the present invention, which may comprise or consist of all or a portion of a wild-type guide-polynucleotide structural component (e.g. about or more than about 20, 26, 32, 45, 48, 54, 63, 67, 85, or more nucleotides of a wild-type tracr-sequence) forms part of a CRISPR-Cas complex; e.g. by hybridization of at least a portion of a tracr-sequence according to the present invention to all or a portion of a tracr-mate sequence according to the present invention and preferably operably linked to a guide-sequence according to the present invention. A tracr-sequence according to the present invention has sufficient complementarity to a tracr-mate sequence according to the present invention to hybridize, preferably under physiological condition as in a host cell, and facilitate formation of a CRISPR-Cas complex. As with the target-sequence according to the present invention, it is believed that complete complementarity is not needed, provided there is sufficient complementarity to be functional. Preferably, the tracr-sequence according to the present invention has at least 50%, 60%, 70%, 80%, 90%, 95% or 99% sequence identity along the length of the tracr-mate sequence according to the present invention when optimally aligned. Optimal alignment may be determined using any suitable algorithm for aligning sequences, preferably an algorithm as defined herein under "Sequence identity". In general, a tracr mate sequence according to the present invention includes any sequence that has sufficient complementarity with a tracr sequence according to the present invention to promote formation of a CRISPR-Cas complex at a target-sequence, wherein the CRISPR-Cas complex comprises the tracr mate sequence according to the present invention hybridized to the tracr sequence according to the present invention. The degree of complementarity of the tracr sequence according to the present invention and the tracr mate sequence according to the present invention is preferably defined with respect to optimal alignment of the tracr mate sequence and tracr sequence along the length of the shorter of the two sequences. Optimal alignment may be determined using any suitable algorithm for aligning sequences, preferably an algorithm as defined herein under "Sequence identity". Preferably, with respect to a tracr mate sequence according to the present invention and a tracr sequence according to the present invention, secondary structures are taken into account, such as self-complementarity within either the tracr sequence or tracr mate sequence. Preferably, the degree of complementarity between the tracr sequence according to the present invention and tracr mate sequence according to the present invention along the length of the shorter of the two sequences when optimally aligned is higher than 50%, 60%, 75%, 80%, 85%, 90%, 95%, 97.5%, 99% sequence identity. Preferably, the tracr mate sequence according to the present invention is 5, 6, 7, 8, 9, 10, 11, 12, 13, 14, 15, 16, 17, 18, 19, 20, 25, 30, 40, 50, or more nucleotides in length. Preferably, the tracr sequence according to the present invention is 5, 6, 7, 8, 9, 10, 11, 12, 13, 14, 15, 16, 17, 18, 19, 20, 25, 30, 40, 50, or more nucleotides in length. Preferably, the tracr sequence according to the present invention and tracr mate sequence, i.e. the guide-polynucleotide structural component according to the present invention are comprised within a single transcript, such that hybridization between the two produces a hybridization complex comprising a secondary structure, such as a hairpin. Such hybridization complex may also be formed when the tracr sequence and the tracr mate sequence are not comprised in a single transcript. Preferred loop forming sequences in a tracr sequence according to the present invention and/or a tracr mate sequence according to the present invention and/or guide-polynucleotide structural component according to the present invention for formation of hairpin structures are four nucleotides in length, and most preferably have the sequence GAAA; longer or shorter loop sequences may be used, as may alternative sequences. The loop sequences preferably include a nucleotide triplet (for example, AAA), and an additional nucleotide (for example C or G). Examples of loop forming sequences include CAAA and AAAG. Preferably, a tracr sequence according to the present invention and/or tracr mate sequence according to the present invention or hybridization complex thereof and/or guide-polynucleotide structural component according to the present invention comprise or is able to form at least two or more hairpins. More preferably, a tracr sequence according to the present invention and/or tracr mate sequence according to the present invention or hybridization complex thereof and/or guide-polynucleotide structural component according to the present invention comprises or is able to form two, three, four or five hairpins. Preferably, a tracr sequence according to the present invention and/or tracr mate sequence according to the present invention or hybridization complex thereof and/or guide-polynucleotide structural component according to the present invention comprises or is able to form at most five hairpins. Preferably, the single transcript of a tracr sequence according to the present invention and a tracr-mate sequence according to the present invention or hybridization complex of a tracr sequence according to the present invention and a tracr mate sequence according to the present invention and/or guide-polynucleotide structural component according to the present invention further comprises a transcription termination sequence; preferably this is a polyT sequence, for example six T nucleotides or, preferred for yeast, TTTTTTTGTTTTTTATGTCT (SEQ ID NO: 1). As said, guide-polynucleotide structural components are known to the person skilled in the art; background information can e.g. be found in Gaj et al, 2013.

In the context of all embodiments according to the present invention, the term "target-polynucleotide" refers to a target-sequence as defined herewith to which a guide-sequence as defined herewith is designed to have complementarity, where hybridization between a target-sequence as defined herewith and a guide-sequence as defined herewith promotes the formation of a CRISPR-Cas complex. Full complementarity is not necessarily required, provided there is sufficient complementarity to cause hybridization and promote formation of a CRISPR-Cas complex. Preferably, a guide-sequence as herewith defined targets a target-sequence that is unique in the target. Preferably, a guide-sequence has 100% sequence identity with the 8, 9, 10, 11, 12, 13, 14, 15, 16, 17, 18, 19 or 20, more preferably 8, 9, 10, 11 or 12 nucleotides in the target-polynucleotide immediately adjacent to a PAM sequence. A target-polynucleotide as defined herewith may comprise any polynucleotide, such as DNA or RNA polynucleotides and may be single or double stranded. When the target-polynucleotide is a double strand polynucleotide, a guide-sequence as defined herewith, may be able to hybridize with either strand of the target-polynucleotide e.g. a coding strand or a non-coding strand.
A target-polynucleotide as defined herewith may be located in the nucleus or cytoplasm of a cell. A target-polynucleotide as defined herewith may be located in an organelle of a host cell, for example in a mitochondrion or chloroplast. A target-polynucleotide as defined herewith may be comprised in a genome, may be comprised in a chromosome or may be extra-chromosomal, may be comprised in an artificial chromosome such a Yeast Artificial Chromosome (YAC), may be present in any chromosomal entity or extra-chromosomal entity such as an autosomal replicating entity such as an episomal plasmid or vector. A target-polynucleotide as defined herewith may be native or foreign to the host cell. A target-polynucleotide as defined herewith is preferably associated with a protospacer adjacent motif (PAM), which is a short polynucleotide recognized by the CRISPR-Cas complex. Preferably, the target-polynucleotide and PAM are linked wherein the PAM is preferably immediately downstream (3') of the target-polynucleotide. The exact sequence and length of the PAM may vary, e.g. different Cas proteins may require different PAM's. A preferred PAM according to the present invention is a polynucleotide of 2 to 8 nucleotides in length. A preferred PAM is selected from the group consisting of 5'-XGG-3', 5'-XGGXG-3', 5'-XXAGAAW-3', 5'-XXXXGATT-3', 5'-XXAGAA-3', 5'-XAAAAC-3', wherein X can be any nucleotide or analog thereof, preferably any nucleotide; and W is A or T. A more preferred PAM is 5'-XGG-3'. The PAM is preferably matched with the Cas protein. The most widely used CAS/CRISPR system is derived from S. *pyogenes* and the matching PAM sequence 5'-XGG-3' is located immediately downstream (3') of the target-sequence. A preferred PAM for a *Neisseria meningitidis* Cas protein is 5'-XXXXGATT-3'; a preferred PAM for a *Streptococcus thermophilus* Cas protein is 5'-XXAGAA-3'; a preferred PAM for a *Treponema denticola* is 5'-XAAAAC-3'. A preferred PAM matches the Cas protein used. A Cas protein according to the present invention may be engineered to match a different PAM than the native PAM matching the wild-type Cas protein. As such, the CRISPR-Cas system according to the present invention may be used for customized specific targeting.

Preferred genomes are those of *Aspergillus, Penicillium, Rasamsonia, Trichoderma, Yarrowia, Saccharomyces, Kluyveromyces, Mortierella, Bacillus, Escherichia, Pichia* and *Issatchenkia,* respectively. Non-limiting examples of genomes of said microorganisms can be accessed through their Genbank accession IDs numbers at for example (http://www.ncbi.nlm.nih.gov/) as follows: *A. niger* ATCC 1015 NCBI accession number ACJE00000000, A. *niger* CBS 513.88 genome sequence is accessible through EMBL accession numbers AM269948 to AM270415; *Aspergillus oryzae* RIB40 BA000049 to BA000056; *Penicillium chrysogenum* Wisconsin 54-1255 complete genome, accession numbers AM920416 to AM920464; *Kluyveromyces lactis* CBS2359 NRRL Y-1140 accession nos AJ229366 to AJ230023; *Kluyveromyces lactis* NRRL Y-1140 Accesion ID NC_006037 to NC_006042; *Saccharomyces cerevisiae* CEN.PK113-7D Accesion ID CM001522 to CM001537; *Saccharomyces cerevisiae* S288c Accesion ID NC_001133 to NC_001148; *Rasamsonia Emersonii* CBS 393.64 Accesion ID LASV01000001 to LASV01000862; *Yarrowia lipolitica* PO1f Accession ID CM002778 to CM002783; *Yarrowia lipolitica* CLIB122 Accession ID CR382127 to CR382132; *Bacillus subtilis* subsp. *subtilis* str. 168 Accession ID AL009126; *Escherichia coli str.* K-12 substr. MG1655 Accession Id U00096.3; *Pichia pastoris* Accession Id FN392319-FN392325, *Pichia kudriavzevii* M12 (also known as *Issatchenkia orientalis*)*,* accession number: GenBank: ALNQ00000000.1 , *Trichoderma reesei* QM6a GL985056- GL985132.

Unknown or ambiguous nucleotides in a genome (such as a nucleotide depicted with "n") are preferably excluded as polynucleotide sequence target.

The terms "CRISPR system", "CRISPR-Cas system" and "CRISPR enzyme system" are used interchangeably herein and refer in the context of all embodiments of the present invention to a collection of elements required to form, together with a target-polynucleotide, a CRISPR-Cas complex; these elements comprise but are not limited to a Cas protein and a guide-polynucleotide.

The terms "polynucleotide", "(poly)nucleotide sequence" and "nucleic acid" are used interchangeably herein and refer in the context of all embodiments of the present invention to a polymeric form of nucleotides of any length, either deoxyribonucleotides or ribonucleotides, or mixes or analogs thereof. Polynucleotides may have any three dimensional structure, and may perform any function, known or unknown. The following are non-limiting examples of polynucleotides: coding or non-coding regions of a gene or gene fragment, loci (locus) defined from linkage analysis, exons, introns, messenger RNA (mRNA), transfer RNA (tRNA), ribosomal RNA (rRNA), short interfering RNA (siRNA), short-hairpin RNA (shRNA), micro-RNA (miRNA), ribozymes, cDNA, recombinant polynucleotides, branched polynucleotides, plasmids, vectors, isolated DNA of any sequence, isolated RNA of any sequence, nucleic acid probes, oligonucleotides and primers. A polynucleotide may comprise one or more modified nucleotides, such as a methylated nucleotide and a nucleotide analogue or nucleotide equivalent wherein a nucleotide analogue or equivalent is defined as a residue having a modified base, and/or a modified backbone, and/or a non-natural internucleoside linkage, or a combination of these modifications. Preferred nucleotide analogues and equivalents are described in the section "General definitions". As desired, modifications to the nucleotide structure may be introduced before or after assembly of the polynucleotide. A polynucleotide may be further modified after polymerization, such as by conjugation with a labeling compound. A polynucleotide may be single stranded or double stranded. When referring to polynucleotide coding/encoding for another polynucleotide, generally the term polynucleotide will include in its meaning the double strand polynucleotide, even though reference will only be made to one of the two strands (i.e. reference will be made to the coding strand).

The method to produce a circular vector according to the first aspect comprises in a first step:
- providing two or more polynucleotides, wherein the two or more polynucleotides comprise a polynucleotide sequence to be extended, wherein the polynucleotide sequence to be extended comprises at the 5'-end and/or at the 3'-end to be extended a fragment of a guide-polynucleotide expression cassette.

The two or more polynucleotides may be a polynucleotide as defined herein above. Each of said two or more polynucleotides comprises a polynucleotide sequence to be extended. In the context of the present invention a polynucleotide sequence to be extended is a polynucleotide sequence comprised in each of said one or more polynucleotides which is to be extended by overlap-extension PCR reaction in the second step of the method to produce a vector, preferably a circular vector according to the invention and which become spliced/hybridized with the nucleotide sequence of the primers used in the reaction. The resulting extended polynucleotide sequence comprises the polynucleotide sequence to be extended which is spliced/hybridized with the nucleotide sequence of the primers used in the reaction. Said extended polynucleotide sequence is herewith indicated as hybrid linear polynucleotide.

The polynucleotide sequence to be extended comprised in each of said two or more polynucleotides which undergoes the overlap-extension PCR reaction may be a sub-sequence of said polynucleotide or it may comprise the whole polynucleotide sequence. Therefore in one embodiment the polynucleotide which undergoes the overlap-extension PCR reaction consists of the polynucleotide sequence to be extended.

The polynucleotide sequence to be extended comprises at the 5'-end and/or at the 3'-end to be extended a fragment of a guide-polynucleotide expression cassette.

A "guide-polynucleotide expression cassette", as defined herein before, is a polynucleotide encoding a guide-polynucleotide operably linked to one or more control sequences which direct the expression of said guide-polynucleotide in a host cell.

The guide-polynucleotide expression cassette contains all the control sequences required for expression of the guide-polynucleotide in a host cell, wherein said control sequences are operably linked to said guide-polynucleotide. Typically a guide-polynucleotide expression cassette will comprise at least a promoter operably linked to the coding sequence of the guide-polynucleotide to be expressed in a host cell and a terminator sequence.

The term "operably linked" as used herein refers to two or more nucleic acid sequence elements that are physically linked and are in a functional relationship with each other. For instance, a control sequence, e.g. a promoter, is operably linked to a DNA coding sequence if the promoter is able to initiate or regulate the transcription or expression of a coding sequence, i.e. in a configuration in which the control sequence is appropriately placed at a position relative to the coding sequence such that the control sequence directs the production of an RNA, e.g. a mRNA or a gRNA and optionally of a polypeptide translated from said mRNA, in which case the coding sequence should be understood as being "under the control of the promoter. Generally, when two nucleic acid sequences are operably linked, they will be in the same orientation and usually also in the same reading frame. They usually will be essentially contiguous, although this may not be required.

The term "control sequences" is defined herein to include all components, which are necessary or advantageous for the expression of RNA and / or a polypeptide (in case the control sequences are operably linked to a polyncleotide coding a compound of interest or involved in the production of a compound of interest) either in vitro or in a host cell. Such control sequences include, but are not limited to, a leader, Shine-Delgarno sequence (also indicated as Ribosome Binding Site), a polyadenylation sequence, a pro-peptide sequence, a pre-pro-peptide sequence, a promoter, a signal sequence, and a transcription terminator.

The control sequences may be provided with linkers for the purpose of introducing specific restriction sites facilitating ligation of the control sequences with the coding region of the guide polynucleotide.

The control sequence may be an appropriate promoter sequence (promoter).

The control sequence may also be a suitable transcription terminator (terminator) sequence, a sequence recognized by a host cell to terminate transcription. The terminator sequence is operably linked to the 3'-terminus of the nucleic acid sequence encoding the guide-polynucleotide or encoding a compound of interest. Any terminator, which is functional in the cell, may be used in the present invention. The person skilled in the art knows which types of terminators can be used in the host cell as described herein.

The control sequence may also be a 5'-untranslated sequence (also known as leader sequence), a non-translated region of a mRNA which is important for translation by the host cell. The translation initiation sequence or 5'-untranslated sequence is operably linked to the 5'-terminus of the coding sequence encoding a compound of interest or a compound related to the compound of interest as defined elsewhere herewith. Any leader sequence, which is functional in the cell, may be used in the present invention.

The control sequence may also be a polyadenylation sequence, a sequence which is operably linked to the 3'-terminus of the nucleic acid sequence and which, when transcribed, is recognized by the host cell (mutated or parent) as a signal to add polyadenosine residues to transcribed mRNA. Any polyadenylation sequence, which is functional in the cell, may be used in the present invention.

The term "promoter" is defined herein as a DNA sequence that binds RNA polymerase and directs the polymerase to the correct downstream transcriptional start site of a nucleic acid sequence encoding a biological compound, e.g. a compound of interest or the guide-polynucleotide, to initiate transcription. RNA polymerase effectively catalyzes the assembly of guide RNA or of messenger RNA complementary to the appropriate DNA strand of a coding region. The term "promoter" will also be understood to include the 5'-non-coding region (between promoter and translation start) for translation after transcription into mRNA (when the promoter is operably linked to a polynucleotide coding a compound of interest or coding a compound involved in the synthesis of a compound of interest), cis-acting transcription control elements such as enhancers, and other nucleotide sequences capable of interacting with transcription factors.

In the context of the present invention a "fragment of a guide-polynucleotide expression cassette" is a polynucleotide which comprises only a part of a (functional) guide-polynucleotide expression cassette. In one embodiment the fragment of a guide-polynucleotide expression cassette comprises a fragment including the 5'-terminus of the guide-polynucleotide expression cassette. In another embodiment the fragment of a guide-polynucleotide expression cassette comprises a fragment including the 3'-terminus of the guide-polynucleotide expression cassette. In one embodiment the polynucleotide sequence to be extended comprises at the 5'-end a fragment of a guide polynulcleotide expression cassette comprising the 3'-terminus of said expression cassette. In another embodiment the polynucleotide sequence to be extended comprises at the 3'-end a fragment of a guide polynulcleotide expression cassette comprising the 5'-terminus of a guide polynucleotide expression cassette. In one embodiment a fragment including the 5'-terminus of the guide-polynucleotide expression cassette comprises a control sequence of the guide-polynucleotide expression cassette, more preferably comprises a promoter. In another embodiment a fragment including the 3'-terminus of a guide polynucleotide expression cassette comprises a guide-polynucleotide structural element and a control sequence, more preferably comprises a guide-polynucleotide structural element and a terminator. In a preferred embodiment the fragment of a guide-polynucleotide expression cassette at the 5'-end and/or at the 3'-end of the polynucleotide sequence to be extended, does not comprise a polynucleotide coding for a guide sequence. This latter embodiment has the advantage of allowing use of standardized vector elements which together with overlap-extension-PCR (OE-PCR) allow introduction of the variable guide sequences. Therefore functional and multi-gRNA cassettes can be assembled straightforward at low cost. In a second step, the method to produce a circular vector comprising two or more guide-polynucleotide expression cassettes according to the first aspect comprises:
- performing two or more overlap-extension PCR reactions by subjecting in each reaction one of said one or more polynucleotides and two suitable polynucleotide primers to yield one hybrid linear polynucleotide,
   wherein the two or more polynucleotide sequence to be extended and the suitable polynucleotide primers are selected so that each hybrid linear polynucleotide obtained in the one or more overlap-extension PCR reactions comprises at least a polynucleotide coding for a guide sequence and suitable 5'-termini and 3'-termini which allow assembly *in vivo* of the one or more hybrid linear polynucleotides and optionally of one or more additional linear polynucleotides with each other in a pre-defined order to yield a circular vector comprising two or more functional guide-polynucleotide expression cassettes.

Throughout the invention the vector comprising two or more guide-polynucleotide expression cassettes, assembled *in vivo* according to the methods of the invention is preferably an autonomously replicating circular vector. In alternative said vector comprising one or more guide polynucleotide expression cassettes may be an autonomously replicating linear vectors. Autonomously replicanting linear vectors are known to those skilled in the art. (Finbarr Hayes (2003); Takahashi S *et al* (2011); Burke DT, et al (1987)).

Therefore in the context of the present invention a one or more polynucleotide which undergoes an overlap-extension PCR reaction is a polynucleotide which comprises a polynucleotide sequence to be extended in the overlap-extension PCR reaction.

In the context of the present invention the terms "overlap-extension PCR" (OE-PCR), "PCR-mediated overlap extension", "PCR-based overlap extension", "splicing by overlap extension (SOE)", "gene splicing by overlap extension", "splicing by overlap extension PCR" (SOE-PCR)", "gene splicing by overlap extension PCR", "gene splicing by PCR-mediated overlap extension", "gene splicing by PCR-driven overlap extension", and similar wording have the same meaning and are used interchangeably herewith. Overlap extension PCR (abbreviated herewith as OE-PCR) is a polymerase chain reaction-based technique well known to those skilled in the art and developed in the late 1980's (see Horton *et al*.). The technique is described in among others the above-mentioned Horton *et al.,* and in Heckman and Pease. The mechanism of overlap extension PCR is for example described in Fig. 1 of Horton *et al* and in Fig. 1b of Heckman and Pease.

In one embodiment, said performing one or more overlap-extension PCR reactions by subjecting in each reaction one of said one or more polynucleotides and two suitable polynucleotide primers to yield one hybrid linear polynucleotide,
wherein the one or more polynucleotide sequence to be extended and the suitable polynucleotide primers are selected so that each hybrid linear polynucleotide obtained in the one or more overlap-extension PCR reactions comprises at least a polynucleotide coding for a guide sequence and suitable 5'-termini and 3'-termini which allow assembly *in vivo* of the one or more hybrid linear polynucleotides and optionally of one or more additional linear polynucleotides with each other in a pre-established order to yield a vector, preferarbly a circular vector comprising one or more functional guide-polynucleotide expression cassettes, comprises the steps of:
a) subjecting in each reaction one of said one or more polynucleotides and one suitable polynucleotide primer to overlap-extension PCR reaction, wherein the 3'-terminus of said polynucleotide primer contains a complementary sequence to the 3'-end of the polynucleotide sequence to be extended comprised in said one or more polynucleotide or wherein the 3'-terminus of the complement of said polynucleotide primer contains a complementary sequence to the 3'-end of the complement of the polynucleotide sequence to be extended comprised in said one or more polynucleotide, respectively, to yield one hybrid linear polynucleotide having a sequence comprising the polynucleotide sequence to be extended spliced at its 3'-terminus or at its 5'-terminus, respectively, with the polynucleotide sequence of the suitable primer,
b) optionally subjecting the hybrid linear polynucleotide obtained in step a) and another suitable primer to a second overlap-extension PCR reaction, wherein the 3'-terminus of said polynucleotide primer contains a complementary sequence to the 3'-end of the hybrid linear polynucleotide having a sequence comprising the polynucleotide sequence to be extended spliced at its 3'-terminus or at its 5'-terminus, respectively, with the polynucleotide sequence of the first suitable primer, or wherein the 3'-terminus of the complement of the other polynucleotide primer contains a complementary sequence to the 3'-end of the complement of the hybrid linear polynucleotide having a sequence comprising the polynucleotide sequence to be extended spliced at its 3'-terminus or at its 5'-terminus, respectively, with the polynucleotide sequence of the first suitable primer, respectively, to yield one hybrid linear polynucleotide having a sequence comprising the polynucleotide sequence to be extended spliced at its 3'-terminus and at its 5'-terminus, respectively, with the polynucleotide sequences of the suitable primers,
   wherein the one or more polynucleotide sequence to be extended and the suitable polynucleotide primers are selected so that each hybrid linear polynucleotide obtained in the one or more overlap-extension PCR reactions comprises at least a polynucleotide coding for a guide sequence and suitable 5'-termini and 3'-termini which allow assembly *in vivo* of the one or more hybrid linear polynucleotides and optionally of one or more additional linear polynucleotides with each other in a pre-defined order to yield a vector, preferarbly a circular vector comprising one or more functional guide-polynucleotide expression cassettes.

The one or more polynucleotide sequence to be extended and the suitable polynucleotide primers used in the overlap extension PCR reaction are selected so that
1) each hybrid linear polynucleotide obtained in the one or more overlap-extension PCR reactions comprises at least a polynucleotide coding for a guide sequence;
2) said one or more hybrid linear polynucleotides obtained in the one or more overlap-extension PCR reaction comprise suitable 5'-termini and 3'-termini which allow assembly of the one or more hybrid linear polynucleotides and optionally of one or more additional linear polynucleotides with each other to yield a vector, preferarbly a circular vector comprising one or more functional guide-polynucleotide expression cassettes
3) said one or more hybrid linear polynucleotides obtained in the one or more overlap-extension PCR reaction comprise suitable 5'-termini and 3'-termini which allow assembly *in vivo* of the one or more hybrid linear polynucleotides and optionally of one or more additional linear polynucleotides with each other in a pre-defined order.

In order to satisfy condition 1), the polynucleotide sequence to be extended, comprising at the 5'-end and/or at the 3'-end to be extended a fragment of a guide-polynucleotide sequence may comprise a polynucleotide coding for a guide polynucleotide sequence. Alternatively in order to satisfy condition 1) a suitable primer used in the overlap extension PCR reaction may comprise a polynucleotide coding for a guide sequence. In yet another embodiment both the polynucleotide sequence to be extended and the suitable primer used in the overlap extension PCR reaction comprise a part of a polynucleotide coding for a guide sequence capable to yield the full polynucleotide coding for the guide sequence upon splicing of the two sequences in the OE-PCR reaction. In one preferred embodiment the fragment of a guide-polynucleotide expression cassette at the 5'-end and/or at the 3'-end of the polynucleotide sequence to be extended, does not comprise a polynucleotide coding for a guide sequence. In one preferred embodiment a suitable primer used in the overlap extension PCR reaction comprises a polynucleotide coding for a guide sequence.

In order to satisfy condition 2), the polynucleotide sequence to be extended in the one or more OE-PCR reaction, comprises at the 5'-end and/or at the 3'-end to be extended a fragment of a guide-polynucleotide expression cassette and the primer or primers used in the one or more OE-PCR reaction may comprise a second fragment of said guide-polynucleotide expression cassette such that upon OE-PCR the hybrid linear polynucleotide obtained comprises at least one functional guide-polynucleotide expression cassette at its 5'-terminus or at its 3'-terminus. Alternatively, the polynucleotide sequence to be extended in the one or more OE-PCR reaction, comprises at the 5'-end and at the 3'-end to be extended a fragment of a guide-polynucleotide expression cassette and the primers used in the one or more OE-PCR reaction may comprise a second fragment of said guide-polynucleotide expression cassette such that upon OE-PCR the hybrid linear polynucleotide obtained comprises a functional guide-polynucleotide both at the 5'-terminus and at the 3'-terminus. In yet an alternative embodiment the hybrid linear polynucleotides obtained in the one or more overlap-extension PCR reactions comprise at its 5'-terminus and/or 3'-terminus a fragment of a guide polynucleotide expression cassette and functional expression cassette are generated upon assembly of the one or more hybrid linear polynucleotides with each other and optionally with one or more additional linear polynucleotide. Therefore in one embodiment of the invention the hybrid linear polynucleotide obtained in the overlap-extension PCR reaction does not comprise a functional guide-polynucleotide expression cassette. The hybrid linear polyncuelcitde preferably comprises a fragment of a guide-polynucleotide expression cassette which fragment comprises a polynucleotide coding for a guide sequence.

In the context of the present invention said one or more additional linear polynucleotide is a linear polynucleotide defined as herein before which do not comprise a nucleotide coding for a guide-sequence.

In order to satisfy condition 3) the 5'-termini and 3'-termini present in the one or more hybrid linear polynucleotides and optionally in the one or more additional linear polynucleotides to be assembled are designed so as to allow homologous recombination *in vivo* between the one or more hybrid linear polynucleotides and optionally the one or more additional linear polynucleotides in a pre-defined order, leading to the formation of one type of product via homologous recombination.

Suitable primers are used in the OE-PCR reaction together with the one or more polynucleotides to yield hybrid linear polynucleotides as defined herewith above.

In one embodiment of a method to produce a circular vector according to the invention primers used in each overlap-extension PCR reaction are at most 100 nucleotides long, preferably at most 80, 70, 60, 50, 40, 30 nucleotides long. In another embodiment of a method to produce a circular vector according to the invention primers used in each overlap-extension PCR reaction are at least 30 nucleotides long, preferably at least 40, 50, 60 nucleotides long.

The primers used in the method to produce a circular vector according to the invention are selected, such as selected and designed, so that so that each hybrid linear polynucleotide obtained in the one or more overlap-extension PCR reactions comprises at least a polynucleotide coding for a guide sequence and suitable 5'-termini and 3'-termini which allow assembly *in vivo* of the two or more hybrid linear polynucleotides and optionally of one or more additional linear polynucleotides with each other in a pre-defined order to yield a vector, preferably a circular vector comprising one or more functional guide-polynucleotide expression cassettes.

Therefore in one embodiment the suitable primers used in the OE-PCR reaction comprises at least a polynucleotide coding for a guide sequence. In another embodiment the suitable primers used in the OE-PCR reaction comprises at least a polynucleotide coding for a guide sequence and a terminus, such as a 3'-terminus containing a complementary sequence to the 3'-end of the polynucleotide sequence to be extended. In another embodiment the suitable primers used in the OE-PCR reaction comprises at least a polynucleotide coding for a guide sequence and a terminus, such as a 3'-terminus containing a complementary sequence to the 3'-end of a hybrid polynucleotide. In yet another embodiment the suitable primers used in the OE-PCR reaction comprises comprises at least a polynucleotide coding for a guide sequence, a 3'-terminus containing a complementary sequence to the 3'-end of the polynucleotide sequence to be extended or a 3'-terminus containing a complementary sequence to the 3'-end of a hybrid polynucleotide to be extended, and a 5'-terminus sharing homologous sequences with the 3'-terminus of a hybrid linear polynucleotide or additional linear polynucleotide to be used in the assembly step.

The last step of the method to produce a circular vector according to the first aspect comprises:
- subjecting the two or more hybrid linear polynucleotides obtained in the overlap-extension PCR and optionally one or more additional linear polynucleotides to an assembly reaction yielding a circular vector comprising one or more functional guide-polynucleotide expression cassettes,
   wherein the assembly reaction occurs *in vivo.*

According to the invention the assembly reaction occurs in a host cell. In other words according to the invention the assembly reaction occurs *in vivo.* Assembly of the two or more hybrid linear polynucleotides obtained in the overlap-extension PCR and optionally one or more additional linear polynucleotides will occur *in vivo* by homologous recombination. This step exploits the homologous recombination-mediated double strand brake DNA repair pathway in the host cell where the assembly reaction occurs. A suitable host cell where the assembly reaction may occur is S. *cerevisiae.* Methods to produce linear or circular vectors exploiting homologous recombination mediated DNA repair pathways are knonw to those skilled in the art and e.g. described in Raymond *et al..*

"Homologous recombination" refers to a reaction between nucleotide sequences having corresponding sites containing a similar nucleotide sequence (i.e., homologous sequences) through which the molecules can interact (recombine) to form a new, recombinant nucleic acid sequence. The sites of similar nucleotide sequence are each referred to herein as a "homologous sequence". Generally, the frequency of homologous recombination increases as the length of the homology sequence increases. Thus, while homologous recombination can occur between two nucleic acid sequences that are less than identical, the recombination frequency (or efficiency) declines as the divergence between the two sequences increases.

Recombination may be accomplished using one homology sequence on each of two molecules to be combined, thereby generating a "single-crossover" recombination product. Alternatively, two homology sequences may be placed on each of two molecules to be recombined. Recombination between two homology sequences on the donor with two homology sequences on the target generates a "double-crossover" recombination product.

Therefore in order for two polynucleotide sequences to be recombined by homologous recombination with each other, both polynucleotides need to share a region of homology with each other. These regions of homology are called interchangeably herewith "flanking regions", "flanking sequences", "overlapping regions", "overlapping sequences", "homologous regions", "homologous sequences". In order for homologous recombination to take place the homologous sequences do not need to be identical. However the efficiency of homologous recombination increases with the level of sequence identity between the homolgous sequences. Preferably the homologous sequence will be at least 50% identical, preferably at least 60%, 70%, 80%, 85%, 90%, 95%, identical with each other, more preferably the homologous sequences will be 100% identical with each other. It is known to those skilled in the art that efficiency of homologous recombination increases with the length of the homologous sequences between the polynucleotides to be recombined. In one embodiment the homologous sequences are at least 10 bp long, preferably at least 20 bp, 30bp, 40bp, 50bp, 100bp, 500bp, 1000bp or more. It is known to those skilled in the art that while in *S*. *cerevisiae* the efficiency of homologous recombination is very high as double strand DNA brakes are mainly repaired by this mechanism, in other host cell homologous recombination is less efficient, requiring longer homologous sequences. In filamentous fungi said homologous sequences may be several thousands of nucleobases long. The skilled person will know how to design sufficiently long homologous sequences sharing sufficient sequence identity to allow efficient homologous recombination. Preferably the homologous sequences will share at least 80% sequence identity and will be at least 20 bp long. In a preferred embodiment the assembly reaction *in vivo* is performed in a host cell belonging to *S*. *cerevisiae.*

In an embodiment of the method to produce a circular vector comprising two or more guide-polynucleotide expression cassettes according to the first aspect, the two or more polynucleotide sequence to be extended or additional linear polynucleotide comprises one or more elements selected from the group consisting of: an origin of replication or a fragment thereof, a selectable marker or a fragment thereof, a CAS9 expression cassette or a fragment thereof, a donor polynucleotide or a fragment thereof. In another embodiment of the method to produce a circular vector comprising two or more guide-polynucleotide expression cassettes according to the first aspect, the two or more polynucleotide sequence to be extended or additional linear polynucleotide comprises one or more elements selected from the group consisting of: an origin of replication or a fragment thereof, a selectable marker or a fragment thereof, a CAS9 expression cassette or a fragment thereof, a donor polynucleotide or a fragment thereof, a control sequence or fragment thereof. Preferably the one or more elements comprise standardized and/or re-usable vector elements namely, standardized origin of replications or fragments thereof, standardised selectable marker cassettes or fragments thereof, standardised control sequences (e.g. standardised promoters or terminators) or fragments thereof.

An "origin of replication" is herewith defined as a sequence of DNA at which replication is initiated on a chromosome, plasmid or virus. The term "origin of replication" or "plasmid replicator" or " replicon" is defined herein as a nucleotide sequence that enables a plasmid or vector to replicate in vivo. For the vector according to the invention to be maintained in a host cell an origin of replication (ORI), also know as autonomously replicating sequence (ARS) or replicon is necessary. The autonomous replication sequence may be any suitable sequence available to the person skilled in the art that allows for plasmid replication that is independent of chromosomal replication. The origin of replication may be any plasmid replicator mediating autonomous replication that functions in a cell. Examples of bacterial origins of replication are the origins of replication of plasmids pBR322, pUC19, pACYC177, and pACYC184 permitting replication in *E*. *coli,* RSF1010 permitting replication in Pseudomonas is described, e.g., by F. Heffron et al., in Proc. Nat'l Acad. Sci. USA 72(9):3623-27 (Sep 1975), and pUB110, pE194, pTA1060, and pAMβ1 permitting replication in *Bacillus.* For the efficient expression of gRNA in yeast an ORI which leads to high copy number of the resulting plasmid is highly desirable. Preferred origin of replications used in S. *cerevisiae* is the 2-micron (2µ) plasmid sequence (Broach et al.). The autonomous replication sequence may be any suitable sequence available to the person skilled in the art that it confers to the plasmid replication that is independent of chromosomal replication. Preferably, the autonomous replication sequence is the *AMA1* replicon (Gems *et al*.).

A "selectable marker" is a gene which permit easy selection of transformed cells, the product of which provides for biocide or viral resistance, resistance to heavy metals, prototrophy to auxotrophs, and the like. The selectable marker may be introduced into the cell on the vector as an expression cassette or may be introduced on a separate vector. Some chromogenic or fluorescent protein may be used as selectable markers. There are many varieties of chromogenic or fluorescent proteins such as Red Fluorescent Protein (RFP), Green Fluorescent Protein (GFP), mCherry, dsRed, etcetera.

A selectable marker for use in a filamentous fungal cell may be selected from the group including, but not limited to, amdS (acetamidase), argB (ornithine carbamoyltransferase), bar (phosphinothricinacetyltransferase), bleA (phleomycin binding), hygB (hygromycinphosphotransferase), niaD (nitrate reductase), pyrG (orotidine-5'-phosphate decarboxylase), sC (sulfate adenyltransferase), NAT or NTC (Nourseothricin) and trpC (anthranilate synthase), KanMX (resistance to G418/geneticin; the selection marker kanMX is a hybrid gene consisting of a bacterial aminoglycoside phosphotransferase (kanr from transposon Tn903) under control of the strong TEF promoter from *Ashbya gossypii*; mammalian cells, yeast, and other eukaryotes acquire resistance to geneticin (= G418, an aminoglycoside antibiotic similar to kanamycin) when transformed with a kanMX marker; in yeast, the kanMX marker avoids the requirement of auxotrophic markers; in addition, the kanMX marker renders *E. coli* resistant to kanamycin.) as well as equivalents from other species. Preferred for use in an *Aspergillus* and *Penicillium* cell are the amdS (see for example EP 635574 B1, EP0758020A2, EP1799821A2, WO 97/06261A2) and pyrG genes of A. *nidulans* or *A*. *oryzae* and the bar gene of *Streptomyces hygroscopicus.* More preferably an amdS gene is used, even more preferably an amdS gene from *A. nidulans* or *A*. *niger.* A most preferred selectable marker gene is the *A.nidulans* amdS coding sequence fused to the A. *nidulans* gpdA promoter (see EP 635574 B1). Other preferred AmdS markers are those described in WO2006/040358. AmdS genes from other filamentous fungi may also be used (WO 97/06261).

Markers which can be used in a prokaryotic host cell include ATP synthetase, subunit 9 (*oliC*)*,* orotidine-5'-phosphatedecarboxylase *(pvrA),* , the ampicillin resistance gene (*E*. *coli),* resistance genes for neomycin, kanamycin, tetracycline, spectinomycin, erythromycin, chloramphenicol, phleomycin (*Bacillus*) and the *E. coli uidA* gene, coding for β-gtucuronidase (GUS). Vectors may be used *in vitro,* for example for the *in vitro* production of RNA in an *in vitro* transcription system or used to transfect or transform a host cell.

Versatile marker genes that can be used for transformation of most filamentous fungi and yeasts such as acetamidase genes or cDNAs (the *amdS, niaD, facA* genes or cDNAs from A. *nidulans, A. oryzae* or *A*. *niger*)*,* or genes providing resistance to antibiotics like G418, hygromycin, bleomycin, kanamycin, methotrexate, phleomycin orbenomyl resistance (benA). Alternatively, specific selection markers can be used such as auxotrophic markers which require corresponding mutant host strains: e. g. D-alanine racemase (from *Bacillus*)*,* URA3 (from *S*. *cerevisiae* or analogous genes from other yeasts), *pyrG* or *pyrA* (from *A*. *nidulans* or *A*. *niger), argB* (from *A*. *nidulans* or *A*. *niger)* or *trpC.*

The procedures used to ligate elements described above to construct a vector according to the present invention are well known to one skilled in the art (see, e.g. Sambrook & Russell, Molecular Cloning: A Laboratory Manual, 3rd Ed., CSHL Press, Cold Spring Harbor, NY, 2001; and Ausubel et al., Current Protocols in Molecular Biology, Wiley InterScience, NY, 1995).

A "CAS9 expression cassette" is a polynucleotide sequence comprising a polynucleotide coding for a CAS9 protein operably linked to control sequences which allow expression of said protein in a suitable host cell. Control sequences and operably linked have been herewith above defined.

A Cas protein in the context of all embodiments of the present invention refers to any Cas protein suitable for the purpose of the invention. A Cas protein may comprise enzymatic activity or may not comprise enzymatic activity. Non-limiting examples of Cas proteins include CasI, CasI B, Cas2, Cas3, Cas4, Cas5, Cas6, Cas7, Cas8, Cas9 (also known as CsnI and CsxI2), CaslO, Csyl, Csy2, Csy3, Csel, Cse2, Cscl, Csc2, Csa5, Csn2, Csm2, Csm3, Csm4, Csm5, Csm6, Cmrl, Cmr3, Cmr4, Cmr5, Cmr6, CsbI, Csb2, Csb3, CsxI7, CsxI4, CsxIO, CsxI6, CsaX, Csx3, CsxI, CsxIS, CsfI, Csf2, Csf3, Csf4, homologs thereof or modified versions thereof (Slaymaker et al 2016; Kleinstiver et al 2016; Nelson and Gersbach 2016). In alternative to a Cas protein as describred herein above, a Cpf1 protein (as described in Zetsche et al 2015) can also be used in the method according to the invention, optionally in combination with adaptation of the guide-polynucleotide expression cassette. These Cas proteins are known to the person skilled in the art; for example, the amino acid sequence of S. *pyogenes* Cas9 protein may be found in the SwissProt database under accession number Q99ZW2. Preferably, an unmodified Cas protein according to the present invention has DNA cleavage activity, such as e.g. Cas9. Preferably, a Cas protein according is Cas9, and may be Cas9 from *S*. *pyogenes* or *S*. *pneumoniae* or a Cas9 from *Staphylococcus aureus* (Ran et al 2015). Preferably, a Cas protein according to the present invention directs cleavage of one or both polynucleotide strands at the location of the target-polynucleotide, such as within the target-polynucleotide and/or within the reverse complement of the target-polynucleotide. At the location of the target-polynucleotide is herein defined as within about 1, 2, 3, 4, 5, 6, 7, 8, 9, 10, 15, 20, 25, 50, 100, 200, 500, or more nucleotides from the first or last nucleotide of a target-polynucleotide; more preferably, within 1, 2, 3, 4, 5, 6, 7, 8, 9, 10, 15, 20, 25, 50, 100, 200, 500, or more nucleotides from the first or last nucleotide of a target-polynucleotide; even more preferably, within 1, 2, 3, 4, 5, 6, 7, 8, 9, 10, 15, 20, 25, 50 nucleotides from the first or last nucleotide of a target-polynucleotide. Accordingly, a Cas protein according to the present invention preferably directs cleavage of one or both polynucleotide strands within about 1, 2, 3, 4, 5, 6, 7, 8, 9, 10, 15, 20, 25, 50, 100, 200, 500, or more nucleotides from the first or last nucleotide of a target-polynucleotide; more preferably, within 1, 2, 3, 4, 5, 6, 7, 8, 9, 10, 15, 20, 25, 50, 100, 200, 500, or more nucleotides from the first or last nucleotide of a target-polynucleotide; even more preferably, within 1, 2, 3, 4, 5, 6, 7, 8, 9, 10, 15, 20, 25, 50 nucleotides from the first or last nucleotide of a target-polynucleotide. Typically, a target-polynucleotide according to the present invention is associated with a PAM sequence (defined elsewhere herein) and the PAM sequence is preferably immediately downstream (3') of the target-sequence; the formation of the CRISPR-Cas complex typically results in cleavage of one or both polynucleotide strands 3 base pairs upstream (5') of the PAM sequence.

Preferably, a Cas protein has activity for directing cleavage of both polynucleotide strands at the location of the target-polynucleotide. Cas nuclease activity is typically performed by two separate catalytic domains, namely RuvC and HNH. Each domain cuts one polynucleotide strand each domain can be inactivated by a single point mutation. A Cas protein as defined herewith may thus conveniently be mutated with respect to a corresponding wild-type Cas protein such that the mutated Cas protein has altered nuclease activity and lacks the ability to cleave one or both strands of a target-polynucleotide. For example, an aspartate-to-alanine substitution (D10A) in the RuvC I catalytic domain of Cas9 from S. *pyogenes* converts Cas9 from a nuclease that cleaves both strands to a nickase, which is herein defined as a Cas protein that cleaves a single strand of a target-polynucleotide. Other examples of mutations that render Cas9 into a nickase include, but are not limited to H840A, N854A, and N863A. In the context of the present invention, a Cas protein having nickase activity may be used for genome editing via homologous recombination, preferably the double nicking technique according to Ran et al., 2013. Accordingly, a preferred Cas protein according to the present invention comprises at least one mutation, such that the protein has altered nuclease activity compared to the corresponding wild-type Cas protein, preferably having activity to direct cleavage of a single polynucleotide strand at the location of the target-sequence. Such so-called nickase mutant can conveniently be used in duplex set-up, i.e. in a composition according to the present invention comprising a Cas protein nickase mutant with RuvC mutated and a Cas protein nickase mutant wherein NHN is mutated, such that the one Cas protein mutant nicks one strand of the polynucleotide target and the other Cas protein mutant nicks the other strand of the polynucleotide target. Depending on the two guide-polynucleotides used, the two different CRISPR-Cas complexes will effectively result in two single-strand nicks in the polynucleotide target; these nicks may be several nucleotides up to 5, 10, 20, 30 or more apart. Such double nicking method greatly enhances specificity of NEJH. Background information on double nicking can be found in e.g. Ran et al, 2013.

A Cas protein according to the present invention may comprise two or more mutated catalytic domains of Cas9, such as RuvC I, RuvC II and/or RuvC III to result in a mutated Cas9 substantially lacking all DNA cleavage activity. In some embodiments, a D10A mutation is combined with one or more of H840A, N854A, or N863A mutations to produce a Cas9 enzyme substantially lacking all DNA cleavage activity. Preferably, a Cas protein is considered to substantially lack all DNA cleavage activity when the DNA cleavage activity of the mutated enzyme is less than about 25%, 10%, 5%, 1%, 0.1%, 0.01%, or lower with respect to its non-mutated form. A Cas protein lacking substantially all enzyme activity can conveniently be used for gene silencing or down regulation of expression since the CRISPR-CAS complex will hamper transcription from the target-polynucleotide. Other mutations may be useful; where the Cas9 or other Cas protein is from a species other than *S*. *pyogenes,* mutations in corresponding amino acids may be made to achieve similar effects; the person skilled in the art knows how to identify these corresponding amino acids.

A Cas protein according to the present invention may be a fusion protein and comprise at least one heterologous functional domain, such domain preferably is a domain comprising *Fok*I activity such as described by Aggarwal et al (Aggarwal, A. K.; Wah, D. A.; Hirsch, J. A.; Dorner, L. F.; Schildkraut, I. (1997). "Structure of the multimodular endonuclease Fokl bound to DNA". Nature 388 (6637): 97-100). The enzyme *Fok*I is naturally found in *Flavobacterium okeanokoites* and is a bacterial type IIS restriction endonuclease consisting of an N-terminal DNA-binding domain and a non-specific DNA cleavage domain at the C-terminal (Durai et al., 2005). When the *Fok*I protein is bound to double stranded DNA via its DNA-binding domain at the 5'-GGATG-3':3'-CATCC-5' recognition site, the DNA cleavage domain is activated and cleaves, without further sequence specificity, the first strand 9 nucleotides downstream and the second strand 13 nucleotides upstream of the nearest nucleotide of the recognition site (Wah et al., 1998. Cas9-*Fok*I fusions have been described *inter alia* in Guilinger et al., 2014; and in Tsai et al., 2014.

A Cas fusion protein according to the present invention may comprise 1, 2, 3, 4, 5, 6, 7, 8, 9, 10, or more domains in addition to the Cas protein. Examples of protein domains that may be fused to a Cas protein include, but are not limited to, epitope tags, reporter gene sequences, and protein domains having one or more of the following activities: methylase activity, demethylase activity, transcription activation activity, transcription repression activity, transcription release factor activity, historic modification activity, RNA cleavage activity and nucleic acid binding activity. Non-limiting examples of epitope tags include histidine (His) tags, V5 tags, FLAG tags, influenza hemagglutinin (HA) tags, Myc tags, VSV-G tags, and thioredoxin (Trx) tags. Examples of reporter genes include, but are not limited to, glutathione-S-transferase (GST), horseradish peroxidase (HRP), chloramphenicol acetyltransferase (CAT) beta-galactosidase, beta-glucuronidase, luciferase, green fluorescent protein (GFP), HcRed, DsRed, cyan fluorescent protein (CFP), yellow fluorescent protein (YFP), and autofluorescent proteins including blue fluorescent protein (BFP). A Cas protein may be fused to a gene sequence encoding a protein or a fragment of a protein that bind DNA molecules or bind other cellular molecules, including but not limited to, maltose binding protein (MBP), S-tag, Lex A DNA binding domain (DBD) fusions, GAL4 DNA binding domain fusions, and herpes simplex virus (HSV) BP 16 protein fusions. Additional domains that may form part of a fusion protein comprising a CRISPR enzyme are described in US20110059502. A tagged Cas protein may be used to identify the location of a target-polynucleotide. A preferred Cas fusion protein according to the present invention comprises a *Fok*I domain as defined here above.

A preferred Cas protein according to the present invention comprises a nuclear localization sequence, preferably a heterologous nuclear localization sequence. Such nuclear localization sequence is also referred as a nuclear localization signal. Preferably, such nuclear localization signal confers to the CRISPR-Cas complex sufficient strength to drive accumulation of said CRISPR-Cas complex in a detectable amount in the nucleus of a host cell. Without wishing to be bound by theory, it is believed that a nuclear localization sequence is not necessary for CRISPR-Cas activity in a host cell, but that including such sequences enhances activity of the system, especially as to targeting nucleic acid molecules into the nucleus. Such nuclear localization sequence is preferably present in the Cas protein, but may also be present anywhere else such that targeting of the CRISPR-Cas system to the nucleus is facilitated. A preferred nuclear localization sequence is the SV40 nuclear localization sequence. A polynucleotide coding for a Cas protein, e.g. a polynucleotide coding for a Cas9 protein, is preferably codon optimized for the host cell it is to be expressed in, more preferably the Cas protein encoding polynucleotide is codon pair optimized. In general, codon optimization refers to a process of modifying a nucleic acid sequence for enhanced expression in a host cell of interest by replacing at least one codon (e.g. more than 1, 2, 3, 4, 5, 10, 15, 20, 25, 50, or more codons) of a native sequence with codons that are more frequently or most frequently used in the genes of that host cell while maintaining the native amino acid sequence. Various species exhibit particular bias for certain codons of a particular amino acid. Codon bias (differences in codon usage between organisms) often correlates with the efficiency of translation of messenger RNA (mRNA), which is in turn believed to be dependent on, among other things, the properties of the codons being translated and the availability of particular transfer RNA (tRNA) molecules. The predominance of selected tRNAs in a cell is generally a reflection of the codons used most frequently in peptide synthesis. Accordingly, genes can be tailored for optimal gene expression in a given organism based on codon optimization. Codon usage tables are readily available, for example, at the "Codon Usage Database", and these tables can be adapted in a number of ways. See e.g. Nakamura, Y., et al., 2000. Computer algorithms for codon optimizing a particular sequence for expression in a particular host cell are also available, such as Gene Forge (Aptagen; Jacobus, PA), are also available. Preferably, one or more codons (e.g. 1, 2, 3, 4, 5, 10, 15, 20, 25, 50, or more, or all codons) in a sequence encoding a Cas protein correspond to the most frequently used codon for a particular amino acid. Preferred methods for codon optimization are described in WO2006/077258 and WO2008/000632). WO2008/000632 addresses codon-pair optimization. Codon-pair optimization is a method wherein the nucleotide sequences encoding a polypeptide have been modified with respect to their codon-usage, in particular the codon-pairs that are used, to obtain improved expression of the nucleotide sequence encoding the polypeptide and/or improved production of the encoded polypeptide. Codon pairs are defined as a set of two subsequent triplets (codons) in a coding sequence.

A "donor polynucleotide" as defined herewith is an exogenous polynucleotide, also referred to as donor DNA when the polynucleotide is a DNA, or repair template, that upon cleavage of the target-polynucleotide by the CRISPR-Cas complex recombines with the target-polynucleotide, resulting in a modified target-polynucleotide. Such exogenous polynucleotide is herein referred to as an exogenous polynucleotide or donor nucleotide according to the present invention and may be single-stranded or double-stranded. The exogenous polynucleotide may be present on a vector or may be present as such, may be encoded by another polynucleotide or may be operably linked to the guide-polynucleotide and may have sequence identity to a part of the target-polynucleotide upstream of the PAM associated with the guide-sequence (i.e. on the 5' side of the PAM) or may have sequence identity to a part of the target-polynucleotide downstream of the PAM associated with the guide-sequence (i.e. on the 5' side of the PAM). The donor polynucleotide may be comprised in a separate vector. A vector carrying an exogenous polynucleotide may be any vector described herein below. The exogenous polynucleotide may be present on a vector that comprises a polynucleotide encoding a Cas protein as defined herewith and/or comprising a guide-polynucleotide or a polynucleotide encoding a guide-polynucleotide as defined herewith.

In one embodiment according to the method to produce a circular vector comprising two or more guide polynucleotide expression cassettes, the circular vector obtained after assembly comprises one or more elements selected from the group consisting of: an origin of replication, a selectable marker, a CAS9 expression cassette, a donor polynucleotide or a combination of one or more of said elements.

In yet another embodiment according to the method to produce a circular vector comprising two or more guide polynucleotide expression cassettes, the circular vector obtained after assembly comprises an origin of replication, preferably an origin of replication and a selectable marker, even more preferably an origin of replication, a selectable marker and a CAS 9 expression cassette and/or a donor polynucleotide.

Accordingly, in an embodiment, the present invention provides for a vector wherein a polynucleotide encoding a Cas protein, a guide-polynucleotide or a polynucleotide encoding a guide-polynucleotide are present on a single vector, which may further comprise any elements necessary for expressing the encoded products such as promoter and terminator elements. Such single (all-in-one) vector has the advantage that all components necessary for a CRISPR-Cas system are present together; in addition, a single transformation event, optionally in combination with a donor polynucleotide, suffices to introduce the components into a host cell.

In another embodiment of the method to produce a circular vector according to the first aspect the vector, preferably the circular vector comprises two or more guide-polynucleotide expression cassettes.

Therefore the invention provides a method to produce a vector, preferably a circular vector comprising two or more guide-polynucleotide expression cassettes, wherein said two or more guide-polynucleotide expression cassettes comprise a polynucleotide encoding a guide-polynucleotide operably linked to one or more control sequences which direct the expression of said guide-polynucleotide in a host cell, wherein said guide-polynucleotide comprises a guide-sequence that essentially is the reverse complement of a target-polynucleotide in a host cell and wherein the guide-polynucleotide can direct binding of a Cas protein at a target-polynucleotide in a host cell to form a CRISPR-Cas complex, wherein the method preferably comprises the following steps:
- providing two or more polynucleotides, wherein the two or more polynucleotides comprise a polynucleotide sequence to be extended, wherein the polynucleotide sequence to be extended comprises at the 5'-end and/or at the 3'-end to be extended a fragment of a guide-polynucleotide expression cassette;
- performing two or more overlap-extension PCR reactions by subjecting in each reaction one of said two or more polynucleotides and two suitable polynucleotide primers to yield one hybrid linear polynucleotide,
   wherein the two or more polynucleotide sequence to be extended and the suitable polynucleotide primers are selected so that each hybrid linear polynucleotide obtained in the two or more overlap-extension PCR reactions comprises at least a polynucleotide coding for a guide sequence and suitable 5'-termini and 3'-termini which allow assembly *in vivo* of the two or more hybrid linear polynucleotides and optionally of one or more additional linear polynucleotides with each other in a pre-defined order to yield a vector, preferably a circular vector comprising one or more functional guide-polynucleotide expression cassettes;
- subjecting the two or more hybrid linear polynucleotides obtained in the overlap-extension PCR and optionally one or more additional linear polynucleotides to an assembly reaction yielding a vector, preferably a circular vector comprising two or more functional guide-polynucleotide expression cassettes,
   preferably wherein the assembly reaction occurs *in vivo.*

In a second aspect, the present invention provides a method of assembling a circular vector comprising two or more guide-polynucleotide expression cassettes *in vivo,* wherein said two or more guide-polynucleotide expression cassette comprises a polynucleotide encoding a guide-polynucleotide operably linked to one or more control sequences which direct the expression of said guide-polynucleotide in a host cell, wherein said guide-polynucleotide comprises a guide-sequence that essentially is the reverse complement of a target-polynucleotide in a host cell and the guide-polynucleotide can direct binding of a Cas protein at a target-polynucleotide in a host cell to form a CRISPR-Cas complex, wherein the method comprises the following steps:
- providing two or more linear polynucleotides, wherein said two or more linear polynucleotides comprise at the 5'-terminus and/or at the 3'-terminus at least one guide-polynucleotide expression cassette or a fragment thereof comprising at least the guide sequence;
- transforming said one or more linear polynucleotides, and optionally one or more additional linear polynucleotides into a host cell;
- allowing an homologous recombination reaction to take place between said one or more linear polynucleotides and optionally one or more additional linear polynucleotides, wherein said linear polynucleotides and additional linear polynucleotides have been selected to comprise suitable 5'-termini and 3'-termini which allow homologous recombination of said one or more linear polynucleotides and optionally of said one or more additional linear polynucleotides with each other in a pre-defined order to yield a vector, preferably a circular vector comprising one or more functional guide-polynucleotide expression cassettes;
- selecting a host cell comprising a circular vector and wherein said vector comprises one or more functional guide-polynucleotide expression cassettes;
- optionally recovering the vector, preferably the circular vector.

In a first step a method of assembling a circular vector comprising two or more guide-polynucleotide expression cassettes *in vivo* according to the second aspect of the invention comprises:
- providing two or more linear polynucleotides, wherein said two or more linear polynucleotides comprise at the 5'-terminus and/or at the 3'-terminus at least one guide-polynucleotide expression cassette or a fragment thereof comprising at least the guide sequence.

A guide-polynucleotide expression cassette, a CRISPR-CAS complex, a guide-polynucleotide, a guide-sequence, a target-polynucleotide, a CRISPR-CAS system, polynucleotide, a control sequence, "operably linked", promoter, fragment of a guide-polynucleotide expression cassette, homologous recombination, homologous sequence, have been defined herewith above in the first aspect of the invention. These definitions are valid throughout the whole specification.

The one or more linear polynucleotides are polynucleotides as defined herewith which comprise at the 5'-terminus and/or at the 3'-terminus at least one guide-polynucleotide expression cassette or a fragment thereof comprising at least the guide sequence.

The one or more linear polynucleotides, the one or more polynucleotides comprising a polynucleotide sequence to be extended and the one ore more additional linear polynucleotides as defined herewith may be produced according to methods known in the art. E.g the polynucleotides suitable for use in the invention may typically be generated by any amplification process known in the art (e.g., PCR, RT-PCR and the like).

The one or more linear polynucleotides and optionally the one or more additional polynucleotides used in the second aspect may comprise one or more elements selected from the group consisting of: an origin of replication or a fragment thereof, a selectable marker or a fragment thereof, a CAS9 expression cassette or a fragment thereof, a donor polynucleotide or a fragment thereof, as defined herein before.

In a second step, a method of assembling a circular vector comprising two or more guide-polynucleotide expression cassettes *in vivo* according to the second aspect of the invention comprises:
- transforming said one or more linear polynucleotides, and optionally one or more additional linear polynucleotides into a host cell.

Transformation of said one or more linear polynucleotides and optionally one or more additional linear polynucleotides into a host cell may occur according to various techniques known to those skilled in the art. Non-limiting examples of methods used to introduce heterologus nucleic acids into various organisms include; transformation, transfection, transduction, electroporation, ultrasound-mediated transformation, particle bombardment, microprojectile bombardment, protoplast method, *Agrobacterium* mediated transformation (AMT) and the like. In some instances the addition of carrier molecules can increase the uptake of DNA in cells typically thought to be difficult to transform by conventional methods. Conventional methods of transformation are readily available to the skilled person. Preferably the protoplast method is used for filamentous fungi. Procedures for transformation are *inter alia* described by J.R.S. Fincham, Transformation in fungi. 1989, Microbiological reviews. 53, 148-170. Transformation may involve a process consisting of protoplast formation, transformation of the protoplasts, and regeneration of the cell wall in a manner known *per se.* Suitable procedures for transformation of *Aspergillus* cells are described in EP 238 023 and Yelton et al., 1984, Proceedings of the National Academy of Sciences USA 81:1470-1474. Suitable procedures for transformation of *Aspergillus* and other filamentous fungal host cells using *Agrobacterium tumefaciens* are described in e.g. De Groot et al., Agrobacterium tumefaciens-mediated transformation of filamentous fungi. Nat Biotechnol. 1998, 16:839-842. Erratum in: Nat Biotechnol 1998 16:1074. A suitable method of transforming *Fusarium* species is described by Malardier et al., 1989, Gene 78:147156 or in WO 96/00787. Other methods can be applied such as a method using biolistic transformation as described in: Christiansen et al., Biolistic transformation of the obligate plant pathogenic fungus, Erysiphe graminis f.sp. hordei. 1995, Curr Genet. 29:100-102. Yeast may be transformed using any method known in the art such as the procedures described by Becker and Guarente, In Abelson, J. N. and Simon, 1983; Hinnen et al., 1978, and Gietz RD, Woods RA. 2002.

In a third step, a method of assembling a circular vector comprising one or more guide-polynucleotide expression cassettes *in vivo* according to the second aspect of the invention comprises:
- allowing an homologous recombination reaction to take place between said one or more linear polynucleotides and optionally one or more additional linear polynucleotides, wherein said linear polynucleotides and additional linear polynucleotides have been selected to comprise suitable 5'-termini and 3'-termini which allow homologous recombination of said one or more linear polynucleotides and optionally of said one or more additional linear polynucleotides with each other in a pre-defined order to yield a vector, preferably a circular vector comprising one or more functional guide-polynucleotide expression cassettes.

The one more more linear polynucleotides and one more more additional linear polynucleotides have been selected to comprise suitable 5'-termini and 3'-termini which allow homologous recombination of said one or more linear polynucleotides and optionally of said one or more additional linear polynucleotides with each other in a pre-established order. How to select linear polynucleotides and additional linear polynucleotides to comprise suitable 5'-termini and 3'-termini to allow homologous recombination is performed by providing the two polynucleotide sequences to be recombined by homologous recombination with each other, with homologous sequences as defined herein before. Preferably the homologous sequence will be at least 50% identical, preferably at least 60%, 70%, 80%, 85%, 90%, 95%, identical with each other, more preferably the homologous sequences will be 100% identical with each other. In one embodiment the homologous sequences are at least 10 bp long, preferably at least 20 bp, 30bp, 40bp, 50bp, 100bp, 500bp, 1000bp or more. The skilled person will know how to design sufficiently long homologous sequences sharing sufficient sequence identity to allow efficient homologous recombination in the host cell. Preferably the homologous sequences will share at least 80% sequence identity and will be at least 20 bp long. In a preferred embodiment the method of the second aspect of the invention is performed in a host cell belonging to *S*. *cerevisiae.*

In a fourth optional step, a method of assembling a circular vector comprising one or more guide-polynucleotide expression cassettes *in vivo* according to the second aspect of the invention comprises:
- optionally selecting a host cell comprising a circular vector and wherein said vector comprises two or more functional guide-polynucleotide expression cassettes.

In a fifth optional step, a method of assembling a circular vector comprising two or more guide-polynucleotide expression cassettes *in vivo* according to the second aspect of the invention comprises:
- optionally recovering the circular vector.

Selection of host cells comprising the circular vector may be performed with various techniques knonw to those skilled in the art. Typically the one or more linear polynucleotides and optionally one or more additional linear polynucleotides used in the assembly reaction will comprise one or more selectable markers which are incorporated in the polynucleotide sequence of the circular vector and allow differential selection of host cells comprising a vector comprising all of the one or more functional guide-polynucleotide expression cassettes from other vectors.

Once the host cell comprising the circular vector have been slected, the circular vector may be recovered therefrom according to methods known to those skilled in the art. Typically host cell comprising the correct vector may be propagated in a suitable medium, after sufficient cell growth the culture may be harvested, host cell may be lysed and the circular vector may be purified from other cellular components, e.g. by chromatography such as chromatography using silica gel or anion exchange resins. The vector can be further recovered after chromatography using standard techniques, such as ethanol precipitation.

In one embodiment of the method according to the second aspect the circular vector comprises two or more guide-polynucleotide expression cassettes, preferably the method comprises the following steps:
- providing two or more polynucleotides, wherein the two or more polynucleotides comprise a polynucleotide sequence to be extended, wherein the polynucleotide sequence to be extended comprises at the 5'-end and/or at the 3'-end to be extended a fragment of a guide-polynucleotide expression cassette;
- performing two or more overlap-extension PCR reactions by subjecting in each reaction one of said two or more polynucleotides and two suitable polynucleotide primers to yield one hybrid linear polynucleotide,
   wherein the two or more polynucleotide sequence to be extended and the suitable polynucleotide primers are selected so that each hybrid linear polynucleotide obtained in the two or more overlap-extension PCR reactions comprises at least a polynucleotide coding for a guide sequence and suitable 5'-termini and 3'-termini which allow assembly *in vivo* of the two or more hybrid linear polynucleotides and optionally of one or more additional linear polynucleotides with each other in a pre-defined order to yield a vector, preferably a circular vector comprising one or more functional guide-polynucleotide expression cassettes;
- subjecting the two or more hybrid linear polynucleotides obtained in the overlap-extension PCR and optionally one or more additional linear polynucleotides to an assembly reaction yielding a vector, preferably a circular vector comprising two or more functional guide-polynucleotide expression cassettes,
   preferabaly wherein the assembly reaction occurs *in vivo.*

In a third aspect the invention provides a method for producing a recombinant host cell comprising a circular vector, wherein said vector comprises two or more guide-polynucleotide expression cassettes, wherein said two or more guide-polynucleotide expression cassette comprises a polynucleotide encoding a guide-polynucleotide operably linked to one or more control sequences which direct the expression of said guide-polynucleotide in a host cell, wherein said guide-polynucleotide comprises a guide-sequence that essentially is the reverse complement of a target-polynucleotide in a host cell and the guide-polynucleotide can direct binding of a Cas protein at a target-polynucleotide in a host cell to form a CRISPR-Cas complex, wherein the method comprises the following steps:
- providing one or more linear polynucleotides, wherein said one or more linear polynucleotides comprise at the 5'-terminus and/or at the 3'-terminus at least one guide-polynucleotide expression cassette or a fragment thereof comprising at least the guide sequence;
- transforming said one or more linear polynucleotides, and optionally one or more additional linear polynucleotides into a host cell;
- allowing an homologous recombination reaction to take place between said one or more linear polynucleotides and optionally one or more additional linear polynucleotides, wherein said linear polynucleotides and additional linear polynucleotides have been selected to comprise suitable 5'-termini and 3'-termini which allow homologous recombination of said one or more linear polynucleotides and optionally of said one or more additional linear polynucleotides with each other in a pre-defined order to yield a vector, preferably a circular vector comprising one or more functional guide-polynucleotide expression cassettes;
- optionally selecting a host cell comprising a circular vector and wherein said vector comprises one or more functional guide-polynucleotide expression cassettes
   wherein the one or more linear polynucleotides are hybrid linear polynucleotides obtained by
- providing two or more polynucleotides, wherein the two or more polynucleotides comprise a polynucleotide sequence to be extended, wherein the polynucleotide sequence to be extended comprises at the 5'-end and/or at the 3'-end to be extended a fragment of a guide-polynucleotide expression cassette, preferably wherein the fragment of a guide-polynucleotide expression cassette at the 5'-end and/or at the 3'-end of the polynucleotide sequence to be extended, does not comprise a polynucleotide coding for a guide sequence;
- performing two or more overlap-extension PCR reactions by subjecting in each reaction one of said two or more polynucleotides and two suitable polynucleotide primers to yield one hybrid linear polynucleotide,
   wherein the two or more polynucleotide sequence to be extended and the suitable polynucleotide primers are selected so that each hybrid linear polynucleotide obtained in the two or more overlap-extension PCR reactions comprises at least a polynucleotide coding for a guide sequence and suitable 5'-termini and 3'-termini which allow assembly *in vivo* of the two or more hybrid linear polynucleotides and optionally of one or more additional linear polynucleotides with each other in a pre-defined order to yield a circular vector comprising one or more functional guide-polynucleotide expression cassettes.

The first four steps of the method according to the third aspect may be performed in analogy with the corresponding steps of the method according to the second aspect of the invention.

In one embodiment, the method according to the third aspect of the invention further comprises the steps of:
- recovering the circular vector;
- transforming the circular vector in a second host cell;
- optionally isolating the second host cell comprising the circular vector.

Recovery of the circular vector in the method according to the third aspect, transformation of the circular vector in a second host cell and isolation of the second host cell comprising the the circular vector may be performed according to methods analogous to those described herein above.

In a fourth aspect, the invention provides a method for producing a recombinant host cell comprising a circular vector, wherein said vector comprises two or more guide-polynucleotide expression cassettes, wherein said two or more guide-polynucleotide expression cassette comprises a polynucleotide encoding a guide-polynucleotide operably linked to one or more control sequences which direct the expression of said guide-polynucleotide in a host cell, wherein said guide-polynucleotide comprises a guide-sequence that essentially is the reverse complement of a target-polynucleotide in a host cell and the guide-polynucleotide can direct binding of a Cas protein at a target-polynucleotide in a host cell to form a CRISPR-Cas complex, wherein the method comprises the following steps:
- perforimg a method of assembling a circular vector comprising one or more guide-polynucleotide expression cassettes *in vivo* according to the second aspect in a first host cell, preferably a first host cell belonging to the species *S*. *cerevisiae,* wherein said first host cell comprising a circular vector, is selected and the circular vector is recovered, werein said vector comprises one or more functional guide-polynucleotide expression cassettes,
- transforming the circular vector in a second host cell;
- optionally isolating the second host cell comprising the the circular vector.

Recovery of the circular vector in the method according to the forth aspect, transformation of the vector in a second host cell and isolation of the second host cell comprising the vector may be performed according to methods analogous to those described herein above.

In one embodiment of the method according to the third or fourth aspect, the one or more linear polynucleotides are hybrid linear polynucleotides as defined herein before in the first aspect of the invention, obtained by
- providing one or more polynucleotides, wherein the one or more polynucleotides comprise a polynucleotide sequence to be extended, wherein the polynucleotide sequence to be extended comprises at the 5'-end and/or at the 3'-end to be extended a fragment of a guide-polynucleotide expression cassette;
- performing one or more overlap-extension PCR reactions by subjecting in each reaction one of said one or more polynucleotides and two suitable polynucleotide primers to yield one hybrid linear polynucleotide,
   wherein the one or more polynucleotide sequence to be extended and the suitable polynucleotide primers are selected so that each hybrid linear polynucleotide obtained in the one or more overlap-extension PCR reactions comprises at least a polynucleotide coding for a guide sequence and suitable 5'-termini and 3'-termini which allow assembly *in vivo* of the one or more hyrbird linear polynucleotides and optionally of one or more additional linear polynucleotides with each other in a pre-defined order to yield a circular vector comprising one or more functional guide-polynucleotide expression cassettes.

In a preferred embodiment of the method according to the third or forth aspect of the invention, said performing one or more overlap-extension PCR reactions by subjecting in each reaction one of said one or more polynucleotides and two suitable polynucleotide primers to yield one hybrid linear polynucleotide,
wherein the one or more polynucleotide sequence to be extended and the suitable polynucleotide primers are selected so that each hybrid linear polynucleotide obtained in the one or more overlap-extension PCR reactions comprises at least a polynucleotide coding for a guide sequence and suitable 5'-termini and 3'-termini which allow assembly *in vivo* of the one or more hybrid linear polynucleotides and optionally of one or more additional linear polynucleotides with each other to yield a circular vector comprising one or more functional guide-polynucleotide expression cassettes, comprises the steps of:
a) subjecting in each reaction one of said one or more polynucleotides and one suitable polynucleotide primer to overlap-extension PCR reaction, wherein the 3'-terminus of said polynucleotide primer contains a complementary sequence to the 3'-end of the polynucleotide sequence to be extended comprised in said one or more polynucleotide or wherein the 3'-terminus of the complement of said polynucleotide primer contains a complementary sequence to the 3'-end of the complement of the polynucleotide sequence to be extended comprised in said one or more polynucleotide, respectively, to yield one hybrid linear polynucleotide having a sequence comprising the polynucleotide sequence to be extended spliced at its 3'-terminus or at its 5'-terminus, respectively, with the polynucleotide sequence of the suitable primer,
b) optionally subjecting the hybrid linear polynucleotide obtained in step a) and another suitable primer to a second overlap-extension PCR reaction, wherein the 3'-terminus of said polynucleotide primer contains a complementary sequence to the 3'-end of the hybrid linear polynucleotide having a sequence comprising the polynucleotide sequence to be extended spliced at its 3'-terminus or at its 5'-terminus, respectively, with the polynucleotide sequence of the suitable primer, or wherein the 3'-terminus of the complement of said polynucleotide primer contains a complementary sequence to the 3'-end of the complement of the hybrid linear polynucleotide having a sequence comprising the polynucleotide sequence to be extended spliced at its 3'-terminus or at its 5'-terminus, respectively, with the polynucleotide sequence of the other suitable primer, respectively, to yield one hybrid linear polynucleotide having a sequence comprising the polynucleotide sequence to be extended spliced at its 3'-terminus and at its 5'-terminus, respectively, with the polynucleotide sequences of the suitable primers,
   wherein the one or more polynucleotide sequence to be extended and the suitable polynucleotide primers are selected so that each hybrid linear polynucleotide obtained in the one or more overlap-extension PCR reactions comprises at least a polynucleotide coding for a guide sequence and suitable 5'-termini and 3'-termini which allow assembly *in vivo* of the one or more hybrid linear polynucleotides and optionally of one or more additional linear polynucleotides with each other in a pre-defined order to yield a vector, preferably the circular vector comprising one or more functional guide-polynucleotide expression cassettes.

In a preferred embodiment according to the method of the third or forth aspect of the invention the assembly reaction occurs in a host cell belonging to *S*. *cerevisiae* species.

In one embodiment of the method according to the third or forth aspect the second host cell is a prokaryotic, such as a bacterial host cell, or an eukaryotic host cell, preferably a fungal host cell. E. g. the the second host cell is a prokaryotic host cell selected from *Escherichia, Anabaena, Caulobactert, Gluconobacter, Rhodobacter, Pseudomonas, Paracoccus, Bacillus, Brevibacterium, Corynebacterium, Rhizobium (Sinorhizobium), Flavobacterium, Klebsiella, Enterobacter, Lactobacillus, Lactococcus, Methylobacterium, Staphylococcus or Streptomyces.* Alternatively, the second host cell is a fungal host cell, more preferably a filamentous fungal host cell, most preferably a filamentous fungal host cell selected from *Acremonium, Agaricus, Aspergillus, Aureobasidium, Chrysosporium, Coprinus, Cryptococcus, Filibasidium, Fusarium, Humicola, Magnaporthe, Mortierella, Mucor, Myceliophthora, Neocallimastix, Neurospora, Paecilomyces, Penicillium, Piromyces, Panerochaete, Pleurotus, Schizophyllum, Talaromyces, Rasamsonia, Thermoascus, Thielavia, Tolypocladium, and Trichoderma.* In yet another embodiment the fungal host cell is a yeast host cell, preferably a yeast host cell selected from *Candida, Hansenula, Issatchenkia, Kluyveromyces, Pichia, Saccharomyces, Schizosaccharomyces, Yarrowia or Zygosaccharomyces.*

According to one embodiment of any one of the aspects according to the invention the cicular vector comprising one or more functional guide polynucleotides expression cassettes comprises at least 2 or more functional guide polynucleotide expression cassettes, preferably at least 3, 4, 5, 6 or more functional guide polynucleotide expression cassettes.

In the context of all embodiments of the present invention, a vector may be any vector (e.g., a plasmid or virus), which can conveniently be subjected to recombinant DNA procedures and can mediate expression of the one or more guide polynucleotide expression cassettes. The choice of the vector will typically depend on the compatibility of the vector with the host cell into which the vector is to be introduced. Preferred vectors are the vectors used in the examples herein. A vector according to the fifth aspect of the invention is preferably a closed circular plasmid. In other contexts a vector may either be a circular or may be a linear polynucleotide. A vector may be an autonomously replicating vector, i.e., a vector, which exists as an extra-chromosomal entity, the replication of which is independent of chromosomal replication, e.g., a plasmid, an extra-chromosomal element, a mini-chromosome, or an artificial chromosome.

Preferably, the vector is an autonomously replicating vector, preferably a 2µ vector (for yeast) or an AMA-vector (for filamentous fungi). An AMA-vector preferably comprise the AMA1-sequence (see e.g. Aleksenko and Clutterbuck 1997) or a functional variant or equivalent thereof. A vector is preferably not integrated into the genome of the host cell. Preferably the vector according to the fourth aspect is not an integrative vector. A vector according to the fourth aspect of the invention may contain one or more selectable markers, which permit easy selection of transformed cells. The vector comprising one or more guide-polynucleotide expression cassettes, assembled *in vivo* according to the methods of the invention is preferably an autonomously replicating circular vector. A recombinant host cell comprising a vector, preferably a circular vector comprising one or more functional guide polynucleotides expression cassettes may be any host cell as defined herein e.g.in the general definitions. When the host cell is a host cell wherein the assembly of the vector, preferably the circular vector occurs, said host cell is preferebaly a *S*. *cerevisiae* host cell. A preferred host cell is a modified host cell wherein expression of a component associated with non-homologous end joining (NHEJ) is altered compared to the corresponding wild-type host cell; preferably expression of the component associated with NHEJ is lowered. Preferred components associated with NHEJ are the yeast Ku70 and Ku80 and their respective orthologs in preferred non-mammalian host cells according to the present invention. Another preferred component associated with NHEJ is the yeast LIG4 and its respective orthologs in preferred non-mammalian host cells according to the present invention.

The invention provides a non-naturally occurring or engineered composition comprising a source of a CRISPR-Cas system comprising a guide-polynucleotide and a Cas protein, wherein the guide-polynucleotide comprises a guide-sequence that essentially is the reverse complement of a target-polynucleotide in a host cell and the guide-polynucleotide can direct binding of the Cas protein at the target-polynucleotide in the host cell to form a CRISPR-Cas complex, wherein the guide-sequence is essentially the reverse complement of the (N)y part of a 5'-(N)yPAM-3' polynucleotide sequence target in the genome of the host cell, wherein y is an integer of 8-30, more preferably 10-30, more preferably 15-30, more preferably 17-27, more preferably 17-20, more preferably 17, 18, 19, 20, 21, 22, 23, 24, 25, 26, or 27, wherein PAM is a protospacer adjacent motif, wherein the host cell is a prokaryote or a eukaryote, which eukaryote is a filamentous fungus, preferably an Aspergillus, a Penicillium or a Rasamsonia or a Mortierella; or a yeast, preferably a Yarrowia, a Saccharomyces or a Kluyveromyces or Pichia or Issatchenkia; and wherein PAM is preferably a sequence selected from the group consisting of 5'-XGG-3', 5'-XGGXG-3', 5'-XXAGAAW-3', 5'-XXXXGATT-3', 5'-XXAGAA-3', 5'-XAAAAC-3', wherein X can be any nucleotide or analog thereof, preferably X can be any nucleotide; and W is A or T, wherein the guide-polynucleotide is comprised in a vector, preferably a circular vector according to the fifth aspect.

The composition, source, CRISPR-Cas system, guide-polynucleotide, Cas protein, target-polynucleotide, host cell and CRISPR-Cas complex are herein referred to as a composition, source, CRISPR-Cas system, guide-polynucleotide, Cas protein, target-polynucleotide, host cell and CRISPR-Cas complex according to the present invention. For the sake of completeness, since "a" is defined elsewhere herein as "at least one", a composition according to the present invention comprises a source of at least one, i.e. one, two, three or more guide-polynucleotides and/or at least one, i.e. one, two, three or more Cas proteins. Accordingly, the present invention conveniently provides for a multiplex CRISPR-Cas system. Such multiplex CRISPR-Cas system can conveniently be used for introduction of a donor polynucleotide, deletion of a polynucleotide and polynucleotide library insertion into the genome of a host cell.

The term "non-naturally occurring composition" refers in the context of all embodiments of the present invention to a composition that in its form used in the present invention does not occur in nature. The individual elements may e.g. occur as such or in combinations with other elements in nature, but the non-naturally occurring composition comprises e.g. at least one element more or less than a naturally composition.

The term "engineered composition" refers in the context of all embodiments of the present invention to a composition wherein at least one of the elements has been engineered, i.e. modified by man, in such a way that resulting element does not occur in nature. It follows that by virtue of comprising at least one engineered element, an engineered composition does not occur in nature.

The term "hybridization" refers to a reaction in which one or more polynucleotides react to form a complex that is stabilized via hydrogen bonding between the bases of the nucleotide residues. The hydrogen bonding may occur by Watson Crick base pairing, Hoogstein binding, or in any other sequence-specific manner. The complex may comprise two strands forming a duplex structure, three or more strands forming a multi stranded complex, a single self hybridizing strand, or any combination of these. A hybridization reaction may constitute a step in a more extensive process, such as the cleavage of a polynucleotide by an enzyme. Preferred hybridization conditions are physiological conditions as within a host cell according to the present invention.

The term "source" in the context of all embodiments of the present invention refers to any source of a CRISPR-Cas system comprising a guide-polynucleotide and a Cas protein. The guide-polynucleotide and Cas protein may be present in separate sources. In such case, the composition according to the present invention comprises a CRISPR-Cas system comprising a source of a guide-polynucleotide and a source of a Cas-protein. Any source means that the guide-polynucleotide and Cas protein may be present as such in a form that they can function within a CRISPR-Cas system. The guide-polynucleotide and/or the Cas-protein may be provided in its active forms and may e.g. be provided from an inactive form or from another entity. The guide-polynucleotide may e.g. be present on another polynucleotide or may be encoded by a polynucleotide that is transcribed to provide for the actual guide-polynucleotide. The Cas protein may be encoded by a polynucleotide (e.g. DNA or mRNA) that is transcribed and/or translated to provide the actual Cas protein. An encoding polynucleotide may be present in a nucleic acid construct as defined herein and/or in a vector as defined herein. Such nucleic acid construct and vector are herein referred to as a nucleic acid construct according to the present invention and a vector according to the present invention.

Preferably, in the composition, the Cas protein is encoded by a polynucleotide and/or the guide-polynucleotide is encoded by or present on a polynucleotide.

Preferably, in the composition, the Cas protein is encoded by a polynucleotide and/or the guide-polynucleotide is encoded by or present on another polynucleotide and the polynucleotide or polynucleotides are comprised in a vector.

Preferably, in the composition, the Cas protein is encoded by a polynucleotide and the guide-polynucleotide is encoded by or present on another polynucleotide and the polynucleotides are comprised in one vector.
Preferably, in the composition, the Cas protein is encoded by a polynucleotide comprised in a vector and the guide-polynucleotide is encoded by or present on another polynucleotide comprised in another vector.

The invention thus provides for the possibilities that the guide-polynucleotide and the Cas protein are provided as such, or that they are encoded on or present on a vector, such as the vector according to the fifth aspect of the invention. In the latter case, the encoding polynucleotides may each be on a separate vector or may both be on a single vector.

The amount of Cas protein in a source in a composition according to the present invention may vary and may be optimized for optimal performance. It may be convenient to avoid too high levels of Cas protein in a host cell since high levels of Cas protein may be toxic to the host cell, even without a guide-polynucleotide present (see e.g. Ryan et al 2014 and Jacobs et al., 2014). A person skilled in the art knows how to regulate expression levels, such as by choosing a weaker promoter or inducible promoter for expression of the Cas protein. Examples of promoters suitable for expression of a protein are depicted elsewhere herein.

In a composition wherein a guide-polynucleotide according to the present invention is encoded by a polynucleotide, expression of the guide-polynucleotide may be facilitated by a promoter operably linked to the encoding polynucleotide. Such promoter may be any suitable promoter known to the person skilled in the art. Several types of promoters can be used. It may be convenient to use an RNA polymerase III promoter or an RNA polymerase II promoter. Background information on RNA polymerase III and its promoters can be found e.g. in Marck et al., 2006. In some cases, such as in *S*. *cerevisiae, S. pombe,* RNA polymerase III promoters include promoter elements in the transcribed region. Accordingly, it may be convenient to use an RNA polymerase II promoter; these are known to the person skilled in the art and reviewed in e.g. Kornberg 1999. However, transcripts from an RNA II polymerase often have complex transcription terminators and transcripts are polyadenylated; this may hamper with the requirements of the guide-polynucleotide which because both its 5' and 3' ends need to be precisely defined in order to achieve the required secondary structure to produce a functional CRISPR-Cas system. These drawbacks can however be circumvented. In case an RNA polymerase II promoter is used, the polynucleotide encoding the guide-polynucleotide may also encode self-processing ribozymes and may be operably linked to an RNA polymerase II promoter; as such the polynucleotide encodes a pre-guide-polynucleotide comprising the guide-polynucleotide and self-processing ribozymes, wherein, when transcribed, the guide-polynucleotide is released by the self-processing ribozymes from the pre-guide-polynucleotide transcript. Preferred constructs comprising a polynucleotide encoding a pre-guide-polynucleotide according to the present invention operably linked to an RNA polymerase II promoter are those depicted in examples 8 - 17 and 18 - 34 herein. Background information on such constructs can be found in e.g. Gao et al, 2014 et al.
Preferably, in a composition wherein the guide-polynucleotide is encoded by a polynucleotide, said polynucleotide is operably linked to an H1 RNA polymerase III promoter, preferably a human H1 RNA polymerase III promoter.

Preferably, in a composition wherein the guide-polynucleotide is encoded by a polynucleotide, said polynucleotide is operably linked to a U6 RNA polymerase III promoter, preferably a human U6 RNA polymerase III promoter.

Preferably, in a composition wherein the guide-polynucleotide is encoded by a polynucleotide, said polynucleotide is operably linked to an SNR52p RNA polymerase III promoter, preferably a yeast SNR52p RNA polymerase III promoter. Such promoter is preferably used when the host is a yeast host cell, such as a *Saccharomyces* or a *Kluyveromyces.*

Preferably, in a composition wherein the guide-polynucleotide is encoded by a polynucleotide, said polynucleotide is operably linked to an RNA polymerase II promoter and encodes a pre-guide-polynucleotide comprising the guide-polynucleotide and self-processing ribozymes, wherein, when transcribed, the guide-polynucleotide is released by the self-processing ribozymes from the pre-guide-polynucleotide transcript.

The composition can conveniently be used to modulate expression of a polynucleotide in a host cell. Accordingly, disclosed is a method of modulating expression of a polynucleotide in a host cell, comprising contacting a host cell with the composition, wherein the guide-polynucleotide directs binding of the Cas protein at the target-polynucleotide in the host cell to form a CRISPR-Cas complex, preferably wherein the host cell comprises a polynucleotide encoding a compound of interest.

The term "expression" in the context of the present invention is herein defined as the process by which a polynucleotide is transcribed from a polynucleotide template (e.g. a DNA template polynucleotide is transcribed into an mRNA polynucleotide transcript or other RNA transcript) and/or the process by which an mRNA transcript is subsequently translated into peptides, polypeptides, or proteins. Transcripts and encoded polypeptides may be collectively referred to as "gene product". If the polynucleotide transcript is derived from a genomic template DNA, expression may include splicing of the mRNA transcript in a host cell. The term "modulating expression" refers herein to increased or reduced expression compared to a parent host cell wherein expressing is not modulated when assayed using the same conditions. Reduced expression may be a reduced amount of transcript such as mRNA and/or a reduced amount of translation product such as a polypeptide. It follows that increased expression may be an enhanced amount of transcript such as mRNA and/or an enhanced amount of translation product such as a polypeptide.

Preferably, the CRISPR-Cas complex cleaves one or both polynucleotide strands at the location of the target-polynucleotide, resulting in modulated expression of the gene product. The CRISPR-Cas complex may also have altered nuclease activity and substantially lack the ability to cleave one or both strands of a target-polynucleotide; in such case, expression is modulated by the binding of the complex to the target-polynucleotide. A Cas protein lacking substantially all enzyme activity can conveniently be used for gene silencing or down regulation of expression since the CRISPR-Cas complex will hamper transcription from the target-polynucleotide. Alternatively, a Cas protein can be modified into a transcription factor for programmable transcriptional activation or silencing of a gene of interest (Larson, et al., 2013).

A composition can conveniently be used for the deletion of polynucleotide. In an embodiment, when the composition according to the eight aspect of the present invention comprises a source of at least one or two guide-polynucleotides and/or a source of at least at least one Cas protein, at least one CRISPR-Cas complex or two different CRISPR-Cas complexes are formed that cleave one or both polynucleotide strands at one location or at different locations of the target-polynucleotide, resulting in deletion of a polynucleotide fragment from the target-polynucleotide. Preferably, such composition according to the present invention comprising at least one or two guide-polynucleotides and/or a source of at least at least one Cas protein, additionally comprises an exogenous polynucleotide as defined herein below that is at least partly complementary to the at least one or two target-polynucleotides targeted by the guide-polynucleotide(s). Such polynucleotide fragment to be deleted or deleted fragment may be several nucleotides in length up to a few thousand nucleotides in length, an entire gene may be deleted or a cluster of genes may be deleted. Accordingly, the present invention provides for a method of modulating expression of a polynucleotide in a host cell, wherein a polynucleotide fragment is deleted from a target-polynucleotide.

The method of modulating expression comprises cleavage of one or both polynucleotide strands at least one location of the target-polynucleotide followed by modification of the target-polynucleotide by homologous recombination with an exogenous polynucleotide. In such case, the composition preferably further comprises such exogenous polynucleotide. Such modification may result in insertion, deletion or substitution of at least one nucleotide in the target-polynucleotide, wherein the insertion or substitution nucleotide may originate from the exogenous polynucleotide. A modification can also be made when the exogenous polynucleotide is a non-integrating entity such as described in Dong et al., and Beetham et al.; in this case the target-polynucleotide is modified but no nucleotide of the exogenous polynucleotide is introduced into the target-polynucleotide. Consequently, the resulting host is a non-recombinant host cell when the Cas-protein is transformed as a protein. The exogenous polynucleotide may be any polynucleotide of interest such as a polynucleotide encoding a compound of interest as defined herein below, or a part of such polynucleotide or a variant thereof. Such exogenous polynucleotide is herein referred to as an exogenous polynucleotide and may single-stranded or double-stranded.

Various applications can be considered by the person skilled in the art for the methods according to the present invention. A polynucleotide (or gene) in a genome may be modified, edited or disrupted using compositions and methods according to the present invention. E.g. when a fully active Cas protein is used that cuts in both strands of the target-polynucleotide and when no exogenous polynucleotide is present as a suitable repair template, the double strand break is repaired by non-homologous end joining repair (NHEJ). During NHEJ insertions and/or deletions (which may be construed as substitution in some cases) of one or several nucleotides may occur, these are randomly inserted or deleted at the repair site; this is characteristic for NHEJ. Such insertions and/or deletions may impact the reading frame of the coding sequence, resulting amino acid changes in the gene product or even a truncated protein in case of genesis of a (premature) stop codon or alteration of a splice site.

A polynucleotide (or gene) in a genome may be modified, edited or disrupted using compositions and methods according to the present invention using homologous end joining repair (HEJ), also known as homology-directed repair (HDR), when an exogenous polynucleotide is present as repair template. E.g. when an exogenous polynucleotide having sequence identity to the target-polynucleotide (i.e. upstream (5') and downstream (3') of the double strand break) is present together with a CRISPR-Cas system according to the present invention, HDR will introduce (or actually reproduce) the corresponding nucleotides of the exogenous polynucleotide at the double strand break in the target-polynucleotide. Preferably, an exogenous polynucleotide according to the present invention does not contain the target sequence itself followed by a functional PAM sequence to avoid the risk of the exogenous polynucleotide itself or the modified target-polynucleotide being (re)cut by the CRISPR-CAS system.

In the embodiments of the present invention, when a CRISPR-Cas system comprises an exogenous polynucleotide (donor polynucleotide, donor DNA, repair template), the CRISPR-Cas system preferably comprises two or more guide-polynucleotides encoded by or present on one or more separate polynucleotides or vectors, and two or more exogenous polynucleotides are provided together with said CRISPR-Cas system enabling the formation of two or more CRISPR-CAS complexes. In a method, such CRISPR-Cas systems according to the present invention can conveniently be used to modulate expression at two or more target-polynucleotides, i.e. a method to target multiple target sites. Such CRISPR-Cas system will by chance form one, two or more CRISPR-CAS complexes at one or more target-polynucleotides. Such method can be used to generate one or more insertions, deletions, substitutions, optionally in combination with the one or more exogenous polynucleotides, in the genome of the host cell, or to modulate expression of genes via the formed CRISPR-CAS complexes.

Preferably, the CRISPR-Cas complex cleaves one or both polynucleotide strands at the location of the target-polynucleotide, resulting in modulated expression of the gene product. The CRISPR-Cas complex may also have altered nuclease activity and lack the ability to cleave one or both strands of a target-polynucleotide; in such case, expression is modulated by the binding of the complex to the target-polynucleotide.

At least one CRISPR-Cas complex or two different CRISPR-CAS complexes are formed that cleave one or both polynucleotide strands at one location or at different locations of the target-polynucleotide, resulting in deletion of a polynucleotide fragment from the target-polynucleotide. Preferably, such composition comprising at least one or two guide-polynucleotides and/or a source of at least at least one Cas protein, additionally comprises an exogenous polynucleotide as defined herein below that is at least partly complementary to the at least one or two target-polynucleotides targeted by the guide-polynucleotide(s). Such polynucleotide fragment to be deleted or deleted fragment may be from several nucleotides in length up to a few thousand nucleotides in length, an entire gene may be deleted or a cluster of genes may be deleted. Accordingly, the present invention provides for a method of modulating expression of a polynucleotide in a host cell, wherein a polynucleotide fragment is deleted from a target-polynucleotide.

Provided is a method of modulating expression of a polynucleotide in a host cell, wherein a polynucleotide fragments is deleted from a target-polynucleotide, comprises contacting a host cell with a composition as descried herein, wherein the guide-polynucleotide directs binding of the Cas protein at the target-polynucleotide in the host cell to form a CRISPR-Cas complex. Preferably a method of modulating expression of a polynucleotide in a host cell, wherein a polynucleotide fragments is deleted from a target-polynucleotide, comprises contacting a host cell with a composition as descried herein, wherein the guide-polynucleotide directs binding of the Cas protein at the target-polynucleotide in the host cell to form a CRISPR-Cas complex, wherein the host cell is a modified host cell deficient in a component associated with NHEJ. In anoter preferred embodiment a method of modulating expression of a polynucleotide in a host cell, wherein a polynucleotide fragments is deleted from a target-polynucleotide, comprises contacting a host cell with a composition as descried herein, wherein the guide-polynucleotide directs binding of the Cas protein at the target-polynucleotide in the host cell to form a CRISPR-Cas complex, wherein the host cell is a modified host cell deficient in a component associated with NHEJ, wherein the composition as described herein does not comprise an exogenous or donor polynucleotide. In one preferred embodiment the component associated with NHEJ is a yeast Ku70 or a yeast Ku80 or a yeast LIG4 or its respective ortholog in the host cells according to the present invention. In another embodiment the host cell is a filamentous fungal host cell. In another embodiment of the method of modulating expression of a polynucleotide in a host cell the composition is comprised in an AMA vector.

Disclosed is a method of modulating expression of a polynucleotide in a cell, wherein a polynucleotide fragment is deleted from a target-polynucleotide, comprising contacting a host cell with the composition as described herein but preferably not comprising a donor polynucleotide as defined herein, wherein the guide-polynucleotide directs binding of the Cas protein at the target-polynucleotide in the host cell to form a CRISPR-Cas complex, wherein the host cell is deficient in a component assosicated with NHEJ, preferably a yeast Ku70 or yeast Ku80 or a yeast LIG4 or its respective ortholog in the host cells.

Surprisingly it has been found that in a host cell deficient in a gene involved in NHEJ it is possible to obtain deletions in the host cell genome in a controlled way by using the CRISPR/CAS9 system when regions of homology are present at both sites of the intended cleavage site and wherein the composition as described herein does not comprise a donor DNA, in a method of modulating expression of a polynucleotide in a cell, wherein a polynucleotide fragment is deleted from a target-polynucleotide, as described herein.

Disclosed is a method of modulating expression of a polynucleotide in a cell, wherein a polynucleotide fragment is deleted from a target-polynucleotide, comprising contacting a host cell with a non-naturally occurring or engineered composition comprising a source of a CRISPR-Cas system comprising a guide-polynucleotide and a Cas protein, wherein the guide-polynucleotide comprises a guide-sequence that essentially is the reverse complement of a target-polynucleotide in a host cell and the guide-polynucleotide can direct binding of the Cas protein at the target-polynucleotide in the host cell to form a CRISPR-Cas complex, wherein the guide-sequence is essentially the reverse complement of the (N)y part of a 5'-(N)yPAM-3' polynucleotide sequence target in the genome of the host cell, wherein y is an integer of 8-30, wherein PAM is a protospacer adjacent motif, wherein the host cell is a eukaryote, which eukaryote is a filamentous fungus, preferably an Aspergillus, a Penicillium, a Rasamsonia or a Mortierella and wherein PAM is preferably a sequence selected from the group consisting of 5'-XGG-3', 5'-XGGXG-3', 5'-XXAGAAW-3', 5'-XXXXGATT-3', 5'-XXAGAA-3', 5'-XAAAAC-3', wherein X can be any nucleotide or analog thereof, preferably X can be any nucleotide; and W is A or T herein but preferably not comprising a donor polynucleotide as defined herein, wherein the guide-polynucleotide directs binding of the Cas protein at the target-polynucleotide in the host cell to form a CRISPR-Cas complex, wherein the host cell is deficient in a component assosicated with NHEJ, preferably a yeast Ku70 or yeast Ku80 or a yeast LIG4 or its respective ortholog in the host cells, wherein the Cas protein has activity for directing cleavage of both polynucleotide strands at the location of the target-sequence and wherein the cleavage occurs in a region of the genome comprised between two homologous regions which upon cleavage by the Cas protein recombine with each other resulting in the deletion of a polynucleotide comprised between said regions.
Preferably the degree of homology between the two homologous regions is such to allow homologous recombination. Preferably the two homologous regions have at least 60%, 70%, 80%, 90%, 99% or 100% sequence identity over the whole length of the homologous regions. It has been surprisingly found that the length of homologous region can be very short even in filamentous fungi, wherein usually a length of at leat 1 or several kb is necessary to allow homologous recombination. Therefore in a preferred emdbodiment the length of the homologous regions is preferably at most 1 kb, at most 0,5 kb, at most 100 bp, at most 50 bp, at most 40 bp, at most 30 bp, at most 20 bp, at most 10 bp.
Preferably the distance between the two homologous regions is at most 10 kb, at most 9, at most 8 kb, at most 7 kb, at most 6 kb, at most 5 kb, at most 4 kb, at most 3 kb, at most 2 kb, at most 1 kb, at most 0,5 kb, at most 100bp, at most 50bp, at most 40bp, at most 30, 20, 10kb.
In one aspect, disclosed are software algorithms able to identify PAM sites in the genome comprised between homology regions of about 7-20 bp in a neighbourhood of the PAM site to design a method to target one or more PAM sites and create deletion of polynucleotides without use of a donor DNA.
The above method can be used for efficient removal of polynuclotide sequences in a designed way. For example upon introducing a Cas9 expression cassette at the genomic DNA and after several rounds of modifications mediated by the CRISPR/CAS9 system, one can remove the CAS9 from the genome by the introduction of a gRNA targeting a site in the Cas9 expression casette and wherein the Cas9 expression cassette is comprised between two homologous regions as defined above, preferably 100-bp long, more preferably 20-bp, 15-bp long or shorter and cleave out the Cas9 open reading frame or a large part of the expression cassette.
The above method can also be used for transient inactivation of a gene. Eg. one could for example make a gene, e.g. a Ku70 polynucleotide non-functional by inserting a polynucleotide sequence in the ORF of the Ku70 gene, comprising two homologous regions at its 5'-end and 3'end respectively, whrein preferably the homologous regions are 100-bp, more preferably 20-bp, 15-bp long or shorter. The Ku70 gene can be made functional again using a CRISPR-Cas9 system without donor DNA as described above.

The method of modulating expression comprises cleavage of one or both polynucleotide strands at at least one location of the target-polynucleotide followed by modification of the target-polynucleotide by homologous recombination with an exogenous polynucleotide. In such case, the composition according to the first aspect of the present invention preferably further comprises such exogenous polynucleotide. Such modification may result in insertion, deletion or substitution of at least one nucleotide in the target-polynucleotide, wherein the insertion or substitution nucleotide may or may not originate from the exogenous polynucleotide. In one embodiment the exogenous polynucleotide comprises regions of homology with the target-polynucleotide. Preferably the degree of homology between these homologous regions is such to allow homologous recombination. Preferably the homologous regions have at least 60%, 70%, 80%, 90%, 99% or 100% sequence identity over the whole length of the homologous regions. In one embodiment, wherin the host cell is deficient in a component involve in NHEJ as defined herewith, the homologous regions are preferably at most 1 kb, at most 0,5 kb, at most 100 bp, at most 50 bp, at most 40 bp, at most 30 bp, at most 20 bp, at most 10 bp.
A modification can also be made when the exogenous polynucleotide is a non-integrating entity; in this case the target-polynucleotide is modified but no nucleotide of the exogenous polynucleotide is introduced into the target-polynucleotide. Consequently, the resulting host is a non-recombinant host when the Cas-protein according to the present invention is transformed as a protein. In a method according to this aspect of the present invention, the host cell may thus be a recombinant host cell or may be a non-recombinant host cell. The exogenous polynucleotide may be any polynucleotide of interest such as a polynucleotide encoding a compound of interest as defined herein, or a part of such polynucleotide or a variant thereof.

Disclosed is a method of producing a host cell, comprising contacting a host cell with the composition, wherein the guide-polynucleotide directs binding of the Cas protein at the target-polynucleotide in the host cell to form a CRISPR-Cas complex. A host cell in this embodiment of the present invention may be any type of host cell as defined herein and may comprise a polynucleotide encoding a compound of interest as defined elsewhere herein. A preferred method of producing a host cell according to the present invention comprises a step to produce an offspring host cell, wherein in said offspring host cell no components of a CRISPR-Cas system according to the present invention are present anymore. A further preferred host cell is a modified host cell wherein expression of a component associated with NHEJ as depicted above is altered compared to the corresponding wild-type host cell; preferably expression of the component associated with NHEJ is lowered.

The composition may be any such composition as defined herein. Contacting a host cell with a composition according to the present invention may be performed by any means known to the person skilled in the art. A host cell according to the present invention may simply be brought into a solution comprising a composition according to the present invention. Specific means of delivering a composition according to the present invention into a host cell may be used.

Disclosed is a method for the production of a compound of interest, comprising culturing under conditions conducive to the compound of interest a host cell or a host cell obtainable or obtained by a method according to the third or forth aspect of the present invention, and optionally purifying or isolating the compound of interest.

A compound of interest in the context of all embodiments of the present invention may be any biological compound. The biological compound may be biomass or a biopolymer or a metabolite. The biological compound may be encoded by a single polynucleotide or a series of polynucleotides composing a biosynthetic or metabolic pathway or may be the direct result of the product of a single polynucleotide or products of a series of polynucleotides, the polynucleotide may be a gene, the series of polynucleotide may be a gene cluster. In all embodiments of the present invention, the single polynucleotide or series of polynucleotides encoding the biological compound of interest or the biosynthetic or metabolic pathway associated with the biological compound of interest, are preferred targets for the compositions and methods according to the present invention. The biological compound may be native to the host cell or heterologous to the host cell.

The term "heterologous biological compound" is defined herein as a biological compound which is not native to the cell; or a native biological compound in which structural modifications have been made to alter the native biological compound.

The term "biopolymer" is defined herein as a chain (or polymer) of identical, similar, or dissimilar subunits (monomers). The biopolymer may be any biopolymer. The biopolymer may for example be, but is not limited to, a nucleic acid, polyamine, polyol, polypeptide (or polyamide), or polysaccharide.

The biopolymer may be a polypeptide. The polypeptide may be any polypeptide having a biological activity of interest. The term "polypeptide" is not meant herein to refer to a specific length of the encoded product and, therefore, encompasses peptides, oligopeptides, and proteins. The term polypeptide refers to polymers of amino acids of any length. The polymer may he linear or branched, it may comprise modified amino acids, and it may be interrupted by non amino acids. The terms also encompass an amino acid polymer that has been modified; for example, disulfide bond formation, glycosylation, lipidation, acetylation, phosphorylation, or any other manipulation, such as conjugation with a labeling component. As used herein the term "amino acid" includes natural and/or unnatural or synthetic amino acids, including glycine and both the D or L optical isomers, and amino acid analogs and peptidomimetics. Polypeptides further include naturally occurring allelic and engineered variations of the above- mentioned polypeptides and hybrid polypeptides. The polypeptide may be native or may be heterologous to the host cell. The polypeptide may be a collagen or gelatine, or a variant or hybrid thereof. The polypeptide may be an antibody or parts thereof, an antigen, a clotting factor, an enzyme, a hormone or a hormone variant, a receptor or parts thereof, a regulatory protein, a structural protein, a reporter, or a transport protein, protein involved in secretion process, protein involved in folding process, chaperone, peptide amino acid transporter, glycosylation factor, transcription factor, synthetic peptide or oligopeptide, intracellular protein. The intracellular protein may be an enzyme such as, a protease, ceramidases, epoxide hydrolase, aminopeptidase, acylases, aldolase, hydroxylase, aminopeptidase, lipase. The polypeptide may also be an enzyme secreted extracellularly. Such enzymes may belong to the groups of oxidoreductase, transferase, hydrolase, lyase, isomerase, ligase, catalase, cellulase, chitinase, cutinase, deoxyribonuclease, dextranase, esterase. The enzyme may be a carbohydrase, e.g. cellulases such as endoglucanases, β-gtucanases, cellobiohydrolases or β-gtucosidases, hemicellulases or pectinolytic enzymes such as xylanases, xylosidases, mannanases, galactanases, galactosidases, pectin methyl esterases, pectin lyases, pectate lyases, endo polygalacturonases, exopolygalacturonases rhamnogalacturonases, arabanases, arabinofuranosidases, arabinoxylan hydrolases, galacturonases, lyases, or amylolytic enzymes; hydrolase, isomerase, or ligase, phosphatases such as phytases, esterases such as lipases, proteolytic enzymes, oxidoreductases such as oxidases, transferases, or isomerases. The enzyme may be a phytase. The enzyme may be an aminopeptidase, asparaginase, amylase, a maltogenic amylase, carbohydrase, carboxypeptidase, endo-protease, metallo-protease, serine-protease catalase, chitinase, cutinase, cyclodextrin glycosyltransferase, deoxyribonuclease, esterase, alpha-galactosidase, beta-galactosidase, glucoamylase, alpha-glucosidase, beta-glucosidase, haloperoxidase, protein deaminase, invertase, laccase, lipase, mannosidase, mutanase, oxidase, pectinolytic enzyme, peroxidase, phospholipase, galactolipase, chlorophyllase, polyphenoloxidase, ribonuclease, transglutaminase, or glucose oxidase, hexose oxidase, monooxygenase.

According to the present invention, a compound of interest can be a polypeptide or enzyme with improved secretion features as described in WO2010/102982. According to the present invention, a compound of interest can be a fused or hybrid polypeptide to which another polypeptide is fused at the N-terminus or the C-terminus of the polypeptide or fragment thereof. A fused polypeptide is produced by fusing a nucleic acid sequence (or a portion thereof) encoding one polypeptide to a nucleic acid sequence (or a portion thereof) encoding another polypeptide.

Techniques for producing fusion polypeptides are known in the art, and include, ligating the coding sequences encoding the polypeptides so that they are in frame and expression of the fused polypeptide is under control of the same promoter(s) and terminator. The hybrid polypeptides may comprise a combination of partial or complete polypeptide sequences obtained from at least two different polypeptides wherein one or more may be heterologous to the host cell. Example of fusion polypeptides and signal sequence fusions are for example as described in WO2010/121933.

The biopolymer may be a polysaccharide. The polysaccharide may be any polysaccharide, including, but not limited to, a mucopolysaccharide (e. g., heparin and hyaluronic acid) and nitrogen-containing polysaccharide (e.g., chitin). In a preferred option, the polysaccharide is hyaluronic acid.

A polynucleotide coding for the compound of interest or coding for a compound involved in the production of the compound of interest according to the invention may encode an enzyme involved in the synthesis of a primary or secondary metabolite, such as organic acids, carotenoids, (beta-lactam) antibiotics, and vitamins. Such metabolite may be considered as a biological compound according to the present invention.

The term "metabolite" encompasses both primary and secondary metabolites; the metabolite may be any metabolite. Preferred metabolites are citric acid, gluconic acid, adipic acid, fumaric acid, itaconic acid and succinic acid.

A metabolite may be encoded by one or more genes, such as in a biosynthetic or metabolic pathway. Primary metabolites are products of primary or general metabolism of a cell, which are concerned with energy metabolism, growth, and structure. Secondary metabolites are products of secondary metabolism (see, for example, R. B. Herbert, The Biosynthesis of Secondary Metabolites, Chapman and Hall, New York, 1981).

A primary metabolite may be, but is not limited to, an amino acid, fatty acid, nucleoside, nucleotide, sugar, triglyceride, or vitamin.

A secondary metabolite may be, but is not limited to, an alkaloid, coumarin, flavonoid, polyketide, quinine, steroid, peptide, or terpene. The secondary metabolite may be an antibiotic, antifeedant, attractant, bacteriocide, fungicide, hormone, insecticide, or rodenticide. Preferred antibiotics are cephalosporins and beta-lactams. Other preferred metabolites are exo-metabolites. Examples of exo-metabolites are Aurasperone B, Funalenone, Kotanin, Nigragillin, Orlandin, Other naphtho-γ-pyrones, Pyranonigrin A, Tensidol B, Fumonisin B2 and Ochratoxin A.

The biological compound may also be the product of a selectable marker. A selectable marker is a product of a polynucleotide of interest which product provides for biocide or viral resistance, resistance to heavy metals, prototrophy to auxotrophs, and the like. Selectable markers include, but are not limited to, amdS (acetamidase), argB (ornithinecarbamoyltransferase), bar (phosphinothricinacetyltransferase), hygB (hygromycin phosphotransferase), niaD (nitrate reductase), pyrG (orotidine-5'-phosphate decarboxylase), sC (sulfate adenyltransferase), trpC (anthranilate synthase), ble (phleomycin resistance protein), hyg (hygromycin), NAT or NTC (Nourseothricin) as well as equivalents thereof.

A compound of interest is preferably a polypeptide as described in the list of compounds of interest.

A compound of interest is preferably a metabolite.

The host cell may already be capable of producing the compound of interest. The mutant microbial host cell may also be provided with a homologous or heterologous nucleic acid construct that encodes a polypeptide wherein the polypeptide may be the compound of interest or a polypeptide involved in the production of the compound of interest. The person skilled in the art knows how to modify a microbial host cell such that it is capable of producing the compound of interest

More than one copy of a polynucleotide according to the present invention may be inserted into the microbial host cell to mediate production of the product encoded by said polynucleotide. This can be done, preferably by integrating multiple copies of the polynucleotide into the genome of the host cell, more preferably by targeting the integration of the polynucleotide at one of the highly expressed loci defined in the former paragraph. Alternatively, integration of multiple copies can be achieved by including an amplifiable selectable marker gene with a polynucleotide according to the present invention, such that cells containing amplified copies of the selectable marker gene (and thereby additional copies of the nucleic acid sequence) can be selected for by cultivating the cells in the presence of the appropriate selectable agent. To increase the number of copies of a polynucleotide according the present invention even more, the technique of gene conversion as described in WO98/46772 may be used.

### General definitions

Throughout the present specification and the accompanying claims, the words "comprise", "include" and "having" and variations such as "comprises", "comprising", "includes" and "including" are to be interpreted inclusively. That is, these words are intended to convey the possible inclusion of other elements or integers not specifically recited, where the context allows.
The terms "a" and "an" are used herein to refer to one or to more than one (i.e. to one or at least one) of the grammatical object of the article. By way of example, "an element" may mean one element or more than one element.
The word "about" or "approximately" when used in association with a numerical value (e.g. about 10) preferably means that the value may be the given value (of 10) more or less 1% of the value.

A preferred nucleotide analogue or equivalent comprises a modified backbone. Examples of such backbones are provided by morpholino backbones, carbamate backbones, siloxane backbones, sulfide, sulfoxide and sulfone backbones, formacetyl and thioformacetyl backbones, methyleneformacetyl backbones, riboacetyl backbones, alkene containing backbones, sulfamate, sulfonate and sulfonamide backbones, methyleneimino and methylenehydrazino backbones, and amide backbones. It is further preferred that the linkage between a residue in a backbone does not include a phosphorus atom, such as a linkage that is formed by short chain alkyl or cycloalkyl internucleoside linkages, mixed heteroatom and alkyl or cycloalkyl internucleoside linkages, or one or more short chain heteroatomic or heterocyclic internucleoside linkages.
A preferred nucleotide analogue or equivalent comprises a Peptide Nucleic Acid (PNA), having a modified polyamide backbone (Nielsen, et al. (1991) Science 254, 1497-1500). PNA-based molecules are true mimics of DNA molecules in terms of base-pair recognition. The backbone of the PNA is composed of N-(2-aminoethyl)-glycine units linked by peptide bonds, wherein the nucleobases are linked to the backbone by methylene carbonyl bonds. An alternative backbone comprises a one-carbon extended pyrrolidine PNA monomer (Govindaraju and Kumar (2005) Chem. Commun, 495-497). Since the backbone of a PNA molecule contains no charged phosphate groups, PNA-RNA hybrids are usually more stable than RNA-RNA or RNA-DNA hybrids, respectively (Egholm et al (1993) Nature 365, 566-568).
A further preferred backbone comprises a morpholino nucleotide analog or equivalent, in which the ribose or deoxyribose sugar is replaced by a 6-membered morpholino ring. A most preferred nucleotide analog or equivalent comprises a phosphorodiamidate morpholino oligomer (PMO), in which the ribose or deoxyribose sugar is replaced by a 6-membered morpholino ring, and the anionic phosphodiester linkage between adjacent morpholino rings is replaced by a non-ionic phosphorodiamidate linkage.
A further preferred nucleotide analogue or equivalent comprises a substitution of at least one of the non-bridging oxygens in the phosphodiester linkage. This modification slightly destabilizes base-pairing but adds significant resistance to nuclease degradation. A preferred nucleotide analogue or equivalent comprises phosphorothioate, chiral phosphorothioate, phosphorodithioate, phosphotriester, aminoalkylphosphotriester, H-phosphonate, methyl and other alkyl phosphonate including 3'-alkylene phosphonate, 5'-alkylene phosphonate and chiral phosphonate, phosphinate, phosphoramidate including 3'-amino phosphoramidate and aminoalkylphosphoramidate, thionophosphoramidate, thionoalkylphosphonate, thionoalkylphosphotriester, selenophosphate or boranophosphate.
A further preferred nucleotide analogue or equivalent comprises one or more sugar moieties that are mono- or disubstituted at the 2', 3' and/or 5' position such as a -OH; -F; substituted or unsubstituted, linear or branched lower (C1-C10) alkyl, alkenyl, alkynyl, alkaryl, allyl, aryl, or aralkyl, that may be interrupted by one or more heteroatoms; O-, S-, or N-alkyl; O-, S-, or N-alkenyl; O-, S-or N-alkynyl; O-, S-, or N-allyl; O-alkyl-O-alkyl, -methoxy, -aminopropoxy; aminoxy, methoxyethoxy; - dimethylaminooxyethoxy; and -dimethylaminoethoxyethoxy. The sugar moiety can be a pyranose or derivative thereof, or a deoxypyranose or derivative thereof, preferably a ribose or a derivative thereof, or deoxyribose or derivative thereof. Such preferred derivatized sugar moieties comprise Locked Nucleic Acid (LNA), in which the 2'-carbon atom is linked to the 3' or 4' carbon atom of the sugar ring thereby forming a bicyclic sugar moiety. A preferred LNA comprises 2'-O,4'-C-ethylene-bridged nucleic acid (Morita et al. 2001. Nucleic Acid Res Supplement No. 1: 241-242). These substitutions render the nucleotide analogue or equivalent RNase H and nuclease resistant and increase the affinity for the target.
"Sequence identity" or "identity" in the context of the present invention of an amino acid- or nucleic acid-sequence is herein defined as a relationship between two or more amino acid (peptide, polypeptide, or protein) sequences or two or more nucleic acid (nucleotide, oligonucleotide, polynucleotide) sequences, as determined by comparing the sequences. In the art, "identity" also means the degree of sequence relatedness between amino acid or nucleotide sequences, as the case may be, as determined by the match between strings of such sequences. Within the present invention, sequence identity with a particular sequence preferably means sequence identity over the entire length of said particular polypeptide or polynucleotide sequence. "Similarity" between two amino acid sequences is determined by comparing the amino acid sequence and its conserved amino acid substitutes of one peptide or polypeptide to the sequence of a second peptide or polypeptide. In a preferred embodiment, identity or similarity is calculated over the whole sequence (SEQ ID NO:) as identified herein. "Identity" and "similarity" can be readily calculated by known methods, including but not limited to those described in Computational Molecular Biology, Lesk, A. M., ed., Oxford University Press, New York, 1988; Biocomputing: Informatics and Genome Projects, Smith, D. W., ed., Academic Press, New York, 1993; Computer Analysis of Sequence Data, Part I, Griffin, A. M., and Griffin, H. G., eds., Humana Press, New Jersey, 1994; Sequence Analysis in Molecular Biology, von Heine, G., Academic Press, 1987; and Sequence Analysis Primer, Gribskov, M. and Devereux, J., eds., M Stockton Press, New York, 1991; and Carillo, H., and Lipman, D., SIAM J. Applied Math., 48:1073 (1988).
Preferred methods to determine identity are designed to give the largest match between the sequences tested. Methods to determine identity and similarity are codified in publicly available computer programs. Preferred computer program methods to determine identity and similarity between two sequences include e.g. the GCG program package (Devereux, J., et al., Nucleic Acids Research 12 (1): 387 (1984)), BestFit, BLASTP, BLASTN, and FASTA (Altschul, S. F. et al., J. Mol. Biol. 215:403-410 (1990). The BLAST X program is publicly available from NCBI and other sources (BLAST Manual, Altschul, S., et al., NCBI NLM NIH Bethesda, MD 20894; Altschul, S., et al., J. Mol. Biol. 215:403-410 (1990). The well-known Smith Waterman algorithm may also be used to determine identity.
Preferred parameters for polypeptide sequence comparison include the following: Algorithm: Needleman and Wunsch, J. Mol. Biol. 48:443-453 (1970); Comparison matrix: BLOSSUM62 from Hentikoff and Hentikoff, Proc. Natl. Acad. Sci. USA. 89:10915-10919 (1992); Gap Penalty: 12; and Gap Length Penalty: 4. A program useful with these parameters is publicly available as the "Ogap" program from Genetics Computer Group, located in Madison, WI. The aforementioned parameters are the default parameters for amino acid comparisons (along with no penalty for end gaps).
Preferred parameters for nucleic acid comparison include the following: Algorithm: Needleman and Wunsch, J. Mol. Biol. 48:443-453 (1970); Comparison matrix: matches=+10, mismatch=0; Gap Penalty: 50; Gap Length Penalty: 3. Available as the Gap program from Genetics Computer Group, located in Madison, Wis. Given above are the default parameters for nucleic acid comparisons.
Optionally, in determining the degree of amino acid similarity, the skilled person may also take into account so-called "conservative" amino acid substitutions, as will be clear to the skilled person. Conservative amino acid substitutions refer to the interchangeability of residues having similar side chains. For example, a group of amino acids having aliphatic side chains is glycine, alanine, valine, leucine, and isoleucine; a group of amino acids having aliphatic-hydroxyl side chains is serine and threonine; a group of amino acids having amide-containing side chains is asparagine and glutamine; a group of amino acids having aromatic side chains is phenylalanine, tyrosine, and tryptophan; a group of amino acids having basic side chains is lysine, arginine, and histidine; and a group of amino acids having sulphur-containing side chains is cysteine and methionine. Preferred conservative amino acids substitution groups are: valine-leucine-isoleucine, phenylalanine-tyrosine, lysine-arginine, alanine-valine, and asparagine-glutamine. Substitutional variants of the amino acid sequence disclosed herein are those in which at least one residue in the disclosed sequences has been removed and a different residue inserted in its place. Preferably, the amino acid change is conservative. Preferred conservative substitutions for each of the naturally occurring amino acids are as follows: Ala to ser; Arg to lys; Asn to gln or his; Asp to glu; Cys to ser or ala; Gln to asn; Glu to asp; Gly to pro; His to asn or gin; Ile to leu or val; Leu to ile or val; Lys to arg; gln or glu; Met to leu or ile; Phe to met, leu or tyr; Ser to thr; Thr to ser; Trp to tyr; Tyr to trp or phe; and, Val to ile or leu.

A polynucleotide according to the present invention is represented by a nucleotide sequence. A polypeptide according to the present invention is represented by an amino acid sequence. A nucleic acid construct according to the present invention is defined as a polynucleotide which is isolated from a naturally occurring gene or which has been modified to contain segments of polynucleotides which are combined or juxtaposed in a manner which would not otherwise exist in nature. Optionally, a polynucleotide present in a nucleic acid construct according to the present invention is operably linked to one or more control sequences, which direct the production or expression of the encoded product in a host cell or in a cell-free system.
The sequence information as provided herein should not be so narrowly construed as to require inclusion of erroneously identified bases. The skilled person is capable of identifying such erroneously identified bases and knows how to correct for such errors.
All embodiments of the present invention, i.e. a composition according to the present invention, a method of modulating expression, a host cell comprising a composition according to the present invention, a method of producing a host cell according to the present invention, a host cell according to the present invention and a method for the production of a compound of interest according to the present invention preferably refer to host cell, not to a cell-free *in vitro* system; in other words, the CRISPR-Cas systems according to the present invention are preferably host cell systems, not cell-free *in vitro* systems.
In all embodiments of the present invention, e.g. a composition according to the present invention, a method of modulating expression, a host cell comprising a composition according to the present invention, a method of producing a host cell according to the present invention, a host cell according to the present invention and a method for the production of a compound of interest according to the present invention, the host cell preferably is a microbial cell, which microbial host cell preferably is a prokaryotic or a eukaryotic host cell. When the host cell according to the present invention is a eukaryotic host cell, the host cell may or not be a mammalian host cell; in the latter case the eukaryotic host cell according to the present invention is not a mammalian host cell. A host cell according to the present invention may be a haploid, diploid or polyploid host cell. The host cell according to the present invention may be a prokaryotic host cell. Preferably, the prokaryotic host cell according to the present invention is a bacterial host cell. The term "bacterial host cell" includes both Gram-negative and Gram-positive microorganisms. Preferably, a bacterial host cell according to the present invention is from a genus selected from the group consisting of *Escherichia, Anabaena, Caulobactert, Gluconobacter, Rhodobacter, Pseudomonas, Paracoccus, Bacillus, Brevibacterium, Corynebacterium, Rhizobium* (*Sinorhizobium), Flavobacterium, Klebsiella, Enterobacter, Lactobacillus, Lactococcus, Methylobacterium, Staphylococcus* or *Streptomyces.* More preferably, the bacterial host cell is selected from the group consisting of *B*. *subtilis, B. amyloliquefaciens, B. licheniformis, B. puntis, B. megaterium, B. halodurans, B. pumilus, G. oxydans, Caulobactert crescentus CB 15, Methylobacterium extorquens, Rhodobacter sphaeroides, Pseudomonas zeaxanthinifaciens, Paracoccus denitrificans, Escherichia coli, Corynebacterium glutamicum, Staphylococcus carnosus, Streptomyces lividans, Sinorhizobium melioti* and *Rhizobium radiobacter.*
The host cell according to the present invention may be a eukaryotic host cell. Preferably, the eukaryotic host cell according to the present invention is a mammalian, insect, plant, fungal, or algal host cell, more preferably, the eukaryotic host cell according to the present invention is an insect, plant, fungal, or algal host cell, even more preferably, the eukaryotic host cell according to the present invention is a fungal, or algal host cell such as a *Schizochitrium,* even more preferably, the eukaryotic host cell according to the present invention is a fungal host cell, even more preferably, the eukaryotic host cell according to the present invention is a filamentous fungal host cell. Preferred mammalian host cells are selected from the group consisting of Chinese hamster ovary (CHO) cells, COS cells, 293 cells, PerC6 cells, and hybridomas. Preferred insect host cells are selected from the group consisting of Sf9 and Sf21 cells and derivatives thereof. A preferred fungal host cell is a yeast host cell, a preferred yeast host cell is from a genus selected from the group consisting of *Candida, Hansenula, Issatchenkia, Kluyveromyces, Pichia, Saccharomyces, Schizosaccharomyces,Yarrowia or Zygosaccharomyces;* more preferably a yeast host cell is selected from the group consisting of *Kluyveromyces lactis, Kluyveromyces lactis* NRRL Y-1140, *Kluyveromyces marxianus, Kluyveromyces. thermotolerans, Candida krusei, Candida sonorensis, Candida glabrata, Saccharomyces cerevisiae, Saccharomyces cerevisiae* CEN.PK113-7D, *Schizosaccharomyces pombe, Hansenula polymorpha, Issatchenkia orientalis, Yarrowia lipolytica, Yarrowia lipolytica* CLIB122, Yarrowia lipolytica ML324 (deposited as ATCC18943), *Pichia stipidis, Scheffersomyces stipitis,* and *Pichia pastoris.* A more preferred fungal host cell is a filamentous fungal cell. Filamentous fungi as defined herein include all filamentous forms of the subdivision *Eumycota* and *Oomycota* (as defined by Hawksworth et al., In, Ainsworth and Bisby's Dictionary of The Fungi, 8th edition, 1995, CAB International, University Press, Cambridge, UK). The filamentous fungi are characterized by a mycelial wall composed of chitin, cellulose, glucan, chitosan, mannan, and other complex polysaccharides. Vegetative growth is by hyphal elongation and carbon catabolism is obligatory aerobic. Filamentous fungal strains include, but are not limited to, strains of *Acremonium, Agaricus, Aspergillus, Aureobasidium, Chrysosporium, Coprinus, Cryptococcus, Filibasidium, Fusarium, Humicola, Magnaporthe, Mortierella, Mucor, Myceliophthora, Neocallimastix, Neurospora, Paecilomyces, Penicillium, Piromyces, Panerochaete, Pleurotus, Schizophyllum, Talaromyces, Rasamsonia, Thermoascus, Thielavia, Tolypocladium,* and *Trichoderma.* A preferred filamentous fungal host cell according to the present invention is from a genus selected from the group consisting of *Acremonium, Aspergillus, Chrysosporium, Myceliophthora, Penicillium, Talaromyces, Rasamsonia, Thielavia, Fusarium* and *Trichoderma;* more preferably from a species selected from the group consisting of *Aspergillus niger, Acremonium alabamense, Aspergillus awamori, Aspergillus foetidus, Aspergillus sojae, Aspergillus fumigatus, Talaromyces emersonii, Rasamsonia emersonii, Rasamsonia emersonii* CBS393.64, *Aspergillus oryzae, Chrysosporium lucknowense, Fusarium oxysporum, Mortierella alpina, Mortierella alpina* ATCC 32222, *Myceliophthora thermophila, Trichoderma reesei, Thielavia terrestris, Penicillium chrysogenum* and *P*. *chrysogenum* Wisconsin 54-1255(ATCC28089); even more preferably the filamentous fungal host cell according to the present invention is an *Aspergillus niger.* When the host cell according to the present invention is an *Aspergillus niger* host cell, the host cell preferably is CBS 513.88, CBS124.903 or a derivative thereof.
Several strains of filamentous fungi are readily accessible to the public in a number of culture collections, such as the American Type Culture Collection (ATCC), Deutsche Sammlung von Mikroorganismen und Zellkulturen GmbH (DSM), Centraalbureau Voor Schimmelcultures (CBS), Agricultural Research Service Patent Culture Collection, Northern Regional Research Center (NRRL), and All-Russian Collection of Microorganisms of Russian Academy of Sciences, (abbreviation in Russian - VKM, abbreviation in English - RCM), Moscow, Russia. Preferred strains as host cells according to the present invention are *Aspergillus niger* CBS 513.88, CBS124.903, *Aspergillus oryzae* ATCC 20423, IFO 4177, ATCC 1011, CBS205.89, ATCC 9576, ATCC14488-14491, ATCC 11601, ATCC12892, *P. chrysogenum* CBS 455.95, *P. chrysogenum* Wisconsin54-1255(ATCC28089), *Penicillium citrinum* ATCC 38065, *Penicillium chrysogenum* P2, *Thielavia terrestris* NRRL8126, *Rasamsonia emersonii* CBS393.64, *Talaromyces emersonii* CBS 124.902, *Acremonium chrysogenum* ATCC 36225 or ATCC 48272, *Trichoderma reesei* ATCC 26921 or ATCC 56765 or ATCC 26921, *Aspergillus sojae* ATCC11906, *Myceliophthora thermophila* C1, Garg 27K, VKM-F 3500 D, *Chrysosporium lucknowense* C1, Garg 27K, VKM-F 3500 D, ATCC44006 and derivatives thereof.
Preferably, and more preferably when the microbial host cell according to the invention is a filamentous fungal host cell, a host cell according to the present invention further comprises one or more modifications in its genome such that the host cell is deficient in the production of at least one product selected from glucoamylase (glaA), acid stable alpha-amylase (amyA), neutral alpha-amylase (amyBI and amyBII), oxalic acid hydrolase (oahA), a toxin, preferably ochratoxin and/or fumonisin, a protease transcriptional regulator prtT, PepA, a product encoded by the gene hdfA and/or hdfB, a non-ribosomal peptide synthase *npsE* if compared to a parent host cell and measured under the same conditions.
Oxalic acid hydrolase (oahA) is a component of the synthesis pathway of oxalic acid in many host cells. A host cell deficient in oahA will be deficient in oxalic acid. Oxalic acid is an unwanted byproduct in many applications such as food applications. Furthermore, oxalic acid lowers the pH of the medium cultivations of host cell producing this component, resulting in lowered yields; i.e. yield is increased in oxalic acid deficient host cells. It is therefore advantageous if a host cell according to the present invention is deficient in oahA. OahA deficient host cells and preferred methods of producing said host cells are extensively described in WO 2000/50576 and WO2004/070022. A preferred method to produce an oahA deficient host cell is the recombinant method of disruption described in WO 2000/50576. Preferably, a host cell according to the present invention is deficient in oahA. Preferably, the oahA is a fungal oahA. More preferably, the oahA is the oahA from *Aspergillus.* Even more preferably the oahA is the oahA from *Aspergillus niger.* Even more preferably the oahA is the oahA from *Aspergillus niger* CBS 513.88. Most preferably, the oahA comprises the sequence of An10g00820.
prtT is a transcriptional activator of proteases in eukaryotic cells. Several fungal transcriptional activators of proteases have been recently described in WO 00/20596, WO 01/68864, WO 2006/040312 and WO 2007/062936. These transcriptional activators were isolated from *Aspergillus niger* (*A. niger*), *Aspergillus fumigatus* (*A. fumigatus*), *Penicillium chrysogenum* (*P. chrysogenum*) and *Aspergillus oryzae* (*A. oryzae*). These transcriptional activators of protease genes can be used to improve a method for producing a polypeptide in a host cell, wherein the polypeptide is sensitive for protease degradation. When a host cell according to the present invention is deficient in prtT, the host cell will produce less proteases that are under transcriptional control of prtT. It is therefore advantageous when a host cell according to the invention is deficient in prtT. prtT deficient hosts and preferred methods to produce these hosts are extensively described in WO 01/68864, WO 2006/040312. WO 01/68864 and WO 2006/040312 describe recombinant and classic methods to disrupt the prtT coding sequence. WO 2007/062936 describes disruption of the prtT binding site in a protease promoter. Disruption of the binding site impedes binding of prtT to the binding site. Consequently, the transcription of the protease is not activated by prtT and less protease is produced.
Preferably, a host cell according to the present invention comprises a polynucleotide encoding prtT, said polynucleotide comprising a modification such that the host cell is deficient in the production of prtT compared to a parent cell it originates from when cultivated under comparable conditions. Preferably, the prtT is a fungal prtT. More preferably, the prtT is the prtT from *Aspergillus.* Even more preferably the prtT is the prtT from *Aspergillus niger.* Even more preferably the prtT is the prtT from *Aspergillus niger* CBS 513.88. Most preferably, the prtT comprises the sequence of An04g06940.
The term "glucoamylase" (glaA) is identical to the term "amyloglucosidase" and is defined herein as an enzyme having dextrin 6-alpha-D-glucanohydrolase activity which catalyses the endo hydrolysis of 1, 6-alpha-D-glucoside linkages at points of branching in chains of 1, 4-linked alpha-D-glucose residues and terminal 1, 4-linked alpha-D-glucose residues. Glucoamylase activity can be measured as AGIU/ml by determining the liberation of paranitrofenol from the substrate p-nitrophenyl-a-D-glucopyranoside (Sigma). This results in a yellow colour, whose absorbance can be measured at 405 nm using a spectrophotometer. 1 AGIU is the quantity of enzyme, which produces 1 µmole of glucose per minute at pH 4.3 and 60°C from a soluble starch substrate. In WO98/46772 additional details of the assay can be found.
Preferably, a host cell according to the present invention comprises a polynucleotide encoding glaA, said polynucleotide comprising a modification such that the host cell is deficient in the production of glaA compared to a parent cell it originates from when cultivated under comparable conditions. Preferably, the glaA is a fungal glaA. More preferably, the glaA is the glaA from *Aspergillus.* Even more preferably the glaA is the glaA from *Aspergillus niger.* Even more preferably the glaA is the glaA from Aspergillus niger CBS 513.88. Most preferably, the glaA comprises the sequence of An03g06550.
The term "alpha-amylase" is defined herein as 1, 4-alpha-D-glucan glucanohydrolase activity which catalyzes the endohydrolysis of polysaccharides with three or more alpha-1, 4-linked glucose units in the presence of water to malto-oligosaccharides. To determine the (neutral) alpha-amylase activity, the Megazyme cereal alpha-amylase kit is used (Megazyme, CERALPHA alpha amylase assay kit, catalogus. ref. K-CERA, year 2000-2001), according a protocol of the supplier. The measured activity is based on hydrolysis of non-reducing-endblocked p-nitrophenyl maltoheptaoside in the presence of excess glucoamylase and α-glucosidase at a pH of 7.0. The amount of formed p-nitrophenol is a measure for alpha-amylase activity present in a sample.
The term "acid stable alpha-amylase" (amyA) is defined herein as an enzyme having alpha-amylase activity with optimal activity in the acid pH range. To determine the acid stable alpha-amylase activity, also the Megazyme cereal alpha-amylase kit is used (Megazyme, CERALPHA alpha amylase assay kit, catalogus. ref. K-CERA, year 2000-2001), according a protocol of the supplier but at an acid pH. The measured activity is based on hydrolysis of non-reducing-endblocked p-nitrophenyl maltoheptaoside in the presence of excess glucoamylase and α-glucosidase at a pH of 4.5. The amount of formed p-nitrophenol is a measure for acid stable alpha-amylase activity present in a sample.
Preferably, a host cell according to the present invention comprises a polynucleotide encoding AmyA, said polynucleotide comprising a modification, wherein the host cell is deficient in amyA compared to the parent cell it originates from when cultivated under comparable conditions. Preferably, the amyA is a fungal amyA. More preferably, the amyA is the amyA from Aspergillus. Even more preferably the amyA is the amyA from Aspergillus niger. Even more preferably the amyA is the amyA from Aspergillus niger CBS 513.88. Most preferably, the amyA comprises the sequence of An11g03340.
The term "neutral alpha-amylase activity" (amy) is defined herein as an enzyme having alpha-amylase activity with optimal activity in the neutral pH range.
Preferably, a host cell according to the present invention comprises a polynucleotide encoding AmyB, said polynucleotide comprising a modification, wherein the host cell is deficient in amyBI and/or amyBII compared to the parent cell it originates from when cultivated under comparable conditions. More preferably, a host cell according to the present invention is deficient in amyBI and amy BII. Preferably, the amyB a is a fungal amyB. More preferably, the amyB is the amyB from *Aspergillus.* Even more preferably the amyB is the amyBI from *Aspergillus niger.* Even more preferably the amyB is the amyBI from *Aspergillus niger* CBS 513.88. Most preferably, the amyBI comprises the sequence of An12g06930. Even more preferably the amyB is the amyBII from *Aspergillus niger.* Even more preferably the amyB is the amyBII from *Aspergillus niger* CBS 513.88. Most preferably, the amyBII comprises the sequence of An05g02100.
The term toxin associated polynucleotide is defined herein as a gene cluster, a multitude of genes, a gene or part thereof encoding a compound, or biochemical pathway responsible for the biosynthesis or secretion of at least one toxin or toxin intermediate compound. Said compound may e.g. be a polypeptide, which may be an enzyme.
A number of host cells, especially filamentous fungal host cells, which are used as for the production of polypeptides of interest, comprise genes encoding enzymes involved in the biosynthesis of various toxins. For example, cyclopiazonic acid, kojic acid, 3-nitropropionic acid and aflatoxins are known toxins, which are formed in, e.g., *Aspergillus flavus.* Similarly, trichothecenes are formed in a number of filamentous fungi, e.g., in *Fusarium sp.* such as *Fusarium venenatum* as well as in *Trichoderma;* ochratoxin may be produced by *Aspergillus.* Recently, sequencing of the genome of an industrial *Aspergillus niger* host strain revealed an inactive fumonisin gene cluster (Pel et al., "Genome sequencing and analysis of the versatile cell factory Aspergillus niger CBS 513.88". Nat Biotechnol. 2007 Feb; 25 (2):221-231). The formation of such toxins during the fermentation of compounds of interest is highly undesirable as these toxins may present a health hazard to operators, customers and the environment. Consequently, a toxin deficient host cell enables toxin-free production of a compound of interest. The toxin-free compound is easier to produce since no toxin has to be removed from the product. Furthermore, the regulatory approval procedure for the compound is easier.
Preferably, a host cell according to the present invention comprises a toxin associated polynucleotide encoding a compound (which may e.g. be a polypeptide which may be an enzyme) or biochemical pathway, said toxin associated polynucleotide comprising a modification, wherein the host cell is deficient in the production of said toxin or a toxin intermediate compound compared to the parent cell it originates from when cultivated under comparable conditions. Preferably, the toxin or toxin intermediate compound is a fungal toxin or toxin intermediate compound. More preferably, the toxin or toxin intermediate compound is a toxin or toxin intermediate compound from *Aspergillus.* Even more preferably the toxin or the toxin intermediate compound is a toxin or toxin intermediate compound from *Aspergillus niger.* Even more preferably the toxin or toxin intermediate compound is a toxin or toxin intermediate compound from *Aspergillus niger* CBS 513.88. Even more preferably, the toxin or the toxin intermediate compound is fumonisin or a fumonisin intermediate compound. Even more preferably, the toxin or the toxin intermediate compound is ochratoxin or an ochratoxin intermediate compound. Most preferably, the toxin or the toxin intermediate compound is ochratoxin or fumonisin or an ochratoxin or a fumonisin intermediate compound.
Preferably, the toxin associated polynucleotide encodes a compound (which may e.g. be a polypeptide which may be an enzyme) or a biochemical pathway which is involved in the production of a fungal toxin or toxin intermediate compound. More preferably, said toxin or toxin intermediate compound is from *Aspergillus.* Even more preferably, said toxin or toxin intermediate compound is from *Aspergillus niger.* Even more preferably, said toxin or toxin intermediate compound is from *Aspergillus niger* CBS 513.88. Even more preferably, said toxin or toxin intermediate compound is a fumonisin or a fumonisin intermediate compound; even more preferably, a fumonisin-B or a fumonisin-B intermediate compound; even more preferably, a fumonisin-B2 or a fumonisin-B2 intermediate compound. Preferably, the toxin associated polynucleotide comprises the sequence of the fumonisin cluster from An01g06820 until An01g06930; more preferably, the toxin associated polynucleotide comprises the sequence of An01g06930. Alternatively or in combination when the toxin or toxin intermediate compound is a fumonisin or a fumonisin intermediate compound, the toxin associated polynucleotide encodes a compound (which may e.g. be a polypeptide which may be an enzyme) or a biochemical pathway, which is involved in ochratoxin or an ochratoxin intermediate compound; preferably, an ochratoxin A or an ochratoxin A intermediate compound; more preferably, the toxin associated polynucleotide comprises the sequence of the cluster from An15g07880 until An15g07930; most preferably, the toxin associated polynucleotide comprises the sequence of An15g07910 and/or the sequence of An15g07920.
Preferably, a host cell according to the present invention comprises at least one toxin associated polynucleotide encoding a compound (which may e.g. be a polypeptide which may be an enzyme) or biochemical pathway, said toxin associated polynucleotide comprising at least one modification, wherein the host cell is deficient in the production of a toxin or, toxin intermediate compound compared to the parent cell it originates from when cultivated under comparable conditions. More preferably, a host cell according to the present invention comprises two toxin associated polynucleotides, said two toxin associated polynucleotides each comprising at least one modification, wherein the host cell is preferably deficient in the production of fumonisin and ochratoxin compared to the parent cell it originates from when cultivated under comparable conditions. Even more preferably, a mutant microbial host cell according to the invention comprises three or more toxin associated polynucleotides, said three or more toxin associated polynucleotides each comprising at least one modification, wherein the host cell is preferably deficient in the production of fumonisin, ochratoxin and at least one additional toxin or toxin intermediate compound compared to the parent cell it originates from when cultivated under comparable conditions.
Preferably, a host cell according to the present invention comprises one or more modifications in its genome to result in a deficiency in the production of the major extracellular aspartic protease PepA. Preferably, the host cell according to the present invention comprises a disruption of the pepA gene encoding the major extracellular aspartic protease PepA; more preferably, the pepA is the pepA from *Aspergillus;* even more preferably the pepA is the pepA from *Aspergillus niger;* even more preferably the pepA is the pepA from *Aspergillus niger* CBS 513.88; most preferably, the pepA comprises the sequence of An14g04710.

Preferably, the efficiency of targeted integration of a polynucleotide to a pre-determined site into the genome of a host cell according to the invention is increased by rendering the cell deficient in a component in NHEJ (non-homologous recombination). Preferably, a host cell according to the invention comprises a polynucleotide encoding an NHEJ component comprising a modification, wherein said host cell is deficient in the production of said NHEJ component compared to a parent cell it originates from when cultivated under the same conditions.
The NHEJ component to be modified can be any NHEJ component known to the person skilled in the art. Preferred NHEJ components to be modified are selected from the group of filamentous fungal homologues of yeast KU70, KU80, MRE11, RAD50, RAD51, RAD52, XRS2, SIR4, LIG4. More preferred NHEJ components to be modified are filamentous fungal homologues of yeast KU70 and KU80, preferably hdfA (homologue of yeast KU70) or homologues thereof and hdfB (homologue of yeast KU80) or homologues thereof. The most preferred NHEJ component to be modified is KU70 or hdfA, or a homologue thereof. Another preferred NHEJ component to be modified is KU80 or hdfB, or a homologue thereof. Yet another preferred NHEJ component to be modified is a filamentous fungal homologue of yeast LIG4, or a homologue thereof. Methods to obtain such host cell deficient in a component involved in NHEJ are known to the skilled person and are extensively described in WO2005/095624. Preferably, the hdfA gene is the hdfA gene from *A*. *niger,* more preferably the hdfA from *A*. *niger* according to SEQ ID NO: 1 of WO2005/095624. In another preferred embodiment the hdfB gene is the hdfB gene from *A*. *niger,* more preferably the hdfB from *A. niger* according to SEQ ID NO: 4 of WO2005/095624.
When a host cell according to the present invention is a filamentous fungal host cell, said host cell preferably additionally comprises one or more modifications in its genome to result in a deficiency in the production of the product encoded by the hdf A gene (as depicted in SEQ ID NO: 3 of WO 2005/095624) and/or hdfB gene (as depicted in SEQ ID NO: 6 of WO 2005/095624). A host cell according to the present invention preferably further comprises a disruption of the hdfA and/or hdfB gene. Filamentous fungal host cells which are deficient in a product encoded by the hdfA and/or hdfB gene have been described in WO 2005/095624.
When a host cell according to the present invention is a filamentous fungal host cell, said host cell preferably further comprises a modification in its genome which results in the deficiency in the production of the non-ribosomal peptide synthase npsE, preferably the npsE depicted in SEQ ID NO: 38 of WO2012/001169. Such host cells deficient in the production of non-ribosomal peptide synthase npsE have been described in WO2012/001169 (npsE has a genomic sequence as depicted in SEQ ID NO: 35, a coding sequence as depicted in SEQ ID NO: 36, an mRNA as depicted in SEQ ID NO: 37 and the nrps protein as depicted in SEQ ID NO: 38 of WO2012/001169).
A host cell according to the present invention preferably further comprises a modification in its genome which results in the deficiency in the production of the α-amylase amyC, preferably the mature AmyC protein shown in SEQ ID NO: 4 and 8 of WO2014/013073. Such host cells deficient in the production of the α-amylase *amy*C have been described in WO2014/013073. amyC has a genomic sequence as depicted in SEQ ID NO: 1 or 5 and a coding sequence depicted in SEQ ID NO: 2 or 6 and the AmyC protein as depicted in SEQ ID NO: 3 or 7 with the mature AmyC protein shown in SEQ ID NO: 4 and 8 of WO2014/013073).
A host cell according to the present invention preferably further comprises a modification in its genome which results in the deficiency in the production of the AgsE protein, preferably the mature AgsE protein shown in SEQ ID NO: 3 or comprised in SEQ ID NO: 3 of WO2014/013074. Such host cells deficient in the production of the AgsE protein have been described in WO2014/013074. AgsE has a genomic sequence as depicted in SEQ ID NO: 1 and a coding sequence depicted in SEQ ID NO: 2 and the AgsE protein as depicted in SEQ ID NO: 3 with the mature AgsE protein comprised in SEQ ID NO: 3 of WO2014/013074).
The deficiency in the production of at least one product selected from glucoamylase (glaA), acid stable alpha-amylase (amyA), neutral alpha-amylase (amyBI and amyBII), oxalic acid hydrolase (oahA), a toxin, preferably ochratoxin and/or fumonisin, a protease transcriptional regulator prtT, PepA, a product encoded by the gene hdfA and/or hdfB, a non-ribosomal peptide synthase *npsE,* amylase amyC if compared to a parent host cell and measured under the same conditions may already be present in a parent host cell from which a host cell according to the present invention that is deficient in a further product selected from the group consisting of glucoamylase (glaA), acid stable alpha-amylase (amyA), neutral alpha-amylase (amyBI and amyBII), oxalic acid hydrolase (oahA), a toxin, preferably ochratoxin and/or fumonisin, a protease transcriptional regulator prtT, PepA, a product encoded by the gene hdfA and/or hdfB, a non-ribosomal peptide synthase *nps*E*,* amylase amyC is derived. The deficiency in the production of at least one product selected from glucoamylase (glaA), acid stable alpha-amylase (amyA), neutral alpha-amylase (amyBI and amyBII), oxalic acid hydrolase (oahA), a toxin, preferably ochratoxin and/or fumonisin, a protease transcriptional regulator prtT, PepA, a product encoded by the gene hdfA and/or hdfB, a non-ribosomal peptide synthase *npsE,* amylase amyC, protein AgsE if compared to a parent host cell and measured under the same conditions may already be present in a parent host cell from which a host cell according to the present invention that is deficient in a further product selected from the group consisting of glucoamylase (glaA), acid stable alpha-amylase (amyA), neutral alpha-amylase (amyBI and amyBII), oxalic acid hydrolase (oahA), a toxin, preferably ochratoxin and/or fumonisin, a protease transcriptional regulator prtT, PepA, a product encoded by the gene hdfA and/or hdfB, a non-ribosomal peptide synthase *npsE,* amylase amyC, protein AgsE is derived.A preferred host cell according to the present invention comprises a deficiency in the production of glaA and optionally at least another product selected from the group consisting of acid stable alpha-amylase (amyA), neutral alpha-amylase (amyBI and amyBII), oxalic acid hydrolase (oahA), a toxin, preferably ochratoxin and/or fumonisin, a protease transcriptional regulator prtT, PepA, a product encoded by the gene hdfA and/or hdfB, a non-ribosomal peptide synthase *npsE,* amylase amyC if compared to a parent host cell and measured under the same conditions.

A further preferred host cell according to the present invention comprises a deficiency in the production of glaA, PepA and optionally at least another product selected from the group consisting of acid stable alpha-amylase (amyA), neutral alpha-amylase (amyBI and amyBII), oxalic acid hydrolase (oahA), a toxin, preferably ochratoxin and/or fumonisin, a protease transcriptional regulator prtT, a product encoded by the gene hdfA and/or hdfB, a non-ribosomal peptide synthase *npsE,* amylase amyC if compared to a parent host cell and measured under the same conditions.
A further preferred host cell according to the present invention comprises a deficiency in the production of glaA, PepA, acid stable alpha-amylase (amyA) and optionally at least another product selected from the group consisting of neutral alpha-amylase (amyBI and amyBII), oxalic acid hydrolase (oahA), a toxin, preferably ochratoxin and/or fumonisin, a protease transcriptional regulator prtT, a product encoded by the gene hdfA and/or hdfB, a non-ribosomal peptide synthase *npsE,* amylase amyC if compared to a parent host cell and measured under the same conditions.
A further preferred host cell according to the present invention comprises a deficiency in the production of glaA, PepA, acid stable alpha-amylase (amyA), neutral alpha-amylase amyBI and optionally at least another product selected from the group consisting of neutral alpha-amylase amyBII, oxalic acid hydrolase (oahA), a toxin, preferably ochratoxin and/or fumonisin, a protease transcriptional regulator prtT, a product encoded by the gene hdfA and/or hdfB, a non-ribosomal peptide synthase *npsE,* amylase amyC if compared to a parent host cell and measured under the same conditions.
A further preferred host cell according to the present invention comprises a deficiency in the production of glaA, PepA, acid stable alpha-amylase (amyA), neutral alpha-amylase amyBI and amyBII, and optionally at least another product selected from the group consisting of oxalic acid hydrolase (oahA), a toxin, preferably ochratoxin and/or fumonisin, a protease transcriptional regulator prtT, a product encoded by the gene hdfA and/or hdfB, a non-ribosomal peptide synthase *npsE,* amylase amyC if compared to a parent host cell and measured under the same conditions.
A further preferred host cell according to the present invention comprises a deficiency in the production of glaA, PepA, acid stable alpha-amylase (amyA), neutral alpha-amylase amyBI and amyBII, a product encoded by the gene hdfA and optionally at least another product selected from the group consisting of oxalic acid hydrolase (oahA), a toxin, preferably ochratoxin and/or fumonisin, a protease transcriptional regulator prtT, a product encoded by the gene hdfB, a non-ribosomal peptide synthase *npsE,* amylase amyC if compared to a parent host cell and measured under the same conditions.
A further preferred host cell according to the present invention comprises a deficiency in the production of glaA, PepA, acid stable alpha-amylase (amyA), neutral alpha-amylase amyBI and amyBII, a product encoded by the gene hdfA, oxalic acid hydrolase (oahA) and optionally at least another product selected from the group consisting of, a toxin, preferably ochratoxin and/or fumonisin, a protease transcriptional regulator prtT, a product encoded by the gene hdfB, a non-ribosomal peptide synthase *nps*E*,* amylase amyC if compared to a parent host cell and measured under the same conditions.
A further preferred host cell according to the present invention comprises a deficiency in the production of glaA, PepA, acid stable alpha-amylase (amyA), neutral alpha-amylase amyBI and amyBII, a product encoded by the gene hdfA, oxalic acid hydrolase (oahA), ochratoxin, fumonisin, and optionally at least another product selected from the group consisting of a protease transcriptional regulator prtT, a product encoded by the gene hdfB, a non-ribosomal peptide synthase *nps*E*,* amylase amyC if compared to a parent host cell and measured under the same conditions.
A further preferred host cell according to the present invention comprises a deficiency in the production of glaA, PepA, acid stable alpha-amylase (amyA), neutral alpha-amylase amyBI and amyBII, a product encoded by the gene hdfA, oxalic acid hydrolase (oahA), ochratoxin, fumonisin, a protease transcriptional regulator prtT and optionally at least another product selected from the group consisting of a product encoded by the gene hdfB, a non-ribosomal peptide synthase *nps*E*,* amylase amyC if compared to a parent host cell and measured under the same conditions.
A further preferred host cell according to the present invention comprises a deficiency in the production of glaA, PepA, acid stable alpha-amylase (amyA), neutral alpha-amylase amyBI and amyBII, a product encoded by the gene hdfA, oxalic acid hydrolase (oahA), ochratoxin, fumonisin, a protease transcriptional regulator prtT, a non-ribosomal peptide synthase *nps*E and optionally at least another product selected from the group consisting of a product encoded by the gene hdfB, amylase amyC if compared to a parent host cell and measured under the same conditions.
A further preferred host cell according to the present invention comprises a deficiency in the production of glaA, PepA, acid stable alpha-amylase (amyA), neutral alpha-amylase amyBI and amyBII, a product encoded by the gene hdfA, oxalic acid hydrolase (oahA), ochratoxin, fumonisin, a protease transcriptional regulator prtT, amylase amyC and optionally at least another product selected from the group consisting of a product encoded by the gene hdfB, a non-ribosomal peptide synthase *nps*E*,* if compared to a parent host cell and measured under the same conditions.
A further preferred host cell according to the present invention comprises a reduced amylase background and comprises a deficiency in the production of glaA, acid stable alpha-amylase (amyA), neutral alpha-amylase amyBI and amyBII, if compared to a parent host cell and measured under the same conditions. Such host cell preferably also comprises a deficiency in the production of a filamentous fungal homolog of KU70 or KU80. Such host cell preferably also comprises a deficiency in the production of a toxin. Such a host cell preferably also comprises a deficiency in the production of a filamentous fungal homolog of KU70 or KU80 and a deficiency in the production of a toxin.

A further preferred host cell according to the present invention comprises a reduced amylase background and further comprises a deficiency in the production of glaA, acid stable alpha-amylase (amyA), neutral alpha-amylase amyBI, amyBII and amyC if compared to a parent host cell and measured under the same conditions. Such a host cell may preferably also comprises a filamentous fungal homolog of KU70 or KU80. Such host cell preferably also comprises a deficiency in the production of a toxin. Such host cell preferably also comprises a deficiency in the production of a filamentous fungal homolog of KU70 or KU80 and a deficiency in the production of a toxin.
A preferred host cell according to the present invention is a filamentous fungal host cell which comprises a deficiency in the production of glaA and optionally at least another product selected from the group consisting of acid stable alpha-amylase (amyA), neutral alpha-amylase (amyBI and amyBII), oxalic acid hydrolase (oahA), a toxin, preferably ochratoxin and/or fumonisin, a protease transcriptional regulator prtT, PepA, a product encoded by the gene hdfA and/or hdfB, a non-ribosomal peptide synthase *nps*E*,* amylase amyC, a protein AgsE if compared to a parent host cell and measured under the same conditions.
In one embodiment the host cell according to the present invention comprises a deficiency in the production of glaA, PepA, and optionally at least another product selected from the group consisting of acid stable alpha-amylase (amyA), neutral alpha-amylase (amyBI and amyBII), oxalic acid hydrolase (oahA), a toxin, preferably ochratoxin and/or fumonisin, a protease transcriptional regulator prtT, a product encoded by the gene hdfA and/or hdfB, a non-ribosomal peptide synthase *nps*E*,* amylase amyC, a protein AgsE if compared to a parent host cell and measured under the same conditions.
In one embodiment the host cell according to the present invention comprises a deficiency in the production of glaA, PepA, acid stable alpha-amylase (amyA) and optionally at least another product selected from the group consisting of neutral alpha-amylase (amyBI and amyBII), oxalic acid hydrolase (oahA), a toxin, preferably ochratoxin and/or fumonisin, a protease transcriptional regulator prtT, a product encoded by the gene hdfA and/or hdfB, a non-ribosomal peptide synthase *nps*E*,* amylase amyC, a protein AgsE if compared to a parent host cell and measured under the same conditions.
In one embodiment the host cell according to the present invention comprises a deficiency in the production of glaA, PepA, acid stable alpha-amylase (amyA), neutral alpha-amylase amyBI and optionally at least another product selected from the group consisting of neutral alpha-amylase amyBII, oxalic acid hydrolase (oahA), a toxin, preferably ochratoxin and/or fumonisin, a protease transcriptional regulator prtT, a product encoded by the gene hdfA and/or hdfB, a non-ribosomal peptide synthase *nps*E*,* amylase amyC, a protein AgsE if compared to a parent host cell and measured under the same conditions.
In one embodiment the host cell according to the present invention comprises a deficiency in the production of glaA, PepA, acid stable alpha-amylase (amyA), neutral alpha-amylase amyBI and amyBII and optionally at least another product selected from the group consisting of oxalic acid hydrolase (oahA), a toxin, preferably ochratoxin and/or fumonisin, a protease transcriptional regulator prtT, a product encoded by the gene hdfA and/or hdfB, a non-ribosomal peptide synthase *nps*E*,* amylase amyC, a protein AgsE if compared to a parent host cell and measured under the same conditions.
In one embodiment the host cell according to the present invention comprises a deficiency in the production of glaA, PepA, acid stable alpha-amylase (amyA), neutral alpha-amylase amyBI and amyBII, a product encoded by the gene hdfA and optionally at least another product selected from the group consisting of oxalic acid hydrolase (oahA), a toxin, preferably ochratoxin and/or fumonisin, a protease transcriptional regulator prtT, a product encoded by the gene hdfB, a non-ribosomal peptide synthase *nps*E*,* amylase amyC, a protein AgsE if compared to a parent host cell and measured under the same conditions.
In one embodiment the host cell according to the present invention comprises a deficiency in the production of glaA, PepA, acid stable alpha-amylase (amyA), neutral alpha-amylase amyBI and amyBII, a product encoded by the gene hdfA, oxalic acid hydrolase (oahA), and optionally at least another product selected from the group consisting of a toxin, preferably ochratoxin and/or fumonisin, a protease transcriptional regulator prtT, a product encoded by the gene hdfB, a non-ribosomal peptide synthase *nps*E*,* amylase amyC, a protein AgsE if compared to a parent host cell and measured under the same conditions.
In one embodiment the host cell according to the present invention comprises a deficiency in the production of glaA, PepA, acid stable alpha-amylase (amyA), neutral alpha-amylase amyBI and amyBII, a product encoded by the gene hdfA, oxalic acid hydrolase (oahA), a protein AgsE and optionally at least another product selected from the group consisting of a toxin, preferably ochratoxin and/or fumonisin, a protease transcriptional regulator prtT, a product encoded by the gene hdfB, a non-ribosomal peptide synthase *nps*E*,* amylase amyC, if compared to a parent host cell and measured under the same conditions.
In one embodiment the host cell according to the present invention comprises a deficiency in the production of glaA, PepA, acid stable alpha-amylase (amyA), neutral alpha-amylase amyBI and amyBII, a product encoded by the gene hdfA, oxalic acid hydrolase (oahA), a protein AgsE, a toxin, preferably ochratoxin and/or fumonisin, and optionally at least another product selected from the group consisting of a protease transcriptional regulator prtT, a product encoded by the gene hdfB, a non-ribosomal peptide synthase *nps*E*,* amylase amyC, if compared to a parent host cell and measured under the same conditions.
In one embodiment the host cell according to the present invention comprises a deficiency in the production of glaA, PepA, acid stable alpha-amylase (amyA), neutral alpha-amylase amyBI and amyBII, a product encoded by the gene hdfA, oxalic acid hydrolase (oahA), a protein AgsE, a toxin, preferably ochratoxin and/or fumonisin, amylase amyC, and optionally at least another product selected from the group consisting of a protease transcriptional regulator prtT, a product encoded by the gene hdfB, a non-ribosomal peptide synthase *nps*E*,* if compared to a parent host cell and measured under the same conditions.
In one embodiment the host cell according to the present invention comprises a deficiency in the production of glaA, PepA, acid stable alpha-amylase (amyA), neutral alpha-amylase amyBI and amyBII, a product encoded by the gene hdfA, a toxin, preferably ochratoxin and/or fumonisin, and optionally at least another product selected from the group consisting of oxalic acid hydrolase (oahA), a protease transcriptional regulator prtT, a product encoded by the gene hdfB, a non-ribosomal peptide synthase *nps*E*,* amylase amyC, a protein AgsE if compared to a parent host cell and measured under the same conditions.
In one embodiment the host cell according to the present invention comprises a deficiency in the production of glaA, PepA, acid stable alpha-amylase (amyA), neutral alpha-amylase amyBI and amyBII, a product encoded by the gene hdfA, a toxin, preferably ochratoxin and/or fumonisin, amylase amyC, and optionally at least another product selected from the group consisting of oxalic acid hydrolase (oahA), a protease transcriptional regulator prtT, a product encoded by the gene hdfB, a non-ribosomal peptide synthase *nps*E*,* a protein AgsE if compared to a parent host cell and measured under the same conditions.
In one embodiment the host cell according to the present invention comprises a deficiency in the production of glaA, PepA, acid stable alpha-amylase (amyA), neutral alpha-amylase amyBI and amyBII, a product encoded by the gene hdfA, a toxin, preferably ochratoxin and/or fumonisin, a non-ribosomal peptide synthase *nps*E*,* and optionally at least another product selected from the group consisting of oxalic acid hydrolase (oahA), a protease transcriptional regulator prtT, a product encoded by the gene hdfB, amylase amyC, a protein AgsE if compared to a parent host cell and measured under the same conditions.
In one embodiment the host cell according to the present invention comprises a deficiency in the production of glaA, PepA, acid stable alpha-amylase (amyA), neutral alpha-amylase amyBI and amyBII, a product encoded by the gene hdfA, a toxin, preferably ochratoxin and/or fumonisin, a protein AgsE, and optionally at least another product selected from the group consisting of oxalic acid hydrolase (oahA), a protease transcriptional regulator prtT, a product encoded by the gene hdfB, a non-ribosomal peptide synthase *nps*E*,* amylase amyC, if compared to a parent host cell and measured under the same conditions.
In one embodiment the host cell according to the present invention comprises a deficiency in the production of glaA, PepA, acid stable alpha-amylase (amyA), neutral alpha-amylase amyBI and amyBII, a product encoded by the gene hdfA, a toxin, preferably ochratoxin and/or fumonisin, a protein AgsE, amylase amyC, and optionally at least another product selected from the group consisting of oxalic acid hydrolase (oahA), a protease transcriptional regulator prtT, a product encoded by the gene hdfB, a non-ribosomal peptide synthase *nps*E*,* if compared to a parent host cell and measured under the same conditions.

In one embodiment the host cell according to the present invention comprises a deficiency in the production of glaA, PepA, acid stable alpha-amylase (amyA), neutral alpha-amylase amyBI and amyBII, a product encoded by the gene hdfA, a toxin, preferably ochratoxin and/or fumonisin, a protein AgsE, a non-ribosomal peptide synthase *nps*E*,* and optionally at least another product selected from the group consisting of oxalic acid hydrolase (oahA), a protease transcriptional regulator prtT, a product encoded by the gene hdfB, amylase amyC, if compared to a parent host cell and measured under the same conditions.
In one embodiment the host cell according to the present invention comprises a deficiency in the production of glaA, PepA, acid stable alpha-amylase (amyA), neutral alpha-amylase amyBI and amyBII, a product encoded by the gene hdfA, a toxin, preferably ochratoxin and/or fumonisin, amylase amyC, a non-ribosomal peptide synthase *nps*E*,* and optionally at least another product selected from the group consisting of oxalic acid hydrolase (oahA), a protease transcriptional regulator prtT, a protein AgsE, a product encoded by the gene hdfB, if compared to a parent host cell and measured under the same conditions.
In one embodiment the host cell according to the present invention comprises a deficiency in the production of glaA, PepA, acid stable alpha-amylase (amyA), neutral alpha-amylase amyBI and amyBII, a product encoded by the gene hdfA, a toxin, preferably ochratoxin and/or fumonisin, a protein AgsE, amylase amyC, a non-ribosomal peptide synthase *nps*E*,* and optionally at least another product selected from the group consisting of oxalic acid hydrolase (oahA), a protease transcriptional regulator prtT, a product encoded by the gene hdfB, if compared to a parent host cell and measured under the same conditions.
In one embodiment the host cell according to the present invention comprises a deficiency in the production of glaA, PepA, acid stable alpha-amylase (amyA), neutral alpha-amylase amyBI and amyBII, a product encoded by the gene hdfA, a toxin, preferably ochratoxin and/or fumonisin, oxalic acid hydrolase (oahA), and optionally at least another product selected from the group consisting of a protease transcriptional regulator prtT, a product encoded by the gene hdfB, a non-ribosomal peptide synthase *nps*E*,* amylase amyC, a protein AgsE if compared to a parent host cell and measured under the same conditions.
In one embodiment the host cell according to the present invention comprises a deficiency in the production of glaA, PepA, acid stable alpha-amylase (amyA), neutral alpha-amylase amyBI and amyBII, a product encoded by the gene hdfA, a toxin, preferably ochratoxin and/or fumonisin, oxalic acid hydrolase (oahA), a non-ribosomal peptide synthase *nps*E*,* and optionally at least another product selected from the group consisting of a protease transcriptional regulator prtT, a product encoded by the gene hdfB, amylase amyC, a protein AgsE if compared to a parent host cell and measured under the same conditions.
A further preferred host cell according to the present invention comprises a reduced alpha-amylase background and comprises a deficiency in the production of acid stable alpha-amylase (amyA), neutral alpha-amylase amyBI and amyBII and, optionally, amyC if compared to a parent host cell and measured under the same conditions. Such host cell preferably also comprises a filamentous fungal homolog of KU70 or KU80. Such host cell preferably also comprise a deficiency in the production of a toxin. Such host cell preferably also comprises a deficiency in the production of a filamentous fungal homolog of KU70 or KU80 and a deficiency in the production of a toxin.
When a host cell according to the present invention is a filamentous fungal host cell, the host cell preferably further comprises at least two substantially homologous DNA domains suitable for integration of one or more copies of a polynucleotide according to the present invention or of a polynucleotide encoding a compound of interest, wherein at least one of the at least two substantially homologous DNA domains is adapted to have enhanced integration preference for the polynucleotide encoding a compound of interest compared to the substantially homologous DNA domain it originates from, and wherein the substantially homologous DNA domain where the adapted substantially homologous DNA domain originates from has a gene conversion frequency that is at least 10% higher than one of the other of the at least two substantially homologous DNA domains. Such host cells have extensively been described in WO2011/009700. Strains containing two or more copies of these substantially homologous DNA domains are also referred herein as strain containing two or more amplicons. Examples of host cells comprising such amplicons are *inter alia* described in van Dijck et al, 2003, Regulatory Toxicology and Pharmacology 28; 27-35: *On the safety of a new generation of DSM Aspergillus niger enzyme production strains.* In van Dijck et al, an *Aspergillus niger* strain is described that comprises 7 amplified glucoamylase gene loci, i.e. 7 amplicons. Preferred host cells according to the present invention are filamentous fungus host cells, preferably *A*. *niger* host cells, comprising two or more amplicons, preferably two or more Δ*g*/*a*A amplicons, more preferably comprising 2, 3, 4, 5, 6, 7 Δ*g*/*a*A amplicons, wherein the amplicon which has the highest frequency of gene conversion has been adapted to have enhanced integration preference for the polynucleotide according to the present invention or the polynucleotide encoding a compound of interest, compared to the amplicon it originates from. Adaptation of the amplicon can be performed according to any one of the methods described in WO2011/009700. Host cells comprising two or more amplicons wherein one amplicon has been adapted to have enhanced integration preference for a polynucleotide encoding a compound of interest compared to the amplicon it originates from are herein referred as host cells comprising an adapted amplicon. Preferred host cells with adapted amplicons, described in WO2011/009700, are host cells comprising three Δ*g*/*a*A amplicons being a *Bam*HI truncated amplicon, a Sa*l*I truncated amplicon and a *Bgl*II truncated amplicon and wherein the *Bam*HI amplicon has been adapted to have enhanced integration preference for a polynucleotide according to the present invention or polynucleotide encoding a compound of interest, compared to the *Bam*HI amplicon it originates from.
When a host cell according to the present invention is a filamentous fungal host cell, the host cell according to the present invention preferably further comprises a modification of Sec61. A preferred SEC61 modification is a modification which results in a one-way mutant of SEC61; i.e. a mutant wherein the *de novo* synthesized protein can enter the ER via SEC61, but the protein cannot leave the ER via SEC61. Such modifications are extensively described in WO2005/123763. In a preferred embodiment the mutant microbial host cell comprises a modification in a Sec61 as depicted in SEQ ID NO: 3 of WO2005/123763. Most preferably, the SEC 61 modification is the S376W mutation in which Serine 376 is replaced by Tryptophan in SEQ ID NO: 3 of WO2005/123763.
A modification, preferably in the genome, is construed herein as one or more modifications. A modification, preferably in the genome of a host cell according to the present invention, can either be effected by
a) subjecting a parent host cell to recombinant genetic manipulation techniques; and/or
b) subjecting a parent host cell to (classical) mutagenesis; and/or
c) subjecting a parent host cell to an inhibiting compound or composition. Modification of a genome of a host cell is herein defined as any event resulting in a change in a polynucleotide sequence in the genome of the host cell.
Preferably, a host cell according to the present invention has a modification, preferably in its genome which results in a reduced or no production of an undesired compound as defined herein if compared to the parent host cell that has not been modified, when analysed under the same conditions.
A modification can be introduced by any means known to the person skilled in the art, such as but not limited to classical strain improvement, random mutagenesis followed by selection. Modification can also be introduced by site-directed mutagenesis.
Modification may be accomplished by the introduction (insertion), substitution (replacement) or removal (deletion) of one or more nucleotides in a polynucleotide sequence. A full or partial deletion of a polynucleotide coding for an undesired compound such as a polypeptide may be achieved. An undesired compound may be any undesired compound listed elsewhere herein; it may also be a protein and/or enzyme in a biological pathway of the synthesis of an undesired compound such as a metabolite. Alternatively, a polynucleotide coding for said undesired compound may be partially or fully replaced with a polynucleotide sequence which does not code for said undesired compound or that codes for a partially or fully inactive form of said undesired compound. In another alternative, one or more nucleotides can be inserted into the polynucleotide encoding said undesired compound resulting in the disruption of said polynucleotide and consequent partial or full inactivation of said undesired compound encoded by the disrupted polynucleotide.
In one embodiment the mutant microbial host cell according to the invention comprises a modification in its genome selected from
a) a full or partial deletion of a polynucleotide encoding an undesired compound,
b) a full or partial replacement of a polynucleotide encoding an undesired compound with a polynucleotide sequence which does not code for said undesired compound or that codes for a partially or fully inactive form of said undesired compound.
c) a disruption of a polynucleotide encoding an undesired compound by the insertion of one or more nucleotides in the polynucleotide sequence and consequent partial or full inactivation of said undesired compound by the disrupted polynucleotide.
This modification may for example be in a coding sequence or a regulatory element required for the transcription or translation of said undesired compound. For example, nucleotides may be inserted or removed so as to result in the introduction of a stop codon, the removal of a start codon or a change or a frame-shift of the open reading frame of a coding sequence. The modification of a coding sequence or a regulatory element thereof may be accomplished by site-directed or random mutagenesis, DNA shuffling methods, DNA reassembly methods, gene synthesis (see for example Young and Dong, (2004), Nucleic Acids Research 32, (7) electronic access http://nar.oupjournals.org/cgi/reprint/32/7/e59 *or* Gupta et al. (1968), Proc. Natl. Acad. Sci USA, 60: 1338-1344*;* Scarpulla et al. (1982), Anal. Biochem. 121: 356-365*;* Stemmer et al. (1995), Gene 164: 49-53), or PCR generated mutagenesis in accordance with methods known in the art. Examples of random mutagenesis procedures are well known in the art, such as for example chemical (NTG for example) mutagenesis or physical (UV for example) mutagenesis. Examples of site-directed mutagenesis procedures are the QuickChange™ site-directed mutagenesis kit (Stratagene Cloning Systems, La Jolla, CA), the 'The Altered Sites® II in vitro Mutagenesis Systems' (Promega Corporation) or by overlap extension using PCR as described in Gene. 1989 Apr 15;77(1):51-9. (Ho SN, Hunt HD, Horton RM, Pullen JK, Pease LR "Site-directed mutagenesis by overlap extension using the polymerase chain reaction") or using PCR as described in Molecular Biology: Current Innovations and Future Trends. (Eds. A.M. Griffin and H.G.Griffin. ISBN 1-898486-01-8;1995 Horizon Scientific Press, PO Box 1, Wymondham, Norfolk, U.K.).
Preferred methods of modification are based on recombinant genetic manipulation techniques such as partial or complete gene replacement or partial or complete gene deletion.
For example, in case of replacement of a polynucleotide, nucleic acid construct or expression cassette, an appropriate DNA sequence may be introduced at the target locus to be replaced. The appropriate DNA sequence is preferably present on a cloning vector. Preferred integrative cloning vectors comprise a DNA fragment, which is homologous to the polynucleotide and / or has homology to the polynucleotides flanking the locus to be replaced for targeting the integration of the cloning vector to this pre-determined locus. In order to promote targeted integration, the cloning vector is preferably linearized prior to transformation of the cell. Preferably, linearization is performed such that at least one but preferably either end of the cloning vector is flanked by sequences homologous to the DNA sequence (or flanking sequences) to be replaced. This process is called homologous recombination and this technique may also be used in order to achieve (partial) gene deletion.
For example a polynucleotide corresponding to the endogenous polynucleotide may be replaced by a defective polynucleotide, that is a polynucleotide that fails to produce a (fully functional) polypeptide. By homologous recombination, the defective polynucleotide replaces the endogenous polynucleotide. It may be desirable that the defective polynucleotide also encodes a marker, which may be used for selection of transformants in which the nucleic acid sequence has been modified.
Alternatively or in combination with other mentioned techniques, a technique based on *in vivo* recombination of cosmids in *E*. *coli* can be used, as described in: A rapid method for efficient gene replacement in the filamentous fungus Aspergillus nidulans (2000) Chaveroche, M-K., Ghico, J-M. and d'Enfert C; Nucleic acids Research, vol 28, no 22*.*
Alternatively, modification, wherein said host cell produces less of or no protein such as the polypeptide having amylase activity, preferably α-amylase activity as described herein and encoded by a polynucleotide as described herein, may be performed by established anti-sense techniques using a nucleotide sequence complementary to the nucleic acid sequence of the polynucleotide. More specifically, expression of the polynucleotide by a host cell may be reduced or eliminated by introducing a nucleotide sequence complementary to the nucleic acid sequence of the polynucleotide, which may be transcribed in the cell and is capable of hybridizing to the mRNA produced in the cell. Under conditions allowing the complementary anti-sense nucleotide sequence to hybridize to the mRNA, the amount of protein translated is thus reduced or eliminated. An example of expressing an antisense-RNA is shown in Appl. Environ. Microbiol. 2000 Feb; 66(2):775-82. (Characterization of a foldase, protein disulfide isomerase A, in the protein secretory pathway of Aspergillus niger. Ngiam C, Jeenes DJ, Punt PJ, Van Den Hondel CA, Archer DB) or (Zrenner R, Willmitzer L, Sonnewald U. Analysis of the expression of potato uridinediphosphate-glucose pyrophosphorylase and its inhibition by antisense RNA. Planta. (1993); 190(2):247-52*.*).
A modification resulting in reduced or no production of undesired compound is preferably due to a reduced production of the mRNA encoding said undesired compound if compared with a parent microbial host cell which has not been modified and when measured under the same conditions. A modification which results in a reduced amount of the mRNA transcribed from the polynucleotide encoding the undesired compound may be obtained via the RNA interference (RNAi) technique (Mouyna et al., 2004). In this method identical sense and antisense parts of the nucleotide sequence, which expression is to be affected, are cloned behind each other with a nucleotide spacer in between, and inserted into an expression vector. After such a molecule is transcribed, formation of small nucleotide fragments will lead to a targeted degradation of the mRNA, which is to be affected. The elimination of the specific mRNA can be to various extents. The RNA interference techniques described in WO2008/053019, WO2005/05672A1, WO2005/026356A1, Oliveira et al.,; Crook et al., 2014; and/or Barnes et al., may be used at this purpose.
A modification which results in decreased or no production of an undesired compound can be obtained by different methods, for example by an antibody directed against such undesired compound or a chemical inhibitor or a protein inhibitor or a physical inhibitor (Tour O. et al, (2003) Nat. Biotech: Genetically targeted chromophore-assisted light inactivation. Vol.21. no. 12:1505-1508) or peptide inhibitor or an anti-sense molecule or RNAi molecule (R.S. Kamath_et al, (2003) Nature: Systematic functional analysis of the Caenorhabditis elegans genome using RNAi.vol. 421, 231-237).
In addition of the above-mentioned techniques or as an alternative, it is also possible to inhibiting the activity of an undesired compound, or to re-localize the undesired compound such as a protein by means of alternative signal sequences (Ramon de Lucas, J., Martinez O, Perez P., Isabel Lopez, M., Valenciano, S. and Laborda, F. The Aspergillus nidulans carnitine carrier encoded by the acuH gene is exclusively located in the mitochondria. FEMS Microbiol Lett. 2001 Jul 24;201(2):193-8.) or retention signals (Derkx, P. M. and Madrid, S. M. The foldase CYPB is a component of the secretory pathway of Aspergillus niger and contains the endoplasmic reticulum retention signal HEEL. Mol. Genet. Genomics. 2001 Dec;266(4):537-545), or by targeting an undesired compound such as a polypeptide to a peroxisome which is capable of fusing with a membrane-structure of the cell involved in the secretory pathway of the cell, leading to secretion outside the cell of the polypeptide (e.g. as described in WO2006/040340).
Alternatively or in combination with above-mentioned techniques, decreased or no production of an undesired compound can also be obtained, e.g. by UV or chemical mutagenesis (Mattern, I.E., van Noort J.M., van den Berg, P., Archer, D. B., Roberts, I.N. and van den Hondel, C. A., Isolation and characterization of mutants of Aspergillus niger deficient in extracellular proteases. Mol Gen Genet. 1992 Aug;234(2):332-6.) or by the use of inhibitors inhibiting enzymatic activity of an undesired polypeptide as described herein (e.g. nojirimycin, which function as inhibitor for β-glucosidases (Carrel F.L.Y. and Canevascini G. Canadian Journal of Microbiology (1991) 37(6): 459-464; Reese E.T., Parrish F.W. and Ettlinger M. Carbohydrate Research (1971) 381-388)).
In an embodiment of the present invention, the modification in the genome of the host cell according to the invention is a modification in at least one position of a polynucleotide encoding an undesired compound.
A deficiency of a cell in the production of a compound, for example of an undesired compound such as an undesired polypeptide and/or enzyme is herein defined as a mutant microbial host cell which has been modified, preferably in its genome, to result in a phenotypic feature wherein the cell: a) produces less of the undesired compound or produces substantially none of the undesired compound and/or b) produces the undesired compound having a decreased activity or decreased specific activity or the undesired compound having no activity or no specific activity and combinations of one or more of these possibilities as compared to the parent host cell that has not been modified, when analysed under the same conditions.
Preferably, a modified host cell according to the present invention produces 1% less of the un-desired compound if compared with the parent host cell which has not been modified and measured under the same conditions, at least 5% less of the un-desired compound, at least 10% less of the un-desired compound, at least 20% less of the un-desired compound, at least 30% less of the un-desired compound, at least 40% less of the un-desired compound, at least 50% less of the un-desired compound, at least 60% less of the un-desired compound, at least 70% less of the un-desired compound, at least 80% less of the un-desired compound, at least 90% less of the un-desired compound, at least 91% less of the un-desired compound, at least 92% less of the un-desired compound, at least 93% less of the un-desired compound, at least 94% less of the un-desired compound, at least 95% less of the un-desired compound, at least 96% less of the un-desired compound, at least 97% less of the un-desired compound, at least 98% less of the un-desired compound, at least 99% less of the un-desired compound, at least 99.9% less of the un-desired compound, or most preferably 100% less of the un-desired compound.

The present invention is further illustrated by the following examples:

### EXAMPLES

The described invention combines the more direct approach of introducing multiple gRNA cassettes and the efficiency of resulting multiple genome modifications by connecting each new gRNA sequence to a standard component of a to-be-recombined expression vector functional in yeast. These standard DNA fragments would bear the sequences of components of a yeast expression vector (e.g. 2-micron, dominant resistance or auxotrophic marker, colour marker). The gRNA sequences are introduced to the DNA fragments by overlap extension PCR using oligonucleotide primers of e.g. 60 nucleotides in length needing no additional costly purification methods at the oligonucleotide supplier. After transformation of four DNA fragments flanked on each side with individual guide sequences and subsequent *in vivo* recombination of the plasmid, correct transformants are selected based on complementation of an auxotrophic marker or antibiotics (facilitated by the presence of a marker on one of the DNA fragments) and fluoresense /colouration of the colony (facilitated by the presence of a colour marker on one of the DNA fragments). Correct transformants are screened on the targeted modifications by colony PCR, restriction analysis and/or sequencing of the target locus.

### EXAMPLE 1

### Construction of Cas9-expressing pentose-fermenting yeast strain

For demonstration of *S*. *cerevisiae* strain modification using a CRISPR-Cas9 system including a vector according to the present invention, the genetically modified strain BIE272 (described in US20140141473) was used. This strain is able to ferment both hexose sugars (glucose, mannose, galactose) and pentose sugars (xylose and arabinose) because of the introduction of heterologous utilization pathways. Prior to the introduction of a 4gRNA self-assembling vector system according to the invention (see below), BIE272 was modified to express the *Streptococcus pyogenes* Cas9 protein (DiCarlo et al., 2013). The Cas9 expression cassette was integrated in combination with the *nat*MX marker on an integration locus on chromosome 14. The complete sequence of the integration fragment including flanks to the integration locus, Cas9 expression cassette and the *nat*MX marker is included in here as SEQ ID NO 2.

### EXAMPLE 2

### Description of the standard Biobricks for 4gRNA-vector assembly in S. cerevisiae

For the expression of gRNA sequences into *S*. *cerevisiae* a gRNA expression cassette as previously described (DiCarlo et al., 2013) was used. The gRNA expression cassettes comprises the *SNR52* promoter, the gRNA sequence consisting of the guide-sequence (crRNA; 20 nt) and the structural component, followed by the *SUP4* terminator.
For the assembly of a episomally expressed plasmid in yeast there are certain elements which are required for the replication of the plasmid in yeast, or upon which selection can be exerted. The plasmid is assembled from 4 different polynucleotides (hereafter indicated as biobricks) each one corresponding to a polynucleotide consisting of a polynucleotide sequence to be extended in the overlap-extension PCR reaction described in Example 3.
Each biobrick is flanked:
- on the 5'-end terminus by a fragment of the guide RNA expression cassette corresponding to the structural component of the gRNA and SUP4 terminator.
- on the 3'-end terminus by a fragment of the guide RNA expression cassette corresponding to part of the *SNR52* promoter for type III RNA polymerase transcription.

The assembly approach described in this example may be also compatible with hybrid gRNA platforms, like, for example, the Alt-R™ CRISPR-Cas9 System (Integrated DNA Technologies, Leuven, Belgium), by adapting the 5' ends/termini of the BioBrick flanks replacing the structural component sequence by a crRNA sequence similar to a Alt-R™ CRISPR crRNA sequence that excludes the guide-sequence (also named protospacer element). In that case, the generic Alt-R™ CRISPR tracrRNA (Integrated DNA technologies, Leuven, Belgium) has to be included in the transformation or has to be part of one of the BioBricks to form a functional Alt-R™ crRNA:tracrRNA complex.

The biobricks (the polynucleotide sequences to be extended) used in the assembly comprise the following elements:
Brick 1 (ORI-yeast)
   For the plasmid to be maintained in yeast an origin of replication (ORI) is necessary. For the efficient expression of gRNA in yeast an ORI which leads to high copy number of the resulting plasmid is highly desirable. Therefore, the 2-micron (2µ) plasmid sequence (Broach et al.) was selected as element for the plasmid in Brick 1 (SEQ ID NO. 3).
Brick 2 (color marker)
   For easy visual identification of correct transformants a chromogenic or fluorescent protein may be used. There are many varieties of chromogenic or fluorescent proteins such as Red Fluorescent Protein (RFP), Green Fluorescent Protein (GFP), mCherry, dsRed, etcetera. For Brick 2 an expression cassette of Dasher GFP (DNA2.0, Palo Alto, CA, USA) flanked upstream by the *S*. *cerevisae TDH3* promoter, and flanked downstream by the *S*. *cerevisiae ADH1* terminator sequence (SEQ ID NO 4) was selected.
Brick 3 (dominant resistance or auxotrophic marker)
   To allow for selection of transformants any suitable markers suitable for *S*. *cerevisiae* may be used.
   One group of markers are the auxotrophic markers. Upon introduction or complementation of the gene sequence encoding the auxotrophic marker, a previously lacking enzymatic activity in a metabolic pathway towards an essential S. cerevisiae building block (e.g. adenine, leucine, uracil, histidine). Drawback is the need for an auxotrophic strain to be able to use the auxotrophic marker.
   Another group of markers which can be used in prototrophic strains is a dominant resistance marker (DRM). Upon transformation of the gene sequence of a DRM to a host cell gives rise to resistance to an added compound (e.g. antibiotic, antifungicide) in the growth medium whereas the untransformed host cell which did not express the gene sequence would remain sensitive and would not grow. Examples of functional DRMs in yeast are e.g. *kanMX, hphMX,* and *nat*MX (Goldstein & McCusker, 1999). In this example, for Brick 3, *kanMX* was selected as dominant resistance marker in *S*. *cerevisiae* (SEQ ID NO 5).
Brick 4 (bacterial *ampR*/*kanR* and ORI)
   To enable *E*. *coli*-based plasmid amplification for sub-sequential use in another host organism, a biobrick containing a bacterial selection marker (*ampR* or *kanR*) and ORI (e.g. pUC) may be used. In this example, for Brick 4 the *kanR* gene, a bacterial selection marker, and pUC ORI were selected (SEQ ID NO 6).
   Bricks 1, 2, 3, and 4 were produced synthetically (e.g. DNA2.0, Palo Alto, CA, USA) and served as standard templates for the introduction of the 20bp-long polunucleotide coding for the gRNA-sequences by overlap-extension PCR method.

### EXAMPLE 3

### Using the 4gRNA assembly system to delete HXT genes in BIE272

In this example the 4gRNA-vector assembly system was used to delete four hexose transporter gene clusters (*HXT3-HXT6-HXT7* on chromosome IV, *HXTS-HXT1-HXT4* on chromosome VIII, *HXT2* on chromosome XIII, *GAL2* on chromosome XII) in BIE272 rendering this pentose-fermenting strain unable to grow on pentose sugars since the pentose uptake capacity is eliminated with deletion of of the genes *hxt1-hxt2-hxt3-hxt4-hxt5-hxt6-hxt7* (indicated as *hxt1-7* hereafter) and *gal2* (Nijland et al., 2014). The gRNA sequences for *HXT1* (to delete cluster *HXT5-HXT1-HXT4* cluster), *HXT2* (to delete *HXT2*), *HXT3* (to delete cluster *HXT3-HXT6-HXT7*) and *GAL2* (to delete *GAL2*) were selected on the DNA2.0 website (https://www.dna20.com/eCommerce/cas9/input). The guide sequences with score 100 were selected for implementation into the 4gRNA assembly system.

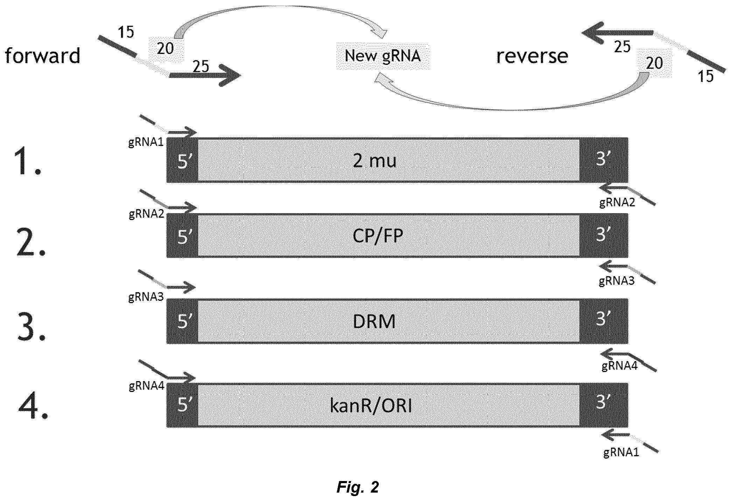

*Extension of Biobricks with gRNA sequences targeting HXT genes by overlap extension PCR* 20-bp polynucleotides coding for the four selected gRNA guide-sequences were added to both the 5'-terminus and the 3'-terminus of each of the Bricks 1 to 4 (previously described above, SEQ ID NOs: 3-6) by overlap extension PCR (see Figure 2 for a schematic representation). The sequences of coding for the four guide-sequences were present in the primer sequences. Table 1, depicts to which Brick the different gRNA sequences were introduced.

**Table 1: orientation of the gRNA sequences to the Bricks**

| **Biobrick** | **5'-flank** | **3'-flank** |
|---|---|---|
| **Brick 1 (2µ)** | ***gRNA1 (HXT1)*** | **gRNA2 (*HXT2*)** |
| **Brick 2 (GFP)** | **gRNA2 (*HXT2*)** | **gRNA3 (*HXT3*)** |
| **Brick 3 (*kanMX*)** | **gRNA3 (*HXT3*)** | **gRNA4 (*GAL2*)** |
| **Brick 4 (*kanR*/ORI)** | **gRNA4 (*GAL2*)** | **gRNA1 (*HXT1*)** |

Generally, for extending the Biobricks with gRNA sequences, oligonucleotide primers consisted of the following parts going from 5'-end to 3'-end direction (see Figure 2 for schematic representation):
a) forward primers (annealing to 5' end of Biobrick)
   - 15 nucleotides of the *SNR52* promoter sequence directly upstream of the gRNA sequence
   - 20 nucleotides of the gRNA sequence, and
   - 25 nucleotides of the structural component directly downstream of the gRNA sequence, namely the nucleotides of the template Biobrick to which the oligonucleotide primer annealed
b) reverse primers (annealing to 3'end of Biobrick)
   - 15 nucleotides of the structural component of the gRNA directly downstream of the gRNA sequence
   - 20 nucleotides of the gRNA sequence, and
   - 25 nucleotides of the *SNR52* promoter sequence directly upstream of the gRNA sequence, namely the nucleotides of the template Biobrick to which the oligonucleotide annealed

Table 2 depicts the sequences in the different parts of the oligonucleotide primers (the 15, the 20, and the 25 nt part, respectively) and Table 3 depicts the full sequences of the oligonucleotide primers used in the OE-PCR reactions.

**Table 2: standardized setup of oligonucleotide primers used in this example**

| **Primer orientation** | **15 (5'→ 3')** | **20 (5'→ 3')** | **25 (5'→ 3')** |
|---|---|---|---|
| **Forward** | | | |
| **Reverse** | | | |

**Table 3: oligonucleotide primers used to fuse gRNA sequences to Biobricks for HXT gene deletions**

| **primer** | **SEQ ID NO** | **Sequence (5'to 3'; in *italics* and underlined the gRNA sequence is depicted )** |
|---|---|---|
| gR1-HXT1-f | 7 | |
| gR2-HXT2-f | 8 | |
| gR3-HXT3-f | 9 | |
| gR4-GAL2-f | 10 | |
| gR1-HXT1-r | 11 | |
| gR2-HXT2-r | 12 | |
| gR3-HXT3-r | 13 | |
| gR4-GAL2-r | 14 | |

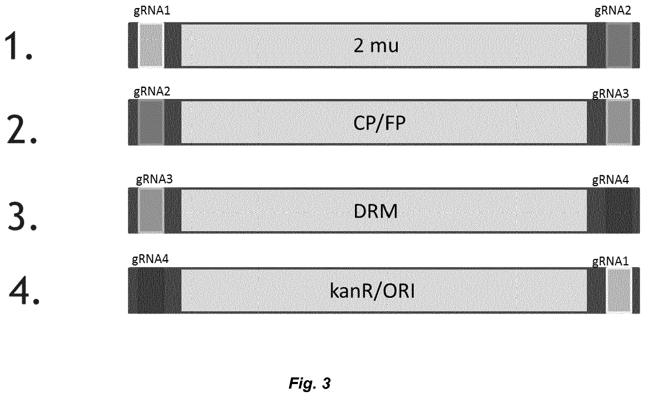

Using the primer design as displayed in Table 2 we have designed forward and reverse primers specifically comprising the gRNA sequences targeting *HXT1, HXT2, HXT3,* and *GAL2,* which were selected as explained above (see SEQ ID NO. 7 to 14). Bricks 1, 2, 3, 4 were extended with the gRNA sequences of gRNA1 (*HXT1*), gRNA2 (*HXT2*), gRNA3 (*HXT3*) or gRNA4 (*GAL2*) in the design as depicted in Table 1 and Figure 2), by overlap extension PCR reaction. In Table 4, the specific combinations of forward primer, reverse primer and template Biobrick were given. PCR reactions were carried out using Phusion High-Fidelity DNA Polymerase (Thermoscientific Landsmeer, the Netherlands) according to the instructions of the supplier. As annealing temperature 60°C was chosen. The resulting PCR products (hybrid linear polynucleotides) with SEQ ID NOs listed in Table 4 were subsequently purified using a standard PCR purification kit (Promega). DNA concentrations were measured on a Nanodrop device (Thermoscientific Landsmeer, the Netherlands). By this way 4 double-stranded DNA products were amplified corresponding to the hybrid linear polynucleotides (indiacated in the examples as extended Biobricks), being 4 linear DNA elements to be used for yeast transformation and *in-vivo* assembly (see Figure 3).

**Table 4: primer combinations for PCR amplifications of gRNA fusion to Bricks 1-4**

| **Brick** | **Template DNA** | **Forward primer (SEQ ID NO)** | **Reverse primer (SEQ ID NO)** | **gRNA 5'** | **gRNA 3'** | **PCR product (SEQ ID NO)** |
|---|---|---|---|---|---|---|
| **1** | **2pORI** | **7** | **12** | ***HXT1*** | ***HXT2*** | **15** |
| **2** | **GFP** | **8** | **13** | ***HXT2*** | ***HXT3*** | **16** |
| **3** | **kanMX** | **9** | **14** | ***HXT3*** | ***GAL2*** | **17** |
| **4** | **kanR/ORI** | **10** | **11** | ***GAL2*** | ***HXT1*** | **18** |

### Co-transformation of extended Biobricks and donor DNA fragments and in vivo assembly of 4gRNA-vector in yeast

To enable the deletion of the four targeted HXT clusters after the targeting of the Cas9-gRNA complex four donor DNA fragments were acquired (IDT, Leuven, Belgium). The donor DNA fragments SEQ ID NO: 19 (*HXTS-HXT1-HXT4-repair*), SEQ ID NO: 20 (*HXT2*-repair), SEQ ID NO: 21 (*HXT3-HXT6-HXT7-repair*), SEQ ID NO: 22 (GAL2-repair) facilitate the repair of the double-stranded break made by Cas9 at the targeted loci of *HXT1, HXT2, HXT3* or *GAL2,* respectively, by homologous recombination, thereby deleting the coding sequences of the targeted HXT genes.

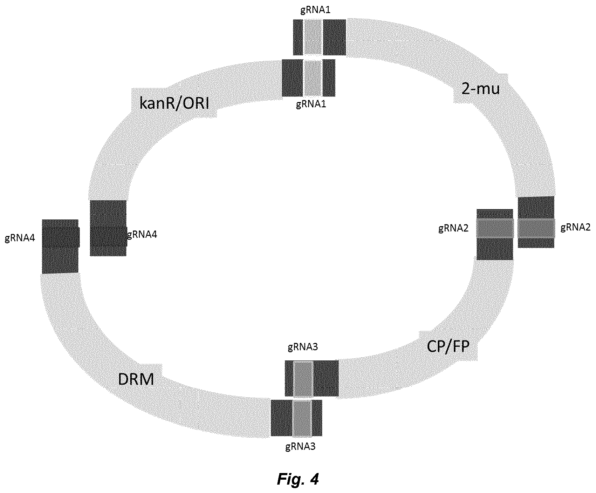

In six separate transformations, the Cas9-expressing BIE272 strain (described in Example 1) was transformed with the extended Biobricks (each transformation with a different concentration namely 31.25, 62.5, 125, 250, 500 and 1000 ng per extended Bricks) obtained above (SEQ ID NOs: 15 to 18), and the donor DNA fragments (1000 ng per donor DNA fragment; SEQ ID NO: 19 to 22). Yeast transformation was done according to the method described by Schiestl and Gietz (Current Genetics (1989), Volume 16, 339-346). 1000x-diluted suspensions of transformed cells were plated on rich growth agar medium (YePhM; 1% w/v yeast extract, 2% w/v phytone, 2% v/v maltose) supplemented with 200 mg/L G418. After 2-3 days of growth at 30°C, colonies appeared on the selection plates. Upon transformation, the PCR products consisting of the 4 extended Bricks have to be assembled in the correct configuration in *S*. *cerevisiae* due to the flanking regions of homology present at the 5'- and 3- termini of each extended biobrick and which comprise the gRNA sequences (see Figure 4 and SEQ ID NOs: 15 to 18). Plates were inspected on a blue light transilluminators for GFP-emitting colonies. Thirty-five colonies of which thirty, which remained GFP-emitting colonies after re-streaking, were selected and were restreaked on yeast nitrogen-base (6.7 g/L yeast nitrogen base w/o amino acids, Sigma-Aldrich, Zwijndrecht) agar (15 g/L) medium supplemented with 2% maltose (YNB-maltose) or 2% xylose (YNB-xylose), and rich growth agar medium (YePhM; 1% w/v yeast extract, 2% w/v phytone, 2% v/v maltose) supplemented with 200 mg/L G418 (YePhM-G418). The restreaked colonies that were able to grow on YePhM-G418, in constract to the untransformed strain BIE272-Cas9, and emitted GFP fluorescence indicated the successful assembly of the extended Biobricks indicating the presence of Brick 3 (*kanMX*) due to the ability to grow on G418, the presence of Brick 2 (GFP) due to the emitted fluorescence, and the presence of Brick 1 (2µ ORI) due to the combined traits (growth on G418 and emitted fluorescence) which could only be possible by the replication of the plasmid in *S*. *cerevisiae* enabled by the 2µ ORI. The restreaked colonies were checked for the intended modifications of the HXT genes by diagnostic colony PCR. Four colonies out of 30 showed an impaired or absent growth profile on YNB-xylose while maintaining growth on YePhM-G418 and YNB-maltose indicating an effect on growth on xylose by the introduction of the transformed extended Biobricks and donor DNA fragments.

Cell material samples of the 30 restreaked colonies growing on YePhM-G418 and GFP-emitting fluorescence were resupended in Y-PER™ (Yeast Protein Extraction Reagent, Life Technologies), according to manufacturer's recommendations, and subsequently boiled to lyse cells. PCR reactions were carried out using Phusion High-Fidelity DNA Polymerase (Thermoscientific Landsmeer, the Netherlands) according to the instructions of the supplier. Suitable oligonucleotide primers were used in the PCR reactions to show the deletions of the different HXT clusters.

In Table 5, the numbers of colonies per intended modification are shown.

**Table 5: Number of colonies showing specific deletions of HXT genes/cluster based on diagnostic colony PCR**

| Deletion | Number of colonies with deletion (x out of 30) |
|---|---|
| *HXT5-HX1-HX74* | 21 |
| *HXT2* | 8 |
| *HXT3-HXT6-HXT7* | 2 |
| *GAL2* | 2 |

Based on these results it can be concluded that by using the 4 gRNA assembly system in BIE272 all intended modifications at the gene loci (*HXT5-HXT1-HXT4, HXT2, HXT3-HXT6-HXT7* and *GAL2*) targeted by the expressed gRNA sequences could be conducted indicating succesfull assembly of plasmids expressing the gRNA sequences.

### EXAMPLE 4

### Using the 4gRNA-vector assembly system to introduce three expression cassettes encoding together a beta-carotene production pathway at three different loci

In this example, the 4gRNA-vector assembly system was used to target three intergenic, non-coding integration loci (INT1, INT59, YPRCtau3) in CEN.PK113-7D to integrate three individual expression casettes, encompassing the three genes of the beta-carotene pathway (crtE, crtYB and crtl from *Xanthophyllomyces dendrorhous*) with each casette targeted to a different integration locus. Upon successful integration and expression of all three genes in the *Saccharomyces cerevisiae* genome, successful transformants will produce colored carotenoid compounds resulting in yellow-, orange- or red-colored transformants (Verwaal *et al.,* 2007). Coloring of the cells is a result of carotenoid production and can be achieved by expressing crtE, crtYB and crtl using promoters and terminators functional in *S*. *cerevisiae* to express these genes (Verwaal *et al.,* 2007). This example demonstrates that by using the 4gRNA-vector assembly method of the invention, the three beta-carotene pathway gene expression cassettes can be transformed to 3 different loci simultaneously, allowing multiplex genome engineering in the genomic DNA of *S*. *cerevisiae,* resulting in colored transformants, reflecting correctly edited cells.

### Construction of a CAS9-expressing CEN.PK113-7D strain

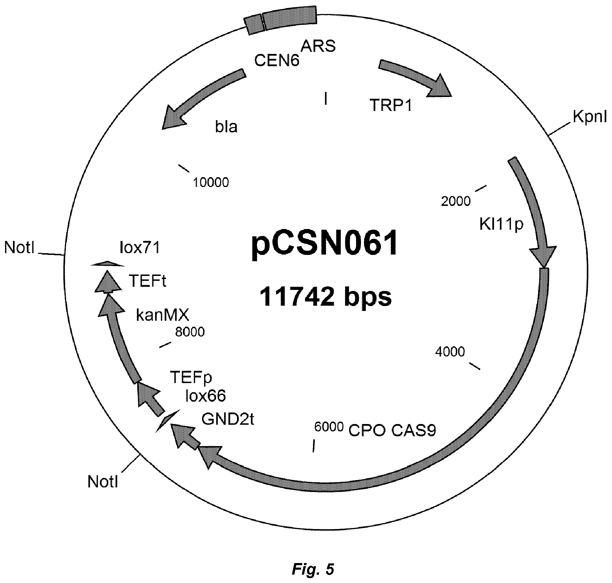

Vector pCSN061 (SEQ ID NO: 23, graphical depiction in Figure 5) containing a CAS9 cassette was first transformed to *S*. *cerevisiae* strain CEN.PK113-7D (MAT*a URA3 HIS3 LEU2 TRP1 MAL2-8 SUC2)* using the LiAc/salmon sperm (SS) carrier DNA/PEG method (Gietz and Woods, 2002). Strain CEN.PK113-7D is available from the EUROSCARF collection (http://www.euroscarf.de, Frankfurt, Germany) or from the Centraal Bureau voor Schimmelcultures (Utrecht, the Netherlands, entry number CBS 8340). The origin of the CEN.PK family of strains is described by van Dijken *et al., 2000.* In the transformation mixture 1 microgram of vector pCNS061 was used. The transformation mixture was plated on YPD-agar (10 grams per litre of yeast extract, 20 grams per litre of peptone, 20 grams per litre of dextrose, 20 grams per litre of agar) containing 200 microgram (µg) G418 (Sigma Aldrich, Zwijndrecht, the Netherlands) per ml. After two to four days of growth at 30°C colonies appeared on the transformation plate. A yeast colony conferring resistance to G418 on the plate, now referred as strain CSN001, was inoculated on YPD-G418 medium (10 grams per litre of yeast extract, 20 grams per litre of peptone, 20 grams per litre of dextrose, 200 µg G418 (Sigma Aldrich, Zwijndrecht, the Netherlands) per ml)..

### Extension of Biobricks with guide sequences by overlap extension PCR

In this example Brick 3b (SEQ ID NO: 24) was used bearing the *nat*MX dominant resistance marker because the *kanMX* marker was used to express Cas9 from episomal plasmid pCSN061 (Figure 5). Different from Example 3, in this example two of the four Bricks, Brick 1 and Brick 2, were extended on both the 5'-terminus and the 3'-terminus with 20-bp polynucleotides coding the selected guide sequences (indicated in Table 6 and in this example as gRNA's). The other two Bricks, Brick 3b and Brick 4, were extended with selected gRNA sequences on either the 5'-terminus or the 3'-terminus, respectively (see Table 6) using the primers as given in Table 7. The other termini (*i.e*. 3'-terminus of Brick 3b and 5'-terminus of Brick 4) were not extended with gRNA sequences, but on each Brick with non-coding 25 nucleotide sequences that are homologous to each other to facilitate *in vivo* recombination.

**Table 6. Orientation of the gRNA sequences to the Bricks**

| **Biobrick** | **5'-terminus** | **3'-terminus** |
|---|---|---|
| Brick 1 (2µ) | gRNA1 (INT59) | gRNA2 (YPRCtau3) |
| Brick 2 (GFP) | gRNA2 (YPRCtau3) | gRNA3 (INT1) |
| Brick 3b (natMX) | gRNA3 (INT1) | No gRNA |
| Brick 4 (*kanR*/ORI) | No gRNA | gRNA1 (INT59) |

**Table 7. Oligonucleotide primers used to fuse guide sequences (or overlapping sequences between Brick 3b and Brick 4) to Biobricks to target integration loci INT59, YPRCtau3, and INT1.**

| **primer** | **SEQ ID NO** | **Sequence (5'to 3'; in *italics* and underlined the gRNA sequence is depicted; grey boxed text is hybridizing nucleotides to respective biobrick)** |
|---|---|---|
| gRl-INT59-f | 25 | |
| gR2-YPRc-f | 26 | |
| gR3-INT1-f | 27 | |
| Brick4_ to3-f | 28 | GCCATCCAGTGTCGAAAACGAGCTCGCGAGCGGTATCAGCTCACTCAAAG |
| gRl-INT59-r | 29 | |
| gR2-YPRc-r | 30 | |
| gR3-INT1-r | 31 | |
| Brick3_ to4-r | 32 | CTTTGAGTGAGCTGATACCGCTCGCGAGCTCGTTTTCGACACTGGATGGC |

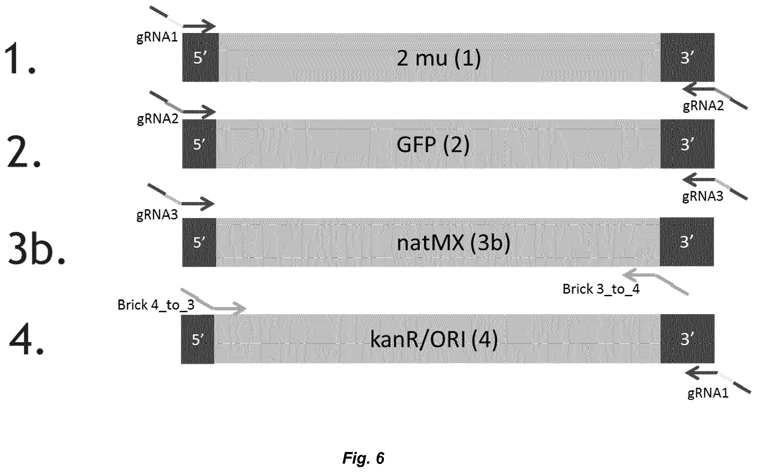

Using the primer design as displayed in Table 2 we have designed forward and reverse primers specifically comprising the gRNA sequences targeting the *INT59, YPRCtau3* and *INT1* loci that were selected for CRISPR/CAS9-mediated integration of crtYB, crtI and crtE expression cassettes as explained above. Bricks 1, 2, 3, 4 were extended with the gRNA sequences of gRNA1 (*INT59*), gRNA2 (*YPRCtau3*), or gRNA3 (*INT1*) by overlap extension PCR reaction in the design as depicted in Table 8. The position for gRNA4 (as depicted in Figure 6) was omitted. Instead, a reverse 50 nt primer was designed hybridizing to a 25 nt sequence directly upstream of the *SNR52* promoter on Brick 3b (primer Brick3_to4-r, SEQ ID NO: 32), and a 50 nt forward primer was designed hybridizing to a 25 nt sequence directly downstream of the *SUP4* terminator on Brick 4 (primer Brick4_to3-f, SEQ ID NO: 28). The remaining 25 nt flanks on each primer facilitated a 50 nt overlap between the resulting PCR-amplification products using Brick 3b (PCR product SEQ ID NO: 35) and Brick 4 (PCR product SEQ ID NO: 36) as templates.

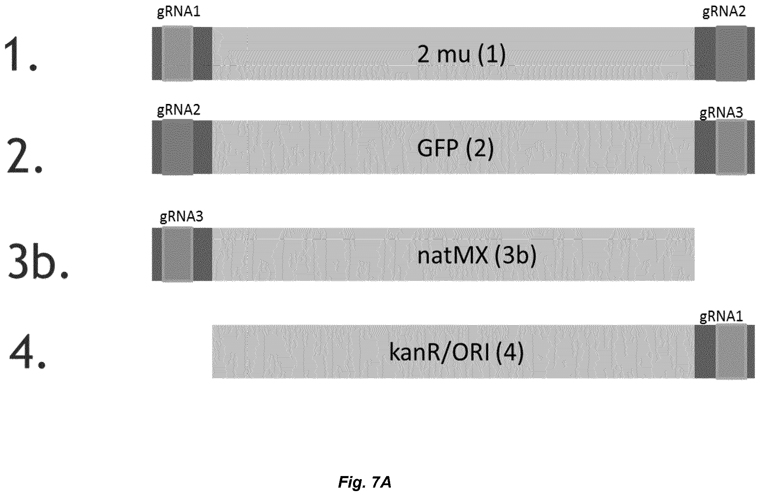

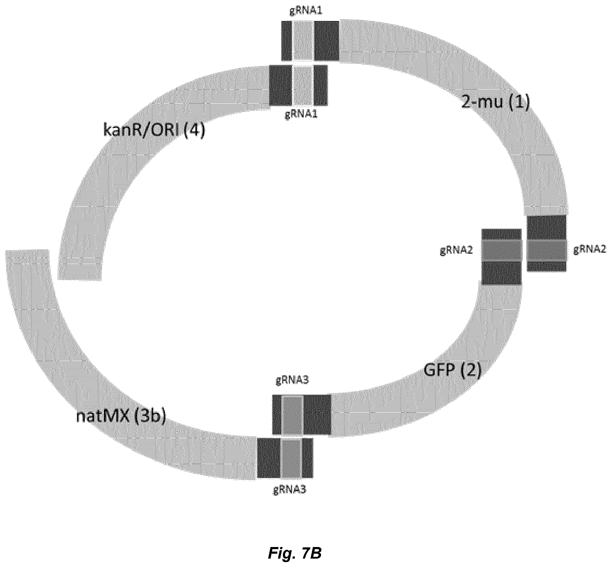

In Table 8, the specific combinations of forward primer, reverse primer and template Biobrick are provided. PCR reactions were carried out using Phusion High-Fidelity DNA Polymerase (Thermoscientific Landsmeer, the Netherlands) according to the instructions of the supplier. As annealing temperature 60°C was chosen. The resulting PCR products (hybrid linear polynucleotides) with SEQ ID NOs listed in Table 8 were subsequently purified using a standard PCR purification kit (Promega). DNA concentrations were measured on a Nanodrop device (Thermoscientific Landsmeer, the Netherlands). By this way 4 double-stranded DNA products were amplified corresponding to the hybrid linear polynucleotides (indicated in the examples as extended Biobricks), being 4 linear DNA elements to be used for yeast transformation as depicted in Figure 7A and *in vivo* assembly into a circular yeast expression vector (see Figure 7B) resulting in SEQ ID NO: 33-36 (see Table 8).

**Table 8. Primer combinations for PCR amplifications of gRNA fusion to Bricks 1, 2, 3b and 4**

| **Brick** | **Template DNA** | **Forward primer (SEQ ID NO)** | **Reverse primer (SEQ ID NO)** | **gRNA 5'** | **gRNA 3'** | **PCR product (SEQ ID NO)** |
|---|---|---|---|---|---|---|
| 1 | **2pORI** | **25** | **30** | ***INT59*** | ***YPRcτ3*** | **33** |
| 2 | **GFP** | **26** | **31** | ***YPRcτ3*** | ***INT1*** | **34** |
| 3b | **natMX** | **27** | **32** | ***INT1*** | **No gRNA** | **35** |
| 4 | **kanR/ORI** | **28** | **29** | **No gRNA** | ***INT59*** | **36** |

### Co-transformation of extended Biobricks together with donor DNA fragments, and in vivo assembly of 4gRNA-vector and multiplex integration of three donor DNA expression cassettes in yeast

To enable multiplex integration of three beta-carotene pathway genes expression cassettes encoding crtE, crtYB and crtl, at the three different integration loci being INT1, INT59 and YPRCtau3, the CRISPR/CAS9 system combined with the 4gRNA-vector assembly system was used. The INT1 integration site is located at the non-coding region between NTR1 (YOR071c) and GYP1 (YOR070c) located on chromosome XV. The INT59 (in this example named INT2) integration site is a non-coding region between SRP40 (YKR092C) and PTR2 (YKR093W) located on chromosome XI. The YPRCtau3 (in this example named INT3) integration site is a Ty4 long terminal repeat, located on chromosome XVI, and has been described by Flagfeldt *et al.* (2009).

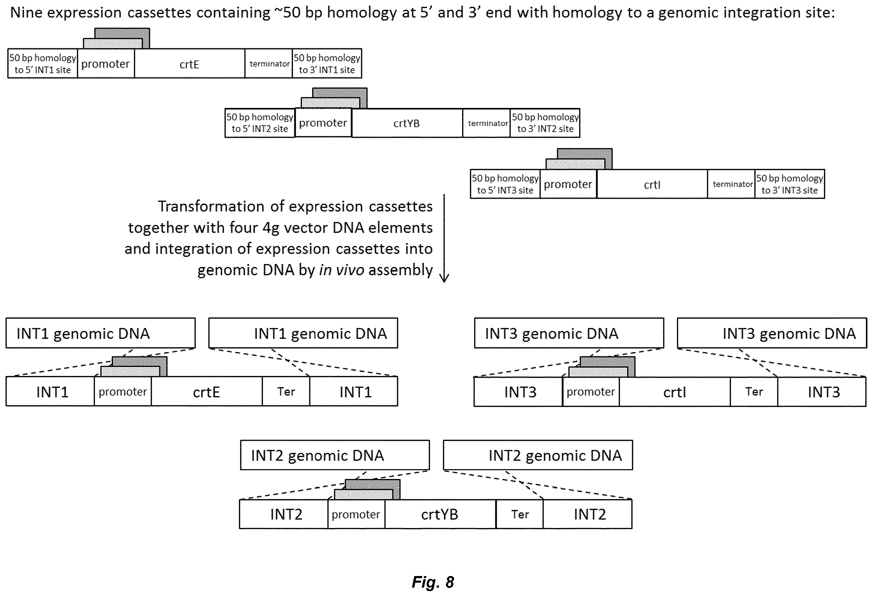

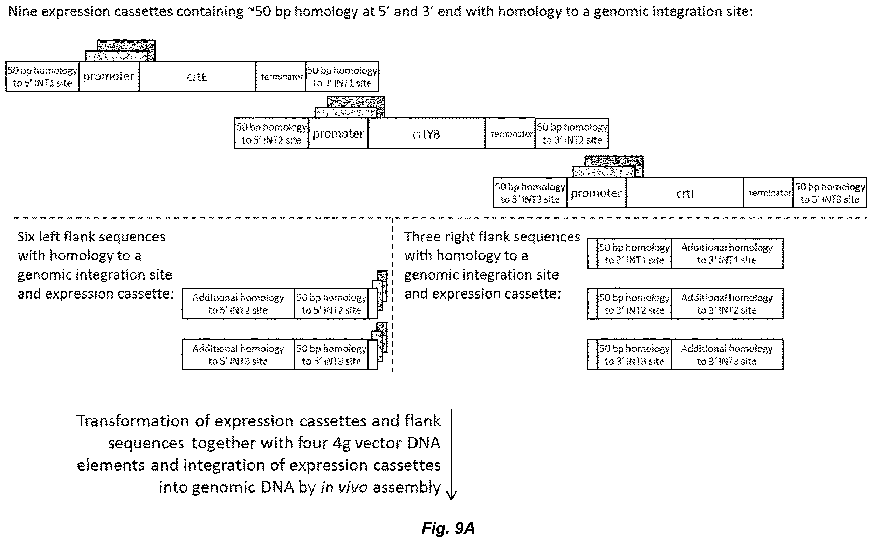

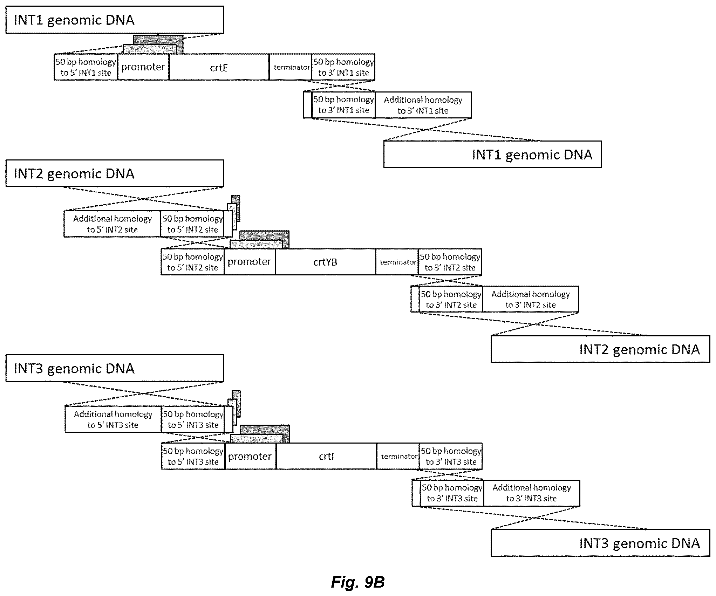

First, donor DNA required for this example was obtained as described below. This donor DNA was subsequently used in two different transformation experiments, which basically differ in the length of DNA homology flanks sequences added in the transformation experiment, as described below and depicted in Figure 8 and Figure 9A and B.

### Donor DNA expression cassette sequences

Carotenoid gene donor DNA expression cassettes, being different crtE, crtYB and crtl expression cassettes, of which the sequences are set out in SEQ ID NO: 47 to SEQ ID NO: 55 (Table 9), were obtained by PCR and were used as donor DNA expression cassettes that were integrated into genomic DNA using the approaches described in this example. PCR fragments of the donor DNA expression cassette and flank sequences were generated by PCR using Phusion DNA polymerase (New England Biolabs, USA) according to manufacturer's instructions. In case of the expression cassettes of the beta-carotene pathway genes, the synthetic DNA provided by DNA2.0 (Menlo Park, USA) was used as a template in the PCR reaction, using the specific forward and reverse primer combinations depicted in Table 9, wherein the PCR reaction is performed according to standard methods known to those skilled in the art. For example, in order to obtain the PCR fragment set out in SEQ ID NO: 47, the synthetic DNA construct SEQ ID NO: 38 provided by DNA2.0 was used as a template, using primer sequences set out in SEQ ID NO: 56 and SEQ ID NO: 57. In total, nine different donor DNA sequences containing the carotenoid gene expression cassettes were generated by PCR, as set out in SEQ ID NO: 47; 48; 49; 50; 51; 52; 53; 54 and 55. All donor DNA PCR fragments were purified using the NucleoSpin Gel and PCR Clean-up kit (Machery-Nagel, distributed by Bioké, Leiden, the Netherlands) according to manufacturer's instructions. DNA concentrations were measured on a Nanodrop device (Thermoscientific Landsmeer, the Netherlands).

Carotenoid gene expression cassettes used in transformation experiment #1 (Figure 8, Table 9) were composed of the following elements: at the 5' and 3' positions of the DNA sequence, approximately 50 basepair (bp) flank sequences were present that contain homology with the desired genomic integration site (INT1, INT2 or INT3). In this example INT1 is the INT1 integration site, INT2 is the INT59 integration site, and INT3 is the YPRCtau3 integration site. The presence of flank sequences allowed introduced of carotenoid expression cassettes into the genomic DNA. As a result, different donor DNA fragments assembled into the genomic DNA at different desired location, as is depicted in Figure 8. A promoter sequence, which can be homologous (*i.e*. from *S*. *cerevisiae*) or heterologous (*e.g*. from *Kluyveromyces lactis*) and a terminator sequences derived from *S*. *cerevisiae,* were used to control the expression of the beta-carotene pathway genes *crtE, crtYB* or *crtI.* As described in Table 9 the promoters were expected to have different expression strengths, resulting in low, medium or high expression levels of crtE, crtYB or crtI. As shown in Example 9 of PCT/EP2016/050136, a combination of crtE, crtYB and crtI expression cassettes with low strength promoters gave the lowest production levels of total carotenoids, medium strength promoters gave higher production levels of total carotenoids and strong promoters gave the highest levels of total carotenoids. The crtE, crtYB and crtI nucleotide sequences were codon pair optimized for expression in *S*. *cerevisiae* as described in WO2008/000632.

**Table 9. Overview of different donor DNA expression cassette sequences used in transformation experiment #1 and transformation experiment #2. Under description, the following elements are indicated: The promoter including the relative expected expression strengths (Low p = low strength promoter, Med p = medium strength promoter, Strong p = high strength promoter). The ORF name, crtE, crtYB or crtI, and the terminator sequence. This table includes the SEQ ID NO's of the primers used to obtain the donor DNA expression cassette sequences by amplification by PCR. INT1: INT1 integration site. INT2: INT59 integration site. INT3: YPRCtau3 integration site.**

| **Donor DNA (expression cassettes)** | **Promoter strength** | **Description** | **Targeting to** | **Synthetic DNA template for PCR** | **Forward primer** | **Reverse primer** |
|---|---|---|---|---|---|---|
| SEQ ID NO: 47 | L | Homology to INT1 - Low p (KITDH2p) - crtE - ScTDH3t - Homology to INT1 | INT1 | SEQ ID NO: 38 | SEQ ID NO: 56 | SEQ ID NO: 57 |
| SEQ ID NO: 48 | M | Homology to INT1 - Med p (KIPGK1 p) - crtE - ScTDH3t - Homology to INT1 | INT1 | SEQ ID NO: 39 | SEQ ID NO: 58 | SEQ ID NO: 57 |
| SEQ ID NO: 49 | S | Homology to INT1 - Strong p (ScFBA1p) - crtE - ScTDH3t - Homology to INT1 | INT1 | SEQ ID NO: 40 | SEQ ID NO: 59 | SEQ ID NO: 57 |
| SEQ ID NO: 50 | L | Homology to INT2 - Low p (KIYDR1 p) - crtYB - ScPDC1t - Homology to INT2 | INT2 | SEQ ID NO: 41 | SEQ ID NO: 60 | SEQ ID NO: 61 |
| SEQ ID NO: 51 | M | Homology to INT2 - Med p (KITEF2p) - crtYB - ScPDC1t - Homology to INT2 | INT2 | SED ID NO: 42 | SEQ ID NO: 62 | SEQ ID NO: 61 |
| SEQ ID NO: 52 | S | Homology to INT2 - Strong p (ScTEF1p) - crtYB - ScPDC1t - Homology to INT2 | INT2 | SEQ ID NO: 43 | SEQ ID NO: 63 | SEQ ID NO: 61 |
| SEQ ID NO: 53 | L | Homology to INT3 - Low p (ScPRE3p) - crtl - ScTAL1t - Homology to INT3 | INT3 | SEQ ID NO: 44 | SED ID NO: 64 | SEQ ID NO: 65 |
| SEQ ID NO: 54 | M | Homology to INT3 - Med p (ScACT1p) - crtl - ScTAL1t - Homology to INT3 | INT3 | SEQ ID NO: 45 | SED ID NO: 66 | SEQ ID NO: 65 |
| SEQ ID NO: 55 | S | Homology to INT3 - Strong p (KIENO1 p) - crtl - ScTAL1t - Homology to INT3 | INT3 | SEQ ID NO: 46 | SED ID NO: 67 | SEQ ID NO: 65 |

### Donor DNA flank sequences

Nine donor DNA flank sequences PCR fragments containing homology with the genomic integration site and the donor DNA expression cassette were included in transformation experiment 2 (Figure 9, Table 10). The donor DNA flanks sequences were obtained as follows: Genomic gDNA was isolated from the yeast strain CEN.PK113-7D (MATa *URA3 HIS3 LEU2 TRP1 MAL2-8 SUC2*) using the lithium acetate SDS method (Lõoke et al., 2011). This genomic DNA was used as a template to obtain the PCR fragments that were used as donor for DNA flanking sequences (comprising the overlap with the genomic DNA for genomic integration), using the specific forward and reverse primer combinations depicted in Table 10. PCR fragments of the donor DNA flank sequences were generated by PCR using Phusion DNA polymerase (New England Biolabs, USA) according to manufacturer's instructions. For example, in order to obtain the Right Flank (RF) INT1 (sequence of the PCR fragment is set out in SEQ ID NO: 68), genomic DNA isolated from strain CEN.PK113-7D was used as a template, using primer sequences set out in SEQ ID NO: 77 and SEQ ID NO: 78. For example, in order to obtain the Left Flank (LF) INT2 sequence with additional homology to the KIYDR2 promoter used for expression of crtYB (sequence of the PCR fragment is set out in SEQ ID NO: 69), genomic DNA isolated from strain CEN.PK113-7D was used as a template, using primer sequences set out in SEQ ID NO: 79 and SEQ ID NO: 80. Six different donor LF DNA flank sequences were generated by PCR, as set out in SEQ ID NO: 69; 70; 71; 73; 74 and 75. Three different RF donor DNA flank sequences were generated by PCR, as set out in SEQ ID NO: 68; 72 and 76. All donor DNA PCR fragments were purified using the NucleoSpin Gel and PCR Clean-up kit (Machery-Nagel, distributed by Bioké, Leiden, the Netherlands) according to manufacturer's instructions. DNA concentrations were measured on a Nanodrop device (Thermoscientific Landsmeer, the Netherlands).

The donor DNA flank sequences contain homology with sequences present in the integration sites and donor DNA expression cassette sequences and allows *in vivo* recombination between integration sites and donor DNA in yeast as depicted in Figure 9. The LF sequences contain at their 5' end homology with the INT2 or INT3 integration site (part of the INT2 and INT3 sequence is present on the crtYB or crtl expression cassette PCR fragment), and contain at their 3' end homology with the specific promoters used in the crtYB and crtl expression cassettes (depicted in Figure 9). The RF sequences contain at their 5' end homology with the specific terminators used in the crtE, crtYB or crtl expression cassettes and contain at the 3' end homology with the INT1, INT2 or INT3 integration site (part of the INT1, INT2 or INT3 sequence is present on the crtE, crtYB and crtI expression cassette PCR fragment respectively; depicted in Figure 9).

**Table 10. Overview of different donor DNA flank sequences additionally used in transformation experiment #2. The homology with genomic DNA indicates the overlap of the flank sequence with the genomic integration site in basepairs (bp).**

| **Donor DNA (flank sequences)** | **Flank** | **Targeting to** | **Description** | **Homology with genomic DNA (bp)** | **Forward primer** | **Reverse primer** |
|---|---|---|---|---|---|---|
| SEQ ID NO: 68 | RF (3') | INT1 | RF INT1 (all crtE cassettes) | 524 | SEQ ID NO: 77 | SEQ ID NO: 78 |
| SEQ ID NO: 69 | LF (5') | INT2 | LF INT2 (crtYB KIYDR1 p) | 322 | SEQ ID NO: 79 | SEQ ID NO: 80 |
| SEQ ID NO: 70 | LF (5') | INT2 | LF INT2 (crtYB KITEF2p) | 322 | SEQ ID NO: 79 | SEQ ID NO: 81 |
| SEQ ID NO: 71 | LF (5') | INT2 | LF INT2 (crtYB ScTEF1p) | 322 | SEQ ID NO: 79 | SEQ ID NO: 82 |
| SEQ ID NO: 72 | RF (3') | INT2 | RF INT2 (all crtYB cassettes) | 496 | SEQ ID NO: 83 | SEQ ID NO: 84 |
| SEQ ID NO: 73 | LF (5') | INT3 | LF INT3 (crtI KIYDR1 p) | 599 | SEQ ID NO: 85 | SEQ ID NO: 86 |
| SEQ ID NO: 74 | LF (5') | INT3 | LF INT3 (crtI KITEF2p) | 599 | SEQ ID NO: 85 | SEQ ID NO: 87 |
| SEQ ID NO: 75 | LF (5') | INT3 | LF INT3 (crtI ScTEF1p) | 599 | SEQ ID NO: 85 | SEQ ID NO: 88 |
| SEQ ID NO: 76 | RF (3) | INT3 | RF INT3 (all crtl cassettes) | 625 | SEQ ID NO: 89 | SEQ ID NO: 90 |

### Transformation experiments

*S*. *cerevisiae* strain CSN001, which is described above under "Construction of a CAS9-expressing CEN.PK113-7D strain", was grown and transformed with the four linear 4gRNA-vector DNA elements outlined in Figure 7 (SEQ ID NO: 33, SEQ ID NO: 34, SEQ ID NO: 35, SEQ ID NO: 36)in two different transformation experiments. In addition, in transformation experiment #1, nine different donor DNA elements comprising beta-carotene expression cassettes (Table 10, Table 11) were transformed as graphically depicted in Figure 8. In addition, in transformation experiment #2, in total eighteen donor DNA elements were transformed, including nine different beta-carotene expression cassettes, six different left flank and three different right flank sequences (Table 10, Table 11) as graphically depicted in Figure 9. Donor DNA expression cassette PCR fragments (see Table 9 for SEQ ID NO's) were used in the transformation experiment #1 and transformation experiment #2. Donor DNA flank PCR fragments (see Table 11 for SEQ ID NO's) were only used in transformation experiment #2. Additional flank sequences were added in transformation experiment #2 to determine whether additional homology of donor DNA by including separate larger flank sequences allows for a higher genome editing efficiency.

**Table 11. Overview of donor DNA and 4gRNA-vector DNA elements used in transformation experiment #1 and transformation experiment #2.**

| **Transformation experiment** | **Description experiment** | **4gRNA-vector DNA elements** | **Donor DNA expression cassettes** | **Flanks sequences** |
|---|---|---|---|---|
| #1 | Nine crt expression cassettes (PCR fragments) containing different low-, medium- and high-strength promoters targeted to INT1, INT2 and INT3 | SEQ ID NO: 33 | SEQ ID NO: 47 | |
| | | SEQ ID NO: 34 | SEQ ID NO: 48 | |
| | | SEQ ID NO: 35 | SEQ ID NO: 49 | |
| | | SEQ ID NO: 36 | SEQ ID NO: 50 | |
| | | | SEQ ID NO: 51 | |
| | | | SEQ ID NO: 52 | |
| | | | SEQ ID NO: 53 | |
| | | | SEQ ID NO: 54 | |
| | | | SEQ ID NO: 55 | |
| #2 | Nine crt expression cassettes (PCR fragments) containing different low-, medium- and high-strength promoters together with six LF and three RF PCR fragments targeted to INT1, INT2 and INT3, together with six LF and three RF PCR fragments | SEQ ID NO: 33 | SEQ ID NO: 47 | SEQ ID NO: 68 |
| | | SEQ ID NO: 34 | SEQ ID NO: 48 | SEQ ID NO: 69 |
| | | SEQ ID NO: 35 | SEQ ID NO: 49 | SEQ ID NO: 70 |
| | | SEQ ID NO: 36 | SEQ ID NO: 50 | SEQ ID NO: 71 |
| | | | SEQ ID NO: 51 | SEQ ID NO: 72 |
| | | | SEQ ID NO: 52 | SEQ ID NO: 73 |
| | | | SEQ ID NO: 53 | SEQ ID NO: 74 |
| | | | SEQ ID NO: 54 | SEQ ID NO: 75 |
| | | | SEQ ID NO: 55 | SEQ ID NO: 76 |

Strain CSN001 was transformed using the LiAc/salmon sperm (SS) carrier DNA/PEG method (Gietz and Woods, 2002). 200 ng of each of the nine beta-carotene pathway donor DNA expression cassettes was included in transformation experiments #1 and #2. 250 ng of each 4gRNA-vector part was included in transformation experiments #1 and #2. 100 ng of each of the nine donor DNA flanks sequences was included in transformation experiment #2. The transformation mixtures were plated on YPD-agar (10 grams per litre of yeast extract, 20 grams per litre of peptone, 20 grams per litre of dextrose, 20 grams per litre of agar) containing 200 µg nourseothricin (NatMX, Jena Bioscience, Germany) and 200 µg G418 (Sigma Aldrich, Zwijndrecht, the Netherlands) per ml. After two to four days of growth at 30°C, colonies appeared on the transformation plates.

After transformation, the total number of colonies on a transformation plate were counted. The transformants were colored and / or non-colored. In case of colored transformants, the *crtE, crtYB* and *crt*I expression cassettes were successfully integrated into the genomic DNA of the yeast cells enabling production of carotenoids (Verwaal et al., 2007; PCT/EP2016/050136). In case of non-colored transformants, *crtE, crtYB* and *crt*I expression cassettes were not successfully integrated into the genomic DNA of the yeast cells. The percentage of successfully engineered cells, i.e. transformants that have integrated the *crtE, crtYB* and *crt*I expression cassettes into genomic DNA, was calculated by dividing the number of colored transformants by the number of total transformants. The results are shown in Table 12.

**Table 12. Percentage colored cells obtained in the two different transformation experiments plated on YPD (2%) + G418 + NatMX agar plates (double selection) to allow selection on both the CAS9 and guide RNA containing vectors.**

| **Transformation experiment** | **Description experiment** | **% Colored cells** |
|---|---|---|
| #1 | Donor DNA with ∼50 bp homology flanks | 37% |
| #2 | Donor DNA with ∼50 bp homology flanks and additional donor DNA flank sequences | 59% |

Based on these results it can be concluded that the 4gRNA-vector assembly system can be used to integrate multiple expression cassettes within the genomic DNA of yeast at different integration sites, and demonstrate that the 4gRNA-vector was successfully assembled in yeast, allowing expressing of multiple functional guide RNA's. These results also demonstrate that including LF and RF flank sequences, creating a higher homology of the donor DNA expression cassettes with the intended chromosomal integration site, increases the genome editing efficiency from 37% to 59%.

This transformation and 4gRNA-vector assembly system in combination with transformation of different donor DNA expression cassettes also allows to obtain a large variety in different transformants that can be obtained. Because nine different donor DNA expression cassettes were included in the transformation, the beta-carotene pathway expression cassettes can be combined to 27 different combinations, resulting in different expression levels of crtE, crtYB and crtI, because of the different promoters used in the expression constructs (Table 13). This resulted in transformants with different yellow, orange or red colors that were obtained after transformation (data not shown), indicative of different combinations of expression cassettes integrated into the genomic DNA of the transformants, and these different transformants are expected to produce different levels of carotenoids. This approach is beneficial for screening purposes, where a large number of different transformants could be screened for different properties, for example for different production levels of a compound or compounds of interests, in this case for different carotenoid production levels.

**Table 13. 27 different combinations can be made when nine different crt expression cassettes (see Table 11) were transformed.**

| **Combination** | **Promoter strength** | **Promoter** | **ORF** |
|---|---|---|---|
| 1 | L | KITDH2 | crtE |
| | L | KIYDR1 | crtYB |
| | L | ScPRE3 | crtI |
| 2 | L | KITDH2 | crtE |
| | L | KIYDR1 | crtYB |
| | M | ScTAL1 | crtI |
| 3 | L | KITDH2 | crtE |
| | L | KIYDR1 | crtYB |
| | H | KIENO1 | crtI |
| 4 | L | KITDH2 | crtE |
| | M | KITEF2 | crtYB |
| | L | ScPRE3 | crtI |
| 5 | L | KITDH2 | crtE |
| | M | KITEF2 | crtYB |
| | M | ScTAL1 | crtI |
| 6 | L | KITDH2 | crtE |
| | M | KITEF2 | crtYB |
| | H | KIENO1 | crtI |
| 7 | L | KITDH2 | crtE |
| | H | ScTEF1 | crtYB |
| | L | ScPRE3 | crtI |
| 8 | L | KITDH2 | crtE |
| | H | ScTEF1 | crtYB |
| | M | ScTAL1 | crtI |
| 9 | L | KITDH2 | crtE |
| | H | ScTEF1 | crtYB |
| | H | KIENO1 | crtI |
| 10 | M | KIPGK1 | crtE |
| | L | KIYDR1 | crtYB |
| | L | ScPRE3 | crtI |
| 11 | M | KIPGK1 | crtE |
| | L | KIYDR1 | crtYB |
| | M | ScTAL1 | crtI |
| 12 | M | KIPGK1 | crtE |
| | L | KIYDR1 | crtYB |
| | H | KIENO1 | crtI |
| 13 | M | KIPGK1 | crtE |
| | M | KITEF2 | crtYB |
| | L | ScPRE3 | crtI |
| 14 | M | KIPGK1 | crtE |
| | M | KITEF2 | crtYB |
| | M | ScTAL1 | crtI |
| 15 | M | KIPGK1 | crtE |
| | M | KITEF2 | crtYB |
| | H | KIENO1 | crtI |
| 16 | M | KIPGK1 | crtE |
| | H | ScTEF1 | crtYB |
| | L | ScPRE3 | crtI |
| 17 | M | KIPGK1 | crtE |
| | H | ScTEF1 | crtYB |
| | M | ScTAL1 | crtI |
| 18 | M | KIPGK1 | crtE |
| | H | ScTEF1 | crtYB |
| | H | KIENO1 | crtI |
| 19 | H | ScFBA1 | crtE |
| | L | KIYDR1 | crtYB |
| | L | ScPRE3 | crtI |
| 20 | H | ScFBA1 | crtE |
| | L | KIYDR1 | crtYB |
| | M | ScTAL1 | crtI |
| 21 | H | ScFBA1 | crtE |
| | L | KIYDR1 | crtYB |
| | H | KIENO1 | crtI |
| 22 | H | ScFBA1 | crtE |
| | M | KITEF2 | crtYB |
| | L | ScPRE3 | crtI |
| 23 | H | ScFBA1 | crtE |
| | M | KITEF2 | crtYB |
| | M | ScTAL1 | crtI |
| 24 | H | ScFBA1 | crtE |
| | M | KITEF2 | crtYB |
| | H | KIENO1 | crtI |
| 25 | H | ScFBA1 | crtE |
| | H | ScTEF1 | crtYB |
| | L | ScPRE3 | crtI |
| 26 | H | ScFBA1 | crtE |
| | H | ScTEF1 | crtYB |
| | M | ScTAL1 | crtI |
| 27 | H | ScFBA1 | crtE |
| | H | ScTEF1 | crtYB |
| | H | KIENO1 | crtI |

### EXAMPLE 5

### Using the 1gRNA-vector assembly system to delete chromosomal DNA

In this example, a 1gRNA-vector assembly system was used to target a GFP expression cassette to the INT1 locus and to achieve deletion of approximately 1000 basepairs (1 kilobase pairs (kb)) or approximately 10,000 basepairs (10 kb) of genomic DNA at the INT1 locus. The INT1 integration site is located at the non-coding region between NTR1 (YOR071c) and GYP1 (YOR070c) located on chromosome XV.

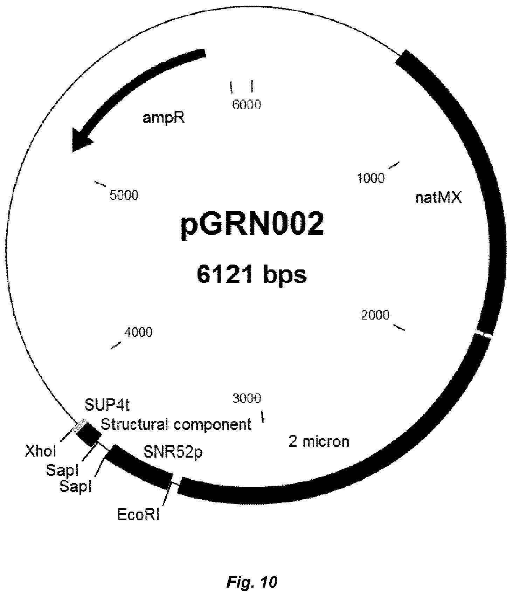

pRN1120 (SEQ ID NO: 91) is a yeast multi-copy vector (2 micron) that contains a functional NatMX marker cassette conferring resistance against nourseothricin. The backbone of this vector is based on pRS305 (Sikorski and Hieter, 1989), including a functional 2 micron ORI sequence and a functional NatMX (nourseothricin resistance) marker cassette (see http://www.euroscarf.de). The Sapl restriction site was removed from the pRN1120 backbone by PCR using the primers set out in SEQ ID NO: 92 and SEQ ID NO: 93, changing the *Sap*I restriction site from GCTCTTC to cCTCTTC. Recircularization of the intermediate PCR fragment without a Sapl site was performed using the KLD enzyme mix of the Q5 site directed mutagenesis kit (New England Biolabs, supplied by Bioké, Leiden, the Netherlands. Catno. E0554S) according to the supplier's manual. The resulting plasmid was digested by *Eco*RI and *Xho*I*.* By Gibson assembly a gBlock containing amongst others a SNR52 promoter, a guide RNA structural component and a SUP4 terminator sequence (Integrated DNA Technologies, Leuven, Belgium), sequence provided in SEQ ID NO: 94, was added to the pRN1120-Sapl backbone. Gibson assembly is performed using Gibson Assembly HiFi 1 Step Kit (SGi-DNA, La Jolla, CA, USA. Catno. GA1100-50) according to supplier's manual. The resulting plasmid was designated pGRN002 (SEQ ID NO: 95, Figure 10), containing a SNR52 promoter, a guide RNA structural component and a SUP4 terminator sequence in which the guide sequence can be cloned/assembled by making use of the Sapl sites. For the expression of gRNA sequences in *S*. *cerevisiae,* a gRNA expression cassette as previously described by DiCarlo *et al.,* 2013 was used. The gRNA expression cassettes comprises the *SNR52* promoter, the gRNA sequence consisting of the guide-sequence or guide sequence, and the guide RNA structural component, followed by the *SUP4* terminator. The guide sequence used in this example will guide the CAS9 protein to the INT1 location in genomic DNA of *S*. *cerevisiae.*

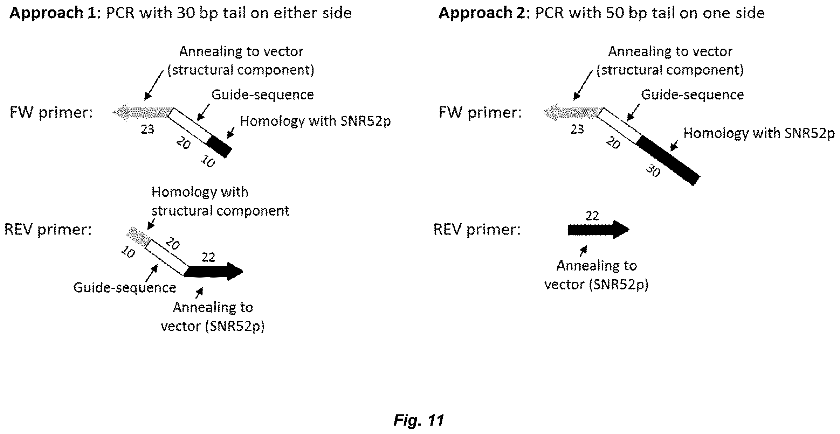

Vector pGRN002 was used as a template in two different PCR reactions using oligonucleotide primers indicated in Table 14 and graphically depicted in Figure 11. The oligonucleotide primers consist of the following parts going from the 5'-end to the 3'-end direction:
a) Forward primer used to obtain PCR fragment #1 (SEQ ID NO: 96):
   - 10 nucleotides of the *SNR52* promoter sequence directly upstream of the guide sequence,
   - 20 nucleotides of the guide sequence, and
   - 23 nucleotides of the structural component of the gRNA directly downstream of the guide sequence, to which the oligonucleotide annealed in the pGRN002 vector.
b) Reverse primer used to obtain PCR fragment #1 (SEQ ID NO: 97):
   - 10 nucleotides of the structural component of the gRNA directly downstream of the guide sequence,
   - 20 nucleotides of the guide sequence, and
   - 22 nucleotides of the *SNR52* promoter sequence directly upstream of the guide sequence, to which the oligonucleotide annealed in the pGRN002 vector.
c) Forward primer used to obtain PCR fragment #2 (SEQ ID NO: 99):
   - 30 nucleotides of the *SNR52* promoter sequence directly upstream of the guide sequence,
   - 20 nucleotides of the guide sequence, and
   - 23 nucleotides of the structural component of the gRNA directly downstream of the guide sequence, to which the oligonucleotide annealed in the pGRN002 vector.
d) Reverse primer used to obtain PCR fragment #2 (SEQ ID NO: 100):
   - 22 nucleotides of the *SNR52* promoter sequence directly upstream of the guide sequence, to which the oligonucleotide annealed in the pGRN002 vector.

The Q5 DNA polymerase (part of the Q5® High-Fidelity 2X Master Mix, New England Biolabs, supplied by Bioké, Leiden, the Netherlands. Catno. M0492S) was used in the PCR reaction, which was performed according to manufacturer's instructions.

**Table 14. Primer combinations for PCR amplifications to obtain 1gRNA-vector PCR fragment #1 and PCR fragment #2.**

| **Description** | **Template DNA** | **Forward primer (SEQ ID NO:)** | **Reverse primer (SEQ ID NO:)** | **Guide sequence** | **PCR product (SEQ ID NO:)** |
|---|---|---|---|---|---|
| 1gRNA PCR fragment #1 | pGRN002 | 96 | 97 | INT1 | 98 |
| 1gRNA PCR fragment #2 | pGRN002 | 99 | 100 | INT1 | 101 |

The resulting PCR fragment #1 contains on the 5'-end/terminus a part of the guide RNA expression cassette corresponding to the *SNR52* promoter and the guide sequence, and on the 3'-end/terminus a fragment of the guide RNA expression cassette corresponding to the guide sequence and the structural component of the **gRNA** (Figure 12, SEQ ID NO: 98). The resulting PCR fragment #2 contains on the 5'-end/terminus a part of the guide RNA expression cassette corresponding to the *SNR52* promoter and the guide sequence, and on the 3'-end/terminus a fragment of the guide RNA expression cassette corresponding to the *SNR52* promoter (Figure 12). The presence of homologous sequences allows *in vivo* recombination in S. *cerevisiae,* resulting in a circular vector as graphically depicted in Figure 12, encoding a functional guide RNA. The NatMX marker present on the circular vector can be used for selection transformants on nourseothricin. PCR fragment #1 was used for 1gRNA-vector assembly approach 1, where homology for *in vivo* recombination was obtained by part of the SNR52 promoter, guide sequence and part of the guide RNA structural component sequences. PCR fragment #2 was used for 1gRNA-vector assembly approach 2, where homology for *in vivo* recombination was obtained by part of the SNR52 promoter sequence only.

To achieve deletion up to approximately 1kb pr 10 kb around the INT1 locus in the genomic DNA of yeast, PCR fragment #1 or PCR fragment #2 were transformed together with donor DNA sequences (left flank, GFP expression cassette, right flank, Table 15 and Table 16 to strain CSN001, which is pre-expressing CAS9 from a single copy KanMX containing vector (see Example 4). A schematic representation is shown in Figure 13. Transformations were performed using the LiAc/SS carrier DNA/PEG method (Gietz and Woods, 2002). Deletion of 1 kb of genomic DNA around the INT1 integration site (approximately 500 bp upstream, approximately 500 bp downstream of the INT1 genomic target) or deletion of 10 kb of genomic DNA around the INT1 integration site (approximately 5 kb upstream, approximately 5 kb downstream of the INT1 genomic target) was expected to result in viable transformants, because no essential genes were fully or partially removed from the genomic DNA (source *Saccharomyces* genome database, http://www.yeastgenome.org/).

Deletion of 1 kb of genomic DNA is exemplified below and is depicted in Figure 13: PCR fragment A (SEQ ID NO: 104), containing 450 bp homology with genomic DNA (5'flank A), was generated using the oligonucleotide sequences as set out in SEQ ID NO: 102 and SEQ ID NO: 103 and using a gBlock (SEQ ID NO: 104) as template (IDT, Leuven, Belgium). PCR fragment C (SEQ ID NO: 109), containing green fluorescent protein (GFP) expression cassette expressed from a *S*. *cerevisiae* TDH3 promoter and *S*. *cerevisiae* EN01 terminator, was generated using the oligonucleotide sequences as set out in SEQ ID NO: 107 and SEQ ID NO: 108, using SEQ ID NO: 109 as template (a synthetic DNA cassette synthesized by DNA 2.0, Menlo Park, CA, USA). PCR fragment D (SEQ ID NO: 112), containing 581 bp homology with genomic DNA (3'flank A), was generated using the oligonucleotide sequences as set out in SEQ ID NO: 110 and SEQ ID NO: 111 using genomic DNA isolated from *S*. *cerevisiae* strain CEN.PK113-7D as template (genomic DNA was isolated according to the method described by Lõoke *et al.,* 2011). PCR reactions were performed by methods known by the person skilled in the art. Due to the presence of connector sequences, the 3' part of PCR fragment A has homology with the 5' part of PCR fragment C, and the 5' part of PCR fragment D has homology with the 3' part of fragment C, which allows homologous recombination into the genome of the yeast *Saccharomyces cerevisiae* (see Figure 13) as is described in WO2013144257A1. Because CAS9 was targeted to the INT1 sequence present in the genomic DNA, a double strand break was introduced. The presence of homologous sequences will promote homologous recombination, and thus repair of the double stranded break and integration of the donor DNA sequences.

Deletion of 10 kb of genomic DNA is exemplified below and is depicted in Figure 13: PCR fragment B (SEQ ID NO: 106), containing 450 bp homology with genomic DNA (5'flank B), was generated using the oligonucleotide sequences as set out in SEQ ID NO: 105 and SEQ ID NO: 103 and using a gBlock (SEQ ID NO: 107) as template (IDT, Leuven, Belgium). PCR fragment C (SEQ ID NO: 109), containing green fluorescent protein (GFP) expression cassette expressed from a S. *cerevisiae* TDH3 promoter and *S*. *cerevisiae* ENO1 terminator, was generated using the oligonucleotide sequences as set out in SEQ ID NO: 107 and SEQ ID NO: 108, using SEQ ID NO: 109 as template (a synthetic DNA cassette synthesized by DNA 2.0, Menlo Park, CA, USA). PCR fragment E (SEQ ID NO: 115), containing 607 bp homology with genomic DNA (3'flank B), was generated using the oligonucleotide sequences as set out in SEQ ID NO: 113 and SEQ ID NO: 114 using genomic DNA isolated from *S*. *cerevisiae* strain CEN.PK113-7D as template (genomic DNA was isolated according to the method described by Lõoke *et al.,* 2011). PCR reactions were performed by methods known by the person skilled in the art. Due to the presence of connector sequences, the 3' part of PCR fragment B has homology with the 5' part of PCR fragment C, and the 5' part of PCR fragment E has homology with the 3' part of fragment C, which allows homologous recombination into the genome of the yeast *Saccharomyces cerevisiae* (see Figure 13) as is described in WO2013144257A1. Because CAS9 was targeted to the INT1 sequence present in the genomic DNA, a double strand break was introduced. The presence of homologous sequences will promote homologous recombination, and thus repair of the double stranded break and integration of the donor DNA sequences.

All donor DNA PCR fragments were purified using the NucleoSpin Gel and PCR Clean-up kit (Machery-Nagel, distributed by Bioké, Leiden, the Netherlands) according to manufacturer's instructions.

**Table 15: Overview of PCR primers used to generate the PCR fragments (left flank (LF) GFP expression cassette, right flank (RF)), used to delete 1 kb or 10 kb of genomic DNA surrounding the INT1 integration site (see Figure 13).**

| **Name** | **Description** | **Template** | **Forward primer (SEQ ID NO:)** | **Reverse primer (SEQ ID NO:)** | **PCR product (SEQ ID NO:)** |
|---|---|---|---|---|---|
| Fragment A | LF 1 kb deletion | gBlock | 102 | 103 | 104 |
| Fragment B | LF 10 kb deletion | gBlock | 105 | 103 | 106 |
| Fragment C | GFP expression cassette | Synthetic gene | 107 | 108 | 109 |
| Fragment D | RF 1 kb deletion | Genomic DNA | 110 | 111 | 112 |
| Fragment E | RF 10 kb deletion | Genomic DNA | 113 | 114 | 115 |

Four transformation experiments were performed in *Saccharomyces cerevisiae* strain CSN0001 (Example 4), according to the combinations of donor DNA and the 1gRNA-vector component as presented in Table 16. Transformation was performed using the LiAc/SS carrier DNA/PEG method (Gietz and Woods, 2002). Transformation mixtures were plated on YPD-agar (10 grams per litre of yeast extract, 20 grams per litre of peptone, 20 grams per litre of dextrose, 20 grams per litre of agar) containing 200 µg G418 (Sigma Aldrich) and 200 µg nourseothricin (NatMX, Jena Bioscience, Germany) per ml. KanMX conferred resistance to transformants containing vector pCSN061, expressing CAS9. NatMX conferred resistance to transformants containing the *in vivo* assembled circular vector of 1gRNA-vector approach 1 and 1gRNA-vector approach 2. After two to four days of growth at 30°C, colonies appeared on the transformation plates.

**Table 16: PCR fragments included in the transformation experiments.**

| **Transformation** | **Description** | **1gRNA-vector** | **Left Flank** | **GFP cassette** | **Right Flank** |
|---|---|---|---|---|---|
| #1 | 1 kb deletion, approach 1 | 1gRNA PCR fragment #1 (SEQ ID NO: 98) | Fragment A (SEQ ID NO: 104) | Fragment C (SEQ ID NO: 109) | Fragment D (SEQ ID NO: 112) |
| #2 | 1 kb deletion, approach 2 | 1gRNA PCR fragment #2 (SEQ ID NO: 101) | Fragment A (SEQ ID NO: 104) | Fragment C (SEQ ID NO: 109) | Fragment D (SEQ ID NO: 112) |
| #3 | 10 kb deletion, approach 1 | 1gRNA PCR fragment #1 (SEQ ID NO: 98) | Fragment B (SEQ ID NO: 106) | Fragment C (SEQ ID NO: 109) | Fragment E (SEQ ID NO: 115) |
| #4 | 10 kb deletion, approach 2 | 1gRNA PCR fragment #2 (SEQ ID NO: 101) | Fragment B (SEQ ID NO: 106) | Fragment C (SEQ ID NO: 109) | Fragment E (SEQ ID NO: 115) |

Transformation of fragment A (5'flank A-Con5), fragment C (Con5-GFP expression cassette-Con3) and fragment D (Con3-3'flank A) resulted in the introduction of the GFP expression cassette and deletion of approximately 1 kb of the genomic DNA sequence. Transformation of fragment B (5'flank B-Con5), fragment C (Con5-GFP expression cassette-Con3) and fragment E (Con3-3'flank B) resulted in the introduction of the GFP expression cassette and deletion of approximately 10 kb of the genomic DNA sequence.

By UV light (Qpix 450 Colony Picker - Molecular devices LLC) a discrimination was made between green fluorescent colonies, indicating GFP integration, and white colonies, indicating no GFP integration, that appeared on the plates. The total number white and green fluorescent colonies on a transformation plate were counted. In case of green fluorescent transformants, the donor DNA was successfully integrated into the genomic DNA of the yeast cells. InThe percentage of successfully engineered cells, i.e. transformants that have integrated the GFP expression cassette and flank sequences into genomic DNA, was calculated by dividing the number of green fluorescent transformants by the number of total transformants (Table 17).

**Table 17: Results of the transformation experiments.**

| **Transformation** | **Description** | **Number of fluorescent transformants** | **Total number of transformants** | **% Edited transformants** |
|---|---|---|---|---|
| #1 | 1 kb deletion, approach 1 | 8 | 9 | 89 |
| #2 | 1 kb deletion, approach 2 | 8 | 9 | 89 |
| #3 | 10 kb deletion, approach 1 | 2 | 3 | 67 |
| #4 | 10 kb deletion, approach 2 | 4 | 5 | 80 |

The results of the transformation experiments (Table 17) demonstrate that the two different 1gRNA-vector approaches as depicted Figure 12 resulted in obtaining GFP fluorescent strains, indicating that both 1gRNA-vector approaches can be used for CRISPR-CAS9 mediated genome engineering experiments.

This experiment clearly shows that one can apply the 1gRNA system in a fast, cheap and flexible way to create functional gRNA expression vectors by OE-PCR making use of short oligonucleotide primers and *in-vivo* recombination, using a standardized basic vector element that already contains all parts of an autonomously replicating vector and the structural parts of a gRNA sequence and control sequences which allow expression of the gRNA in the host cell, but without the guide sequence.

This method could also be applied to create linear autonomously replicating vectors, by using two fragments with OE- PCR at both left and right flanks to be assembled in a 1gRNA linear vector or optionally in a multiple gRNA linear vector.

### REFERENCES

Aleksenko and Clutterbuck. Fungal Genet. Biol. 1997 21: 373-397. Autonomous plasmid replication in Aspergillus nidulans: AMA1 and MATE elements.
Bao et al., 2015, ACS Synth Biol, volume 4 pp. 585-94. Homology-Integrated CRISPR-Cas (HI-CRISPR) System for One-Step Multigene Disruption in Saccharomyces cerevisiae*.*
Barnes et al., siRNA as a molecular tool for use in Aspergillus niger (2008) Biotechnology Letters 30 (5): 885-890.
Becker and Guarente, In Abelson, J. N. and Simon, M. I., editors, Guide to Yeast Genetics and Molecular Biology, Methods in Enzymology, Volume 194, 182-187, Academic Press, Inc., New York.
Beetham PR, Kipp PB, Sawycky XL, Arntzen CJ and May GD. PNAS 1999, 96, 8774-8778. A tool for functional plant genomics: Chimeric RNA/DNA oligonucleotides cause in vivo gene-specific mutations.
Broach et al., 1991 In: Broach, JR.; Pringle, JR.; Jones, EW., editors. The Molecular Biology of the Yeast Saccharomyces. Genome Dynamics, Protein Synthesis and Energetics. Cold Spring Harbor Laboratory Press; Cold Spring harbor, New York. pp. 287-331
Burke DT, et al. Cloning of large segments of exogenous DNA into yeast by means of artificial chromosome vectors. Science 236: 806-812, 1987
Christianson TW, Sikorski RS, Dante M, Shero JH, Hieter P. Gene. 1992 Jan 2;110(1):119-22. Multifunctional yeast high-copy-number shuttle vectors.
Crook NC, Schmitz AC, Alper HS. ACS Synth Biol. 2014 16;3(5):307-13. Optimization of a yeast RNA interference system for controlling gene expression and enabling rapid metabolic engineering.
DiCarlo JE, Norville JE, Mali P, Rios X, Aach J, Church GM. Nucleic Acids Res. 2013 Apr;41(7):4336-43. Genome engineering in Saccharomyces cerevisiae using CRISPR-Cas systems.
Dong C, Beetham P, Vincent K and Sharp P. 2006 Plant Cell Rep 25: 457-465. Oligonucleotide-directed gene repair in wheat using a transient plasmid repair assay system.
J.A. Doudna, E. Charpentier Science (2104) 346: 1258096 DOI:10.1126/science.1258096. The new frontier of genome engineering with CRISPR-Cas9.
Durai S, Mani M, Kandavelou K, Wu J, Porteus M, Chandrasegaran S. Nucleic Acids Res 2005 33 (18): 5978-90. Zinc finger nucleases: custom-designed molecular scissors for genome engineering of plant and mammalian cells.
Finbarr Hayes (2003). "Chapter 1 - The Function and Organization of Plasmids". In Nicola Casali, Andrew Presto. E. Coli Plasmid Vectors: Methods and Applications. Methods in Molecular Biology, Vol. 235. Humana Press. pp. 1-5. ISBN 978-1-58829-151-6.
Flagfeldt DB, Siewers V, Huang L, Nielsen J. Yeast. 2009 Oct;26(10):545-51. Characterization of chromosomal integration sites for heterologous gene expression in Saccharomyces cerevisiae*.*
Gaj T, Gersbach, C and Barbas C. Trends in Biotechnology, 2013, Vol. 31, No. 7 397-405. ZFN, TALEN, and CRISPR/Cas-based methods for genome engineering.
Gao Y and Zhao Y. J Integr Plant Biol. 2014 Apr;56(4):343-9. Self-processing of ribozyme-flanked RNAs into guide RNAs in vitro and in vivo for CRISPR-mediated genome editing.
Gems D., Johnstone I.L., Clutterbuck A.J. Gene 1991 98(1):61-67. An autonomously replicating plasmid transforms Aspergillus nidulans at high frequency.
Gietz RD, Woods RA. Methods Enzymol. 2002;350:87-96. Transformation of yeast by lithium acetate/single-stranded carrier DNA/polyethylene glycol method.
Goldstein, A.L., and McCusker, J.H. Yeast 1999. 15, 1541-15. Three new dominant drug resistance cassettes for gene disruption in Saccharomyces cerevisiae.
Guilinger JP, Thompson DB, Liu DR. Nat Biotechnol. 2014 577-582. Fusion of catalytically inactive Cas9 to Fokl nuclease improves the specificity of genome modification.
Güldener, U., Heck, S., Fiedler, T., Beinhauer, J., and Hegemann, J.H. Nucleic Acids Research 1996. 24, 2519-2524. A new efficient gene disruption cassette for repeated use in budding yeast.
Heckman K.L., Pease L.R. Nature Protocols 2007 2(4): 924-932, Gene splicing and mutagenesis by PCR-driven overlap extension
Horton R.M., Hunt H.D., Ho S.N., Pullen K., Pease L.R. Gene 1989 77: 61-68, Engineering bybrid genes without the use of restriction enzymes: gene splicing by overlap extension
Horwitz A.A., Walter J.M., Schubert M.G., Kung S.H., Hawkings K., Platt D.M., Hernday A.D., Mahatdejkul-Meadows T., Szeto W., Chandran S.S., Newman J.D. , Cell Systems 2015 1: 1-9, http://dx.doi.org/10.1016/j.cels.2015.02.001. Efficient Multiplexed Integration of Synergistic Alleles and Metabolic Pathways in Yeasts via CRISPR-Cas.
Hsu PD, Lander ES, Zhang F. Cell. 2014 Jun 5;157(6):1262-78. Development and applications of CRISPR-Cas9 for genome engineering.
Ito et al., 1983, Journal of Bacteriology 153: 163.
Jacobs JZ, Ciccaglione KM, Tournier V, Zaratiegui M. Nat Commun. 2014 Oct 29;5:5344. Implementation of the CRISPR-Cas9 system in fission yeast.
Jakočiunas et al., 2015, ACS Synth Biol, http://dx.doi.org/10.1021/acssynbio.5b00007. CasEMBLR: Cas9-Facilitated Multiloci Genomic Integration of in Vivo Assembled DNA Parts in Saccharomyces cerevisiae*.*
Jørgensen TR, Park J, Arentshorst M, van Welzen AM, Lamers G, Vankuyk PA, Damveld RA, van den Hondel CA, Nielsen KF, Frisvad JC, Ram AF. Fungal Genet Biol. 2011 May;48(5):544-53. The molecular and genetic basis of conidial pigmentation in Aspergillus niger.
Kleinstiver BP, Pattanayak V, Prew MS, Tsai SQ, Nguyen NT, Zheng Z, Joung JK, Nature. 2016 Jan 28;529(7587):490-5. High-fidelity CRISPR-Cas9 nucleases with no detectable genome-wide off-target effects.
Kornberg R. Trends in Cell Biology 1999 9 (12): M46 Eukaryotic transcriptional control.
Kuijpers et al. Microbial Cell Factories 2013, 12:47. A versatile, efficient strategy for assembly of multi-fragment expression vectors in Saccharomyces cerevisiae using 60 bp synthetic recombination sequences.
Larson, M. H.; Gilbert, L. A.; Wang, X; Lim, W. A.; Weissman, J. S.; Qi, L. S. Nature Protocols 2013 8 (11) 2180-96. CRISPR interference (CRISPRi) for sequence-specific control of gene expression.
Lõoke M, Kristjuhan K, Kristjuhan A. Biotechniques. 2011 May;50(5):325-8. Extraction of genomic DNA from yeasts for PCR-based applications.
Mali P, Yang L, Esvelt KM, Aach J, Guell M, DiCarlo JE, Norville JE, Church GM. Science. 2013 Feb 15;339(6121):823-6. RNA-guided human genome engineering via Cas9.
Mans et al., 2015, FEMS Yeast Res, volume 15, doi: 10.1093/femsyr/fov004. CRISPR/Cas9: a molecular Swiss army knife for simultaneous introduction of multiple genetic modifications in Saccharomyces cerevisiae
Marck C, Kachouri-Lafond R, Lafontaine I, Westhof E, Dujon B, Grosjean H. Nucleic Acids Res. 2006 Apr 5;34(6):1816-35. The RNA polymerase III-dependent family of genes in hemiascomycetes: comparative RNomics, decoding strategies, transcription and evolutionary implications.
Mouyna I, Henry C, Doering TL, Latgé JP. FEMS Microbiol Lett. 2004 Aug 15;237(2):317-24. Gene silencing with RNA interference in the human pathogenic fungus Aspergillus fumigatus.
Nakamura, Y., et al. Nucl. Acids Res. 2000 28:292. Codon usage tabulated from the international DNA sequence databases: status for the year 2000.
Nelson CE, Gersbach CA, Nat Biotechnol. 2016 Mar 10;34(3):298-9. Cas9 loosens its grip on off-target sites.
Nijland et al., Engineering of an endogenous hexose transporter into a specific D-xylose transporter facilitates glucose-xylose co-consumption in Saccharomyces cerevisiae, Biotechnol Biofuels 2014, 7, 168
Oliveira et al., Efficient cloning system for construction of gene silencing vectors in Aspergillus niger (2008) Appl. Microbiol. and Biotechnol. 80 (5): 917-924.
Ran FA, Hsu PD, Lin CY, Gootenberg JS, Konermann S, Trevino AE, Scott DA, Inoue A, Matoba S, Zhang Y, Zhang F. Cell 2013 154, 1380-1389. Double nicking by RNA-guided CRISPR Cas9 for enhanced genome editing specificity.
Ran FA, Cong L, Yan WX, Scott DA, Gootenberg JS, Kriz AJ, Zetsche B, Shalem O, Wu X, Makarova KS, Koonin EV, Sharp PA, Zhang F, Nature. 2015 Apr 9;520(7546):186-91. In vivo genome editing using Staphylococcus aureus Cas9.
Raymond C.K., Pownder T.A., Sexson S.L. Biotechniques 1999, 26: 134-141. General method for plasmid construction using homologous recombination.
Ryan et al., eLife, 2014, 3. doi: 10.7554/eLife.03703. Selection of chromosomal DNA libraries using a multiplex CRISPR system
Ryan OW, Skerker JM, Maurer MJ, Li X, Tsai JC, Poddar S, Lee ME, DeLoache W, Dueber JE, Arkin AP, Cate JH. Elife. 2014. 19;3. 03703.
Sambrook & Russell, Molecular Cloning: A Laboratory Manual, 3rd Ed., CSHL Press, Cold Spring Harbor, NY, 2001; and Ausubel et al., Current Protocols in Molecular Biology, Wiley InterScience, NY, 1995
Sander JD, Joung JK. Nat Biotechnol. 2014 Apr;32(4):347-55. doi: 10.1038/nbt.2842. Epub 2014 Mar 2. CRISPR-Cas systems for editing, regulating and targeting genomes.
Sikorski RS, Hieter P. Genetics. 1989 May;122(1):19-27. A system of shuttle vectors and yeast host strains designed for efficient manipulation of DNA in Saccharomyces cerevisiae.
Slaymaker IM, Gao L, Zetsche B, Scott DA, Yan WX, Zhang F, Science. 2016 Jan 1;351(6268):84-8. Rationally engineered Cas9 nucleases with improved specificity.
Takahashi S, Nakajima Y, Imaizumi T, Furuta Y, Ohshiro Y, Abe K, Yamada RH, Kera Y. Development of an autonomously replicating linear vector of the yeast Cryptococcus humicola by using telomere-like sequence repeats. Appl Microbiol Biotechnol. 2011 Feb;89(4):1213-21
Tsai SQ, Wyvekens N, Khayter C, et al. Nat Biotechnol. 2014 32(6):569-576. Dimeric CRISPR RNA-guided Fokl nucleases for highly specific genome editing.
van Dijken JP, Bauer J, Brambilla L, Duboc P, Francois JM, Gancedo C, Giuseppin ML, Heijnen JJ, Hoare M, Lange HC, Madden EA, Niederberger P, Nielsen J, Parrou JL, Petit T, Porro D, Reuss M, van Riel N, Rizzi M, Steensma HY, Verrips CT, Vindeløv J, Pronk JT. An interlaboratory comparison of physiological and genetic properties of four Saccharomyces cerevisiae strains. Enzyme Microb Technol. 2000 Jun 1;26(9-10):706-714.
Verwaal R, Wang J, Meijnen JP, Visser H, Sandmann G, van den Berg JA, van Ooyen AJ. Appl Environ Microbiol. 2007 Jul;73(13):4342-50. Epub 2007 May 11. High-level production of beta-carotene in *Saccharomyces cerevisiae* by successive transformation with carotenogenic genes from *Xanthophyllomyces dendrorhous.*
Wah, D. A.; J. Bitinaite, Schildkraut, I., Aggarwal, A. K. Proc Natl Acad Sci USA 1998 95 (18): 10564-9. Structure of Fokl has implications for DNA cleavage.
Zetsche B, Gootenberg JS, Abudayyeh OO, Slaymaker IM, Makarova KS, Essletzbichler P, Volz SE, Joung J, van der Oost J, Regev A, Koonin EV, Zhang F, Cell. 2015 Oct 22;163(3):759-71. Cpf1 is a single RNA-guided endonuclease of a class 2 CRISPR-Cas system.
Zhang G, Kong II, Kim H, Liu J, Cate JH, Jin YS. Appl Environ Microbiol. 2014 Dec 15;80(24):7694-701. doi: 10.1128/AEM.02310-14. Epub 2014 Oct 3. Construction of a quadruple auxotrophic mutant of an industrial polyploidy Saccharomyces cerevisiae using RNA-guided Cas9 nuclease.

## Claims

1. Method to produce a circular vector comprising two or more guide-polynucleotide expression cassettes, wherein said two or more guide-polynucleotide expression cassettes comprise a polynucleotide encoding a guide-polynucleotide operably linked to one or more control sequences which direct the expression of said guide-polynucleotide in a host cell, wherein said guide-polynucleotide comprises a guide-sequence that essentially is the reverse complement of a target-polynucleotide in a host cell and wherein the guide-polynucleotide can direct binding of a Cas protein at a target-polynucleotide in a host cell to form a CRISPR-Cas complex, wherein the method comprises the following steps:
- providing two or more polynucleotides, wherein the two or more polynucleotides comprise a polynucleotide sequence to be extended, wherein the polynucleotide sequence to be extended comprises at the 5'-end and/or at the 3'-end to be extended a fragment of a guide-polynucleotide expression cassette, wherein said fragment of a guide-polynucleotide expression cassette at the 5'-end and/or at the 3'-end of the polynucleotide sequence to be extended, does not comprise a polynucleotide coding for a guide sequence;
- performing two or more overlap-extension PCR reactions by subjecting in each reaction one of said two or more polynucleotides and two suitable polynucleotide primers wherein at least one suitable primer comprises a polynucleotide coding for a guide sequence to yield one hybrid linear polynucleotide,
wherein the two or more polynucleotide sequence to be extended and the suitable polynucleotide primers are selected so that each hybrid linear polynucleotide obtained in the two or more overlap-extension PCR reactions comprises at least a polynucleotide coding for a guide sequence and suitable 5'-termini and 3'-termini which allow assembly *in vivo* of the two or more hybrid linear polynucleotides and optionally of one or more additional linear polynucleotides with each other in a pre-defined order to yield a circular vector comprising two or more functional guide-polynucleotide expression cassettes;
- subjecting the two or more hybrid linear polynucleotides obtained in the overlap-extension PCR and optionally one or more additional linear polynucleotides to an assembly reaction yielding a circular vector comprising one or more functional guide-polynucleotide expression cassettes,
wherein the assembly reaction occurs *in vivo,*
preferably wherein the guide-polynucleotide is a gRNA.

2. The method according to claim 1 wherein the circular vector comprises three or more guide-polynucleotide expression cassettes.

3. The method according to claim 1 or 2, wherein the one or more polynucleotide sequence to be extended or additional linear polynucleotide comprises one or more elements selected from the group consisting of: an origin of replication or a fragment thereof, a selectable marker or a fragment thereof, a CAS9 expression cassette or a fragment thereof, a donor polynucleotide or a fragment thereof.

4. The method according to claim 3 wherein the circular vector obtained after assembly comprises one or more elements selected from the group consisting of: an origin of replication, a selectable marker, a CAS9 expression cassette, a donor polynucleotide or a combination of one or more of said elements, preferably wherein the circular vector obtained after assembly further comprises an origin of replication, preferably an origin of replication and a selectable marker.

5. A method of assembling a circular vector comprising two or more guide-polynucleotide expression cassettes *in vivo,* wherein said two or more guide-polynucleotide expression cassette comprises a polynucleotide encoding a guide-polynucleotide operably linked to one or more control sequences which direct the expression of said guide-polynucleotide in a host cell, wherein said guide-polynucleotide comprises a guide-sequence that essentially is the reverse complement of a target-polynucleotide in a host cell and wherein the guide-polynucleotide can direct binding of a Cas protein at a target-polynucleotide in a host cell to form a CRISPR-Cas complex, wherein the method comprises the following steps:
- providing two or more polynucleotides, wherein the two or more polynucleotides comprise a polynucleotide sequence to be extended, wherein the polynucleotide sequence to be extended comprises at the 5'-end and/or at the 3'-end to be extended a fragment of a guide polynucleotide expression cassette;
- performing two or more overlap-extension PCR reactions by subjecting in each reaction one of said one or more polynucleotides and two suitable polynucleotide primers wherein at least one suitable primer comprises a polynucleotide coding for a guide sequence to yield one hybrid linear polynucleotide,
- wherein the two or more polynucleotide sequences to be extended and the suitable polynucleotide primers are selected so that each hybrid linear polynucleotide obtained in the two or more overlap-extension PCR reactions comprises at least a polynucleotide coding for a guide sequence and suitable 5'-termini and 3'-termini which allow assembly *in vivo* of the two or more hybrid linear polynucleotides and optionally of one or more additional linear polynucleotides with each other in a pre-defined order to yield a circular vector comprising one or more functional guide-polynucleotide expression cassettes;-subjecting the two or more hybrid linear polynucleotides obtained in the overlap extension PCR and optionally one or more additional linear polynucleotides to an assembly reaction yielding the circular vector;
- providing one or more linear polynucleotides, wherein said one or more linear polynucleotides comprise at the 5'-terminus and/or at the 3'-terminus at least one guide-polynucleotide expression cassette or a fragment of at least one guide-polynucleotide expression cassette comprising at least the guide sequence;
- transforming said one or more linear polynucleotides, and optionally one or more additional linear polynucleotides into a host cell;
- allowing an homologous recombination reaction to take place between said one or more linear polynucleotides and optionally one or more additional linear polynucleotides, wherein said linear polynucleotides and additional linear polynucleotides have been selected to comprise suitable 5'-termini and 3'-termini which allow homologous recombination of said one or more linear polynucleotides and optionally of said one or more additional linear polynucleotides with each other in a pre-defined order to yield a circular vector comprising one or more functional guide-polynucleotide expression cassettes;
- selecting a host cell comprising a circular vector and wherein said vector comprises one or more functional guide-polynucleotide expression cassettes;
- recovering the circular vector.

6. A method for producing a recombinant host cell comprising a circular vector, wherein said vector comprises two or more guide-polynucleotide expression cassettes, wherein said two or more guide-polynucleotide expression cassettes comprises a polynucleotide encoding a guide-polynucleotide operably linked to one or more control sequences which direct the expression of said guide-polynucleotide in a host cell, wherein said guide-polynucleotide comprises a guide-sequence that essentially is the reverse complement of a target-polynucleotide in a host cell and wherein the guide-polynucleotide can direct binding of a Cas protein at a target-polynucleotide in a host cell to form a CRISPR-Cas complex, wherein the method comprises the following steps:
- providing one or more linear polynucleotides, wherein said one or more linear polynucleotides comprise at the 5'-terminus and/or at the 3'-terminus at least one guide-polynucleotide expression cassette or a fragment of at least one guide-polynucleotide expression cassette comprising at least the guide sequence;
- transforming said one or more linear polynucleotides, and optionally one or more additional linear polynucleotides into a host cell;
- allowing an homologous recombination reaction to take place between said one or more linear polynucleotides and optionally one or more additional linear polynucleotides, wherein said linear polynucleotides and additional linear polynucleotides have been selected to comprise suitable 5'-termini and 3'-termini which allow homologous recombination of said one or more linear polynucleotides and optionally of said one or more additional linear polynucleotides with each other in a pre-defined order to yield a circular vector comprising one or more functional guide-polynucleotide expression cassettes;
- optionally selecting a host cell comprising a circular vector and wherein said vector comprises one or more functional guide-polynucleotide expression cassettes,
wherein the one or more linear polynucleotides are hybrid linear polynucleotides obtained by
- providing two or more polynucleotides, wherein the two or more polynucleotides comprise a polynucleotide sequence to be extended, wherein the polynucleotide sequence to be extended comprises at the 5'-end and/or at the 3'-end to be extended a fragment of a guide-polynucleotide expression cassette, preferably wherein the fragment of a guide-polynucleotide expression cassette at the 5'-end and/or at the 3'-end of the polynucleotide sequence to be extended, does not comprise a polynucleotide coding for a guide sequence;
- performing two or more overlap-extension PCR reactions by subjecting in each reaction one of said two or more polynucleotides and two suitable polynucleotide primers to yield one hybrid linear polynucleotide,
wherein the two or more polynucleotide sequence to be extended and the suitable polynucleotide primers are selected so that each hybrid linear polynucleotide obtained in the two or more overlap-extension PCR reactions comprises at least a polynucleotide coding for a guide sequence and suitable 5'-termini and 3'-termini which allow assembly *in vivo* of the two or more hybrid linear polynucleotides and optionally of one or more additional linear polynucleotides with each other in a pre-defined order to yield a circular vector comprising one or more functional guide-polynucleotide expression cassettes.

7. A method for producing a recombinant host cell according to claim 6 further comprising the step of:
- recovering the circular vector;
- transforming the circular vector in a second host cell;
- optionally isolating the second host cell comprising the circular vector.

8. A method for producing a recombinant host cell comprising a circular vector, wherein said vector comprises two or more guide-polynucleotide expression cassettes, wherein said two or more guide-polynucleotide expression cassette comprises a polynucleotide encoding a guide-polynucleotide operably linked to one or more control sequences which direct the expression of said guide-polynucleotide in a host cell, wherein said guide-polynucleotide comprises a guide-sequence that essentially is the reverse complement of a target-polynucleotide in a host cell and wherein the guide-polynucleotide can direct binding of a Cas protein at a target-polynucleotide in a host cell to form a CRISPR-Cas complex, wherein the method comprises the following steps:
- performing a method of assembling a circular vector comprising two or more guide-polynucleotide expression cassettes *in vivo* according to claim 6 in a first host cell, preferably a first host cell belonging to the species *S. cerevisiae,* wherein said first host cell comprising the circular vector, is selected and the circular vector is recovered, wherein said vector comprises two or more functional guide-polynucleotide expression cassettes,
- transforming the circular vector in a second host cell;
- optionally isolating the second host cell comprising the circular vector.

9. A method according to any one of the preceding claims, wherein the assembly reaction occurs in a host cell belonging to *S. cerevisiae* species.

10. A method according to any one of claims 7 to 9 wherein the second host cell is a prokaryotic, such as a bacterial host cell, or an eukaryotic host cell, preferably a fungal host cell.

11. The method according to anyone of claims 1 to 10 wherein the circular vector comprises at least 2 or more functional guide polynucleotide expression cassettes.

## Patentansprüche

1. Verfahren zur Herstellung eines zirkulären Vektors, der zwei oder mehr Guide-Polynukleotid-Expressionskassetten umfasst, wobei die zwei oder mehr Guide-Polynukleotid-Expressionskassetten ein Polynukleotid, das ein Guide-Polynukleotid codiert, in operativer Verknüpfung mit einer oder mehreren Kontrollsequenzen, die die Expression des Guide-Polynukleotids in einer Wirtszelle steuern, umfassen, wobei das Guide-Polynukleotid eine Guide-Sequenz umfasst, bei der es sich im Wesentlichen um das Rückwärtskomplement eines Ziel-Polynukleotids in einer Wirtszelle handelt, und wobei das Guide-Polynukleotid die Bindung eines Cas-Proteins an einem Ziel-Polynukleotid in einer Wirtszelle steuern kann, so dass ein CRISPR-Cas-Komplex gebildet wird, wobei das Verfahren die folgenden Schritte umfasst:
- Bereitstellen von zwei oder mehr Polynukleotiden, wobei die zwei oder mehr Polynukleotide eine zu verlängernde Polynukleotidsequenz umfassen, wobei die zu verlängernde Polynukleotidsequenz am 5'-Ende und/oder am 3'-Ende, das verlängert werden soll, ein Fragment einer Guide-Polynukleotid-Expressionskassette umfasst, wobei das Fragment einer Guide-Polynukleotid-Expressionskassette am 5'-Ende und/oder am 3'-Ende der zu verlängernden Polynukleotidsequenz kein für eine Guide-Sequenz codierendes Polynukleotid umfasst;
- Durchführen von zwei oder mehr Überlappungsverlängerung-PCR-Reaktionen, indem bei jeder Reaktion eines der zwei oder mehr Polynukleotide und zwei geeignete Polynukleotidprimer umgesetzt werden, wobei wenigstens ein geeigneter Primer ein für eine Guide-Sequenz codierendes Polynukleotid umfasst, so dass ein lineares Hybridpolynukleotid erhalten wird,
wobei die zwei oder mehr zu verlängernden Polynukleotidsequenzen und die geeigneten Polynukleotidprimer so ausgewählt sind, dass die bei den zwei oder mehr Überlappungsverlängerung-PCR-Reaktionen erhaltenen linearen Hybridpolynukleotide jeweils wenigstens ein für eine Guide-Sequenz codierendes Polynukleotid und geeignete 5'-Termini und 3'-Termini umfassen, die einen In-vivo-Zusammenbau der zwei oder mehr linearenHybridpolynukleotide und gegebenenfalls von einem oder mehreren zusätzlichen linearen Polynukleotiden in einer vorbestimmten Reihenfolge gestatten, so dass ein zirkulärer Vektor erhalten wird, der zwei oder mehr funktionelle Guide-Polynukleotid-Expressionskassetten umfasst;
- Unterwerfen der zwei oder mehr in der Überlappungsverlängerung-PCR erhaltenen linearen Hybridpolynukleotide und gegebenenfalls eines oder mehrerer zusätzlicher linearer Polynukleotide einer Zusammenbaureaktion, wobei ein zirkulärer Vektor erhalten wird, der eine oder mehrere funktionelle Guide-Polynukleotid-Expressionskassetten umfasst,
wobei die Zusammenbaureaktion in vivo stattfindet, vorzugsweise wobei es sich bei dem Guide-Polynukleotid um eine gRNA handelt.

2. Verfahren nach Anspruch 1, wobei der zirkuläre Vektor drei oder mehr Guide-Polynukleotid-Expressionskassetten umfasst.

3. Verfahren nach Anspruch 1 oder 2, wobei die eine oder mehreren zu verlängernden Polynukleotidsequenzen bzw. ein oder mehreren zusätzlichen linearen Polynukleotide ein oder mehrere Elemente ausgewählt aus der Gruppe bestehend aus einem Replikationsursprung oder einem Fragment davon, einem selektierbaren Marker oder einem Fragment davon, einer CAS9-Expressionskassette oder einem Fragment davon, einem Donor-Polynukleotid oder einem Fragment davon umfassen.

4. Verfahren nach Anspruch 3, wobei der nach Zusammenbau erhaltene zirkuläre Vektor ein oder mehrere Elemente ausgewählt aus der Gruppe bestehend aus einem Replikationsursprung, einem selektierbaren Marker, einer CAS9-Expressionskassette, einem Donor-Polynukleotid oder einer Kombination von einem oder mehreren der Elemente umfasst, vorzugsweise wobei der nach Zusammenbau erhaltene zirkuläre Vektor ferner einen Replikationsursprung, vorzugsweise einen Replikationsursprung und einen selektierbaren Marker umfasst.

5. Verfahren zum Zusammenbauen eines zirkulären Vektors, der zwei oder mehr Guide-Polynukleotid-Expressionskassetten umfasst, *in vivo,* wobei die zwei oder mehr Guide-Polynukleotid-Expressionskassetten ein Polynukleotid, das ein Guide-Polynukleotid codiert, in operativer Verknüpfung mit einer oder mehreren Kontrollsequenzen, die die Expression des Guide-Polynukleotids in einer Wirtszelle steuern, umfassen, wobei das Guide-Polynukleotid eine Guide-Sequenz umfasst, bei der es sich im Wesentlichen um das Rückwärtskomplement eines Ziel-Polynukleotids in einer Wirtszelle handelt, und wobei das Guide-Polynukleotid die Bindung eines Cas-Proteins an einem Ziel-Polynukleotid in einer Wirtszelle steuern kann, so dass ein CRISPR-Cas-Komplex gebildet wird, wobei das Verfahren die folgenden Schritte umfasst:
- Bereitstellen von zwei oder mehr Polynukleotiden, wobei die zwei oder mehr Polynukleotide eine zu verlängernde Polynukleotidsequenz umfassen, wobei die zu verlängernde Polynukleotidsequenz am 5'-Ende und/oder am 3'-Ende, das verlängert werden soll, ein Fragment einer Guide-Polynukleotid-Expressionskassette umfasst,
- Durchführen von zwei oder mehr Überlappungsverlängerung-PCR-Reaktionen, indem bei jeder Reaktion eines der zwei oder mehr Polynukleotide und zwei geeignete Polynukleotidprimer umgesetzt werden, wobei wenigstens ein geeigneter Primer ein für eine Guide-Sequenz codierendes Polynukleotid umfasst, so dass ein lineares Hybridpolynukleotid erhalten wird,
wobei die zwei oder mehr zu verlängernden Polynukleotidsequenzen und die geeigneten Polynukleotidprimer so ausgewählt sind, dass die bei den zwei oder mehr Überlappungsverlängerung-PCR-Reaktionen erhaltenen linearen Hybridpolynukleotide jeweils wenigstens ein für eine Guide-Sequenz codierendes Polynukleotid und geeignete 5'-Termini und 3'-Termini umfassen, die einen In-vivo-Zusammenbau der zwei oder mehr linearenHybridpolynukleotide und gegebenenfalls von einem oder mehreren zusätzlichen linearen Polynukleotiden in einer vorbestimmten Reihenfolge gestatten, so dass ein zirkulärer Vektor erhalten wird, der eine oder mehrere funktionelle Guide-Polynukleotid-Expressionskassetten umfasst;
- Unterwerfen der zwei oder mehr in der Überlappungsverlängerung-PCR erhaltenen linearen Hybridpolynukleotide und gegebenenfalls eines oder mehrerer zusätzlicher linearer Polynukleotide einer Zusammenbaureaktion, wobei der zirkuläre Vektor erhalten wird,
- Bereitstellen eines oder mehrerer linearer Polynukleotide, wobei die ein oder mehreren linearen Polynukleotide am 5'-Terminus und/oder am 3'-Terminus wenigstens eine Guide-Polynukleotid-Expressionskassette oder ein Fragment wenigstens einer Guide-Polynukleotid-Expressionskassette, das wenigstens die Guide-Sequenz umfasst, umfassen;
- Transformieren der ein oder mehreren linearen Polynukleotide und gegebenenfalls eines oder mehrerer zusätzlicher linearer Polynukleotide in eine Wirtszelle;
- Stattfindenlassen einer homologen Rekombinationsreaktion zwischen den ein oder mehreren linearen Polynukleotiden und gegebenenfalls einem oder mehreren zusätzlichen linearen Polynukleotiden, wobei die linearen Polynukleotide und zusätzlichen linearen Polynukleotide danach ausgewählt wurden, dass sie geeignete 5'-Termini und 3'-Termini umfassen, die eine homologe Rekombination der ein oder mehreren linearen Polynukleotide und gegebenenfalls der ein oder mehreren zusätzlichen linearen Polynukleotide miteinander in einer vorbestimmten Reihenfolge gestatten, so dass ein zirkulärer Vektor erhalten wird, der eine oder mehrere funktionelle Guide-Polynukleotid-Expressionskassetten umfasst;
- Selektieren einer Wirtszelle, die einen zirkulären Vektor umfasst, und wobei der Vektor eine oder mehrere funktionelle Guide-Polynukleotid-Expressionskassetten umfasst;
- Gewinnen des zirkulären Vektors.

6. Verfahren zur Erzeugung einer rekombinanten Wirtszelle, die einen zirkulären Vektor umfasst, wobei der Vektor zwei oder mehr Guide-Polynukleotid-Expressionskassetten umfasst, wobei die zwei oder mehr Guide-Polynukleotid-Expressionskassetten ein Polynukleotid, das ein Guide-Polynukleotid codiert, in operativer Verknüpfung mit einer oder mehreren Kontrollsequenzen, die die Expression des Guide-Polynukleotids in einer Wirtszelle steuern, umfassen, wobei das Guide-Polynukleotid eine Guide-Sequenz umfasst, bei der es sich im Wesentlichen um das Rückwärtskomplement eines Ziel-Polynukleotids in einer Wirtszelle handelt, und wobei das Guide-Polynukleotid die Bindung eines Cas-Proteins an einem Ziel-Polynukleotid in einer Wirtszelle steuern kann, so dass ein CRISPR-Cas-Komplex gebildet wird, wobei das Verfahren die folgenden Schritte umfasst:
- Bereitstellen eines oder mehrerer linearer Polynukleotide, wobei die ein oder mehreren linearen Polynukleotide am 5'-Terminus und/oder am 3'-Terminus wenigstens eine Guide-Polynukleotid-Expressionskassette oder ein Fragment wenigstens einer Guide-Polynukleotid-Expressionskassette, das wenigstens die Guide-Sequenz umfasst, umfassen;
- Transformieren der ein oder mehreren linearen Polynukleotide und gegebenenfalls eines oder mehrerer zusätzlicher linearer Polynukleotide in eine Wirtszelle;
- Stattfindenlassen einer homologen Rekombinationsreaktion zwischen den ein oder mehreren linearen Polynukleotiden und gegebenenfalls einem oder mehreren zusätzlichen linearen Polynukleotiden, wobei die linearen Polynukleotide und zusätzlichen linearen Polynukleotide danach ausgewählt wurden, dass sie geeignete 5'-Termini und 3'-Termini umfassen, die eine homologe Rekombination der ein oder mehreren linearen Polynukleotide und gegebenenfalls der ein oder mehreren zusätzlichen linearen Polynukleotide miteinander in einer vorbestimmten Reihenfolge gestatten, so dass ein zirkulärer Vektor erhalten wird, der eine oder mehrere funktionelle Guide-Polynukleotid-Expressionskassetten umfasst;
- gegebenenfalls Selektieren einer Wirtszelle, die einen zirkulären Vektor umfasst, und wobei der Vektor eine oder mehrere funktionelle Guide-Polynukleotid-Expressionskassetten umfasst;
wobei es sich bei den ein oder mehreren linearen Polynukleotiden um lineare Hybridpolynukleotide handelt, die durch Folgendes erhalten werden:
- Bereitstellen von zwei oder mehr Polynukleotiden, wobei die zwei oder mehr Polynukleotide eine zu verlängernde Polynukleotidsequenz umfassen, wobei die zu verlängernde Polynukleotidsequenz am 5'-Ende und/oder am 3'-Ende, das verlängert werden soll, ein Fragment einer Guide-Polynukleotid-Expressionskassette umfasst, vorzugsweise wobei das Fragment einer Guide-Polynukleotid-Expressionskassette am 5'-Ende und/oder am 3'-Ende der zu verlängernden Polynukleotidsequenz kein für eine Guide-Sequenz codierendes Polynukleotid umfasst;
- Durchführen von zwei oder mehr Überlappungsverlängerung-PCR-Reaktionen, indem bei jeder Reaktion eines der zwei oder mehr Polynukleotide und zwei geeignete Polynukleotidprimer umgesetzt werden, so dass ein lineares Hybridpolynukleotid erhalten wird,
wobei die zwei oder mehr zu verlängernden Polynukleotidsequenzen und die geeigneten Polynukleotidprimer so ausgewählt sind, dass die bei den zwei oder mehr Überlappungsverlängerung-PCR-Reaktionen erhaltenen linearen Hybridpolynukleotide jeweils wenigstens ein für eine Guide-Sequenz codierendes Polynukleotid und geeignete 5'-Termini und 3'-Termini umfassen, die einen In-vivo-Zusammenbau der zwei oder mehr linearenHybridpolynukleotide und gegebenenfalls von einem oder mehreren zusätzlichen linearen Polynukleotiden in einer vorbestimmten Reihenfolge gestatten, so dass ein zirkulärer Vektor erhalten wird, der eine oder mehrere funktionelle Guide-Polynukleotid-Expressionskassetten umfasst.

7. Verfahren zur Erzeugung einer rekombinanten Wirtszelle nach Anspruch 6, ferner umfassend den Schritt:
- Gewinnen des zirkulären Vektors;
- Transformieren des zirkulären Vektors in eine zweite Wirtszelle;
- gegebenenfalls Isolieren der den zirkulären Vektor umfassenden zweiten Wirtszelle.

8. Verfahren zur Erzeugung einer rekombinanten Wirtszelle, die einen zirkulären Vektor umfasst, wobei der Vektor zwei oder mehr Guide-Polynukleotid-Expressionskassetten umfasst, wobei die zwei oder mehr Guide-Polynukleotid-Expressionskassetten ein Polynukleotid, das ein Guide-Polynukleotid codiert, in operativer Verknüpfung mit einer oder mehreren Kontrollsequenzen, die die Expression des Guide-Polynukleotids in einer Wirtszelle steuern, umfassen, wobei das Guide-Polynukleotid eine Guide-Sequenz umfasst, bei der es sich im Wesentlichen um das Rückwärtskomplement eines Ziel-Polynukleotids in einer Wirtszelle handelt, und wobei das Guide-Polynukleotid die Bindung eines Cas-Proteins an einem Ziel-Polynukleotid in einer Wirtszelle steuern kann, so dass ein CRISPR-Cas-Komplex gebildet wird, wobei das Verfahren die folgenden Schritte umfasst:
- Durchführen eines Verfahrens zum Zusammenbauen eines zirkulären Vektors, der zwei oder mehr Guide-Polynukleotid-Expressionskassetten umfasst, *in vivo* nach Anspruch 6 in einer ersten Wirtszelle, vorzugsweise einer ersten Wirtszelle, die zur Spezies *S. cerevisiae* gehört, wobei die erste Wirtszelle, die den zirkulären Vektor umfasst, selektiert und der zirkuläre Vektor gewonnen wird, wobei der Vektor zwei oder mehr funktionelle Guide-Polynukleotid-Expressionskassetten umfasst,
- Transformieren des zirkulären Vektors in eine zweite Wirtszelle;
- gegebenenfalls Isolieren der den zirkulären Vektor umfassenden zweiten Wirtszelle.

9. Verfahren nach einem der vorhergehenden Ansprüche, wobei die Zusammenbaureaktion in einer Wirtszelle erfolgt, die zur Spezies *S. cerevisiae* gehört.

10. Verfahren nach einem der Ansprüche 7 bis 9, wobei es sich bei der zweiten Wirtszelle um eine prokaryontische, wie z. B. eine bakterielle, Wirtszelle oder eine eukaryontische Wirtszelle, bevorzugt eine Pilzwirtszelle, handelt.

11. Verfahren nach einem der Ansprüche 1 bis 10, wobei der zirkuläre Vektor wenigstens 2 oder mehr funktionelle Guide-Polynukleotid-Expressionskassetten umfasst.

## Revendications

1. Procédé destiné à produire un vecteur circulaire comprenant deux ou plus cassettes d'expression de polynucléotides guides, dans lequel lesdites deux ou plus cassettes d'expression de polynucléotides guides comprennent un polynucléotide codant pour un polynucléotide guide étant lié, de manière opérationnelle, à une ou plusieurs séquence(s) de contrôle qui dirige/dirigent l'expression dudit polynucléotide guide dans une cellule hôte, dans lequel ledit polynucléotide guide comprend une séquence guide qui est essentiellement le complément inverse d'un polynucléotide cible dans une cellule hôte et dans lequel le polynucléotide guide peut diriger la liaison d'une protéine Cas au niveau d'un polynucléotide cible dans une cellule hôte pour former un complexe CRISPR-Cas, le procédé comprenant les étapes suivantes :
- fournir deux ou plus polynucléotides, les deux ou plus polynucléotides comprenant une séquence de polynucléotides devant être allongée, dans lesquels la séquence de polynucléotides devant être allongée comprend, au niveau de l'extrémité 5' et/ou au niveau de l'extrémité 3' devant être allongée, un fragment d'une cassette d'expression d'un polynucléotide guide, dans lesquels ledit fragment d'une cassette d'expression d'un polynucléotide guide, au niveau de l'extrémité 5' et/ou au niveau de l'extrémité 3' de la séquence de polynucléotides devant être allongée, ne comprend pas un polynucléotide codant pour une séquence guide ;
- exécuter deux réactions ou plus d'ACP à allongements chevauchants en soumettant dans chaque réaction l'un desdits deux ou plus polynucléotides et deux amorces de polynucléotides appropriées, dans lesquelles au moins une amorce appropriée comprend un polynucléotide codant pour une séquence guide, pour produire un polynucléotide linéaire hybride,
dans lesquelles les deux ou plus séquences de polynucléotides devant être allongées et les amorces de polynucléotides appropriées sont choisies de sorte que chaque polynucléotide linéaire hybride, obtenu dans les deux ou plus réactions d'ACP à allongements chevauchants, comprenne au moins un polynucléotide codant pour une séquence guide et des extrémités terminales en 5' et extrémités terminales en 3' appropriées qui permettent d'obtenir un assemblage *in vivo* des deux ou plus polynucléotides linéaires hybrides et, en option, d'un ou de plusieurs polynucléotide(s) linéaire(s) supplémentaire(s) les uns avec les autres dans un ordre prédéfini pour produire un vecteur circulaire comprenant deux ou plus cassettes fonctionnelles d'expression de polynucléotides guides ;
- soumettre les deux ou plus polynucléotides linéaires hybrides obtenus dans l'ACP à allongements chevauchants et, en option, un ou plusieurs polynucléotide(s) linéaire(s) supplémentaire(s) à une réaction d'assemblage produisant un vecteur circulaire comprenant une ou plusieurs cassette(s) fonctionnelle(s) d'expression de polynucléotides guides,
dans lequel la réaction d'assemblage a lieu *in vivo,*
de préférence, dans lequel le polynucléotide guide est un ARNg.

2. Procédé selon la revendication 1, dans lequel le vecteur circulaire comprend trois ou plus cassettes d'expression de polynucléotides guides.

3. Procédé selon les revendications 1 ou 2, dans lequel l'une ou les plusieurs séquence(s) de polynucléotides devant être allongée(s) ou un polynucléotide linéaire supplémentaire comprend/comprennent un ou plusieurs élément(s) choisi(s) dans le groupe constitué par : une origine de réplication ou un fragment de celle-ci, un marqueur sélectionnable ou un fragment de celui-ci, une cassette d'expression de CAS9 ou un fragment de celle-ci, un polynucléotide donneur ou un fragment de celui-ci.

4. Procédé selon la revendication 3, dans lequel le vecteur circulaire obtenu après l'assemblage comprend un ou plusieurs élément(s) choisi(s) dans le groupe constitué par : une origine de réplication, un marqueur sélectionnable, une cassette d'expression de CAS9, un polynucléotide donneur ou une combinaison d'un ou de plusieurs desdits éléments, de préférence dans lequel le vecteur circulaire obtenu après l'assemblage comprend en outre une origine de réplication, de préférence une origine de réplication et un marqueur sélectionnable.

5. Procédé d'assemblage d'un vecteur circulaire comprenant deux ou plus cassettes d'expression de polynucléotides guides *in vivo,* dans lequel lesdites deux ou plus cassettes d'expression de polynucléotides guides comprennent un polynucléotide codant pour un polynucléotide guide étant lié, de manière opérationnelle, à une ou plusieurs séquence(s) de contrôle qui dirige/dirigent l'expression dudit polynucléotide guide dans une cellule hôte, dans lequel ledit polynucléotide guide comprend une séquence guide qui est essentiellement le complément inverse d'un polynucléotide cible dans une cellule hôte et dans lequel le polynucléotide guide peut diriger la liaison d'une protéine Cas au niveau d'un polynucléotide cible dans une cellule hôte pour former un complexe CRISPR-Cas, le procédé comprenant les étapes suivantes :
- fournir deux ou plus polynucléotides, les deux ou plus polynucléotides comprenant une séquence de polynucléotides devant être allongée, dans lesquels la séquence de polynucléotides devant être allongée comprend, au niveau de l'extrémité 5' et/ou au niveau de l'extrémité 3' devant être allongée, un fragment d'une cassette d'expression d'un polynucléotide guide ;
- exécuter deux ou plus réactions d'ACP à allongements chevauchants en soumettant dans chaque réaction l'un dudit un ou desdits plusieurs polynucléotide(s) et deux amorces de polynucléotides appropriées, dans lesquelles au moins une amorce appropriée comprend un polynucléotide codant pour une séquence guide, pour produire un polynucléotide linéaire hybride,
- dans lesquelles les deux ou plus séquences de polynucléotides devant être allongées et les amorces de polynucléotides appropriées sont choisies de sorte que chaque polynucléotide linéaire hybride, obtenu dans les deux ou plus réactions d'ACP à allongements chevauchants, comprenne au moins un polynucléotide codant pour une séquence guide et des extrémités terminales en 5' et extrémités terminales en 3' appropriées qui permettent d'obtenir un assemblage *in vivo* des deux ou plus polynucléotides linéaires hybrides et, en option, d'un ou de plusieurs polynucléotide(s) linéaire(s) supplémentaire(s) les uns avec les autres dans un ordre prédéfini pour produire un vecteur circulaire comprenant une ou plusieurs cassette(s) fonctionnelle(s) d'expression de polynucléotides guides ;
- soumettre les deux ou plus polynucléotides linéaires hybrides obtenus dans l'ACP à allongements chevauchants et, en option, un ou plusieurs polynucléotide(s) linéaire(s) supplémentaire(s) à une réaction d'assemblage produisant le vecteur circulaire ;
- fournir un ou plusieurs polynucléotide(s) linéaire(s), ledit un/lesdits plusieurs polynucléotide (s) linéaire (s) comprenant, au niveau de l'extrémité terminale en 5' et/ou au niveau de l'extrémité terminale en 3', au moins une cassette d'expression d'un polynucléotide guide ou un fragment d'au moins une cassette d'expression d'un polynucléotide guide comprenant au moins la séquence guide ;
- transformer ledit un/lesdits plusieurs polynucléotide(s) linéaire(s) et, en option, un ou plusieurs polynucléotide(s) linéaire(s) supplémentaire(s) dans une cellule hôte ;
- permettre qu'ait lieu une réaction de recombinaison homologue entre ledit un/lesdits plusieurs polynucléotide(s) linéaire(s) et, en option, un ou plusieurs polynucléotide(s) linéaire(s) supplémentaire(s), dans laquelle lesdits polynucléotides linéaires et polynucléotides linéaires supplémentaires ont été choisis pour comprendre des extrémités terminales en 5' et extrémités terminales en 3' appropriées qui permettent d'obtenir une recombinaison homologue dudit un/desdits plusieurs polynucléotide(s) linéaire(s) et, en option, dudit un/desdits plusieurs polynucléotide(s) linéaire(s) supplémentaire(s) les uns avec les autres dans un ordre prédéfini pour produire un vecteur circulaire comprenant une ou plusieurs cassette(s) fonctionnelle(s) d'expression de polynucléotides guides ;
- choisir une cellule hôte comprenant un vecteur circulaire et dans laquelle ledit vecteur comprend une ou plusieurs cassette(s) fonctionnelle(s)d'expression de polynucléotides guides ;
- récupérer le vecteur circulaire.

6. Procédé destiné à produire une cellule hôte recombinante comprenant un vecteur circulaire, dans laquelle ledit vecteur comprend deux ou plus cassettes d'expression de polynucléotides guides, dans lequel lesdites deux ou plus cassettes d'expression de polynucléotides guides comprennent un polynucléotide codant pour un polynucléotide guide étant lié, de manière opérationnelle, à une ou plusieurs séquence(s) de contrôle qui dirige/dirigent l'expression dudit polynucléotide guide dans une cellule hôte, dans lequel ledit polynucléotide guide comprend une séquence guide qui est essentiellement le complément inverse d'un polynucléotide cible dans une cellule hôte et dans lequel le polynucléotide guide peut diriger la liaison d'une protéine Cas au niveau d'un polynucléotide cible dans une cellule hôte pour former un complexe CRISPR-Cas, le procédé comprenant les étapes suivantes :
- fournir un ou plusieurs polynucléotide(s) linéaire(s), ledit un/lesdits plusieurs polynucléotide (s) linéaire(s) comprenant, au niveau de l'extrémité terminale en 5' et/ou au niveau de l'extrémité terminale en 3', au moins une cassette d'expression d'un polynucléotide guide ou un fragment d'au moins une cassette d'expression d'un polynucléotide guide comprenant au moins la séquence guide ;
- transformer ledit un/lesdits plusieurs polynucléotide(s) linéaire(s) et, en option, un ou plusieurs polynucléotide(s) linéaire(s) supplémentaire(s) dans une cellule hôte ;
- permettre qu'ait lieu une réaction de recombinaison homologue entre ledit un/lesdits plusieurs polynucléotide(s) linéaire(s) et, en option, un ou plusieurs polynucléotide(s) linéaire(s) supplémentaire(s), dans laquelle lesdits polynucléotides linéaires et polynucléotides linéaires supplémentaires ont été choisis pour comprendre des extrémités terminales en 5' et extrémités terminales en 3' appropriées qui permettent d'obtenir une recombinaison homologue dudit un/desdits plusieurs polynucléotide(s) linéaire(s) et, en option, dudit un/desdits plusieurs polynucléotide(s) linéaire(s) supplémentaire(s) les uns avec les autres dans un ordre prédéfini pour produire un vecteur circulaire comprenant une ou plusieurs cassette(s) fonctionnelle(s) d'expression de polynucléotides guides ;
- en option, choisir une cellule hôte comprenant un vecteur circulaire et dans laquelle ledit vecteur comprend une ou plusieurs cassette(s) fonctionnelle(s) d'expression de polynucléotides guides,
dans lequel l'un ou les plusieurs polynucléotide(s) linéaire(s) est/sont des polynucléotides linéaires hybrides obtenus par
- la fourniture de deux ou plus polynucléotides, les deux ou plus polynucléotides comprenant une séquence de polynucléotides devant être allongée, dans lesquels la séquence de polynucléotides devant être allongée comprend, au niveau de l'extrémité 5' et/ou au niveau de l'extrémité 3' devant être allongée, un fragment d'une cassette d'expression d'un polynucléotide guide, de préférence dans lesquels le fragment d'une cassette d'expression d'un polynucléotide guide, au niveau de l'extrémité 5' et/ou au niveau de l'extrémité 3' de la séquence de polynucléotides devant être allongée, ne comprend pas un polynucléotide codant pour une séquence guide ;
- exécuter deux ou plus réactions d'ACP à allongements chevauchants en soumettant dans chaque réaction l'un desdits deux ou plus polynucléotides et deux amorces de polynucléotides appropriées pour produire un polynucléotide linéaire hybride,
dans lesquelles les deux ou plus séquences de polynucléotides devant être allongées et les amorces de polynucléotides appropriées sont choisies de sorte que chaque polynucléotide linéaire hybride, obtenu dans les deux ou plus réactions d'ACP à allongements chevauchants, comprenne au moins un polynucléotide codant pour une séquence guide et des extrémités terminales en 5' et extrémités terminales en 3' appropriées qui permettent d'obtenir un assemblage *in vivo* des deux ou plus polynucléotides linéaires hybrides et, en option, d'un ou de plusieurs polynucléotide(s) linéaire(s) supplémentaire(s) les uns avec les autres dans un ordre prédéfini pour produire un vecteur circulaire comprenant une ou plusieurs cassette(s) fonctionnelle(s) d'expression de polynucléotides guides.

7. Procédé destiné à produire une cellule hôte recombinante, selon la revendication 6, comprenant en outre l'étape de :
- récupération du vecteur circulaire ;
- transformation du vecteur circulaire dans une deuxième cellule hôte ;
- en option, isolement de la deuxième cellule hôte comprenant le vecteur circulaire.

8. Procédé destiné à produire une cellule hôte recombinante comprenant un vecteur circulaire, dans laquelle ledit vecteur comprend deux ou plus cassettes d'expression de polynucléotides guides, dans lequel lesdites deux ou plus cassettes d'expression de polynucléotides guides comprennent un polynucléotide codant pour un polynucléotide guide étant lié, de manière opérationnelle, à une ou plusieurs séquence(s) de contrôle qui dirige/dirigent l'expression dudit polynucléotide guide dans une cellule hôte, dans lequel ledit polynucléotide guide comprend une séquence guide qui est essentiellement le complément inverse d'un polynucléotide cible dans une cellule hôte et dans lequel le polynucléotide guide peut diriger la liaison d'une protéine Cas au niveau d'un polynucléotide cible dans une cellule hôte pour former un complexe CRISPR-Cas, le procédé comprenant les étapes suivantes :
- exécuter un procédé d'assemblage d'un vecteur circulaire comprenant deux ou plus cassettes d'expression de polynucléotides guides *in vivo,* selon la revendication 6, dans une première cellule hôte, de préférence une première cellule hôte appartenant à l'espèce *S. cerevisiae,* dans laquelle est choisie ladite première cellule hôte comprenant le vecteur circulaire, et le vecteur circulaire est récupéré, dans lequel ledit vecteur comprend deux ou plus cassettes fonctionnelles d'expression de polynucléotides guides,
- transformer le vecteur circulaire dans une deuxième cellule hôte ;
- en option, isoler la deuxième cellule hôte comprenant le vecteur circulaire.

9. Procédé selon l'une quelconque des revendications précédentes, dans lequel la réaction d'assemblage a lieu dans une cellule hôte appartenant à l'espèce *S. cerevisiae.*

10. Procédé selon l'une quelconque des revendications 7 à 9, dans lequel la deuxième cellule hôte est une cellule hôte procaryote, telle qu'une cellule hôte bactérienne, ou une cellule hôte eucaryote, de préférence une cellule hôte fongique.

11. Procédé selon l'une quelconque des revendications 1 à 10, dans lequel le vecteur circulaire comprend au moins 2 ou plus cassettes fonctionnelles d'expression de polynucléotides guides.
